# EUROPEAN PATENT APPLICATION

(11) **EP 4 818 318 A2**
(43) Date of publication of application: **07.10.2026**
(21) Application number: 26196359.9
(22) Date of filing: 31.03.2017
(51) Int. Cl.: C12N 15/09

(54) **KIT OR DEVICE FOR DETECTING EARLY STAGE PANCREATIC CANCER OR PANCREATIC CANCER PRECURSOR LESIONS AND DETECTION METHOD THEREFOR**

(30) Priority: 31.03.2016 JP 2016073132
(62) Document number(s) of the earlier application(s) in accordance with Art. 76 EPC: 21206740.9 / 3 988 668; 17775579.0 / 3 438 284
(71) Applicant: Toray Industries, Inc., Tokyo 103-8666 (JP); National Cancer Center, Tokyo 104-0045 (JP)
(72) Inventor: KAWAUCHI, Junpei, Kanagawa, 248-8555 (JP); SUDO, Hiroko, Kanagawa, 248-8555 (JP); KOZONO, Satoko, Kanagawa, 248-8555 (JP); OCHIAI, Atsushi, Kanagawa, 248-8555 (JP); KOJIMA, Motohiro, Chiba, 277-8577 (JP)
(74) Representative: Mewburn Ellis LLP

(57) **Abstract**

This application provides a kit or a device for the detection of early pancreatic cancer or a pancreatic cancer precursor lesion, comprising a nucleic acid(s) capable of specifically binding to a miRNA(s) in a sample from a subject, and a method for detecting early pancreatic cancer or a pancreatic cancer precursor lesion, comprising measuring an expression level(s) of the miRNA(s) *in vitro.*

## Description

### TECHNICAL FIELD

The present invention relates to a kit or a device for the detection of early pancreatic cancer or a pancreatic cancer precursor lesion, comprising a nucleic acid capable of specifically binding to a particular miRNA, which is used for examining the presence or absence of early pancreatic cancer or pancreatic cancer precursor lesion in a subject, and a method for detecting early pancreatic cancer or a pancreatic cancer precursor lesion, comprising measuring an expression level of the miRNA using the nucleic acid.

### BACKGROUND ART

The pancreas serves as an exocrine gland that secretes pancreatic juice as a digestive juice and sends the juice into the digestive tract through the pancreatic duct, while also functioning as an endocrine gland that secretes hormones such as insulin and glucagon into blood.

Since the pancreas is surrounded by many organs such as the stomach, the duodenum, the small intestine, the liver, and the gallbladder, pancreatic cancer is not only difficult to detect early but has properties such as a lack of subjective symptoms, very rapid progression, and metastasis to other organs and thus has very poor prognosis as compared with other cancers. According to the 2011 statistics of cancer type-specific mortality in Japan disclosed by the Center for Cancer Control and Information Services, National Cancer Center (Tokyo, Japan), the number of pancreatic cancer deaths climbed to 28,829 people, and 5-year relative survival rates by cancer type from 2003 to 2005 were lowest in pancreatic cancer with 7.1% for males and 6.9% for females.

As described in Non-Patent Literature 1, the basic therapy of pancreatic cancer is practiced by surgery, systemic chemotherapy, radiotherapy, or a combination thereof depending on a stage of progression. Although 15 to 20% pancreatic cancer patients undergo surgery for potential complete cure, the great majority of patients who do not undergo surgery are considered to have local progression or metastasis.

The UICC (Unio Internationalis Contra Cancrum) stages of progression of pancreatic cancer are classified into stages 0, IA, IB, IIA, IIB, III, IVa, and IVb. Stages I to III occupy half or more of the number of 5-year survivals, and stages IVa and IVb occupy 70% or more of the stages of progression at the time of diagnosis. As described in Non-Patent Literature 1, the 5-year survival rate of pancreatic cancer is 45.8% for stage IA, 36.3% for stage IB, 29.4% for stage IIA, 10.6% for stage IIB, 5.9% for stage III, and 4.0% for stage IV, and the prognoses of stage III and stage IV are very poor. Therefore, early detection and treatment of pancreatic cancer are necessary.

As described in Non-Patent Literature 2, abdominal ultrasonography is very useful as convenient and minimally invasive examination in outpatient care or medical examination for the diagnosis of pancreatic cancer. However, it is often difficult to visualize pancreatic cancer having a small tumor size or a lesion on the pancreatic tail side. In ordinary medical checkup, the abnormality detection rate with pancreatic images by abdominal ultrasonography is approximately 1%, and the detection rate of pancreatic cancer is approximately 0.06% or lower. For example, CA19-9, Span-1, CA50, CA242, Dupan-2, TAG-72, and urinary fucose as carbohydrate antigens, and CEA, POA, and TPS as non-carbohydrate antigens are known as tumor markers for the detection of pancreatic cancer. As for how to use these tumor markers, a subject is suspected of having a cancer when their concentrations in blood are higher or lower than predetermined reference values. For example, as described in Non-Patent Literature 3, the reference value of CEA is set to 5 ng/mL, and the reference value of CA19-9 is set to 37 U/mL. A subject is suspected of having a cancer including pancreatic cancer when their concentrations exhibit these values or higher. However, the evaluation of tumor markers mostly examines advanced pancreatic cancer and does not show abnormal values for early pancreatic cancer in many cases. Even combinatorial use of tumor markers and abdominal ultrasonography in medical examination results in low rates of detection of pancreatic cancer. The implementation of such medical examinations for the detection of pancreatic cancer is controversial from the viewpoint of cost effectiveness.

Meanwhile, cystic diseases that occur in the pancreas are known to progress to invasive cancers through malignant transformation and can be regarded as pancreatic cancer precursor lesions. As described in Non-Patent Literature 4, the malignancy of the cystic diseases is evaluated on the basis of cyst diameters, wall thickening, diameters of the main pancreatic duct, mural nodules, stenosis of the main pancreatic duct, enlarged lymph nodes, and cystic lesions, etc. Patients with intraductal papillary-mucinous neoplasms, one type of cystic disease, have a prognosis as poor as 40.4% for malignant cancer and 30.8% for invasive cancer and are therefore recommended to receive follow-up or tumor resection even if the malignancy of the tumors is low when the tumors are detected.

As shown in Patent Literatures 1 to 5 and Non-Patent Literature 4, there are reports, albeit at a research stage, on the determination of pancreatic cancer using the expression levels of microRNAs (miRNAs), or combinations of the expression levels of miRNAs and the expression levels of additional protein markers in biological samples including blood.

Patent Literature 1 discloses a method for detecting pancreatic cancer by combining hsa-miR-125a-3p, hsa-miR-204-3p, and hsa-miR-3648 with several other miRNAs in blood.

Patent Literature 2 discloses a method for detecting pancreatic cancer by combining miRNAs such as hsa-miR-1908-5p, hsa-miR-6729-5p, and hsa-miR-5195-3p in blood.

Patent Literature 3 discloses a method for detecting pancreatic cancer by combining miR-23a-3p with tens of other miRNAs in blood.

Patent Literature 4 lists hsa-miR-1268a, hsa-miR-939-5p, and hsa-miR-642b-3p as miRNAs that have a larger expression level in the blood of pancreatic cancer patients than that in the blood of healthy subjects and discloses a method for detecting pancreatic cancer and a method for evaluating the risk of developing pancreatic cancer by combining these miRNAs with tens of other miRNAs.

Patent Literature 5 discloses a method for detecting pancreatic cancer or a pancreatic cancer precursor lesion and a method for evaluating the risk of developing pancreatic cancer by combining hsa-miR-296-5p with tens of other miRNAs in blood.

Non-Patent Literature 5 lists hsa-miR-638, hsa-miR-3196, hsa-miR-1225-3p, and the like as miRNAs that have a larger expression level in the blood of pancreatic cancer patients than that in the blood of healthy subjects and discloses a method for detecting pancreatic cancer by combining several these miRNAs.

### PRIOR ART LITERATURE

### Patent Literature

Patent Literature 1: JP Patent Publication (Kokai) No. 2015-107091 A (2015)
Patent Literature 2: International Publication No. WO 2015/182781
Patent Literature 3: Published U.S. Patent Application No. 2015/0011414
Patent Literature 4: JP Patent Publication (Kohyo) No. 2015-502176 A (2015)
Patent Literature 5: International Publication No. WO 2015/153679

### Non-Patent Literature

Non-Patent Literature 1: Tetsuya Mine, "Suizo (Pancreas), Journal of the Japan Pancreas Society", Japan Pancreas Society, 2007, Vol. 22, p.10-13
Non-Patent Literature 2: Japan Pancreas Society, "2009 Scientific evidence based clinical practice guidelines for pancreatic cancer" CQ1 diagnosis methods http://www.suizou.org/PCMG2009/cq1/cq1-3.html
Non-Patent Literature 3: Kiyoshi Kurokawa et al. ed., LAB DATA, 2013, p. 633, 636 (Igaku-Shoin Ltd., Tokyo, Japan)
Non-Patent Literature 4: Working Group of the Japan Pancreas Society, International consensus guidelines 2012 for the management of IPMN and MCN of the pancreas., p. 6, 8
Non-Patent Literature 5: Miyamae M. et al., 2015, British Journal of Cancer, Vol. 113, (10) p. 1467-1476

### SUMMARY OF INVENTION

### Problem to be Solved by Invention

An object of the present invention is to find novel tumor markers for early pancreatic cancer or a pancreatic cancer precursor lesion and to provide a method that can effectively detect early pancreatic cancer or a pancreatic cancer precursor lesion using nucleic acids capable of specifically binding to the markers. As described in Non-Patent Literature 2, for example, CA19-9, Span-1, CA50, CA242, Dupan-2, TAG-72, and urinary fucose as carbohydrate antigens and CEA, POA, and TPS as non-carbohydrate antigens are known as tumor markers for the detection of pancreatic cancer. The pancreatic cancer detection sensitivity of these tumor markers is 70 to 80% for CA19-9, 70 to 80% for Span-1, 50 to 60% for Dupan-2, 30 to 60% for CEA, and 60% for CA50. In addition, their specificity is not much high, and their false positive rates are as high as 20 to 30%. Therefore, there may be the possibility of false detection of other cancers and/or benign tumors and/or benign diseases of the pancreas and/or peripancreatic organs, etc. Particularly, the detection sensitivity of early pancreatic cancer is generally low, and the positive rate of CA19-9 is merely 1/2 (52%) for pancreatic cancer having a tumor size of 2 cm or smaller. Therefore, these tumor markers are not useful for the detection of early pancreatic cancer. Furthermore, the tumor markers based on carbohydrate antigens exhibit false negatives in Lewis blood type negative cases, in which the subjects do not produce the antigens. Therefore, this examination is unsuitable for some subjects. The detection rates of intraductal papillary-mucinous neoplasms which are pancreatic cancer precursor lesions by MRI and CT are 19.9% and 1.2 to 2.6%, respectively, and are not sufficient. Thus, use of tumor markers is not recommended for the detection of pancreatic cancer precursor lesions.

As described below, there are reports, albeit at a research stage, on the determination of pancreatic cancer using the expression levels of microRNAs (miRNAs) in biological samples including blood, none of which, however, have yet been brought into practical use as a method for detecting early pancreatic cancer or a pancreatic cancer precursor lesion.

Patent Literature 1 discloses a method for detecting pancreatic cancer by combining miR-125a-3p, miR-204-3p, and miR-3648 with several other miRNAs in blood. In this literature, however, only healthy subjects were used as a negative control group for pancreatic cancer. Furthermore, the literature neither describes cancers in organs other than the pancreas or benign diseases nor describes a specific method for detecting a pancreatic cancer precursor lesion using blood.

Patent Literature 2 discloses a method for detecting pancreatic cancer by combining miRNAs such as hsa-miR-1908-5p, hsa-miR-6729-5p, and hsa-miR-5195-3p in blood. In this literature, however, only several samples from early pancreatic cancer patients were involved. Furthermore, the literature neither describes specific detection performance thereof such as accuracy, sensitivity, or specificity for early pancreatic cancer nor describes a specific method for detecting a pancreatic cancer precursor lesion using blood.

Patent Literature 3 discloses a method for detecting pancreatic cancer by combining hsa-miR-23a-3p with tens of other miRNAs in blood. This literature, however, neither describes specific detection performance thereof such as accuracy, sensitivity, or specificity for early pancreatic cancer nor describes cancers in regions other than the peripancreatic gastrointestinal upper regions as a negative control group for pancreatic cancer. Furthermore, the literature does not describe a method for detecting a pancreatic cancer precursor lesion.

Patent Literature 4 discloses a method for detecting pancreatic cancer or a pancreatic cancer precursor lesion by combining hsa-miR-1268a, hsa-miR-939-5p, and hsa-miR-642b-3p with tens of other miRNAs. This literature, however, neither describes specific detection performance thereof such as accuracy, sensitivity, or specificity for pancreatic cancer nor describes cancers in organs other than the pancreas as a negative control group for pancreatic cancer.

Patent Literature 5 discloses a method for detecting pancreatic cancer or a pancreatic cancer precursor lesion by combining hsa-miR-296-5p with tens of other miRNAs in blood. This literature, however, does not describe specific detection performance thereof such as accuracy, sensitivity, or specificity for early pancreatic cancer. In the literature, cancers in organs other than the pancreas or benign diseases were not measured as a negative control group for pancreatic cancer precursor lesions. Furthermore, the literature does not describe specific specificity.

Non-Patent Literature 5 lists hsa-miR-638, hsa-miR-3196, hsa-miR-1225-3p, and the like as miRNAs that have a larger expression level in the blood of pancreatic cancer patients than that in the blood of healthy subjects and discloses a method for detecting pancreatic cancer by combining several these miRNAs. This literature, however, does not describe cancers in organs other than pancreas as a negative control group for pancreatic cancer.

As mentioned above, the existing tumor markers exhibit low performance in the detection of early pancreatic cancer or a pancreatic cancer precursor lesion, or neither performance nor detection methods are specifically shown as to the markers at a research stage. Therefore, use of these markers might require carrying out needless extra examination due to the false detection of healthy subjects as being early pancreatic cancer or pancreatic cancer precursor lesion patients, or might waste therapeutic opportunity because of overlooking early pancreatic cancer or pancreatic cancer precursor lesion patients. In addition, the measurement of tens to hundreds of miRNAs increases examination costs and is therefore difficult to use in large-scale screening such as medical checkup. Furthermore, the collection of pancreatic tissues for measuring the tumor markers is highly invasive to patients and is not favorable. Hence, there is a demand for a highly accurate early pancreatic cancer or pancreatic cancer precursor lesion marker that is detectable from blood, which can be collected in less invasive manner, and is capable of correctly determining an early pancreatic cancer or pancreatic cancer precursor lesion patient as an early pancreatic cancer or pancreatic cancer precursor lesion patient and a healthy subject as a healthy subject. Particularly, a highly sensitive early pancreatic cancer or pancreatic cancer precursor lesion marker is desired because tumor resection based on early detection is only radical cure for pancreatic cancer.

### Means for Solution of Problem

The present inventors have conducted diligent studies to attain the object and consequently completed the present invention by identifying several genes usable as markers for the detection of early pancreatic cancer or a pancreatic cancer precursor lesion from blood, which can be collected with minimal invasiveness, and finding that early pancreatic cancer or a pancreatic cancer precursor lesion can be significantly detected by using nucleic acids capable of specifically binding to any of these markers.

### <Summary of Invention>

The present invention has the following features:
(1) A kit for the detection of early pancreatic cancer or a pancreatic cancer precursor lesion, comprising a nucleic acid(s) capable of specifically binding to at least one polynucleotides selected from the group consisting of the following early pancreatic cancer or pancreatic cancer precursor lesion markers: miR-6784-5p, miR-1181, miR-671-5p, miR-6857-5p, miR-4276, miR-1914-3p, miR-149-3p, miR-937-5p, miR-4675, miR-6795-5p, miR-4731-5p, miR-5090, miR-3620-5p, miR-1343-5p, miR-6717-5p, miR-6825-5p, miR-6738-5p, miR-6769a-5p, miR-4728-5p, miR-652-5p, miR-4257, miR-6785-5p, miR-7110-5p, miR-6887-5p, miR-887-3p, miR-1228-5p, miR-5572, miR-6782-5p, miR-4298, miR-6786-5p, miR-5010-5p, miR-6087, miR-6765-5p, miR-6732-5p, miR-6787-5p, miR-6737-5p, miR-128-2-5p, miR-4270, miR-6861-5p, miR-6756-5p, miR-1229-5p, miR-6891-5p, miR-6848-5p, miR-1237-5p, miR-30c-1-3p, miR-1233-5p, miR-211-3p, miR-4758-5p, miR-614, miR-6746-5p, miR-1915-5p, miR-4688, miR-3917, miR-5787, miR-4632-5p, miR-6126, miR-135a-3p, miR-8063, miR-5698, miR-6089, miR-498, miR-296-3p, miR-4419b, miR-6802-5p, miR-6829-5p, miR-6803-5p, miR-1199-5p, miR-6840-3p, miR-6752-5p, miR-6798-5p, miR-6131, miR-4667-5p, miR-6510-5p, miR-4690-5p, miR-920, miR-23b-3p, miR-4448, miR-2110, miR-4706, miR-7845-5p, miR-6808-5p, miR-4447, miR-6869-5p, miR-6794-5p, miR-6511a-5p, miR-6824-5p, miR-6766-3p, miR-6511a-5p, and miR-6749-5p.
(2) The kit according to (1), wherein miR-6784-5p is hsa-miR-6784-5p, miR-1181 is hsa-miR-1181, miR-671-5p is hsa-miR-671-5p, miR-6857-5p is hsa-miR-6857-5p, miR-4276 is hsa-miR-4276, miR-1914-3p is hsa-miR-1914-3p, miR-149-3p is hsa-miR-149-3p, miR-937-5p is hsa-miR-937-5p, miR-4675 is hsa-miR-4675, miR-6795-5p is hsa-miR-6795-5p, miR-4731-5p is hsa-miR-4731-5p, miR-5090 is hsa-miR-5090, miR-3620-5p is hsa-miR-3620-5p, miR-1343-5p is hsa-miR-1343-5p, miR-6717-5p is hsa-miR-6717-5p, miR-6825-5p is hsa-miR-6825-5p, miR-6738-5p is hsa-miR-6738-5p, miR-6769a-5p is hsa-miR-6769a-5p, miR-4728-5p is hsa-miR-4728-5p, miR-652-5p is hsa-miR-652-5p, miR-4257 is hsa-miR-4257, miR-6785-5p is hsa-miR-6785-5p, miR-7110-5p is hsa-miR-7110-5p, miR-6887-5p is hsa-miR-6887-5p, miR-887-3p is hsa-miR-887-3p, miR-1228-5p is hsa-miR-1228-5p, miR-5572 is hsa-miR-5572, miR-6782-5p is hsa-miR-6782-5p, miR-4298 is hsa-miR-4298, miR-6786-5p is hsa-miR-6786-5p, miR-5010-5p is hsa-miR-5010-5p, miR-6087 is hsa-miR-6087, miR-6765-5p is hsa-miR-6765-5p, miR-6732-5p is hsa-miR-6732-5p, miR-6787-5p is hsa-miR-6787-5p, miR-6737-5p is hsa-miR-6737-5p, miR-128-2-5p is hsa-miR-128-2-5p, miR-4270 is hsa-miR-4270, miR-6861-5p is hsa-miR-6861-5p, miR-6756-5p is hsa-miR-6756-5p, miR-1229-5p is hsa-miR-1229-5p, miR-6891-5p is hsa-miR-6891-5p, miR-6848-5p is hsa-miR-6848-5p, miR-1237-5p is hsa-miR-1237-5p, miR-30c-1-3p is hsa-miR-30c-1-3p, miR-1233-5p is hsa-miR-1233-5p, miR-211-3p is hsa-miR-211-3p, miR-4758-5p is hsa-miR-4758-5p, miR-614 is hsa-miR-614, miR-6746-5p is hsa-miR-6746-5p, miR-1915-5p is hsa-miR-1915-5p, miR-4688 is hsa-miR-4688, miR-3917 is hsa-miR-3917, miR-5787 is hsa-miR-5787, miR-4632-5p is hsa-miR-4632-5p, miR-6126 is hsa-miR-6126, miR-135a-3p is hsa-miR-135a-3p, miR-8063 is hsa-miR-8063, miR-5698 is hsa-miR-5698, miR-6089 is hsa-miR-6089, miR-498 is hsa-miR-498, miR-296-3p is hsa-miR-296-3p, miR-4419b is hsa-miR-4419b, miR-6802-5p is hsa-miR-6802-5p, miR-6829-5p is hsa-miR-6829-5p, miR-6803-5p is hsa-miR-6803-5p, miR-1199-5p is hsa-miR-1199-5p, miR-6840-3p is hsa-miR-6840-3p, miR-6752-5p is hsa-miR-6752-5p, miR-6798-5p is hsa-miR-6798-5p, miR-6131 is hsa-miR-6131, miR-4667-5p is hsa-miR-4667-5p, miR-6510-5p is hsa-miR-6510-5p, miR-4690-5p is hsa-miR-4690-5p, miR-920 is hsa-miR-920, miR-23b-3p is hsa-miR-23b-3p, miR-4448 is hsa-miR-4448, miR-2110 is hsa-miR-2110, miR-4706 is hsa-miR-4706, miR-7845-5p is hsa-miR-7845-5p, miR-6808-5p is hsa-miR-6808-5p, miR-4447 is hsa-miR-4447, miR-6869-5p is hsa-miR-6869-5p, miR-6794-5p is hsa-miR-6794-5p, miR-6511a-5p is hsa-miR-6511a-5p, miR-6824-5p is hsa-miR-6824-5p, miR-6766-3p is hsa-miR-6766-3p, miR-6511a-5p is hsa-miR-6511a-5p, and miR-6749-5p is hsa-miR-6749-5p.
(3) The kit according to (1) or (2), wherein the nucleic acid(s) is a polynucleotide(s) selected from the group consisting of the following polynucleotides (a) to (e):
   (a) a polynucleotide consisting of a nucleotide sequence represented by any of SEQ ID NOs: 1 to 83, 227 to 229, 246, 248, and 250 or a nucleotide sequence derived from the nucleotide sequence by the replacement of u with t, a variant thereof, a derivative thereof, or a fragment thereof comprising 15 or more consecutive nucleotides;
   (b) a polynucleotide comprising a nucleotide sequence represented by any of SEQ ID NOs: 1 to 83, 227 to 229, 246, 248, and 250;
   (c) a polynucleotide consisting of a nucleotide sequence complementary to a nucleotide sequence represented by any of SEQ ID NOs: 1 to 83, 227 to 229, 246, 248, and 250 or a nucleotide sequence derived from the nucleotide sequence by the replacement of u with t, a variant thereof, a derivative thereof, or a fragment thereof comprising 15 or more consecutive nucleotides;
   (d) a polynucleotide comprising a nucleotide sequence complementary to a nucleotide sequence represented by any of SEQ ID NOs: 1 to 83, 227 to 229, 246, 248, and 250 or a nucleotide sequence derived from the nucleotide sequence by the replacement of u with t; and
   (e) a polynucleotide hybridizing under stringent conditions to any of the polynucleotides (a) to (d).
(4) The kit according to any of (1) to (3), wherein the kit further comprises a nucleic acid(s) capable of specifically binding to at least one polynucleotide selected from the group consisting of other early pancreatic cancer or pancreatic cancer precursor lesion markers miR-1908-5p, miR-6729-5p, miR-5195-3p, miR-638, miR-6125, miR-3178, miR-3196, miR-8069, miR-4723-5p, miR-4746-3p, miR-4689, miR-6816-5p, miR-6757-5p, miR-7109-5p, miR-6724-5p, miR-1225-3p, miR-6875-5p, miR-7108-5p, miR-4508, miR-6085, miR-6779-5p, miR-642a-3p, miR-4695-5p, miR-7847-3p, miR-3197, miR-6769b-5p, miR-7641, miR-187-5p, miR-3185, miR-2861, miR-3940-5p, miR-1203, miR-615-5p, miR-4787-5p, miR-1343-3p, miR-6813-5p, miR-1225-5p, miR-602, miR-4488, miR-125a-3p, miR-5100, miR-4294, miR-1231, miR-6765-3p, miR-4442, miR-718, miR-6780b-5p, miR-6090, miR-6845-5p, miR-4741, miR-4467, miR-4707-5p, miR-4271, miR-4673, miR-3184-5p, miR-1469, miR-4640-5p, miR-663a, miR-6791-5p, miR-6826-5p, miR-4433b-3p, miR-1915-3p, miR-4417, miR-4449, miR-4707-3p, miR-3180-3p, miR-5585-3p, miR-1268a, miR-8072, miR-296-5p, miR-204-3p, miR-4454, miR-6722-3p, miR-1290, miR-3622a-5p, miR-939-5p, miR-675-5p, miR-3131, miR-4648, miR-1268b, miR-6741-5p, miR-6893-5p, miR-3162-5p, miR-642b-3p, miR-4734, miR-150-3p, miR-8089, miR-6805-3p, miR-7113-3p, miR-6850-5p, miR-6799-5p, miR-6768-5p, miR-92b-5p, miR-3679-5p, miR-4792, miR-3656, miR-92a-2-5p, miR-4466, miR-4513, miR-6781-5p, miR-4649-5p, miR-6775-5p, miR-4651, miR-3195, miR-6726-5p, miR-6872-3p, miR-371a-5p, miR-6777-5p, miR-6789-5p, miR-7975, miR-6821-5p, miR-4534, miR-619-5p, miR-7107-5p, miR-1228-3p, miR-6774-5p, miR-6805-5p, miR-23a-3p, miR-4665-5p, miR-4505, miR-4638-5p, miR-24-3p, miR-3135b, miR-4745-5p, miR-128-1-5p, miR-4476, miR-4687-3p, miR-3665, miR-6806-5p, miR-3937, miR-711, miR-3141, miR-3188, miR-4281, miR-5196-5p, miR-6880-5p, miR-3960, miR-3648, miR-6721-5p, miR-4492, miR-744-5p, miR-7704, miR-4749-5p, miR-762, miR-6836-3p, miR-6727-5p, miR-4739, miR-7977, miR-4484, miR-6515-3p, miR-373-5p, miR-4258, miR-4674, miR-3180, miR-6076, miR-1238-5p, miR-4463, miR-4486, miR-4730, miR-4286, and miR-4739.
(5) The kit according to (4), wherein miR-1908-5p is hsa-miR-1908-5p, miR-6729-5p is hsa-miR-6729-5p, miR-5195-3p is hsa-miR-5195-3p, miR-638 is hsa-miR-638, miR-6125 is hsa-miR-6125, miR-3178 is hsa-miR-3178, miR-3196 is hsa-miR-3196, miR-8069 is hsa-miR-8069, miR-4723-5p is hsa-miR-4723-5p, miR-4746-3p is hsa-miR-4746-3p, miR-4689 is hsa-miR-4689, miR-6816-5p is hsa-miR-6816-5p, miR-6757-5p is hsa-miR-6757-5p, miR-7109-5p is hsa-miR-7109-5p, miR-6724-5p is hsa-miR-6724-5p, miR-1225-3p is hsa-miR-1225-3p, miR-6875-5p is hsa-miR-6875-5p, miR-7108-5p is hsa-miR-7108-5p, miR-4508 is hsa-miR-4508, miR-6085 is hsa-miR-6085, miR-6779-5p is hsa-miR-6779-5p, miR-642a-3p is hsa-miR-642a-3p, miR-4695-5p is hsa-miR-4695-5p, miR-7847-3p is hsa-miR-7847-3p, miR-3197 is hsa-miR-3197, miR-6769b-5p is hsa-miR-6769b-5p, miR-7641 is hsa-miR-7641, miR-187-5p is hsa-miR-187-5p, miR-3185 is hsa-miR-3185, miR-2861 is hsa-miR-2861, miR-3940-5p is hsa-miR-3940-5p, miR-1203 is hsa-miR-1203, miR-615-5p is hsa-miR-615-5p, miR-4787-5p is hsa-miR-4787-5p, miR-1343-3p is hsa-miR-1343-3p, miR-6813-5p is hsa-miR-6813-5p, miR-1225-5p is hsa-miR-1225-5p, miR-602 is hsa-miR-602, miR-4488 is hsa-miR-4488, miR-125a-3p is hsa-miR-125a-3p, miR-5100 is hsa-miR-5100, miR-4294 is hsa-miR-4294, miR-1231 is hsa-miR-1231, miR-6765-3p is hsa-miR-6765-3p, miR-4442 is hsa-miR-4442, miR-718 is hsa-miR-718, miR-6780b-5p is hsa-miR-6780b-5p, miR-6090 is hsa-miR-6090, miR-6845-5p is hsa-miR-6845-5p, miR-4741 is hsa-miR-4741, miR-4467 is hsa-miR-4467, miR-4707-5p is hsa-miR-4707-5p, miR-4271 is hsa-miR-4271, miR-4673 is hsa-miR-4673, miR-3184-5p is hsa-miR-3184-5p, miR-1469 is hsa-miR-1469, miR-4640-5p is hsa-miR-4640-5p, miR-663a is hsa-miR-663a, miR-6791-5p is hsa-miR-6791-5p, miR-6826-5p is hsa-miR-6826-5p, miR-4433b-3p is hsa-miR-4433b-3p, miR-1915-3p is hsa-miR-1915-3p, miR-4417 is hsa-miR-4417, miR-4449 is hsa-miR-4449, miR-4707-3p is hsa-miR-4707-3p, miR-3180-3p is hsa-miR-3180-3p, miR-5585-3p is hsa-miR-5585-3p, miR-1268a is hsa-miR-1268a, miR-8072 is hsa-miR-8072, miR-296-5p is hsa-miR-296-5p, miR-204-3p is hsa-miR-204-3p, miR-4454 is hsa-miR-4454, miR-6722-3p is hsa-miR-6722-3p, miR-1290 is hsa-miR-1290, miR-3622a-5p is hsa-miR-3622a-5p, miR-939-5p is hsa-miR-939-5p, miR-675-5p is hsa-miR-675-5p, miR-3131 is hsa-miR-3131, miR-4648 is hsa-miR-4648, miR-1268b is hsa-miR-1268b, miR-6741-5p is hsa-miR-6741-5p, miR-6893-5p is hsa-miR-6893-5p, miR-3162-5p is hsa-miR-3162-5p, miR-642b-3p is hsa-miR-642b-3p, miR-4734 is hsa-miR-4734, miR-150-3p is hsa-miR-150-3p, miR-8089 is hsa-miR-8089, miR-6805-3p is hsa-miR-6805-3p, miR-7113-3p is hsa-miR-7113-3p, miR-6850-5p is hsa-miR-6850-5p, miR-6799-5p is hsa-miR-6799-5p, miR-6768-5p is hsa-miR-6768-5p, miR-92b-5p is hsa-miR-92b-5p, miR-3679-5p is hsa-miR-3679-5p, miR-4792 is hsa-miR-4792, miR-3656 is hsa-miR-3656, miR-92a-2-5p is hsa-miR-92a-2-5p, miR-4466 is hsa-miR-4466, miR-4513 is hsa-miR-4513, miR-6781-5p is hsa-miR-6781-5p, miR-4649-5p is hsa-miR-4649-5p, miR-6775-5p is hsa-miR-6775-5p, miR-4651 is hsa-miR-4651, miR-3195 is hsa-miR-3195, miR-6726-5p is hsa-miR-6726-5p, miR-6872-3p is hsa-miR-6872-3p, miR-371a-5p is hsa-miR-371a-5p, miR-6777-5p is hsa-miR-6777-5p, miR-6789-5p is hsa-miR-6789-5p, miR-7975 is hsa-miR-7975, miR-6821-5p is hsa-miR-6821-5p, miR-4534 is hsa-miR-4534, miR-619-5p is hsa-miR-619-5p, miR-7107-5p is hsa-miR-7107-5p, miR-1228-3p is hsa-miR-1228-3p, miR-6774-5p is hsa-miR-6774-5p, miR-6805-5p is hsa-miR-6805-5p, miR-23a-3p is hsa-miR-23a-3p, miR-4665-5p is hsa-miR-4665-5p, miR-4505 is hsa-miR-4505, miR-4638-5p is hsa-miR-4638-5p, miR-24-3p is hsa-miR-24-3p, miR-3135b is hsa-miR-3135b, miR-4745-5p is hsa-miR-4745-5p, miR-128-1-5p is hsa-miR-128-1-5p, miR-4476 is hsa-miR-4476, miR-4687-3p is hsa-miR-4687-3p, miR-3665 is hsa-miR-3665, miR-6806-5p is hsa-miR-6806-5p, miR-3937 is hsa-miR-3937, miR-711 is hsa-miR-711, miR-3141 is hsa-miR-3141, miR-3188 is hsa-miR-3188, miR-4281 is hsa-miR-4281, miR-5196-5p is hsa-miR-5196-5p, miR-6880-5p is hsa-miR-6880-5p, miR-3960 is hsa-miR-3960, miR-3648 is hsa-miR-3648, miR-6721-5p is hsa-miR-6721-5p, miR-4492 is hsa-miR-4492, miR-744-5p is hsa-miR-744-5p, miR-7704 is hsa-miR-7704, miR-4749-5p is hsa-miR-4749-5p, miR-762 is hsa-miR-762, miR-6836-3p is hsa-miR-6836-3p, miR-6727-5p is hsa-miR-6727-5p, miR-4739 is hsa-miR-4739, miR-7977 is hsa-miR-7977, miR-4484 is hsa-miR-4484, miR-6515-3p is hsa-miR-6515-3p, miR-373-5p is hsa-miR-373-5p, miR-4258 is hsa-miR-4258, miR-4674 is hsa-miR-4674, miR-3180 is hsa-miR-3180, miR-6076 is hsa-miR-6076, miR-1238-5p is hsa-miR-1238-5p, miR-4463 is hsa-miR-4463, miR-4486 is hsa-miR-4486, miR-4730 is hsa-miR-4730, miR-4286 is hsa-miR-4286, and miR-4739 is hsa-miR-4739.
(6) The kit according to (4) or (5), wherein the nucleic acid(s) is a polynucleotide(s) selected from the group consisting of the following polynucleotides (f) to (j):
   (f) a polynucleotide consisting of a nucleotide sequence represented by any of SEQ ID NOs: 84 to 226, 230 to 245, 247, and 249 or a nucleotide sequence derived from the nucleotide sequence by the replacement of u with t, a variant thereof, a derivative thereof, or a fragment thereof comprising 15 or more consecutive nucleotides;
   (g) a polynucleotide comprising a nucleotide sequence represented by any of SEQ ID NOs: 84 to 226, 230 to 245, 247, and 249;
   (h) a polynucleotide consisting of a nucleotide sequence complementary to a nucleotide sequence represented by any of SEQ ID NOs: 84 to 226, 230 to 245, 247, and 249 or a nucleotide sequence derived from the nucleotide sequence by the replacement of u with t, a variant thereof, a derivative thereof, or a fragment thereof comprising 15 or more consecutive nucleotides;
   (i) a polynucleotide comprising a nucleotide sequence complementary to a nucleotide sequence represented by any of SEQ ID NOs: 84 to 226, 230 to 245, 247, and 249 or a nucleotide sequence derived from the nucleotide sequence by the replacement of u with t; and
   (j) a polynucleotide hybridizing under stringent conditions to any of the polynucleotides (f) to (i).
(7) A device for the detection of early pancreatic cancer or a pancreatic cancer precursor lesion, comprising a nucleic acid(s) capable of specifically binding to at least one polynucleotide selected from early pancreatic cancer or pancreatic cancer precursor lesion markers miR-6784-5p, miR-1181, miR-671-5p, miR-6857-5p, miR-4276, miR-1914-3p, miR-149-3p, miR-937-5p, miR-4675, miR-6795-5p, miR-4731-5p, miR-5090, miR-3620-5p, miR-1343-5p, miR-6717-5p, miR-6825-5p, miR-6738-5p, miR-6769a-5p, miR-4728-5p, miR-652-5p, miR-4257, miR-6785-5p, miR-7110-5p, miR-6887-5p, miR-887-3p, miR-1228-5p, miR-5572, miR-6782-5p, miR-4298, miR-6786-5p, miR-5010-5p, miR-6087, miR-6765-5p, miR-6732-5p, miR-6787-5p, miR-6737-5p, miR-128-2-5p, miR-4270, miR-6861-5p, miR-6756-5p, miR-1229-5p, miR-6891-5p, miR-6848-5p, miR-1237-5p, miR-30c-1-3p, miR-1233-5p, miR-211-3p, miR-4758-5p, miR-614, miR-6746-5p, miR-1915-5p, miR-4688, miR-3917, miR-5787, miR-4632-5p, miR-6126, miR-135a-3p, miR-8063, miR-5698, miR-6089, miR-498, miR-296-3p, miR-4419b, miR-6802-5p, miR-6829-5p, miR-6803-5p, miR-1199-5p, miR-6840-3p, miR-6752-5p, miR-6798-5p, miR-6131, miR-4667-5p, miR-6510-5p, miR-4690-5p, miR-920, miR-23b-3p, miR-4448, miR-2110, miR-4706, miR-7845-5p, miR-6808-5p, miR-4447, miR-6869-5p, miR-6794-5p, miR-6511a-5p, miR-6824-5p, miR-6766-3p, miR-6511a-5p, and miR-6749-5p.
(8) The device according to (7), wherein miR-6784-5p is hsa-miR-6784-5p, miR-1181 is hsa-miR-1181, miR-671-5p is hsa-miR-671-5p, miR-6857-5p is hsa-miR-6857-5p, miR-4276 is hsa-miR-4276, miR-1914-3p is hsa-miR-1914-3p, miR-149-3p is hsa-miR-149-3p, miR-937-5p is hsa-miR-937-5p, miR-4675 is hsa-miR-4675, miR-6795-5p is hsa-miR-6795-5p, miR-4731-5p is hsa-miR-4731-5p, miR-5090 is hsa-miR-5090, miR-3620-5p is hsa-miR-3620-5p, miR-1343-5p is hsa-miR-1343-5p, miR-6717-5p is hsa-miR-6717-5p, miR-6825-5p is hsa-miR-6825-5p, miR-6738-5p is hsa-miR-6738-5p, miR-6769a-5p is hsa-miR-6769a-5p, miR-4728-5p is hsa-miR-4728-5p, miR-652-5p is hsa-miR-652-5p, miR-4257 is hsa-miR-4257, miR-6785-5p is hsa-miR-6785-5p, miR-7110-5p is hsa-miR-7110-5p, miR-6887-5p is hsa-miR-6887-5p, miR-887-3p is hsa-miR-887-3p, miR-1228-5p is hsa-miR-1228-5p, miR-5572 is hsa-miR-5572, miR-6782-5p is hsa-miR-6782-5p, miR-4298 is hsa-miR-4298, miR-6786-5p is hsa-miR-6786-5p, miR-5010-5p is hsa-miR-5010-5p, miR-6087 is hsa-miR-6087, miR-6765-5p is hsa-miR-6765-5p, miR-6732-5p is hsa-miR-6732-5p, miR-6787-5p is hsa-miR-6787-5p, miR-6737-5p is hsa-miR-6737-5p, miR-128-2-5p is hsa-miR-128-2-5p, miR-4270 is hsa-miR-4270, miR-6861-5p is hsa-miR-6861-5p, miR-6756-5p is hsa-miR-6756-5p, miR-1229-5p is hsa-miR-1229-5p, miR-6891-5p is hsa-miR-6891-5p, miR-6848-5p is hsa-miR-6848-5p, miR-1237-5p is hsa-miR-1237-5p, miR-30c-1-3p is hsa-miR-30c-1-3p, miR-1233-5p is hsa-miR-1233-5p, miR-211-3p is hsa-miR-211-3p, miR-4758-5p is hsa-miR-4758-5p, miR-614 is hsa-miR-614, miR-6746-5p is hsa-miR-6746-5p, miR-1915-5p is hsa-miR-1915-5p, miR-4688 is hsa-miR-4688, miR-3917 is hsa-miR-3917, miR-5787 is hsa-miR-5787, miR-4632-5p is hsa-miR-4632-5p, miR-6126 is hsa-miR-6126, miR-135a-3p is hsa-miR-135a-3p, miR-8063 is hsa-miR-8063, miR-5698 is hsa-miR-5698, miR-6089 is hsa-miR-6089, miR-498 is hsa-miR-498, miR-296-3p is hsa-miR-296-3p, miR-4419b is hsa-miR-4419b, miR-6802-5p is hsa-miR-6802-5p, miR-6829-5p is hsa-miR-6829-5p, miR-6803-5p is hsa-miR-6803-5p, miR-1199-5p is hsa-miR-1199-5p, miR-6840-3p is hsa-miR-6840-3p, miR-6752-5p is hsa-miR-6752-5p, miR-6798-5p is hsa-miR-6798-5p, miR-6131 is hsa-miR-6131, miR-4667-5p is hsa-miR-4667-5p, miR-6510-5p is hsa-miR-6510-5p, miR-4690-5p is hsa-miR-4690-5p, miR-920 is hsa-miR-920, miR-23b-3p is hsa-miR-23b-3p, miR-4448 is hsa-miR-4448, miR-2110 is hsa-miR-2110, miR-4706 is hsa-miR-4706, miR-7845-5p is hsa-miR-7845-5p, miR-6808-5p is hsa-miR-6808-5p, miR-4447 is hsa-miR-4447, miR-6869-5p is hsa-miR-6869-5p, miR-6794-5p is hsa-miR-6794-5p, miR-6511a-5p is hsa-miR-6511a-5p, miR-6824-5p is hsa-miR-6824-5p, miR-6766-3p is hsa-miR-6766-3p, miR-6511a-5p is hsa-miR-6511a-5p, and miR-6749-5p is hsa-miR-6749-5p.
(9) The device according to (7) or (8), wherein the nucleic acid(s) is a polynucleotide(s) selected from the group consisting of the following polynucleotides (a) to (e):
   (a) a polynucleotide consisting of a nucleotide sequence represented by any of SEQ ID NOs: 1 to 83, 227 to 229, 246, 248, and 250 or a nucleotide sequence derived from the nucleotide sequence by the replacement of u with t, a variant thereof, a derivative thereof, or a fragment thereof comprising 15 or more consecutive nucleotides;
   (b) a polynucleotide comprising a nucleotide sequence represented by any of SEQ ID NOs: 1 to 83, 227 to 229, 246, 248 and 250;
   (c) a polynucleotide consisting of a nucleotide sequence complementary to a nucleotide sequence represented by any of SEQ ID NOs: 1 to 83, 227 to 229, 246, 248, and 250 or a nucleotide sequence derived from the nucleotide sequence by the replacement of u with t, a variant thereof, a derivative thereof, or a fragment thereof comprising 15 or more consecutive nucleotides;
   (d) a polynucleotide comprising a nucleotide sequence complementary to a nucleotide sequence represented by any of SEQ ID NOs: 1 to 83, 227 to 229, 246, 248, and 250 or a nucleotide sequence derived from the nucleotide sequence by the replacement of u with t; and
   (e) a polynucleotide hybridizing under stringent conditions to any of the polynucleotides (a) to (d).
(10) The device according to any of (7) to (9), wherein the device further comprises a nucleic acid(s) capable of specifically binding to at least one polynucleotide selected from the group consisting of other early pancreatic cancer or pancreatic cancer precursor lesion markers miR-1908-5p, miR-6729-5p, miR-5195-3p, miR-638, miR-6125, miR-3178, miR-3196, miR-8069, miR-4723-5p, miR-4746-3p, miR-4689, miR-6816-5p, miR-6757-5p, miR-7109-5p, miR-6724-5p, miR-1225-3p, miR-6875-5p, miR-7108-5p, miR-4508, miR-6085, miR-6779-5p, miR-642a-3p, miR-4695-5p, miR-7847-3p, miR-3197, miR-6769b-5p, miR-7641, miR-187-5p, miR-3185, miR-2861, miR-3940-5p, miR-1203, miR-615-5p, miR-4787-5p, miR-1343-3p, miR-6813-5p, miR-1225-5p, miR-602, miR-4488, miR-125a-3p, miR-5100, miR-4294, miR-1231, miR-6765-3p, miR-4442, miR-718, miR-6780b-5p, miR-6090, miR-6845-5p, miR-4741, miR-4467, miR-4707-5p, miR-4271, miR-4673, miR-3184-5p, miR-1469, miR-4640-5p, miR-663a, miR-6791-5p, miR-6826-5p, miR-4433b-3p, miR-1915-3p, miR-4417, miR-4449, miR-4707-3p, miR-3180-3p, miR-5585-3p, miR-1268a, miR-8072, miR-296-5p, miR-204-3p, miR-4454, miR-6722-3p, miR-1290, miR-3622a-5p, miR-939-5p, miR-675-5p, miR-3131, miR-4648, miR-1268b, miR-6741-5p, miR-6893-5p, miR-3162-5p, miR-642b-3p, miR-4734, miR-150-3p, miR-8089, miR-6805-3p, miR-7113-3p, miR-6850-5p, miR-6799-5p, miR-6768-5p, miR-92b-5p, miR-3679-5p, miR-4792, miR-3656, miR-92a-2-5p, miR-4466, miR-4513, miR-6781-5p, miR-4649-5p, miR-6775-5p, miR-4651, miR-3195, miR-6726-5p, miR-6872-3p, miR-371a-5p, miR-6777-5p, miR-6789-5p, miR-7975, miR-6821-5p, miR-4534, miR-619-5p, miR-7107-5p, miR-1228-3p, miR-6774-5p, miR-6805-5p, miR-23a-3p, miR-4665-5p, miR-4505, miR-4638-5p, miR-24-3p, miR-3135b, miR-4745-5p, miR-128-1-5p, miR-4476, miR-4687-3p, miR-3665, miR-6806-5p, miR-3937, miR-711, miR-3141, miR-3188, miR-4281, miR-5196-5p, miR-6880-5p, miR-3960, miR-3648, miR-6721-5p, miR-4492, miR-744-5p, miR-7704, miR-4749-5p, miR-762, miR-6836-3p, miR-6727-5p, miR-4739, miR-7977, miR-4484, miR-6515-3p, miR-373-5p, miR-4258, miR-4674, miR-3180, miR-6076, miR-1238-5p, miR-4463, miR-4486, miR-4730, miR-4286, and miR-4739.
(11) The device according to (10), wherein miR-1908-5p is hsa-miR-1908-5p, miR-6729-5p is hsa-miR-6729-5p, miR-5195-3p is hsa-miR-5195-3p, miR-638 is hsa-miR-638, miR-6125 is hsa-miR-6125, miR-3178 is hsa-miR-3178, miR-3196 is hsa-miR-3196, miR-8069 is hsa-miR-8069, miR-4723-5p is hsa-miR-4723-5p, miR-4746-3p is hsa-miR-4746-3p, miR-4689 is hsa-miR-4689, miR-6816-5p is hsa-miR-6816-5p, miR-6757-5p is hsa-miR-6757-5p, miR-7109-5p is hsa-miR-7109-5p, miR-6724-5p is hsa-miR-6724-5p, miR-1225-3p is hsa-miR-1225-3p, miR-6875-5p is hsa-miR-6875-5p, miR-7108-5p is hsa-miR-7108-5p, miR-4508 is hsa-miR-4508, miR-6085 is hsa-miR-6085, miR-6779-5p is hsa-miR-6779-5p, miR-642a-3p is hsa-miR-642a-3p, miR-4695-5p is hsa-miR-4695-5p, miR-7847-3p is hsa-miR-7847-3p, miR-3197 is hsa-miR-3197, miR-6769b-5p is hsa-miR-6769b-5p, miR-7641 is hsa-miR-7641, miR-187-5p is hsa-miR-187-5p, miR-3185 is hsa-miR-3185, miR-2861 is hsa-miR-2861, miR-3940-5p is hsa-miR-3940-5p, miR-1203 is hsa-miR-1203, miR-615-5p is hsa-miR-615-5p, miR-4787-5p is hsa-miR-4787-5p, miR-1343-3p is hsa-miR-1343-3p, miR-6813-5p is hsa-miR-6813-5p, miR-1225-5p is hsa-miR-1225-5p, miR-602 is hsa-miR-602, miR-4488 is hsa-miR-4488, miR-125a-3p is hsa-miR-125a-3p, miR-5100 is hsa-miR-5100, miR-4294 is hsa-miR-4294, miR-1231 is hsa-miR-1231, miR-6765-3p is hsa-miR-6765-3p, miR-4442 is hsa-miR-4442, miR-718 is hsa-miR-718, miR-6780b-5p is hsa-miR-6780b-5p, miR-6090 is hsa-miR-6090, miR-6845-5p is hsa-miR-6845-5p, miR-4741 is hsa-miR-4741, miR-4467 is hsa-miR-4467, miR-4707-5p is hsa-miR-4707-5p, miR-4271 is hsa-miR-4271, miR-4673 is hsa-miR-4673, miR-3184-5p is hsa-miR-3184-5p, miR-1469 is hsa-miR-1469, miR-4640-5p is hsa-miR-4640-5p, miR-663a is hsa-miR-663a, miR-6791-5p is hsa-miR-6791-5p, miR-6826-5p is hsa-miR-6826-5p, miR-4433b-3p is hsa-miR-4433b-3p, miR-1915-3p is hsa-miR-1915-3p, miR-4417 is hsa-miR-4417, miR-4449 is hsa-miR-4449, miR-4707-3p is hsa-miR-4707-3p, miR-3180-3p is hsa-miR-3180-3p, miR-5585-3p is hsa-miR-5585-3p, miR-1268a is hsa-miR-1268a, miR-8072 is hsa-miR-8072, miR-296-5p is hsa-miR-296-5p, miR-204-3p is hsa-miR-204-3p, miR-4454 is hsa-miR-4454, miR-6722-3p is hsa-miR-6722-3p, miR-1290 is hsa-miR-1290, miR-3622a-5p is hsa-miR-3622a-5p, miR-939-5p is hsa-miR-939-5p, miR-675-5p is hsa-miR-675-5p, miR-3131 is hsa-miR-3131, miR-4648 is hsa-miR-4648, miR-1268b is hsa-miR-1268b, miR-6741-5p is hsa-miR-6741-5p, miR-6893-5p is hsa-miR-6893-5p, miR-3162-5p is hsa-miR-3162-5p, miR-642b-3p is hsa-miR-642b-3p, miR-4734 is hsa-miR-4734, miR-150-3p is hsa-miR-150-3p, miR-8089 is hsa-miR-8089, miR-6805-3p is hsa-miR-6805-3p, miR-7113-3p is hsa-miR-7113-3p, miR-6850-5p is hsa-miR-6850-5p, miR-6799-5p is hsa-miR-6799-5p, miR-6768-5p is hsa-miR-6768-5p, miR-92b-5p is hsa-miR-92b-5p, miR-3679-5p is hsa-miR-3679-5p, miR-4792 is hsa-miR-4792, miR-3656 is hsa-miR-3656, miR-92a-2-5p is hsa-miR-92a-2-5p, miR-4466 is hsa-miR-4466, miR-4513 is hsa-miR-4513, miR-6781-5p is hsa-miR-6781-5p, miR-4649-5p is hsa-miR-4649-5p, miR-6775-5p is hsa-miR-6775-5p, miR-4651 is hsa-miR-4651, miR-3195 is hsa-miR-3195, miR-6726-5p is hsa-miR-6726-5p, miR-6872-3p is hsa-miR-6872-3p, miR-371a-5p is hsa-miR-371a-5p, miR-6777-5p is hsa-miR-6777-5p, miR-6789-5p is hsa-miR-6789-5p, miR-7975 is hsa-miR-7975, miR-6821-5p is hsa-miR-6821-5p, miR-4534 is hsa-miR-4534, miR-619-5p is hsa-miR-619-5p, miR-7107-5p is hsa-miR-7107-5p, miR-1228-3p is hsa-miR-1228-3p, miR-6774-5p is hsa-miR-6774-5p, miR-6805-5p is hsa-miR-6805-5p, miR-23a-3p is hsa-miR-23a-3p, miR-4665-5p is hsa-miR-4665-5p, miR-4505 is hsa-miR-4505, miR-4638-5p is hsa-miR-4638-5p, miR-24-3p is hsa-miR-24-3p, miR-3135b is hsa-miR-3135b, miR-4745-5p is hsa-miR-4745-5p, miR-128-1-5p is hsa-miR-128-1-5p, miR-4476 is hsa-miR-4476, miR-4687-3p is hsa-miR-4687-3p, miR-3665 is hsa-miR-3665, miR-6806-5p is hsa-miR-6806-5p, miR-3937 is hsa-miR-3937, miR-711 is hsa-miR-711, miR-3141 is hsa-miR-3141, miR-3188 is hsa-miR-3188, miR-4281 is hsa-miR-4281, miR-5196-5p is hsa-miR-5196-5p, miR-6880-5p is hsa-miR-6880-5p, miR-3960 is hsa-miR-3960, miR-3648 is hsa-miR-3648, miR-6721-5p is hsa-miR-6721-5p, miR-4492 is hsa-miR-4492, miR-744-5p is hsa-miR-744-5p, miR-7704 is hsa-miR-7704, miR-4749-5p is hsa-miR-4749-5p, miR-762 is hsa-miR-762, miR-6836-3p is hsa-miR-6836-3p, miR-6727-5p is hsa-miR-6727-5p, miR-4739 is hsa-miR-4739, miR-7977 is hsa-miR-7977, miR-4484 is hsa-miR-4484, miR-6515-3p is hsa-miR-6515-3p, miR-373-5p is hsa-miR-373-5p, miR-4258 is hsa-miR-4258, miR-4674 is hsa-miR-4674, miR-3180 is hsa-miR-3180, miR-6076 is hsa-miR-6076, miR-1238-5p is hsa-miR-1238-5p, miR-4463 is hsa-miR-4463, miR-4486 is hsa-miR-4486, miR-4730 is hsa-miR-4730, miR-4286 is hsa-miR-4286, and miR-4739 is hsa-miR-4739.
(12) The device according to (10) or (11), wherein the nucleic acid(s) is a polynucleotide(s) selected from the group consisting of the following polynucleotides (f) to (j):
   (f) a polynucleotide consisting of a nucleotide sequence represented by any of SEQ ID NOs: 84 to 226, 230 to 245, 247, and 249 or a nucleotide sequence derived from the nucleotide sequence by the replacement of u with t, a variant thereof, a derivative thereof, or a fragment thereof comprising 15 or more consecutive nucleotides;
   (g) a polynucleotide comprising a nucleotide sequence represented by any of SEQ ID NOs: 84 to 226, 230 to 245, 247, and 249;
   (h) a polynucleotide consisting of a nucleotide sequence complementary to a nucleotide sequence represented by any of SEQ ID NOs: 84 to 226, 230 to 245, 247, and 249 or a nucleotide sequence derived from the nucleotide sequence by the replacement of u with t, a variant thereof, a derivative thereof, or a fragment thereof comprising 15 or more consecutive nucleotides;
   (i) a polynucleotide comprising a nucleotide sequence complementary to a nucleotide sequence represented by any of SEQ ID NOs: 84 to 226, 230 to 245, 247, and 249 or a nucleotide sequence derived from the nucleotide sequence by the replacement of u with t; and
   (j) a polynucleotide hybridizing under stringent conditions to any of the polynucleotides (f) to (i).
(13) The device according to any one of (7) to (12), wherein the device is for measurement based on a hybridization technique.
(14) The device according to (13), wherein the hybridization technique is a nucleic acid array technique.
(15) A method for detecting early pancreatic cancer or a pancreatic cancer precursor lesion in a subject, comprising: measuring an expression level(s) of a target nucleic acid(s) in a sample from the subject using a kit according to any of (1) to (6) or a device according to any of (7) to (14); and evaluating *in vitro* whether or not the subject has early pancreatic cancer or a pancreatic cancer precursor lesion using both of the measured expression level(s) and a control expression level(s) in a sample from a healthy subject measured in the same way, to detect the presence or absence of early pancreatic cancer or a pancreatic cancer precursor lesion in the subject.
(16) A method for detecting early pancreatic cancer or a pancreatic cancer precursor lesion in a subject, comprising: measuring an expression level(s) of a target gene(s) in a sample from the subject using a kit according to any of (1) to (6) or a device according to any of (7) to (14); and assigning the expression level(s) of the target gene(s) in the sample from the subject to a discriminant (discriminant function) to evaluate the presence or absence of early pancreatic cancer or a pancreatic cancer precursor lesion, wherein the discriminant is prepared with the gene expression level(s) in a sample(s) from a subject(s) known to have early pancreatic cancer or a pancreatic cancer precursor lesion and the gene expression level(s) in a sample(s) from a healthy subject(s) as supervising samples and is capable of discriminating an early pancreatic cancer or pancreatic cancer precursor lesion patient from a healthy subject.
(17) The method according to (15) or (16), wherein the subject is a human.
(18) The method according to any one of (15) to (17), wherein the sample is blood, serum, or plasma.

### <Definition of Terms>

The terms used herein are defined as described below.

The term "pancreatic cancer" used herein refers to invasive ductal carcinomas. Specifically, the "pancreatic cancer" includes papillary adenocarcinoma, tubular adenocarcinoma, poorly differentiated adenocarcinoma, adenosquamous carcinoma, mucinous carcinoma, anaplastic carcinoma, and the like formed in the pancreas ("Classification of Pancreatic Carcinoma", the 6th edition, revised version, 2013, Japan Pancreas Society, KANEHARA & Co., LTD. (Tokyo, Japan), p. 27-28).

The term "pancreatic cancer precursor lesion" used herein refers to exocrine neoplasms formed in the pancreas. Specifically, the "pancreatic cancer precursor lesion" includes serous cystic neoplasms (SCNs), serous cystadenoma (SCA), serous cystadenocarcinoma (SCC), mucinous cystic neoplasms (MCNs), mucinous cystadenoma (MCA), mucinous cystadenocarcinoma (MCC), intraductal papillary-mucinous neoplasms (IPMNs), intraductal papillary-mucinous adenoma (IPMA), intraductal papillary-mucinous carcinoma (IPMC), and the like ("General Rules for the Study of Pancreatic Cancer", the 6th edition, revised version, 2013, Japan Pancreas Society, KANEHARA & Co., LTD. (Tokyo, Japan), p. 24-27).

The term "stage of progression of pancreatic cancer" used herein is classified into stages 0, IA, IB, IIA, IIB, III, IVa, and IVb according to the local extent of the primary tumor, lymph node metastasis, distant metastasis, etc. ("General Rules for the Study of Pancreatic Cancer", the 6th edition, revised version, 2013, Japan Pancreas Society, KANEHARA & Co., LTD. (Tokyo, Japan), p. 55-57).

The term "early pancreatic cancer" used herein refers to pancreatic cancer of stage 0, IA, IB, IIA, or IIB.

The term "advanced pancreatic cancer" used herein refers to pancreatic cancer of stage III, IVa, or IVb.

The term "benign disease" used herein refers to a disease with a nonmalignant tumor in an organ.

Abbreviations or terms such as "nucleotide", "polynucleotide", "DNA", and "RNA" used herein abide by "Guidelines for the preparation of specification which contains nucleotide and/or amino acid sequences" (edited by Japan Patent Office) and common use in the art.

The term "polynucleotide" used herein refers to a nucleic acid including any of RNA, DNA, and RNA/DNA (chimera). The DNA includes any of cDNA, genomic DNA, and synthetic DNA. The RNA includes any of total RNA, mRNA, rRNA, miRNA, siRNA, snoRNA, snRNA, non-coding RNA and synthetic RNA. Here the "synthetic DNA" and the "synthetic RNA" refer to a DNA and an RNA artificially prepared using, for example, an automatic nucleic acid synthesizer, on the basis of predetermined nucleotide sequences (which may be any of natural and non-natural sequences). Herein, the "non-natural sequence" is intended to be used in a broad sense and includes, for example, a sequence comprising substitution, deletion, insertion, and/or addition of one or more nucleotides (i.e., a variant sequence) and a sequence comprising one or more modified nucleotides (i.e., a modified sequence), which are different from the natural sequence. Herein, the term "polynucleotide" is used interchangeably with the term "nucleic acid."

The term "fragment" used herein is a polynucleotide having a nucleotide sequence that consists of a consecutive portion of a polynucleotide and desirably has a length of 15 or more nucleotides, preferably 17 or more nucleotides, more preferably 19 or more nucleotides.

The term "gene" used herein is intended to include not only RNA and double-stranded DNA but also each single-stranded DNA such as a plus(+) strand (or a sense strand) or a complementary strand (or an antisense strand) constituting the duplex. The gene is not particularly limited by its length.

Thus, the "gene" used herein includes any of double-stranded DNA including human genomic DNA, single-stranded DNA (plus strand), single-stranded DNA having a sequence complementary to the plus strand (complementary strand) (e.g., cDNA), microRNA (miRNA), and their fragments, and their transcripts, unless otherwise specified. The "gene" includes not only a "gene" represented by a particular nucleotide sequence (or SEQ ID NO) but "nucleic acids" encoding RNAs having biological functions equivalent to RNA encoded by the gene, for example, a congener (i.e., a homolog or an ortholog), a variant (e.g., a genetic polymorph), and a derivative. Specific examples of such a "nucleic acid" encoding a congener, a variant, or a derivative can include a "nucleic acid" having a nucleotide sequence hybridizing under stringent conditions described later to a complementary sequence of a nucleotide sequence represented by any of SEQ ID NOs: 1 to 812 or a nucleotide sequence derived from the nucleotide sequence by the replacement of u with t. Regardless whether or not there is a difference in functional region, the "gene" can comprise, for example, expression control regions, coding region, exons, or introns. The "gene" may be contained in a cell or may exist alone after being released from a cell. Alternatively, the "gene" may be in a state enclosed in a vesicle called exosome.

The term "exosome" used herein refers to a vesicle that is encapsulated by lipid bilayer and secreted from a cell. The exosome is derived from a multivesicular endosome and may incorporate biomaterials such as "genes" (e.g., RNA or DNA) or proteins when released into an extracellular environment. The exosome is known to be contained in a body fluid such as blood, serum, plasma, or lymph.

The term "transcript" used herein refers to an RNA synthesized from the DNA sequence of a gene as a template. RNA polymerase binds to a site called promoter located upstream of the gene and adds ribonucleotides complementary to the nucleotide sequence of the DNA to the 3' end to synthesize an RNA. This RNA contains not only the gene itself but the whole sequence from a transcription initiation site to the end of a polyA sequence, including expression control regions, coding region, exons, or introns.

Unless otherwise specified, the term "microRNA (miRNA)" used herein is intended to mean a 15- to 25-nucleotide non-coding RNA that is transcribed as an RNA precursor having a hairpin-like structure, cleaved by a dsRNA-cleaving enzyme having RNase III cleavage activity, and integrated into a protein complex called RISC, and that is involved in the suppression of translation of mRNA. The term "miRNA" used herein includes not only a "miRNA" represented by a particular nucleotide sequence (or SEQ ID NO) but a precursor of the "miRNA" (pre-miRNA or pri-miRNA), and miRNAs having biological functions equivalent thereto, for example, a congener (i.e., a homolog or an ortholog), a variant (e.g., a genetic polymorph), and a derivative. Such a precursor, a congener, a variant, or a derivative can be specifically identified using miRBase Release 20 (http://www.mirbase.org/), and examples thereof can include a "miRNA" having a nucleotide sequence hybridizing under stringent conditions described later to a complementary sequence of any particular nucleotide sequence represented by any of SEQ ID NOs: 1 to 812. The term "miRNA" used herein may be a gene product of a miR gene. Such a gene product includes a mature miRNA (e.g., a 15-to 25-nucleotide or 19- to 25-nucleotide non-coding RNA involved in the suppression of translation of mRNA as described above) or a miRNA precursor (e.g., pre-miRNA or pri-miRNA as described above).

The term "probe" used herein includes a polynucleotide that is used for specifically detecting an RNA resulting from the expression of a gene or a polynucleotide derived from the RNA, and/or a polynucleotide complementary thereto.

The term "primer" used herein includes a polynucleotide that specifically recognizes and amplifies an RNA resulting from the expression of a gene or a polynucleotide derived from the RNA, and/or a polynucleotide complementary thereto.

In this context, the complementary polynucleotide (complementary strand or reverse strand) means a polynucleotide in a complementary relationship based on A:T (U) and G:C base pairs with the full-length sequence of a polynucleotide consisting of a nucleotide sequence defined by any of SEQ ID NOs: 1 to 812 or a nucleotide sequence derived from the nucleotide sequence by the replacement of u with t, or a partial sequence thereof (here, this full-length or partial sequence is referred to as a plus strand for the sake of convenience). The phrase "polynucleotide consisting of a nucleotide sequence complementary" to a nucleotide sequence represented by any of SEQ ID NOs: 1 to 812 or a nucleotide sequence derived from the nucleotide sequence by the replacement of u with t is also basically understood in the same way.

The term "stringent conditions" used herein refers to conditions under which a nucleic acid probe hybridizes to its target sequence to a detectably larger extent (e.g., a measurement value equal to or larger than "(a mean of background measurement values) + (a standard error of the background measurement values) × 2") than that for other sequences. The stringent conditions are dependent on a sequence and differ depending on an environment where hybridization is performed. A target sequence complementary in 100% to the nucleic acid probe can be identified by controlling the stringency of hybridization and/or washing conditions. Specific examples of the "stringent conditions" will be mentioned later.

The term "Tm value" used herein means a temperature at which the double-stranded part of a polynucleotide is denatured into single strands so that the double strands and the single strands exist at a ratio of 1:1.

The term "variant" used herein means, in the case of a nucleic acid, a natural variant attributed to polymorphism, mutation, or the like; a variant containing the deletion, substitution, addition, or insertion of 1 or 2 or more (e.g., 1 to several) nucleotides in a nucleotide sequence represented by any of SEQ ID NOs: 1 to 812 or a nucleotide sequence derived from the nucleotide sequence by the replacement of u with t, or a partial sequence thereof; a variant that exhibits percent (%) identity of approximately 90% or higher, approximately 95% or higher, approximately 97% or higher, approximately 98% or higher, approximately 99% or higher to each of these nucleotide sequences or the partial sequences thereof; or a nucleic acid hybridizing under the stringent conditions defined above to a polynucleotide or an oligonucleotide comprising each of these nucleotide sequences or the partial sequences thereof.

The term "several" used herein means an integer of approximately 10, 9, 8, 7, 6, 5, 4, 3, or 2.

The variant as used herein can be prepared by use of a well-known technique such as site-directed mutagenesis or mutagenesis using PCR.

The term "percent (%) identity" used herein can be determined with or without an introduced gap, using a protein or gene search system based on BLAST or FASTA (Zheng Zhang et al., 2000, J. Comput. Biol., Vol. 7, p. 203-214; Altschul, S.F. et al., 1990, Journal of Molecular Biology, Vol. 215, p. 403-410; and Pearson, W.R. et al., 1988, Proc. Natl. Acad. Sci. U. S. A., Vol. 85, p. 2444-2448).

The term "derivative" used herein is meant to include a modified nucleic acid, for example, unlimitedly, a derivative labeled with a fluorophore or the like, a derivative containing a modified nucleotide (e.g., a nucleotide containing a group such as halogen, alkyl such as methyl, alkoxy such as methoxy, thio, or carboxymethyl, and a nucleotide that has undergone base rearrangement, double bond saturation, deamination, replacement of an oxygen molecule with a sulfur atom, etc.), PNA (peptide nucleic acid; Nielsen, P.E. et al., 1991, Science, Vol. 254, p. 1497-500), and LNA (locked nucleic acid; Obika, S. et al., 1998, Tetrahedron Lett., Vol. 39, p. 5401-5404).

As used herein, the "nucleic acid" capable of specifically binding to a polynucleotide selected from the early pancreatic cancer or pancreatic cancer precursor lesion marker miRNAs described above is a synthesized or prepared nucleic acid and specifically includes a "nucleic acid probe" or a "primer". The "nucleic acid" is utilized directly or indirectly for detecting the presence or absence of early pancreatic cancer or a pancreatic cancer precursor lesion in a subject, for diagnosing the presence or absence or the severity of early pancreatic cancer or a pancreatic cancer precursor lesion, the presence or absence or the degree of amelioration of early pancreatic cancer or a pancreatic cancer precursor lesion, or the therapeutic sensitivity of early pancreatic cancer or a pancreatic cancer precursor lesion, or for screening for a candidate substance useful in the prevention, amelioration, or treatment of early pancreatic cancer or a pancreatic cancer precursor lesion. The "nucleic acid" includes a nucleotide, an oligonucleotide, and a polynucleotide capable of specifically recognizing and binding to a transcript represented by any of SEQ ID NOs: 1 to 812 or a synthetic cDNA nucleic acid thereof *in vivo*, particularly in a sample such as a body fluid (e.g., blood or urine), in relation to the development of early pancreatic cancer or a pancreatic cancer precursor lesion. The nucleotide, the oligonucleotide, and the polynucleotide can be effectively used as probes for detecting the aforementioned gene expressed *in vivo*, in tissues, in cells, or the like on the basis of the properties described above, or as primers for amplifying the aforementioned gene expressed *in vivo*.

The term "detection" used herein is interchangeable with the term "examination", "measurement", "decision", or "decision support". As used herein, the term "evaluation" is meant to include diagnosing or evaluation-supporting on the basis of examination results or measurement results.

The term "subject" used herein means a mammal such as a primate including a human and a chimpanzee, a pet animal including a dog and a cat, a livestock animal including cattle, a horse, sheep, and a goat, a rodent including a mouse and a rat, and an animal that is kept in a zoo. The subject is preferably a human. The term "healthy subject" also means such a mammal without being affected with the cancer to be detected. The healthy subject is preferably a human.

The term "P" or "P value" used herein refers to a probability at which a more extreme statistic than that actually calculated from data under null hypothesis is observed in a statistical test. Thus, with smaller "P" or "P value", it is regarded that there is a more significant difference between subjects to be compared.

The term "sensitivity" used herein means a value of (the number of true positives) / (the number of true positives + the number of false negatives). High sensitivity allows early pancreatic cancer or a pancreatic cancer precursor lesion to be detected early, leading to the complete resection of cancer sites and reduction in the rate of recurrence.

The term "specificity" used herein means a value of (the number of true negatives) / (the number of true negatives + the number of false positives). High specificity prevents needless extra examination for healthy subjects misjudged as being early pancreatic cancer or pancreatic cancer precursor lesion patients, leading to reduction in burden on patients and reduction in medical expense.

The term "accuracy" used herein means a value of (the number of true positives + the number of true negatives) / (the total number of cases). The accuracy indicates the ratio of samples that are identified correctly to all samples, and serves as a primary index for evaluating detection performance.

As used herein, the "sample" that is subject to determination, detection, or diagnosis refers to a tissue and a biological material in which the expression of the gene of the present invention varies as early pancreatic cancer or pancreatic cancer precursor lesion develops, as early pancreatic cancer or pancreatic cancer precursor lesion progresses, or as therapeutic effects on early pancreatic cancer or a pancreatic cancer precursor lesion are exerted. Specifically, the "sample" refers to a pancreatic tissue, a peripancreatic vascular channel, lymph node, and organ, an organ suspected of having metastasis, the skin, a body fluid such as blood, urine, saliva, sweat, or tissue exudates, serum or plasma prepared from blood, feces, hair, and the like. The "sample" further refers to a biological sample extracted therefrom, specifically, a gene such as RNA or miRNA.

The term "hsa-miR-6784-5p gene" or "hsa-miR-6784-5p" used herein includes the hsa-miR-6784-5p gene (miRBase Accession No. MIMAT0027468) shown in SEQ ID NO: 1, a homolog or an ortholog thereof of a different organism species, and the like. The gene can be obtained by a method described in Ladewig E et al., 2012, Genome Res, Vol. 22, p. 1634-1645. Also, "hsa-mir-6784" (miRBase Accession No. MI0022629, SEQ ID NO: 251) having a hairpin-like structure is known as a precursor of "hsa-miR-6784-5p".

The term "hsa-miR-1181 gene" or "hsa-miR-1181" used herein includes the hsa-miR-1181 gene (miRBase Accession No. MIMAT0005826) shown in SEQ ID NO: 2, a homolog or an ortholog thereof of a different organism species, and the like. The gene can be obtained by a method described in Subramanian S et al., 2008, Oncogene, Vol. 27, p. 2015-2026. Also, "hsa-mir-1181" (miRBase Accession No. MI0006274, SEQ ID NO: 252) having a hairpin-like structure is known as a precursor of "hsa-miR-1181".

The term "hsa-miR-671-5p gene" or "hsa-miR-671-5p" used herein includes the hsa-miR-671-5p gene (miRBase Accession No. MIMAT0003880) shown in SEQ ID NO: 3, a homolog or an ortholog thereof of a different organism species, and the like. The gene can be obtained by a method described in Berezikov E et al., 2006, Genome Res, Vol. 16, p. 1289-1298. Also, "hsa-mir-671" (miRBase Accession No. MI0003760, SEQ ID NO: 253) having a hairpin-like structure is known as a precursor of "hsa-miR-671-5p".

The term "hsa-miR-6857-5p gene" or "hsa-miR-6857-5p" used herein includes the hsa-miR-6857-5p gene (miRBase Accession No. MIMAT0027614) shown in SEQ ID NO: 4, a homolog or an ortholog thereof of a different organism species, and the like. The gene can be obtained by a method described in Ladewig E et al., 2012, Genome Res, Vol. 22, p. 1634-1645. Also, "hsa-mir-6857" (miRBase Accession No. MI0022703, SEQ ID NO: 254) having a hairpin-like structure is known as a precursor of "hsa-miR-6857-5p".

The term "hsa-miR-4276 gene" or "hsa-miR-4276" used herein includes the hsa-miR-4276 gene (miRBase Accession No. MIMAT0016904) shown in SEQ ID NO: 5, a homolog or an ortholog thereof of a different organism species, and the like. The gene can be obtained by a method described in Goff LA et al., 2009, PLoS One, Vol. 4, e7192. Also, "hsa-mir-4276" (miRBase Accession No. MI0015882, SEQ ID NO: 255) having a hairpin-like structure is known as a precursor of "hsa-miR-4276".

The term "hsa-miR-1914-3p gene" or "hsa-miR-1914-3p" used herein includes the hsa-miR-1914-3p gene (miRBase Accession No. MIMAT0007890) shown in SEQ ID NO: 6, a homolog or an ortholog thereof of a different organism species, and the like. The gene can be obtained by a method described in Bar M et al., 2008, Stem Cells, Vol. 26, p. 2496-2505. Also, "hsa-mir-1914" (miRBase Accession No. MI0008335, SEQ ID NO: 256) having a hairpin-like structure is known as a precursor of "hsa-miR-1914-3p".

The term "hsa-miR-149-3p gene" or "hsa-miR-149-3p" used herein includes the hsa-miR-149-3p gene (miRBase Accession No. MIMAT0004609) shown in SEQ ID NO: 7, a homolog or an ortholog thereof of a different organism species, and the like. The gene can be obtained by a method described in Lagos-Quintana M et al., 2002, Curr Biol, Vol. 12, p. 735-739. Also, "hsa-mir-149" (miRBase Accession No. MI0000478, SEQ ID NO: 257) having a hairpin-like structure is known as a precursor of "hsa-miR-149-3p".

The term "hsa-miR-937-5p gene" or "hsa-miR-937-5p" used herein includes the hsa-miR-937-5p gene (miRBase Accession No. MIMAT0022938) shown in SEQ ID NO: 8, a homolog or an ortholog thereof of a different organism species, and the like. The gene can be obtained by a method described in Lui WO et al., 2007, Cancer Res, Vol. 67, p. 6031-6043. Also, "hsa-mir-937" (miRBase Accession No. MI0005759, SEQ ID NO: 258) having a hairpin-like structure is known as a precursor of "hsa-miR-937-5p".

The term "hsa-miR-4675 gene" or "hsa-miR-4675" used herein includes the hsa-miR-4675 gene (miRBase Accession No. MIMAT0019757) shown in SEQ ID NO: 9, a homolog or an ortholog thereof of a different organism species, and the like. The gene can be obtained by a method described in Persson H et al., 2011, Cancer Res, Vol. 71, p. 78-86. Also, "hsa-mir-4675" (miRBase Accession No. MI0017306, SEQ ID NO: 259) having a hairpin-like structure is known as a precursor of "hsa-miR-4675".

The term "hsa-miR-6795-5p gene" or "hsa-miR-6795-5p" used herein includes the hsa-miR-6795-5p gene (miRBase Accession No. MIMAT0027490) shown in SEQ ID NO: 10, a homolog or an ortholog thereof of a different organism species, and the like. The gene can be obtained by a method described in Ladewig E et al., 2012, Genome Res, Vol. 22, p. 1634-1645. Also, "hsa-mir-6795" (miRBase Accession No. MI0022640, SEQ ID NO: 260) having a hairpin-like structure is known as a precursor of "hsa-miR-6795-5p".

The term "hsa-miR-4731-5p gene" or "hsa-miR-4731-5p" used herein includes the hsa-miR-4731-5p gene (miRBase Accession No. MIMAT0019853) shown in SEQ ID NO: 11, a homolog or an ortholog thereof of a different organism species, and the like. The gene can be obtained by a method described in Persson H et al., 2011, Cancer Res, Vol. 71, p. 78-86. Also, "hsa-mir-4731" (miRBase Accession No. MI0017368, SEQ ID NO: 261) having a hairpin-like structure is known as a precursor of "hsa-miR-4731-5p".

The term "hsa-miR-5090 gene" or "hsa-miR-5090" used herein includes the hsa-miR-5090 gene (miRBase Accession No. MIMAT0021082) shown in SEQ ID NO: 12, a homolog or an ortholog thereof of a different organism species, and the like. The gene can be obtained by a method described in Ding N et al., 2011, J Radiat Res, Vol. 52, p. 425-432. Also, "hsa-mir-5090" (miRBase Accession No. MI0017979, SEQ ID NO: 262) having a hairpin-like structure is known as a precursor of "hsa-miR-5090".

The term "hsa-miR-3620-5p gene" or "hsa-miR-3620-5p" used herein includes the hsa-miR-3620-5p gene (miRBase Accession No. MIMAT0022967) shown in SEQ ID NO: 13, a homolog or an ortholog thereof of a different organism species, and the like. The gene can be obtained by a method described in Witten D et al., 2010, BMC Biol, Vol. 8, p. 58. Also, "hsa-mir-3620" (miRBase Accession No. MI0016011, SEQ ID NO: 263) having a hairpin-like structure is known as a precursor of "hsa-miR-3620-5p".

The term "hsa-miR-1343-5p gene" or "hsa-miR-1343-5p" used herein includes the hsa-miR-1343-5p gene (miRBase Accession No. MIMAT0027038) shown in SEQ ID NO: 14, a homolog or an ortholog thereof of a different organism species, and the like. The gene can be obtained by a method described in Persson H et al., 2011, Cancer Res, Vol. 71, p. 78-86. Also, "hsa-mir-1343" (miRBase Accession No. MI0017320, SEQ ID NO: 264) having a hairpin-like structure is known as a precursor of "hsa-miR-1343-5p".

The term "hsa-miR-6717-5p gene" or "hsa-miR-6717-5p" used herein includes the hsa-miR-6717-5p gene (miRBase Accession No. MIMAT0025846) shown in SEQ ID NO: 15, a homolog or an ortholog thereof of a different organism species, and the like. The gene can be obtained by a method described in Li Y et al., 2012, Gene, Vol. 497, p. 330-335. Also, "hsa-mir-6717" (miRBase Accession No. MI0022551, SEQ ID NO: 265) having a hairpin-like structure is known as a precursor of "hsa-miR-6717-5p".

The term "hsa-miR-6825-5p gene" or "hsa-miR-6825-5p" used herein includes the hsa-miR-6825-5p gene (miRBase Accession No. MIMAT0027550) shown in SEQ ID NO: 16, a homolog or an ortholog thereof of a different organism species, and the like. The gene can be obtained by a method described in Ladewig E et al., 2012, Genome Res, Vol. 22, p. 1634-1645. Also, "hsa-mir-6825" (miRBase Accession No. MI0022670, SEQ ID NO: 266) having a hairpin-like structure is known as a precursor of "hsa-miR-6825-5p".

The term "hsa-miR-6738-5p gene" or "hsa-miR-6738-5p" used herein includes the hsa-miR-6738-5p gene (miRBase Accession No. MIMAT0027377) shown in SEQ ID NO: 17, a homolog or an ortholog thereof of a different organism species, and the like. The gene can be obtained by a method described in Ladewig E et al., 2012, Genome Res, Vol. 22, p. 1634-1645. Also, "hsa-mir-6738" (miRBase Accession No. MI0022583, SEQ ID NO: 267) having a hairpin-like structure is known as a precursor of "hsa-miR-6738-5p".

The term "hsa-miR-6769a-5p gene" or "hsa-miR-6769a-5p" used herein includes the hsa-miR-6769a-5p gene (miRBase Accession No. MIMAT0027438) shown in SEQ ID NO: 18, a homolog or an ortholog thereof of a different organism species, and the like. The gene can be obtained by a method described in Ladewig E et al., 2012, Genome Res, Vol. 22, p. 1634-1645. Also, "hsa-mir-6769a" (miRBase Accession No. MI0022614, SEQ ID NO: 268) having a hairpin-like structure is known as a precursor of "hsa-miR-6769a-5p".

The term "hsa-miR-4728-5p gene" or "hsa-miR-4728-5p" used herein includes the hsa-miR-4728-5p gene (miRBase Accession No. MIMAT0019849) shown in SEQ ID NO: 19, a homolog or an ortholog thereof of a different organism species, and the like. The gene can be obtained by a method described in Persson H et al., 2011, Cancer Res, Vol. 71, p. 78-86. Also, "hsa-mir-4728" (miRBase Accession No. MI0017365, SEQ ID NO: 269) having a hairpin-like structure is known as a precursor of "hsa-miR-4728-5p".

The term "hsa-miR-652-5p gene" or "hsa-miR-652-5p" used herein includes the hsa-miR-652-5p gene (miRBase Accession No. MIMAT0022709) shown in SEQ ID NO: 20, a homolog or an ortholog thereof of a different organism species, and the like. The gene can be obtained by a method described in Cummins JM et al., 2006, Proc Natl Acad Sci U S A, Vol. 103, p. 3687-3692. Also, "hsa-mir-652" (miRBase Accession No. MI0003667, SEQ ID NO: 270) having a hairpin-like structure is known as a precursor of "hsa-miR-652-5p".

The term "hsa-miR-4257 gene" or "hsa-miR-4257" used herein includes the hsa-miR-4257 gene (miRBase Accession No. MIMAT0016878) shown in SEQ ID NO: 21, a homolog or an ortholog thereof of a different organism species, and the like. The gene can be obtained by a method described in Goff LA et al., 2009, PLoS One, Vol. 4, e7192. Also, "hsa-mir-4257" (miRBase Accession No. MI0015856, SEQ ID NO: 271) having a hairpin-like structure is known as a precursor of "hsa-miR-4257".

The term "hsa-miR-6785-5p gene" or "hsa-miR-6785-5p" used herein includes the hsa-miR-6785-5p gene (miRBase Accession No. MIMAT0027470) shown in SEQ ID NO: 22, a homolog or an ortholog thereof of a different organism species, and the like. The gene can be obtained by a method described in Ladewig E et al., 2012, Genome Res, Vol. 22, p. 1634-1645. Also, "hsa-mir-6785" (miRBase Accession No. MI0022630, SEQ ID NO: 272) having a hairpin-like structure is known as a precursor of "hsa-miR-6785-5p".

The term "hsa-miR-7110-5p gene" or "hsa-miR-7110-5p" used herein includes the hsa-miR-7110-5p gene (miRBase Accession No. MIMAT0028117) shown in SEQ ID NO: 23, a homolog or an ortholog thereof of a different organism species, and the like. The gene can be obtained by a method described in Ladewig E et al., 2012, Genome Res, Vol. 22, p. 1634-1645. Also, "hsa-mir-7110" (miRBase Accession No. MI0022961, SEQ ID NO: 273) having a hairpin-like structure is known as a precursor of "hsa-miR-7110-5p".

The term "hsa-miR-6887-5p gene" or "hsa-miR-6887-5p" used herein includes the hsa-miR-6887-5p gene (miRBase Accession No. MIMAT0027674) shown in SEQ ID NO: 24, a homolog or an ortholog thereof of a different organism species, and the like. The gene can be obtained by a method described in Ladewig E et al., 2012, Genome Res, Vol. 22, p. 1634-1645. Also, "hsa-mir-6887" (miRBase Accession No. MI0022734, SEQ ID NO: 274) having a hairpin-like structure is known as a precursor of "hsa-miR-6887-5p".

The term "hsa-miR-887-3p gene" or "hsa-miR-887-3p" used herein includes the hsa-miR-887-3p gene (miRBase Accession No. MIMAT0004951) shown in SEQ ID NO: 25, a homolog or an ortholog thereof of a different organism species, and the like. The gene can be obtained by a method described in Berezikov E et al., 2006, Genome Res, Vol. 16, p. 1289-1298. Also, "hsa-mir-887" (miRBase Accession No. MI0005562, SEQ ID NO: 275) having a hairpin-like structure is known as a precursor of "hsa-miR-887-3p".

The term "hsa-miR-1228-5p gene" or "hsa-miR-1228-5p" used herein includes the hsa-miR-1228-5p gene (miRBase Accession No. MIMAT0005582) shown in SEQ ID NO: 26, a homolog or an ortholog thereof of a different organism species, and the like. The gene can be obtained by a method described in Berezikov E et al., 2007, Mol Cell, Vol. 28, p. 328-336. Also, "hsa-mir-1228" (miRBase Accession No. MI0006318, SEQ ID NO: 276) having a hairpin-like structure is known as a precursor of "hsa-miR-1228-5p".

The term "hsa-miR-5572 gene" or "hsa-miR-5572" used herein includes the hsa-miR-5572 gene (miRBase Accession No. MIMAT0022260) shown in SEQ ID NO: 27, a homolog or an ortholog thereof of a different organism species, and the like. The gene can be obtained by a method described in Tandon M et al., 2012, Oral Dis, Vol. 18, p. 127-131. Also, "hsa-mir-5572" (miRBase Accession No. MI0019117, SEQ ID NO: 277) having a hairpin-like structure is known as a precursor of "hsa-miR-5572".

The term "hsa-miR-6782-5p gene" or "hsa-miR-6782-5p" used herein includes the hsa-miR-6782-5p gene (miRBase Accession No. MIMAT0027464) shown in SEQ ID NO: 28, a homolog or an ortholog thereof of a different organism species, and the like. The gene can be obtained by a method described in Ladewig E et al., 2012, Genome Res, Vol. 22, p. 1634-1645. Also, "hsa-mir-6782" (miRBase Accession No. MI0022627, SEQ ID NO: 278) having a hairpin-like structure is known as a precursor of "hsa-miR-6782-5p".

The term "hsa-miR-4298 gene" or "hsa-miR-4298" used herein includes the hsa-miR-4298 gene (miRBase Accession No. MIMAT0016852) shown in SEQ ID NO: 29, a homolog or an ortholog thereof of a different organism species, and the like. The gene can be obtained by a method described in Goff LA et al., 2009, PLoS One, Vol. 4, e7192. Also, "hsa-mir-4298" (miRBase Accession No. MI0015830, SEQ ID NO: 279) having a hairpin-like structure is known as a precursor of "hsa-miR-4298".

The term "hsa-miR-6786-5p gene" or "hsa-miR-6786-5p" used herein includes the hsa-miR-6786-5p gene (miRBase Accession No. MIMAT0027472) shown in SEQ ID NO: 30, a homolog or an ortholog thereof of a different organism species, and the like. The gene can be obtained by a method described in Ladewig E et al., 2012, Genome Res, Vol. 22, p. 1634-1645. Also, "hsa-mir-6786" (miRBase Accession No. MI0022631, SEQ ID NO: 280) having a hairpin-like structure is known as a precursor of "hsa-miR-6786-5p".

The term "hsa-miR-5010-5p gene" or "hsa-miR-5010-5p" used herein includes the hsa-miR-5010-5p gene (miRBase Accession No. MIMAT0021043) shown in SEQ ID NO: 31, a homolog or an ortholog thereof of a different organism species, and the like. The gene can be obtained by a method described in Hansen TB et al., 2011, RNA Biol, Vol. 8, p. 378-383. Also, "hsa-mir-5010" (miRBase Accession No. MI0017878, SEQ ID NO: 281) having a hairpin-like structure is known as a precursor of "hsa-miR-5010-5p".

The term "hsa-miR-6087 gene" or "hsa-miR-6087" used herein includes the hsa-miR-6087 gene (miRBase Accession No. MIMAT0023712) shown in SEQ ID NO: 32, a homolog or an ortholog thereof of a different organism species, and the like. The gene can be obtained by a method described in Yoo JK et al., 2012, Stem Cells Dev, Vol. 21, p. 2049-2057. Also, "hsa-mir-6087" (miRBase Accession No. MI0020364, SEQ ID NO: 282) having a hairpin-like structure is known as a precursor of "hsa-miR-6087".

The term "hsa-miR-6765-5p gene" or "hsa-miR-6765-5p" used herein includes the hsa-miR-6765-5p gene (miRBase Accession No. MIMAT0027430) shown in SEQ ID NO: 33, a homolog or an ortholog thereof of a different organism species, and the like. The gene can be obtained by a method described in Ladewig E et al., 2012, Genome Res, Vol. 22, p. 1634-1645. Also, "hsa-mir-6765" (miRBase Accession No. MI0022610, SEQ ID NO: 283) having a hairpin-like structure is known as a precursor of "hsa-miR-6765-5p".

The term "hsa-miR-6732-5p gene" or "hsa-miR-6732-5p" used herein includes the hsa-miR-6732-5p gene (miRBase Accession No. MIMAT0027365) shown in SEQ ID NO: 34, a homolog or an ortholog thereof of a different organism species, and the like. The gene can be obtained by a method described in Ladewig E et al., 2012, Genome Res, Vol. 22, p. 1634-1645. Also, "hsa-mir-6732" (miRBase Accession No. MI0022577, SEQ ID NO: 284) having a hairpin-like structure is known as a precursor of "hsa-miR-6732-5p".

The term "hsa-miR-6787-5p gene" or "hsa-miR-6787-5p" used herein includes the hsa-miR-6787-5p gene (miRBase Accession No. MIMAT0027474) shown in SEQ ID NO: 35, a homolog or an ortholog thereof of a different organism species, and the like. The gene can be obtained by a method described in Ladewig E et al., 2012, Genome Res, Vol. 22, p. 1634-1645. Also, "hsa-mir-6787" (miRBase Accession No. MI0022632, SEQ ID NO: 285) having a hairpin-like structure is known as a precursor of "hsa-miR-6787-5p".

The term "hsa-miR-6737-5p gene" or "hsa-miR-6737-5p" used herein includes the hsa-miR-6737-5p gene (miRBase Accession No. MIMAT0027375) shown in SEQ ID NO: 36, a homolog or an ortholog thereof of a different organism species, and the like. The gene can be obtained by a method described in Ladewig E et al., 2012, Genome Res, Vol. 22, p. 1634-1645. Also, "hsa-mir-6737" (miRBase Accession No. MI0022582, SEQ ID NO: 286) having a hairpin-like structure is known as a precursor of "hsa-miR-6737-5p".

The term "hsa-miR-128-2-5p gene" or "hsa-miR-128-2-5p" used herein includes the hsa-miR-128-2-5p gene (miRBase Accession No. MIMAT0031095) shown in SEQ ID NO: 37, a homolog or an ortholog thereof of a different organism species, and the like. The gene can be obtained by a method described in Lagos-Quintana M et al., 2002, Curr Biol, Vol. 12, p. 735-739. Also, "hsa-mir-128-2" (miRBase Accession No. MI0000727, SEQ ID NO: 287) having a hairpin-like structure is known as a precursor of "hsa-miR-128-2-5p".

The term "hsa-miR-4270 gene" or "hsa-miR-4270" used herein includes the hsa-miR-4270 gene (miRBase Accession No. MIMAT0016900) shown in SEQ ID NO: 38, a homolog or an ortholog thereof of a different organism species, and the like. The gene can be obtained by a method described in Goff LA et al., 2009, PLoS One, Vol. 4, e7192. Also, "hsa-mir-4270" (miRBase Accession No. MI0015878, SEQ ID NO: 288) having a hairpin-like structure is known as a precursor of "hsa-miR-4270".

The term "hsa-miR-6861-5p gene" or "hsa-miR-6861-5p" used herein includes the hsa-miR-6861-5p gene (miRBase Accession No. MIMAT0027623) shown in SEQ ID NO: 39, a homolog or an ortholog thereof of a different organism species, and the like. The gene can be obtained by a method described in Ladewig E et al., 2012, Genome Res, Vol. 22, p. 1634-1645. Also, "hsa-mir-6861" (miRBase Accession No. MI0022708, SEQ ID NO: 289) having a hairpin-like structure is known as a precursor of "hsa-miR-6861-5p".

The term "hsa-miR-6756-5p gene" or "hsa-miR-6756-5p" used herein includes the hsa-miR-6756-5p gene (miRBase Accession No. MIMAT0027412) shown in SEQ ID NO: 40, a homolog or an ortholog thereof of a different organism species, and the like. The gene can be obtained by a method described in Ladewig E et al., 2012, Genome Res, Vol. 22, p. 1634-1645. Also, "hsa-mir-6756" (miRBase Accession No. MI0022601, SEQ ID NO: 290) having a hairpin-like structure is known as a precursor of "hsa-miR-6756-5p".

The term "hsa-miR-1229-5p gene" or "hsa-miR-1229-5p" used herein includes the hsa-miR-1229-5p gene (miRBase Accession No. MIMAT0022942) shown in SEQ ID NO: 41, a homolog or an ortholog thereof of a different organism species, and the like. The gene can be obtained by a method described in Berezikov E et al., 2007, Mol Cell, Vol. 28, p. 328-336. Also, "hsa-mir-1229" (miRBase Accession No. MI0006319, SEQ ID NO: 291) having a hairpin-like structure is known as a precursor of "hsa-miR-1229-5p".

The term "hsa-miR-6891-5p gene" or "hsa-miR-6891-5p" used herein includes the hsa-miR-6891-5p gene (miRBase Accession No. MIMAT0027682) shown in SEQ ID NO: 42, a homolog or an ortholog thereof of a different organism species, and the like. The gene can be obtained by a method described in Ladewig E et al., 2012, Genome Res, Vol. 22, p. 1634-1645. Also, "hsa-mir-6891" (miRBase Accession No. MI0022738, SEQ ID NO: 292) having a hairpin-like structure is known as a precursor of "hsa-miR-6891-5p".

The term "hsa-miR-6848-5p gene" or "hsa-miR-6848-5p" used herein includes the hsa-miR-6848-5p gene (miRBase Accession No. MIMAT0027596) shown in SEQ ID NO: 43, a homolog or an ortholog thereof of a different organism species, and the like. The gene can be obtained by a method described in Ladewig E et al., 2012, Genome Res, Vol. 22, p. 1634-1645. Also, "hsa-mir-6848" (miRBase Accession No. MI0022694, SEQ ID NO: 293) having a hairpin-like structure is known as a precursor of "hsa-miR-6848-5p".

The term "hsa-miR-1237-5p gene" or "hsa-miR-1237-5p" used herein includes the hsa-miR-1237-5p gene (miRBase Accession No. MIMAT0022946) shown in SEQ ID NO: 44, a homolog or an ortholog thereof of a different organism species, and the like. The gene can be obtained by a method described in Berezikov E et al., 2007, Mol Cell, Vol. 28, p. 328-336. Also, "hsa-mir-1237" (miRBase Accession No. MI0006327, SEQ ID NO: 294) having a hairpin-like structure is known as a precursor of "hsa-miR-1237-5p".

The term "hsa-miR-30c-1-3p gene" or "hsa-miR-30c-1-3p" used herein includes the hsa-miR-30c-1-3p gene (miRBase Accession No. MIMAT0004674) shown in SEQ ID NO: 45, a homolog or an ortholog thereof of a different organism species, and the like. The gene can be obtained by a method described in Lagos-Quintana M et al., 2002, Curr Biol, Vol. 12, p. 735-739. Also, "hsa-mir-30c-1" (miRBase Accession No. MI0000736, SEQ ID NO: 295) having a hairpin-like structure is known as a precursor of "hsa-miR-30c-1-3p".

The term "hsa-miR-1233-5p gene" or "hsa-miR-1233-5p" used herein includes the hsa-miR-1233-5p gene (miRBase Accession No. MIMAT0022943) shown in SEQ ID NO: 46, a homolog or an ortholog thereof of a different organism species, and the like. The gene can be obtained by a method described in Berezikov E et al., 2007, Mol Cell, Vol. 28, p. 328-336. Also, "hsa-mir-1233-1 and hsa-mir-1233-2" (miRBase Accession Nos. MI0006323 and MI0015973, SEQ ID NOs: 296 and 297) having a hairpin-like structure are known as a precursor of "hsa-miR-1233-5p".

The term "hsa-miR-211-3p gene" or "hsa-miR-211-3p" used herein includes the hsa-miR-211-3p gene (miRBase Accession No. MIMAT0022694) shown in SEQ ID NO: 47, a homolog or an ortholog thereof of a different organism species, and the like. The gene can be obtained by a method described in Lim LP et al., 2003, Science, Vol. 299, p. 1540. Also, "hsa-mir-211" (miRBase Accession No. MI0000287, SEQ ID NO: 298) having a hairpin-like structure is known as a precursor of "hsa-miR-211-3p".

The term "hsa-miR-4758-5p gene" or "hsa-miR-4758-5p" used herein includes the hsa-miR-4758-5p gene (miRBase Accession No. MIMAT0019903) shown in SEQ ID NO: 48, a homolog or an ortholog thereof of a different organism species, and the like. The gene can be obtained by a method described in Persson H et al., 2011, Cancer Res, Vol. 71, p. 78-86. Also, "hsa-mir-4758" (miRBase Accession No. MI0017399, SEQ ID NO: 299) having a hairpin-like structure is known as a precursor of "hsa-miR-4758-5p".

The term "hsa-miR-614 gene" or "hsa-miR-614" used herein includes the hsa-miR-614 gene (miRBase Accession No. MIMAT0003282) shown in SEQ ID NO: 49, a homolog or an ortholog thereof of a different organism species, and the like. The gene can be obtained by a method described in Cummins JM et al., 2006, Proc Natl Acad Sci U S A, VOL. 103, p. 3687-3692. Also, "hsa-mir-614" (miRBase Accession No. MI0003627, SEQ ID NO: 300) having a hairpin-like structure is known as a precursor of "hsa-miR-614".

The term "hsa-miR-6746-5p gene" or "hsa-miR-6746-5p" used herein includes the hsa-miR-6746-5p gene (miRBase Accession No. MIMAT0027392) shown in SEQ ID NO: 50, a homolog or an ortholog thereof of a different organism species, and the like. The gene can be obtained by a method described in Ladewig E et al., 2012, Genome Res, Vol. 22, p. 1634-1645. Also, "hsa-mir-6746" (miRBase Accession No. MI0022591, SEQ ID NO: 301) having a hairpin-like structure is known as a precursor of "hsa-miR-6746-5p".

The term "hsa-miR-1915-5p gene" or "hsa-miR-1915-5p" used herein includes the hsa-miR-1915-5p gene (miRBase Accession No. MIMAT0007891) shown in SEQ ID NO: 51, a homolog or an ortholog thereof of a different organism species, and the like. The gene can be obtained by a method described in Bar M et al., 2008, Stem Cells, Vol. 26, p. 2496-2505. Also, "hsa-mir-1915" (miRBase Accession No. MI0008336, SEQ ID NO: 302) having a hairpin-like structure is known as a precursor of "hsa-miR-1915-5p".

The term "hsa-miR-4688 gene" or "hsa-miR-4688" used herein includes the hsa-miR-4688 gene (miRBase Accession No. MIMAT0019777) shown in SEQ ID NO: 52, a homolog or an ortholog thereof of a different organism species, and the like. The gene can be obtained by a method described in Persson H et al., 2011, Cancer Res, Vol. 71, p. 78-86. Also, "hsa-mir-4688" (miRBase Accession No. MI0017321, SEQ ID NO: 303) having a hairpin-like structure is known as a precursor of "hsa-miR-4688".

The term "hsa-miR-3917 gene" or "hsa-miR-3917" used herein includes the hsa-miR-3917 gene (miRBase Accession No. MIMAT0018191) shown in SEQ ID NO: 53, a homolog or an ortholog thereof of a different organism species, and the like. The gene can be obtained by a method described in Creighton CJ et al., 2010, PLoS One, Vol. 5, e9637. Also, "hsa-mir-3917" (miRBase Accession No. MI0016423, SEQ ID NO: 304) having a hairpin-like structure is known as a precursor of "hsa-miR-3917".

The term "hsa-miR-5787 gene" or "hsa-miR-5787" used herein includes the hsa-miR-5787 gene (miRBase Accession No. MIMAT0023252) shown in SEQ ID NO: 54, a homolog or an ortholog thereof of a different organism species, and the like. The gene can be obtained by a method described in Yoo H et al., 2011, Biochem Biophys Res Commun, Vol. 415, p. 567-572. Also, "hsa-mir-5787" (miRBase Accession No. MI0019797, SEQ ID NO: 305) having a hairpin-like structure is known as a precursor of "hsa-miR-5787".

The term "hsa-miR-4632-5p gene" or "hsa-miR-4632-5p" used herein includes the hsa-miR-4632-5p gene (miRBase Accession No. MIMAT0022977) shown in SEQ ID NO: 55, a homolog or an ortholog thereof of a different organism species, and the like. The gene can be obtained by a method described in Persson H et al., 2011, Cancer Res, Vol. 71, p. 78-86. Also, "hsa-mir-4632" (miRBase Accession No. MI0017259, SEQ ID NO: 306) having a hairpin-like structure is known as a precursor of "hsa-miR-4632-5p".

The term "hsa-miR-6126 gene" or "hsa-miR-6126" used herein includes the hsa-miR-6126 gene (miRBase Accession No. MIMAT0024599) shown in SEQ ID NO: 56, a homolog or an ortholog thereof of a different organism species, and the like. The gene can be obtained by a method described in Smith JL et al., 2012, J Virol, Vol. 86, p. 5278-5287. Also, "hsa-mir-6126" (miRBase Accession No. MI0021260, SEQ ID NO: 307) having a hairpin-like structure is known as a precursor of "hsa-miR-6126".

The term "hsa-miR-135a-3p gene" or "hsa-miR-135a-3p" used herein includes the hsa-miR-135a-3p gene (miRBase Accession No. MIMAT0004595) shown in SEQ ID NO: 57, a homolog or an ortholog thereof of a different organism species, and the like. The gene can be obtained by a method described in Lagos-Quintana M et al., 2002, Curr Biol, Vol. 12, p. 735-739. Also, "hsa-mir-135a-1" (miRBase Accession No. MI0000452, SEQ ID NO: 308) having a hairpin-like structure is known as a precursor of "hsa-miR-135a-3p".

The term "hsa-miR-8063 gene" or "hsa-miR-8063" used herein includes the hsa-miR-8063 gene (miRBase Accession No. MIMAT0030990) shown in SEQ ID NO: 58, a homolog or an ortholog thereof of a different organism species, and the like. The gene can be obtained by a method described in Wang HJ et al., 2013, Shock, Vol. 39, p. 480-487. Also, "hsa-mir-8063" (miRBase Accession No. MI0025899, SEQ ID NO: 309) having a hairpin-like structure is known as a precursor of "hsa-miR-8063".

The term "hsa-miR-5698 gene" or "hsa-miR-5698" used herein includes the hsa-miR-5698 gene (miRBase Accession No. MIMAT0022491) shown in SEQ ID NO: 59, a homolog or an ortholog thereof of a different organism species, and the like. The gene can be obtained by a method described in Watahiki A et al., 2011, PLoS One, Vol. 6, e24950. Also, "hsa-mir-5698" (miRBase Accession No. MI0019305, SEQ ID NO: 310) having a hairpin-like structure is known as a precursor of "hsa-miR-5698".

The term "hsa-miR-6089 gene" or "hsa-miR-6089" used herein includes the hsa-miR-6089 gene (miRBase Accession No. MIMAT0023714) shown in SEQ ID NO: 60, a homolog or an ortholog thereof of a different organism species, and the like. The gene can be obtained by a method described in Yoo JK et al., 2012, Stem Cells Dev, Vol. 21, p. 2049-2057. Also, "hsa-mir-6089-1 and hsa-mir-6089-2" (miRBase Accession Nos. MI0020366 and MI0023563, SEQ ID NOs: 311 and 312) having a hairpin-like structure are known as a precursor of "hsa-miR-6089".

The term "hsa-miR-498 gene" or "hsa-miR-498" used herein includes the hsa-miR-498 gene (miRBase Accession No. MIMAT0002824) shown in SEQ ID NO: 61, a homolog or an ortholog thereof of a different organism species, and the like. The gene can be obtained by a method described in Bentwich I et al., 2005, Nat Genet, Vol. 37, p. 766-770. Also, "hsa-mir-498" (miRBase Accession No. MI0003142, SEQ ID NO: 313) having a hairpin-like structure is known as a precursor of "hsa-miR-498".

The term "hsa-miR-296-3p gene" or "hsa-miR-296-3p" used herein includes the hsa-miR-296-3p gene (miRBase Accession No. MIMAT0004679) shown in SEQ ID NO: 62, a homolog or an ortholog thereof of a different organism species, and the like. The gene can be obtained by a method described in Houbaviy HB et al., 2003, Dev Cell, Vol. 5, p. 351-358. Also, "hsa-mir-296" (miRBase Accession No. MI0000747, SEQ ID NO: 314) having a hairpin-like structure is known as a precursor of "hsa-miR-296-3p".

The term "hsa-miR-4419b gene" or "hsa-miR-4419b" used herein includes the hsa-miR-4419b gene (miRBase Accession No. MIMAT0019034) shown in SEQ ID NO: 63, a homolog or an ortholog thereof of a different organism species, and the like. The gene can be obtained by a method described in Jima DD et al., 2010, Blood, Vol. 116, e118-e127. Also, "hsa-mir-4419b" (miRBase Accession No. MI0016861, SEQ ID NO: 315) having a hairpin-like structure is known as a precursor of "hsa-miR-4419b".

The term "hsa-miR-6802-5p gene" or "hsa-miR-6802-5p" used herein includes the hsa-miR-6802-5p gene (miRBase Accession No. MIMAT0027504) shown in SEQ ID NO: 64, a homolog or an ortholog thereof of a different organism species, and the like. The gene can be obtained by a method described in Ladewig E et al., 2012, Genome Res, Vol. 22, p. 1634-1645. Also, "hsa-mir-6802" (miRBase Accession No. MI0022647, SEQ ID NO: 316) having a hairpin-like structure is known as a precursor of "hsa-miR-6802-5p".

The term "hsa-miR-6829-5p gene" or "hsa-miR-6829-5p" used herein includes the hsa-miR-6829-5p gene (miRBase Accession No. MIMAT0027558) shown in SEQ ID NO: 65, a homolog or an ortholog thereof of a different organism species, and the like. The gene can be obtained by a method described in Ladewig E et al., 2012, Genome Res, Vol. 22, p. 1634-1645. Also, "hsa-mir-6829" (miRBase Accession No. MI0022674, SEQ ID NO: 317) having a hairpin-like structure is known as a precursor of "hsa-miR-6829-5p".

The term "hsa-miR-6803-5p gene" or "hsa-miR-6803-5p" used herein includes the hsa-miR-6803-5p gene (miRBase Accession No. MIMAT0027506) shown in SEQ ID NO: 66, a homolog or an ortholog thereof of a different organism species, and the like. The gene can be obtained by a method described in Ladewig E et al., 2012, Genome Res, Vol. 22, p. 1634-1645. Also, "hsa-mir-6803" (miRBase Accession No. MI0022648, SEQ ID NO: 318) having a hairpin-like structure is known as a precursor of "hsa-miR-6803-5p".

The term "hsa-miR-1199-5p gene" or "hsa-miR-1199-5p" used herein includes the hsa-miR-1199-5p gene (miRBase Accession No. MIMAT0031119) shown in SEQ ID NO: 67, a homolog or an ortholog thereof of a different organism species, and the like. The gene can be obtained by a method described in Salvi A et al., 2013, Int J Oncol, Vol. 42, p. 391-402. Also, "hsa-mir-1199" (miRBase Accession No. MI0020340, SEQ ID NO: 319) having a hairpin-like structure is known as a precursor of "hsa-miR-1199-5p".

The term "hsa-miR-6840-3p gene" or "hsa-miR-6840-3p" used herein includes the hsa-miR-6840-3p gene (miRBase Accession No. MIMAT0027583) shown in SEQ ID NO: 68, a homolog or an ortholog thereof of a different organism species, and the like. The gene can be obtained by a method described in Ladewig E et al., 2012, Genome Res, Vol. 22, p. 1634-1645. Also, "hsa-mir-6840" (miRBase Accession No. MI0022686, SEQ ID NO: 320) having a hairpin-like structure is known as a precursor of "hsa-miR-6840-3p".

The term "hsa-miR-6752-5p gene" or "hsa-miR-6752-5p" used herein includes the hsa-miR-6752-5p gene (miRBase Accession No. MIMAT0027404) shown in SEQ ID NO: 69, a homolog or an ortholog thereof of a different organism species, and the like. The gene can be obtained by a method described in Ladewig E et al., 2012, Genome Res, Vol. 22, p. 1634-1645. Also, "hsa-mir-6752" (miRBase Accession No. MI0022597, SEQ ID NO: 321) having a hairpin-like structure is known as a precursor of "hsa-miR-6752-5p".

The term "hsa-miR-6798-5p gene" or "hsa-miR-6798-5p" used herein includes the hsa-miR-6798-5p gene (miRBase Accession No. MIMAT0027496) shown in SEQ ID NO: 70, a homolog or an ortholog thereof of a different organism species, and the like. The gene can be obtained by a method described in Ladewig E et al., 2012, Genome Res, Vol. 22, p. 1634-1645. Also, "hsa-mir-6798" (miRBase Accession No. MI0022643, SEQ ID NO: 322) having a hairpin-like structure is known as a precursor of "hsa-miR-6798-5p".

The term "hsa-miR-6131 gene" or "hsa-miR-6131" used herein includes the hsa-miR-6131 gene (miRBase Accession No. MIMAT0024615) shown in SEQ ID NO: 71, a homolog or an ortholog thereof of a different organism species, and the like. The gene can be obtained by a method described in Dannemann M et al., 2012, Genome Biol Evol, Vol. 4, p. 552-564. Also, "hsa-mir-6131" (miRBase Accession No. MI0021276, SEQ ID NO: 323) having a hairpin-like structure is known as a precursor of "hsa-miR-6131".

The term "hsa-miR-4667-5p gene" or "hsa-miR-4667-5p" used herein includes the hsa-miR-4667-5p gene (miRBase Accession No. MIMAT0019743) shown in SEQ ID NO: 72, a homolog or an ortholog thereof of a different organism species, and the like. The gene can be obtained by a method described in Persson H et al., 2011, Cancer Res, Vol. 71, p. 78-86. Also, "hsa-mir-4667" (miRBase Accession No. MI0017297, SEQ ID NO: 324) having a hairpin-like structure is known as a precursor of "hsa-miR-4667-5p".

The term "hsa-miR-6510-5p gene" or "hsa-miR-6510-5p" used herein includes the hsa-miR-6510-5p gene (miRBase Accession No. MIMAT0025476) shown in SEQ ID NO: 73, a homolog or an ortholog thereof of a different organism species, and the like. The gene can be obtained by a method described in Joyce CE et al., 2011, Hum Mol Genet, Vol. 20, p. 4025-4040. Also, "hsa-mir-6510" (miRBase Accession No. MI0022222, SEQ ID NO: 325) having a hairpin-like structure is known as a precursor of "hsa-miR-6510-5p".

The term "hsa-miR-4690-5p gene" or "hsa-miR-4690-5p" used herein includes the hsa-miR-4690-5p gene (miRBase Accession No. MIMAT0019779) shown in SEQ ID NO: 74, a homolog or an ortholog thereof of a different organism species, and the like. The gene can be obtained by a method described in Persson H et al., 2011, Cancer Res, Vol. 71, p. 78-86. Also, "hsa-mir-4690" (miRBase Accession No. MI0017323, SEQ ID NO: 326) having a hairpin-like structure is known as a precursor of "hsa-miR-4690-5p".

The term "hsa-miR-920 gene" or "hsa-miR-920" used herein includes the hsa-miR-920 gene (miRBase Accession No. MIMAT0004970) shown in SEQ ID NO: 75, a homolog or an ortholog thereof of a different organism species, and the like. The gene can be obtained by a method described in Novotny GW et al., 2007, Int J Androl, Vol. 30, p. 316-326. Also, "hsa-mir-920" (miRBase Accession No. MI0005712, SEQ ID NO: 327) having a hairpin-like structure is known as a precursor of "hsa-miR-920".

The term "hsa-miR-23b-3p gene" or "hsa-miR-23b-3p" used herein includes the hsa-miR-23b-3p gene (miRBase Accession No. MIMAT0000418) shown in SEQ ID NO: 76, a homolog or an ortholog thereof of a different organism species, and the like. The gene can be obtained by a method described in Lagos-Quintana M et al., 2002, Curr Biol, Vol. 12, p. 735-739. Also, "hsa-mir-23b" (miRBase Accession No. MI0000439, SEQ ID NO: 328) having a hairpin-like structure is known as a precursor of "hsa-miR-23b-3p".

The term "hsa-miR-4448 gene" or "hsa-miR-4448" used herein includes the hsa-miR-4448 gene (miRBase Accession No. MIMAT0018967) shown in SEQ ID NO: 77, a homolog or an ortholog thereof of a different organism species, and the like. The gene can be obtained by a method described in Jima DD et al., 2010, Blood, Vol. 116, e118-e127. Also, "hsa-mir-4448" (miRBase Accession No. MI0016791, SEQ ID NO: 329) having a hairpin-like structure is known as a precursor of "hsa-miR-4448".

The term "hsa-miR-2110 gene" or "hsa-miR-2110" used herein includes the hsa-miR-2110 gene (miRBase Accession No. MIMAT0010133) shown in SEQ ID NO: 78, a homolog or an ortholog thereof of a different organism species, and the like. The gene can be obtained by a method described in Zhu JY et al., 2009, J Virol, Vol. 83, p. 3333-3341. Also, "hsa-mir-2110" (miRBase Accession No. MI0010629, SEQ ID NO: 330) having a hairpin-like structure is known as a precursor of "hsa-miR-2110".

The term "hsa-miR-4706 gene" or "hsa-miR-4706" used herein includes the hsa-miR-4706 gene (miRBase Accession No. MIMAT0019806) shown in SEQ ID NO: 79, a homolog or an ortholog thereof of a different organism species, and the like. The gene can be obtained by a method described in Persson H et al., 2011, Cancer Res, Vol. 71, p. 78-86. Also, "hsa-mir-4706" (miRBase Accession No. MI0017339, SEQ ID NO: 331) having a hairpin-like structure is known as a precursor of "hsa-miR-4706".

The term "hsa-miR-7845-5p gene" or "hsa-miR-7845-5p" used herein includes the hsa-miR-7845-5p gene (miRBase Accession No. MIMAT0030420) shown in SEQ ID NO: 80, a homolog or an ortholog thereof of a different organism species, and the like. The gene can be obtained by a method described in Ple H et al., 2012, PLoS One, Vol. 7, e50746. Also, "hsa-mir-7845" (miRBase Accession No. MI0025515, SEQ ID NO: 332) having a hairpin-like structure is known as a precursor of "hsa-miR-7845-5p".

The term "hsa-miR-6808-5p gene" or "hsa-miR-6808-5p" used herein includes the hsa-miR-6808-5p gene (miRBase Accession No. MIMAT0027516) shown in SEQ ID NO: 81, a homolog or an ortholog thereof of a different organism species, and the like. The gene can be obtained by a method described in Ladewig E et al., 2012, Genome Res, Vol. 22, p. 1634-1645. Also, "hsa-mir-6808" (miRBase Accession No. MI0022653, SEQ ID NO: 333) having a hairpin-like structure is known as a precursor of "hsa-miR-6808-5p".

The term "hsa-miR-4447 gene" or "hsa-miR-4447" used herein includes the hsa-miR-4447 gene (miRBase Accession No. MIMAT0018966) shown in SEQ ID NO: 82, a homolog or an ortholog thereof of a different organism species, and the like. The gene can be obtained by a method described in Jima DD et al., 2010, Blood, Vol. 116, e118-e127. Also, "hsa-mir-4447" (miRBase Accession No. MI0016790, SEQ ID NO: 334) having a hairpin-like structure is known as a precursor of "hsa-miR-4447".

The term "hsa-miR-6869-5p gene" or "hsa-miR-6869-5p" used herein includes the hsa-miR-6869-5p gene (miRBase Accession No. MIMAT0027638) shown in SEQ ID NO: 83, a homolog or an ortholog thereof of a different organism species, and the like. The gene can be obtained by a method described in Ladewig E et al., 2012, Genome Res, Vol. 22, p. 1634-1645. Also, "hsa-mir-6869" (miRBase Accession No. MI0022716, SEQ ID NO: 335) having a hairpin-like structure is known as a precursor of "hsa-miR-6869-5p".

The term "hsa-miR-1908-5p gene" or "hsa-miR-1908-5p" used herein includes the hsa-miR-1908-5p gene (miRBase Accession No. MIMAT0007881) shown in SEQ ID NO: 84, a homolog or an ortholog thereof of a different organism species, and the like. The gene can be obtained by a method described in Bar M et al., 2008, Stem Cells, Vol. 26, p. 2496-2505. Also, "hsa-mir-1908" (miRBase Accession No. MI0008329, SEQ ID NO: 336) having a hairpin-like structure is known as a precursor of "hsa-miR-1908-5p".

The term "hsa-miR-6729-5p gene" or "hsa-miR-6729-5p" used herein includes the hsa-miR-6729-5p gene (miRBase Accession No. MIMAT0027359) shown in SEQ ID NO: 85, a homolog or an ortholog thereof of a different organism species, and the like. The gene can be obtained by a method described in Ladewig E et al., 2012, Genome Res, Vol. 22, p. 1634-1645. Also, "hsa-mir-6729" (miRBase Accession No. MI0022574, SEQ ID NO: 337) having a hairpin-like structure is known as a precursor of "hsa-miR-6729-5p".

The term "hsa-miR-5195-3p gene" or "hsa-miR-5195-3p" used herein includes the hsa-miR-5195-3p gene (miRBase Accession No. MIMAT0021127) shown in SEQ ID NO: 86, a homolog or an ortholog thereof of a different organism species, and the like. The gene can be obtained by a method described in Schotte D et al., 2011, Leukemia, Vol. 25, p. 1389-1399. Also, "hsa-mir-5195" (miRBase Accession No. MI0018174, SEQ ID NO: 338) having a hairpin-like structure is known as a precursor of "hsa-miR-5195-3p".

The term "hsa-miR-638 gene" or "hsa-miR-638" used herein includes the hsa-miR-638 gene (miRBase Accession No. MIMAT0003308) shown in SEQ ID NO: 87, a homolog or an ortholog thereof of a different organism species, and the like. The gene can be obtained by a method described in Cummins JM et al., 2006, Proc Natl Acad Sci U S A, Vol. 103, p. 3687-3692. Also, "hsa-mir-638" (miRBase Accession No. MI0003653, SEQ ID NO: 339) having a hairpin-like structure is known as a precursor of "hsa-miR-638".

The term "hsa-miR-6125 gene" or "hsa-miR-6125" used herein includes the hsa-miR-6125 gene (miRBase Accession No. MIMAT0024598) shown in SEQ ID NO: 88, a homolog or an ortholog thereof of a different organism species, and the like. The gene can be obtained by a method described in Smith JL et al., 2012, J Virol, Vol. 86, p. 5278-5287. Also, "hsa-mir-6125" (miRBase Accession No. MI0021259, SEQ ID NO: 340) having a hairpin-like structure is known as a precursor of "hsa-miR-6125".

The term "hsa-miR-3178 gene" or "hsa-miR-3178" used herein includes the hsa-miR-3178 gene (miRBase Accession No. MIMAT0015055) shown in SEQ ID NO: 89, a homolog or an ortholog thereof of a different organism species, and the like. The gene can be obtained by a method described in Stark MS et al., 2010, PLoS One, Vol. 5, e9685. Also, "hsa-mir-3178" (miRBase Accession No. MI0014212, SEQ ID NO: 341) having a hairpin-like structure is known as a precursor of "hsa-miR-3178".

The term "hsa-miR-3196 gene" or "hsa-miR-3196" used herein includes the hsa-miR-3196 gene (miRBase Accession No. MIMAT0015080) shown in SEQ ID NO: 90, a homolog or an ortholog thereof of a different organism species, and the like. The gene can be obtained by a method described in Stark MS et al., 2010, PLoS One, Vol. 5, e9685. Also, "hsa-mir-3196" (miRBase Accession No. MI0014241, SEQ ID NO: 342) having a hairpin-like structure is known as a precursor of "hsa-miR-3196".

The term "hsa-miR-8069 gene" or "hsa-miR-8069" used herein includes the hsa-miR-8069 gene (miRBase Accession No. MIMAT0030996) shown in SEQ ID NO: 91, a homolog or an ortholog thereof of a different organism species, and the like. The gene can be obtained by a method described in Wang HJ et al., 2013, Shock, Vol. 39, p. 480-487. Also, "hsa-mir-8069-1 and hsa-mir-8069-2" (miRBase Accession Nos. MI0025905 and MI0031519, SEQ ID NOs: 343 and 344) having a hairpin-like structure are known as a precursor of "hsa-miR-8069".

The term "hsa-miR-4723-5p gene" or "hsa-miR-4723-5p" used herein includes the hsa-miR-4723-5p gene (miRBase Accession No. MIMAT0019838) shown in SEQ ID NO: 92, a homolog or an ortholog thereof of a different organism species, and the like. The gene can be obtained by a method described in Persson H et al., 2011, Cancer Res, Vol. 71, p. 78-86. Also, "hsa-mir-4723" (miRBase Accession No. MI0017359, SEQ ID NO: 345) having a hairpin-like structure is known as a precursor of "hsa-miR-4723-5p".

The term "hsa-miR-4746-3p gene" or "hsa-miR-4746-3p" used herein includes the hsa-miR-4746-3p gene (miRBase Accession No. MIMAT0019881) shown in SEQ ID NO: 93, a homolog or an ortholog thereof of a different organism species, and the like. The gene can be obtained by a method described in Persson H et al., 2011, Cancer Res, Vol. 71, p. 78-86. Also, "hsa-mir-4746" (miRBase Accession No. MI0017385, SEQ ID NO: 346) having a hairpin-like structure is known as a precursor of "hsa-miR-4746-3p".

The term "hsa-miR-4689 gene" or "hsa-miR-4689" used herein includes the hsa-miR-4689 gene (miRBase Accession No. MIMAT0019778) shown in SEQ ID NO: 94, a homolog or an ortholog thereof of a different organism species, and the like. The gene can be obtained by a method described in Persson H et al., 2011, Cancer Res, Vol. 71, p. 78-86. Also, "hsa-mir-4689" (miRBase Accession No. MI0017322, SEQ ID NO: 347) having a hairpin-like structure is known as a precursor of "hsa-miR-4689".

The term "hsa-miR-6816-5p gene" or "hsa-miR-6816-5p" used herein includes the hsa-miR-6816-5p gene (miRBase Accession No. MIMAT0027532) shown in SEQ ID NO: 95, a homolog or an ortholog thereof of a different organism species, and the like. The gene can be obtained by a method described in Ladewig E et al., 2012, Genome Res, Vol. 22, p. 1634-1645. Also, "hsa-mir-6816" (miRBase Accession No. MI0022661, SEQ ID NO: 348) having a hairpin-like structure is known as a precursor of "hsa-miR-6816-5p".

The term "hsa-miR-6757-5p gene" or "hsa-miR-6757-5p" used herein includes the hsa-miR-6757-5p gene (miRBase Accession No. MIMAT0027414) shown in SEQ ID NO: 96, a homolog or an ortholog thereof of a different organism species, and the like. The gene can be obtained by a method described in Ladewig E et al., 2012, Genome Res, Vol. 22, p. 1634-1645. Also, "hsa-mir-6757" (miRBase Accession No. MI0022602, SEQ ID NO: 349) having a hairpin-like structure is known as a precursor of "hsa-miR-6757-5p".

The term "hsa-miR-7109-5p gene" or "hsa-miR-7109-5p" used herein includes the hsa-miR-7109-5p gene (miRBase Accession No. MIMAT0028115) shown in SEQ ID NO: 97, a homolog or an ortholog thereof of a different organism species, and the like. The gene can be obtained by a method described in Ladewig E et al., 2012, Genome Res, Vol. 22, p. 1634-1645. Also, "hsa-mir-7109" (miRBase Accession No. MI0022960, SEQ ID NO: 350) having a hairpin-like structure is known as a precursor of "hsa-miR-7109-5p".

The term "hsa-miR-6724-5p gene" or "hsa-miR-6724-5p" used herein includes the hsa-miR-6724-5p gene (miRBase Accession No. MIMAT0025856) shown in SEQ ID NO: 98, a homolog or an ortholog thereof of a different organism species, and the like. The gene can be obtained by a method described in Li Y et al., 2012, Gene, Vol. 497, p. 330-335. Also, "hsa-mir-6724-1, hsa-mir-6724-2, hsa-mir-6724-3 and hsa-mir-6724-4" (miRBase Accession Nos. MI0022559, MI0031516, MI0031517 and MI0031518, SEQ ID NOs: 351, 352, 353 and 354) having a hairpin-like structure are known as a precursor of "hsa-miR-6724-5p".

The term "hsa-miR-1225-3p gene" or "hsa-miR-1225-3p" used herein includes the hsa-miR-1225-3p gene (miRBase Accession No. MIMAT0005573) shown in SEQ ID NO: 99, a homolog or an ortholog thereof of a different organism species, and the like. The gene can be obtained by a method described in Berezikov E et al., 2007, Mol Cell, Vol. 28, p. 328-336. Also, "hsa-mir-1225" (miRBase Accession No. MI0006311, SEQ ID NO: 355) having a hairpin-like structure is known as a precursor of "hsa-miR-1225-3p".

The term "hsa-miR-6875-5p gene" or "hsa-miR-6875-5p" used herein includes the hsa-miR-6875-5p gene (miRBase Accession No. MIMAT0027650) shown in SEQ ID NO: 100, a homolog or an ortholog thereof of a different organism species, and the like. The gene can be obtained by a method described in Ladewig E et al., 2012, Genome Res, Vol. 22, p. 1634-1645. Also, "hsa-mir-6875" (miRBase Accession No. MI0022722, SEQ ID NO: 356) having a hairpin-like structure is known as a precursor of "hsa-miR-6875-5p".

The term "hsa-miR-7108-5p gene" or "hsa-miR-7108-5p" used herein includes the hsa-miR-7108-5p gene (miRBase Accession No. MIMAT0028113) shown in SEQ ID NO: 101, a homolog or an ortholog thereof of a different organism species, and the like. The gene can be obtained by a method described in Ladewig E et al., 2012, Genome Res, Vol. 22, p. 1634-1645. Also, "hsa-mir-7108" (miRBase Accession No. MI0022959, SEQ ID NO: 357) having a hairpin-like structure is known as a precursor of "hsa-miR-7108-5p".

The term "hsa-miR-4508 gene" or "hsa-miR-4508" used herein includes the hsa-miR-4508 gene (miRBase Accession No. MIMAT0019045) shown in SEQ ID NO: 102, a homolog or an ortholog thereof of a different organism species, and the like. The gene can be obtained by a method described in Jima DD et al., 2010, Blood, Vol. 116, e118-e127. Also, "hsa-mir-4508" (miRBase Accession No. MI0016872, SEQ ID NO: 358) having a hairpin-like structure is known as a precursor of "hsa-miR-4508".

The term "hsa-miR-6085 gene" or "hsa-miR-6085" used herein includes the hsa-miR-6085 gene (miRBase Accession No. MIMAT0023710) shown in SEQ ID NO: 103, a homolog or an ortholog thereof of a different organism species, and the like. The gene can be obtained by a method described in Voellenkle C et al., 2012, RNA, Vol. 18, p. 472-484. Also, "hsa-mir-6085" (miRBase Accession No. MI0020362, SEQ ID NO: 359) having a hairpin-like structure is known as a precursor of "hsa-miR-6085".

The term "hsa-miR-6779-5p gene" or "hsa-miR-6779-5p" used herein includes the hsa-miR-6779-5p gene (miRBase Accession No. MIMAT0027458) shown in SEQ ID NO: 104, a homolog or an ortholog thereof of a different organism species, and the like. The gene can be obtained by a method described in Ladewig E et al., 2012, Genome Res, Vol. 22, p. 1634-1645. Also, "hsa-mir-6779" (miRBase Accession No. MI0022624, SEQ ID NO: 360) having a hairpin-like structure is known as a precursor of "hsa-miR-6779-5p".

The term "hsa-miR-642a-3p gene" or "hsa-miR-642a-3p" used herein includes the hsa-miR-642a-3p gene (miRBase Accession No. MIMAT0020924) shown in SEQ ID NO: 105, a homolog or an ortholog thereof of a different organism species, and the like. The gene can be obtained by a method described in Cummins JM et al., 2006, Proc Natl Acad Sci U S A, Vol. 103, p. 3687-3692. Also, "hsa-mir-642a" (miRBase Accession No. MI0003657, SEQ ID NO: 361) having a hairpin-like structure is known as a precursor of "hsa-miR-642a-3p".

The term "hsa-miR-4695-5p gene" or "hsa-miR-4695-5p" used herein includes the hsa-miR-4695-5p gene (miRBase Accession No. MIMAT0019788) shown in SEQ ID NO: 106, a homolog or an ortholog thereof of a different organism species, and the like. The gene can be obtained by a method described in Persson H et al., 2011, Cancer Res, Vol. 71, p. 78-86. Also, "hsa-mir-4695" (miRBase Accession No. MI0017328, SEQ ID NO: 362) having a hairpin-like structure is known as a precursor of "hsa-miR-4695-5p".

The term "hsa-miR-7847-3p gene" or "hsa-miR-7847-3p" used herein includes the hsa-miR-7847-3p gene (miRBase Accession No. MIMAT0030422) shown in SEQ ID NO: 107, a homolog or an ortholog thereof of a different organism species, and the like. The gene can be obtained by a method described in Ple H et al., 2012, PLoS One, Vol. 7, e50746. Also, "hsa-mir-7847" (miRBase Accession No. MI0025517, SEQ ID NO: 363) having a hairpin-like structure is known as a precursor of "hsa-miR-7847-3p".

The term "hsa-miR-3197 gene" or "hsa-miR-3197" used herein includes the hsa-miR-3197 gene (miRBase Accession No. MIMAT0015082) shown in SEQ ID NO: 108, a homolog or an ortholog thereof of a different organism species, and the like. The gene can be obtained by a method described in Stark MS et al., 2010, PLoS One, Vol. 5, e9685. Also, "hsa-mir-3197" (miRBase Accession No. MI0014245, SEQ ID NO: 364) having a hairpin-like structure is known as a precursor of "hsa-miR-3197".

The term "hsa-miR-6769b-5p gene" or "hsa-miR-6769b-5p" used herein includes the hsa-miR-6769b-5p gene (miRBase Accession No. MIMAT0027620) shown in SEQ ID NO: 109, a homolog or an ortholog thereof of a different organism species, and the like. The gene can be obtained by a method described in Ladewig E et al., 2012, Genome Res, Vol. 22, p. 1634-1645. Also, "hsa-mir-6769b" (miRBase Accession No. MI0022706, SEQ ID NO: 365) having a hairpin-like structure is known as a precursor of "hsa-miR-6769b-5p".

The term "hsa-miR-7641 gene" or "hsa-miR-7641" used herein includes the hsa-miR-7641 gene (miRBase Accession No. MIMAT0029782) shown in SEQ ID NO: 110, a homolog or an ortholog thereof of a different organism species, and the like. The gene can be obtained by a method described in Yoo JK et al., 2013, Arch Pharm Res, Vol. 36, p. 353-358. Also, "hsa-mir-7641-1 and hsa-mir-7641-2" (miRBase Accession Nos. MI0024975 and MI0024976, SEQ ID NOs: 366 and 367) having a hairpin-like structure are known as a precursor of "hsa-miR-7641".

The term "hsa-miR-187-5p gene" or "hsa-miR-187-5p" used herein includes the hsa-miR-187-5p gene (miRBase Accession No. MIMAT0004561) shown in SEQ ID NO: 111, a homolog or an ortholog thereof of a different organism species, and the like. The gene can be obtained by a method described in Lim LP et al., 2003, Science, Vol. 299, p. 1540. Also, "hsa-mir-187" (miRBase Accession No. MI0000274, SEQ ID NO: 368) having a hairpin-like structure is known as a precursor of "hsa-miR-187-5p".

The term "hsa-miR-3185 gene" or "hsa-miR-3185" used herein includes the hsa-miR-3185 gene (miRBase Accession No. MIMAT0015065) shown in SEQ ID NO: 112, a homolog or an ortholog thereof of a different organism species, and the like. The gene can be obtained by a method described in Stark MS et al., 2010, PLoS One, Vol. 5, e9685. Also, "hsa-mir-3185" (miRBase Accession No. MI0014227, SEQ ID NO: 369) having a hairpin-like structure is known as a precursor of "hsa-miR-3185".

The term "hsa-miR-2861 gene" or "hsa-miR-2861" used herein includes the hsa-miR-2861 gene (miRBase Accession No. MIMAT0013802) shown in SEQ ID NO: 113, a homolog or an ortholog thereof of a different organism species, and the like. The gene can be obtained by a method described in Li H et al., 2009, J Clin Invest, Vol. 119, p. 3666-3677. Also, "hsa-mir-2861" (miRBase Accession No. MI0013006, SEQ ID NO: 370) having a hairpin-like structure is known as a precursor of "hsa-miR-2861".

The term "hsa-miR-3940-5p gene" or "hsa-miR-3940-5p" used herein includes the hsa-miR-3940-5p gene (miRBase Accession No. MIMAT0019229) shown in SEQ ID NO: 114, a homolog or an ortholog thereof of a different organism species, and the like. The gene can be obtained by a method described in Liao JY et al., 2010, PLoS One, Vol. 5, e10563. Also, "hsa-mir-3940" (miRBase Accession No. MI0016597, SEQ ID NO: 371) having a hairpin-like structure is known as a precursor of "hsa-miR-3940-5p".

The term "hsa-miR-1203 gene" or "hsa-miR-1203" used herein includes the hsa-miR-1203 gene (miRBase Accession No. MIMAT0005866) shown in SEQ ID NO: 115, a homolog or an ortholog thereof of a different organism species, and the like. The gene can be obtained by a method described in Marton S et al., 2008, Leukemia, Vol. 22, p. 330-338. Also, "hsa-mir-1203" (miRBase Accession No. MI0006335, SEQ ID NO: 372) having a hairpin-like structure is known as a precursor of "hsa-miR-1203".

The term "hsa-miR-615-5p gene" or "hsa-miR-615-5p" used herein includes the hsa-miR-615-5p gene (miRBase Accession No. MIMAT0004804) shown in SEQ ID NO: 116, a homolog or an ortholog thereof of a different organism species, and the like. The gene can be obtained by a method described in Cummins JM et al., 2006, Proc Natl Acad Sci U S A, Vol. 103, p. 3687-3692. Also, "hsa-mir-615" (miRBase Accession No. MI0003628, SEQ ID NO: 373) having a hairpin-like structure is known as a precursor of "hsa-miR-615-5p".

The term "hsa-miR-4787-5p gene" or "hsa-miR-4787-5p" used herein includes the hsa-miR-4787-5p gene (miRBase Accession No. MIMAT0019956) shown in SEQ ID NO: 117, a homolog or an ortholog thereof of a different organism species, and the like. The gene can be obtained by a method described in Persson H et al., 2011, Cancer Res, Vol. 71, p. 78-86. Also, "hsa-mir-4787" (miRBase Accession No. MI0017434, SEQ ID NO: 374) having a hairpin-like structure is known as a precursor of "hsa-miR-4787-5p".

The term "hsa-miR-1343-3p gene" or "hsa-miR-1343-3p" used herein includes the hsa-miR-1343-3p gene (miRBase Accession No. MIMAT0019776) shown in SEQ ID NO: 118, a homolog or an ortholog thereof of a different organism species, and the like. The gene can be obtained by a method described in Persson H et al., 2011, Cancer Res, Vol. 71, p. 78-86. Also, "hsa-mir-1343" (miRBase Accession No. MI0017320, SEQ ID NO: 375) having a hairpin-like structure is known as a precursor of "hsa-miR-1343-3p".

The term "hsa-miR-6813-5p gene" or "hsa-miR-6813-5p" used herein includes the hsa-miR-6813-5p gene (miRBase Accession No. MIMAT0027526) shown in SEQ ID NO: 119, a homolog or an ortholog thereof of a different organism species, and the like. The gene can be obtained by a method described in Ladewig E et al., 2012, Genome Res, Vol. 22, p. 1634-1645. Also, "hsa-mir-6813" (miRBase Accession No. MI0022658, SEQ ID NO: 376) having a hairpin-like structure is known as a precursor of "hsa-miR-6813-5p".

The term "hsa-miR-1225-5p gene" or "hsa-miR-1225-5p" used herein includes the hsa-miR-1225-5p gene (miRBase Accession No. MIMAT0005572) shown in SEQ ID NO: 120, a homolog or an ortholog thereof of a different organism species, and the like. The gene can be obtained by a method described in Berezikov E et al., 2007, Mol Cell, Vol. 28, p. 328-336. Also, "hsa-mir-1225" (miRBase Accession No. MI0006311, SEQ ID NO: 377) having a hairpin-like structure is known as a precursor of "hsa-miR-1225-5p".

The term "hsa-miR-602 gene" or "hsa-miR-602" used herein includes the hsa-miR-602 gene (miRBase Accession No. MIMAT0003270) shown in SEQ ID NO: 121, a homolog or an ortholog thereof of a different organism species, and the like. The gene can be obtained by a method described in Cummins JM et al., 2006, Proc Natl Acad Sci U S A, Vol. 103, p. 3687-3692. Also, "hsa-mir-602" (miRBase Accession No. MI0003615, SEQ ID NO: 378) having a hairpin-like structure is known as a precursor of "hsa-miR-602".

The term "hsa-miR-4488 gene" or "hsa-miR-4488" used herein includes the hsa-miR-4488 gene (miRBase Accession No. MIMAT0019022) shown in SEQ ID NO: 122, a homolog or an ortholog thereof of a different organism species, and the like. The gene can be obtained by a method described in Jima DD et al., 2010, Blood, Vol. 116, e118-e127. Also, "hsa-mir-4488" (miRBase Accession No. MI0016849, SEQ ID NO: 379) having a hairpin-like structure is known as a precursor of "hsa-miR-4488".

The term "hsa-miR-125a-3p gene" or "hsa-miR-125a-3p" used herein includes the hsa-miR-125a-3p gene (miRBase Accession No. MIMAT0004602) shown in SEQ ID NO: 123, a homolog or an ortholog thereof of a different organism species, and the like. The gene can be obtained by a method described in Lagos-Quintana M et al., 2002, Curr Biol, Vol. 12, p. 735-739. Also, "hsa-mir-125a" (miRBase Accession No. MI0000469, SEQ ID NO: 380) having a hairpin-like structure is known as a precursor of "hsa-miR-125a-3p".

The term "hsa-miR-5100 gene" or "hsa-miR-5100" used herein includes the hsa-miR-5100 gene (miRBase Accession No. MIMAT0022259) shown in SEQ ID NO: 124, a homolog or an ortholog thereof of a different organism species, and the like. The gene can be obtained by a method described in Tandon M et al., 2012, Oral Dis, Vol. 18, p. 127-131. Also, "hsa-mir-5100" (miRBase Accession No. MI0019116, SEQ ID NO: 381) having a hairpin-like structure is known as a precursor of "hsa-miR-5100".

The term "hsa-miR-4294 gene" or "hsa-miR-4294" used herein includes the hsa-miR-4294 gene (miRBase Accession No. MIMAT0016849) shown in SEQ ID NO: 125, a homolog or an ortholog thereof of a different organism species, and the like. The gene can be obtained by a method described in Goff LA et al., 2009, PLoS One, Vol. 4, e7192. Also, "hsa-mir-4294" (miRBase Accession No. MI0015827, SEQ ID NO: 382) having a hairpin-like structure is known as a precursor of "hsa-miR-4294".

The term "hsa-miR-1231 gene" or "hsa-miR-1231" used herein includes the hsa-miR-1231 gene (miRBase Accession No. MIMAT0005586) shown in SEQ ID NO: 126, a homolog or an ortholog thereof of a different organism species, and the like. The gene can be obtained by a method described in Berezikov E et al., 2007, Mol Cell, Vol. 28, p. 328-336. Also, "hsa-mir-1231" (miRBase Accession No. MI0006321, SEQ ID NO: 383) having a hairpin-like structure is known as a precursor of "hsa-miR-1231".

The term "hsa-miR-6765-3p gene" or "hsa-miR-6765-3p" used herein includes the hsa-miR-6765-3p gene (miRBase Accession No. MIMAT0027431) shown in SEQ ID NO: 127, a homolog or an ortholog thereof of a different organism species, and the like. The gene can be obtained by a method described in Ladewig E et al., 2012, Genome Res, Vol. 22, p. 1634-1645. Also, "hsa-mir-6765" (miRBase Accession No. MI0022610, SEQ ID NO: 384) having a hairpin-like structure is known as a precursor of "hsa-miR-6765-3p".

The term "hsa-miR-4442 gene" or "hsa-miR-4442" used herein includes the hsa-miR-4442 gene (miRBase Accession No. MIMAT0018960) shown in SEQ ID NO: 128, a homolog or an ortholog thereof of a different organism species, and the like. The gene can be obtained by a method described in Jima DD et al., 2010, Blood, Vol. 116, e118-e127. Also, "hsa-mir-4442" (miRBase Accession No. MI0016785, SEQ ID NO: 385) having a hairpin-like structure is known as a precursor of "hsa-miR-4442".

The term "hsa-miR-718 gene" or "hsa-miR-718" used herein includes the hsa-miR-718 gene (miRBase Accession No. MIMAT0012735) shown in SEQ ID NO: 129, a homolog or an ortholog thereof of a different organism species, and the like. The gene can be obtained by a method described in Artzi S et al., 2008, BMC Bioinformatics, Vol. 9, p. 39. Also, "hsa-mir-718" (miRBase Accession No. MI0012489, SEQ ID NO: 386) having a hairpin-like structure is known as a precursor of "hsa-miR-718".

The term "hsa-miR-6780b-5p gene" or "hsa-miR-6780b-5p" used herein includes the hsa-miR-6780b-5p gene (miRBase Accession No. MIMAT0027572) shown in SEQ ID NO: 130, a homolog or an ortholog thereof of a different organism species, and the like. The gene can be obtained by a method described in Ladewig E et al., 2012, Genome Res, Vol. 22, p. 1634-1645. Also, "hsa-mir-6780b" (miRBase Accession No. MI0022681, SEQ ID NO: 387) having a hairpin-like structure is known as a precursor of "hsa-miR-6780b-5p".

The term "hsa-miR-6090 gene" or "hsa-miR-6090" used herein includes the hsa-miR-6090 gene (miRBase Accession No. MIMAT0023715) shown in SEQ ID NO: 131, a homolog or an ortholog thereof of a different organism species, and the like. The gene can be obtained by a method described in Yoo JK et al., 2012, Stem Cells Dev, Vol. 21, p. 2049-2057. Also, "hsa-mir-6090" (miRBase Accession No. MI0020367, SEQ ID NO: 388) having a hairpin-like structure is known as a precursor of "hsa-miR-6090".

The term "hsa-miR-6845-5p gene" or "hsa-miR-6845-5p" used herein includes the hsa-miR-6845-5p gene (miRBase Accession No. MIMAT0027590) shown in SEQ ID NO: 132, a homolog or an ortholog thereof of a different organism species, and the like. The gene can be obtained by a method described in Ladewig E et al., 2012, Genome Res, Vol. 22, p. 1634-1645. Also, "hsa-mir-6845" (miRBase Accession No. MI0022691, SEQ ID NO: 389) having a hairpin-like structure is known as a precursor of "hsa-miR-6845-5p".

The term "hsa-miR-4741 gene" or "hsa-miR-4741" used herein includes the hsa-miR-4741 gene (miRBase Accession No. MIMAT0019871) shown in SEQ ID NO: 133, a homolog or an ortholog thereof of a different organism species, and the like. The gene can be obtained by a method described in Persson H et al., 2011, Cancer Res, Vol. 71, p. 78-86. Also, "hsa-mir-4741" (miRBase Accession No. MI0017379, SEQ ID NO: 390) having a hairpin-like structure is known as a precursor of "hsa-miR-4741".

The term "hsa-miR-4467 gene" or "hsa-miR-4467" used herein includes the hsa-miR-4467 gene (miRBase Accession No. MIMAT0018994) shown in SEQ ID NO: 134, a homolog or an ortholog thereof of a different organism species, and the like. The gene can be obtained by a method described in Jima DD et al., 2010, Blood, Vol. 116, e118-e127. Also, "hsa-mir-4467" (miRBase Accession No. MI0016818, SEQ ID NO: 391) having a hairpin-like structure is known as a precursor of "hsa-miR-4467".

The term "hsa-miR-4707-5p gene" or "hsa-miR-4707-5p" used herein includes the hsa-miR-4707-5p gene (miRBase Accession No. MIMAT0019807) shown in SEQ ID NO: 135, a homolog or an ortholog thereof of a different organism species, and the like. The gene can be obtained by a method described in Persson H et al., 2011, Cancer Res, Vol. 71, p. 78-86. Also, "hsa-mir-4707" (miRBase Accession No. MI0017340, SEQ ID NO: 392) having a hairpin-like structure is known as a precursor of "hsa-miR-4707-5p".

The term "hsa-miR-4271 gene" or "hsa-miR-4271" used herein includes the hsa-miR-4271 gene (miRBase Accession No. MIMAT0016901) shown in SEQ ID NO: 136, a homolog or an ortholog thereof of a different organism species, and the like. The gene can be obtained by a method described in Goff LA et al., 2009, PLoS One, Vol. 4, e7192. Also, "hsa-mir-4271" (miRBase Accession No. MI0015879, SEQ ID NO: 393) having a hairpin-like structure is known as a precursor of "hsa-miR-4271".

The term "hsa-miR-4673 gene" or "hsa-miR-4673" used herein includes the hsa-miR-4673 gene (miRBase Accession No. MIMAT0019755) shown in SEQ ID NO: 137, a homolog or an ortholog thereof of a different organism species, and the like. The gene can be obtained by a method described in Persson H et al., 2011, Cancer Res, Vol. 71, p. 78-86. Also, "hsa-mir-4673" (miRBase Accession No. MI0017304, SEQ ID NO: 394) having a hairpin-like structure is known as a precursor of "hsa-miR-4673".

The term "hsa-miR-3184-5p gene" or "hsa-miR-3184-5p" used herein includes the hsa-miR-3184-5p gene (miRBase Accession No. MIMAT0015064) shown in SEQ ID NO: 138, a homolog or an ortholog thereof of a different organism species, and the like. The gene can be obtained by a method described in Stark MS et al., 2010, PLoS One, Vol. 5, e9685. Also, "hsa-mir-3184" (miRBase Accession No. MI0014226, SEQ ID NO: 395) having a hairpin-like structure is known as a precursor of "hsa-miR-3184-5p".

The term "hsa-miR-1469 gene" or "hsa-miR-1469" used herein includes the hsa-miR-1469 gene (miRBase Accession No. MIMAT0007347) shown in SEQ ID NO: 139, a homolog or an ortholog thereof of a different organism species, and the like. The gene can be obtained by a method described in Kawaji H et al., 2008, BMC Genomics, Vol. 9, p. 157. Also, "hsa-mir-1469" (miRBase Accession No. MI0007074, SEQ ID NO: 396) having a hairpin-like structure is known as a precursor of "hsa-miR-1469".

The term "hsa-miR-4640-5p gene" or "hsa-miR-4640-5p" used herein includes the hsa-miR-4640-5p gene (miRBase Accession No. MIMAT0019699) shown in SEQ ID NO: 140, a homolog or an ortholog thereof of a different organism species, and the like. The gene can be obtained by a method described in Persson H et al., 2011, Cancer Res, Vol. 71, p. 78-86. Also, "hsa-mir-4640" (miRBase Accession No. MI0017267, SEQ ID NO: 397) having a hairpin-like structure is known as a precursor of "hsa-miR-4640-5p".

The term "hsa-miR-663a gene" or "hsa-miR-663a" used herein includes the hsa-miR-663a gene (miRBase Accession No. MIMAT0003326) shown in SEQ ID NO: 141, a homolog or an ortholog thereof of a different organism species, and the like. The gene can be obtained by a method described in Cummins JM et al., 2006, Proc Natl Acad Sci U S A, Vol. 103, p. 3687-3692. Also, "hsa-mir-663a" (miRBase Accession No. MI0003672, SEQ ID NO: 398) having a hairpin-like structure is known as a precursor of "hsa-miR-663a".

The term "hsa-miR-6791-5p gene" or "hsa-miR-6791-5p" used herein includes the hsa-miR-6791-5p gene (miRBase Accession No. MIMAT0027482) shown in SEQ ID NO: 142, a homolog or an ortholog thereof of a different organism species, and the like. The gene can be obtained by a method described in Ladewig E et al., 2012, Genome Res, Vol. 22, p. 1634-1645. Also, "hsa-mir-6791" (miRBase Accession No. MI0022636, SEQ ID NO: 399) having a hairpin-like structure is known as a precursor of "hsa-miR-6791-5p".

The term "hsa-miR-6826-5p gene" or "hsa-miR-6826-5p" used herein includes the hsa-miR-6826-5p gene (miRBase Accession No. MIMAT0027552) shown in SEQ ID NO: 143, a homolog or an ortholog thereof of a different organism species, and the like. The gene can be obtained by a method described in Ladewig E et al., 2012, Genome Res, Vol. 22, p. 1634-1645. Also, "hsa-mir-6826" (miRBase Accession No. MI0022671, SEQ ID NO: 400) having a hairpin-like structure is known as a precursor of "hsa-miR-6826-5p".

The term "hsa-miR-4433b-3p gene" or "hsa-miR-4433b-3p" used herein includes the hsa-miR-4433b-3p gene (miRBase Accession No. MIMAT0030414) shown in SEQ ID NO: 144, a homolog or an ortholog thereof of a different organism species, and the like. The gene can be obtained by a method described in Ple H et al., 2012, PLoS One, Vol. 7, e50746. Also, "hsa-mir-4433b" (miRBase Accession No. MI0025511, SEQ ID NO: 401) having a hairpin-like structure is known as a precursor of "hsa-miR-4433b-3p".

The term "hsa-miR-1915-3p gene" or "hsa-miR-1915-3p" used herein includes the hsa-miR-1915-3p gene (miRBase Accession No. MIMAT0007892) shown in SEQ ID NO: 145, a homolog or an ortholog thereof of a different organism species, and the like. The gene can be obtained by a method described in Bar M et al., 2008, Stem Cells, Vol. 26, p. 2496-2505. Also, "hsa-mir-1915" (miRBase Accession No. MI0008336, SEQ ID NO: 402) having a hairpin-like structure is known as a precursor of "hsa-miR-1915-3p".

The term "hsa-miR-4417 gene" or "hsa-miR-4417" used herein includes the hsa-miR-4417 gene (miRBase Accession No. MIMAT0018929) shown in SEQ ID NO: 146, a homolog or an ortholog thereof of a different organism species, and the like. The gene can be obtained by a method described in Jima DD et al., 2010, Blood, Vol. 116, e118-e127. Also, "hsa-mir-4417" (miRBase Accession No. MI0016753, SEQ ID NO: 403) having a hairpin-like structure is known as a precursor of "hsa-miR-4417".

The term "hsa-miR-4449 gene" or "hsa-miR-4449" used herein includes the hsa-miR-4449 gene (miRBase Accession No. MIMAT0018968) shown in SEQ ID NO: 147, a homolog or an ortholog thereof of a different organism species, and the like. The gene can be obtained by a method described in Jima DD et al., 2010, Blood, Vol. 116, e118-e127. Also, "hsa-mir-4449" (miRBase Accession No. MI0016792, SEQ ID NO: 404) having a hairpin-like structure is known as a precursor of "hsa-miR-4449".

The term "hsa-miR-4707-3p gene" or "hsa-miR-4707-3p" used herein includes the hsa-miR-4707-3p gene (miRBase Accession No. MIMAT0019808) shown in SEQ ID NO: 148, a homolog or an ortholog thereof of a different organism species, and the like. The gene can be obtained by a method described in Persson H et al., 2011, Cancer Res, Vol. 71, p. 78-86. Also, "hsa-mir-4707" (miRBase Accession No. MI0017340, SEQ ID NO: 405) having a hairpin-like structure is known as a precursor of "hsa-miR-4707-3p".

The term "hsa-miR-3180-3p gene" or "hsa-miR-3180-3p" used herein includes the hsa-miR-3180-3p gene (miRBase Accession No. MIMAT0015058) shown in SEQ ID NO: 149, a homolog or an ortholog thereof of a different organism species, and the like. The gene can be obtained by a method described in Creighton CJ et al., 2010, PLoS One, Vol. 5, e9637. Also, "hsa-mir-3180-1, hsa-mir-3180-2 and hsa-mir-3180-3" (miRBase Accession Nos. MI0014214, MI0014215 and MI0014217, SEQ ID NOs: 406, 407 and 408) having a hairpin-like structure are known as a precursor of "hsa-miR-3180-3p".

The term "hsa-miR-5585-3p gene" or "hsa-miR-5585-3p" used herein includes the hsa-miR-5585-3p gene (miRBase Accession No. MIMAT0022286) shown in SEQ ID NO: 150, a homolog or an ortholog thereof of a different organism species, and the like. The gene can be obtained by a method described in Friedlander MR et al., 2012, Nucleic Acids Res, Vol. 40, p. 37-52. Also, "hsa-mir-5585" (miRBase Accession No. MI0019142, SEQ ID NO: 409) having a hairpin-like structure is known as a precursor of "hsa-miR-5585-3p".

The term "hsa-miR-1268a gene" or "hsa-miR-1268a" used herein includes the hsa-miR-1268a gene (miRBase Accession No. MIMAT0005922) shown in SEQ ID NO: 151, a homolog or an ortholog thereof of a different organism species, and the like. The gene can be obtained by a method described in Morin RD et al., 2008, Genome Res, Vol. 18, p. 610-621. Also, "hsa-mir-1268a" (miRBase Accession No. MI0006405, SEQ ID NO: 410) having a hairpin-like structure is known as a precursor of "hsa-miR-1268a".

The term "hsa-miR-8072 gene" or "hsa-miR-8072" used herein includes the hsa-miR-8072 gene (miRBase Accession No. MIMAT0030999) shown in SEQ ID NO: 152, a homolog or an ortholog thereof of a different organism species, and the like. The gene can be obtained by a method described in Wang HJ et al., 2013, Shock, Vol. 39, p. 480-487. Also, "hsa-mir-8072" (miRBase Accession No. MI0025908, SEQ ID NO: 411) having a hairpin-like structure is known as a precursor of "hsa-miR-8072".

The term "hsa-miR-296-5p gene" or "hsa-miR-296-5p" used herein includes the hsa-miR-296-5p gene (miRBase Accession No. MIMAT0000690) shown in SEQ ID NO: 153, a homolog or an ortholog thereof of a different organism species, and the like. The gene can be obtained by a method described in Houbaviy HB et al., 2003, Dev Cell, Vol. 5, p. 351-358. Also, "hsa-mir-296" (miRBase Accession No. MI0000747, SEQ ID NO: 412) having a hairpin-like structure is known as a precursor of "hsa-miR-296-5p".

The term "hsa-miR-204-3p gene" or "hsa-miR-204-3p" used herein includes the hsa-miR-204-3p gene (miRBase Accession No. MIMAT0022693) shown in SEQ ID NO: 154, a homolog or an ortholog thereof of a different organism species, and the like. The gene can be obtained by a method described in Lim LP et al., 2003, Science, Vol. 299, p. 1540. Also, "hsa-mir-204" (miRBase Accession No. MI0000284, SEQ ID NO: 413) having a hairpin-like structure is known as a precursor of "hsa-miR-204-3p".

The term "hsa-miR-4454 gene" or "hsa-miR-4454" used herein includes the hsa-miR-4454 gene (miRBase Accession No. MIMAT0018976) shown in SEQ ID NO: 155, a homolog or an ortholog thereof of a different organism species, and the like. The gene can be obtained by a method described in Jima DD et al., 2010, Blood, Vol. 116, e118-e127. Also, "hsa-mir-4454" (miRBase Accession No. MI0016800, SEQ ID NO: 414) having a hairpin-like structure is known as a precursor of "hsa-miR-4454".

The term "hsa-miR-6722-3p gene" or "hsa-miR-6722-3p" used herein includes the hsa-miR-6722-3p gene (miRBase Accession No. MIMAT0025854) shown in SEQ ID NO: 156, a homolog or an ortholog thereof of a different organism species, and the like. The gene can be obtained by a method described in Li Y et al., 2012, Gene, Vol. 497, p. 330-335. Also, "hsa-mir-6722" (miRBase Accession No. MI0022557, SEQ ID NO: 415) having a hairpin-like structure is known as a precursor of "hsa-miR-6722-3p".

The term "hsa-miR-1290 gene" or "hsa-miR-1290" used herein includes the hsa-miR-1290 gene (miRBase Accession No. MIMAT0005880) shown in SEQ ID NO: 157, a homolog or an ortholog thereof of a different organism species, and the like. The gene can be obtained by a method described in Morin RD et al., 2008, Genome Res, Vol. 18, p. 610-621. Also, "hsa-mir-1290" (miRBase Accession No. MI0006352, SEQ ID NO: 416) having a hairpin-like structure is known as a precursor of "hsa-miR-1290".

The term "hsa-miR-3622a-5p gene" or "hsa-miR-3622a-5p" used herein includes the hsa-miR-3622a-5p gene (miRBase Accession No. MIMAT0018003) shown in SEQ ID NO: 158, a homolog or an ortholog thereof of a different organism species, and the like. The gene can be obtained by a method described in Witten D et al., 2010, BMC Biol, Vol. 8, p. 58. Also, "hsa-mir-3622a" (miRBase Accession No. MI0016013, SEQ ID NO: 417) having a hairpin-like structure is known as a precursor of "hsa-miR-3622a-5p".

The term "hsa-miR-939-5p gene" or "hsa-miR-939-5p" used herein includes the hsa-miR-939-5p gene (miRBase Accession No. MIMAT0004982) shown in SEQ ID NO: 159, a homolog or an ortholog thereof of a different organism species, and the like. The gene can be obtained by a method described in Lui WO et al., 2007, Cancer Res, Vol. 67, p. 6031-6043. Also, "hsa-mir-939" (miRBase Accession No. MI0005761, SEQ ID NO: 418) having a hairpin-like structure is known as a precursor of "hsa-miR-939-5p".

The term "hsa-miR-675-5p gene" or "hsa-miR-675-5p" used herein includes the hsa-miR-675-5p gene (miRBase Accession No. MIMAT0004284) shown in SEQ ID NO: 160, a homolog or an ortholog thereof of a different organism species, and the like. The gene can be obtained by a method described in Cai X et al., 2007, RNA, Vol. 13, p. 313-316. Also, "hsa-mir-675" (miRBase Accession No. MI0005416, SEQ ID NO: 419) having a hairpin-like structure is known as a precursor of "hsa-miR-675-5p".

The term "hsa-miR-3131 gene" or "hsa-miR-3131" used herein includes the hsa-miR-3131 gene (miRBase Accession No. MIMAT0014996) shown in SEQ ID NO: 161, a homolog or an ortholog thereof of a different organism species, and the like. The gene can be obtained by a method described in Stark MS et al., 2010, PLoS One, Vol. 5, e9685. Also, "hsa-mir-3131" (miRBase Accession No. MI0014151, SEQ ID NO: 420) having a hairpin-like structure is known as a precursor of "hsa-miR-3131".

The term "hsa-miR-4648 gene" or "hsa-miR-4648" used herein includes the hsa-miR-4648 gene (miRBase Accession No. MIMAT0019710) shown in SEQ ID NO: 162, a homolog or an ortholog thereof of a different organism species, and the like. The gene can be obtained by a method described in Persson H et al., 2011, Cancer Res, Vol. 71, p. 78-86. Also, "hsa-mir-4648" (miRBase Accession No. MI0017275, SEQ ID NO: 421) having a hairpin-like structure is known as a precursor of "hsa-miR-4648".

The term "hsa-miR-1268b gene" or "hsa-miR-1268b" used herein includes the hsa-miR-1268b gene (miRBase Accession No. MIMAT0018925) shown in SEQ ID NO: 163, a homolog or an ortholog thereof of a different organism species, and the like. The gene can be obtained by a method described in Jima DD et al., 2010, Blood, Vol. 116, e118-e127. Also, "hsa-mir-1268b" (miRBase Accession No. MI0016748, SEQ ID NO: 422) having a hairpin-like structure is known as a precursor of "hsa-miR-1268b".

The term "hsa-miR-6741-5p gene" or "hsa-miR-6741-5p" used herein includes the hsa-miR-6741-5p gene (miRBase Accession No. MIMAT0027383) shown in SEQ ID NO: 164, a homolog or an ortholog thereof of a different organism species, and the like. The gene can be obtained by a method described in Ladewig E et al., 2012, Genome Res, Vol. 22, p. 1634-1645. Also, "hsa-mir-6741" (miRBase Accession No. MI0022586, SEQ ID NO: 423) having a hairpin-like structure is known as a precursor of "hsa-miR-6741-5p".

The term "hsa-miR-6893-5p gene" or "hsa-miR-6893-5p" used herein includes the hsa-miR-6893-5p gene (miRBase Accession No. MIMAT0027686) shown in SEQ ID NO: 165, a homolog or an ortholog thereof of a different organism species, and the like. The gene can be obtained by a method described in Ladewig E et al., 2012, Genome Res, Vol. 22, p. 1634-1645. Also, "hsa-mir-6893" (miRBase Accession No. MI0022740, SEQ ID NO: 424) having a hairpin-like structure is known as a precursor of "hsa-miR-6893-5p".

The term "hsa-miR-3162-5p gene" or "hsa-miR-3162-5p" used herein includes the hsa-miR-3162-5p gene (miRBase Accession No. MIMAT0015036) shown in SEQ ID NO: 166, a homolog or an ortholog thereof of a different organism species, and the like. The gene can be obtained by a method described in Stark MS et al., 2010, PLoS One, Vol. 5, e9685. Also, "hsa-mir-3162" (miRBase Accession No. MI0014192, SEQ ID NO: 425) having a hairpin-like structure is known as a precursor of "hsa-miR-3162-5p".

The term "hsa-miR-642b-3p gene" or "hsa-miR-642b-3p" used herein includes the hsa-miR-642b-3p gene (miRBase Accession No. MIMAT0018444) shown in SEQ ID NO: 167, a homolog or an ortholog thereof of a different organism species, and the like. The gene can be obtained by a method described in Witten D et al., 2010, BMC Biol, Vol. 8, p. 58. Also, "hsa-mir-642b" (miRBase Accession No. MI0016685, SEQ ID NO: 426) having a hairpin-like structure is known as a precursor of "hsa-miR-642b-3p".

The term "hsa-miR-4734 gene" or "hsa-miR-4734" used herein includes the hsa-miR-4734 gene (miRBase Accession No. MIMAT0019859) shown in SEQ ID NO: 168, a homolog or an ortholog thereof of a different organism species, and the like. The gene can be obtained by a method described in Persson H et al., 2011, Cancer Res, Vol. 71, p. 78-86. Also, "hsa-mir-4734" (miRBase Accession No. MI0017371, SEQ ID NO: 427) having a hairpin-like structure is known as a precursor of "hsa-miR-4734".

The term "hsa-miR-150-3p gene" or "hsa-miR-150-3p" used herein includes the hsa-miR-150-3p gene (miRBase Accession No. MIMAT0004610) shown in SEQ ID NO: 169, a homolog or an ortholog thereof of a different organism species, and the like. The gene can be obtained by a method described in Lagos-Quintana M et al., 2002, Curr Biol, Vol. 12, p. 735-739. Also, "hsa-mir-150" (miRBase Accession No. MI0000479, SEQ ID NO: 428) having a hairpin-like structure is known as a precursor of "hsa-miR-150-3p".

The term "hsa-miR-8089 gene" or "hsa-miR-8089" used herein includes the hsa-miR-8089 gene (miRBase Accession No. MIMAT0031016) shown in SEQ ID NO: 170, a homolog or an ortholog thereof of a different organism species, and the like. The gene can be obtained by a method described in Wang HJ et al., 2013, Shock, Vol. 39, p. 480-487. Also, "hsa-mir-8089" (miRBase Accession No. MI0025925, SEQ ID NO: 429) having a hairpin-like structure is known as a precursor of "hsa-miR-8089".

The term "hsa-miR-6805-3p gene" or "hsa-miR-6805-3p" used herein includes the hsa-miR-6805-3p gene (miRBase Accession No. MIMAT0027511) shown in SEQ ID NO: 171, a homolog or an ortholog thereof of a different organism species, and the like. The gene can be obtained by a method described in Ladewig E et al., 2012, Genome Res, Vol. 22, p. 1634-1645. Also, "hsa-mir-6805" (miRBase Accession No. MI0022650, SEQ ID NO: 430) having a hairpin-like structure is known as a precursor of "hsa-miR-6805-3p".

The term "hsa-miR-7113-3p gene" or "hsa-miR-7113-3p" used herein includes the hsa-miR-7113-3p gene (miRBase Accession No. MIMAT0028124) shown in SEQ ID NO: 172, a homolog or an ortholog thereof of a different organism species, and the like. The gene can be obtained by a method described in Ladewig E et al., 2012, Genome Res, Vol. 22, p. 1634-1645. Also, "hsa-mir-7113" (miRBase Accession No. MI0022964, SEQ ID NO: 431) having a hairpin-like structure is known as a precursor of "hsa-miR-7113-3p".

The term "hsa-miR-6850-5p gene" or "hsa-miR-6850-5p" used herein includes the hsa-miR-6850-5p gene (miRBase Accession No. MIMAT0027600) shown in SEQ ID NO: 173, a homolog or an ortholog thereof of a different organism species, and the like. The gene can be obtained by a method described in Ladewig E et al., 2012, Genome Res, Vol. 22, p. 1634-1645. Also, "hsa-mir-6850" (miRBase Accession No. MI0022696, SEQ ID NO: 432) having a hairpin-like structure is known as a precursor of "hsa-miR-6850-5p".

The term "hsa-miR-6799-5p gene" or "hsa-miR-6799-5p" used herein includes the hsa-miR-6799-5p gene (miRBase Accession No. MIMAT0027498) shown in SEQ ID NO: 174, a homolog or an ortholog thereof of a different organism species, and the like. The gene can be obtained by a method described in Ladewig E et al., 2012, Genome Res, Vol. 22, p. 1634-1645. Also, "hsa-mir-6799" (miRBase Accession No. MI0022644, SEQ ID NO: 433) having a hairpin-like structure is known as a precursor of "hsa-miR-6799-5p".

The term "hsa-miR-6768-5p gene" or "hsa-miR-6768-5p" used herein includes the hsa-miR-6768-5p gene (miRBase Accession No. MIMAT0027436) shown in SEQ ID NO: 175, a homolog or an ortholog thereof of a different organism species, and the like. The gene can be obtained by a method described in Ladewig E et al., 2012, Genome Res, Vol. 22, p. 1634-1645. Also, "hsa-mir-6768" (miRBase Accession No. MI0022613, SEQ ID NO: 434) having a hairpin-like structure is known as a precursor of "hsa-miR-6768-5p".

The term "hsa-miR-92b-5p gene" or "hsa-miR-92b-5p" used herein includes the hsa-miR-92b-5p gene (miRBase Accession No. MIMAT0004792) shown in SEQ ID NO: 176, a homolog or an ortholog thereof of a different organism species, and the like. The gene can be obtained by a method described in Cummins JM et al., 2006, Proc Natl Acad Sci U S A, Vol. 103, p. 3687-3692. Also, "hsa-mir-92b" (miRBase Accession No. MI0003560, SEQ ID NO: 435) having a hairpin-like structure is known as a precursor of "hsa-miR-92b-5p".

The term "hsa-miR-3679-5p gene" or "hsa-miR-3679-5p" used herein includes the hsa-miR-3679-5p gene (miRBase Accession No. MIMAT0018104) shown in SEQ ID NO: 177, a homolog or an ortholog thereof of a different organism species, and the like. The gene can be obtained by a method described in Creighton CJ et al., 2010, PLoS One, Vol. 5, e9637. Also, "hsa-mir-3679" (miRBase Accession No. MI0016080, SEQ ID NO: 436) having a hairpin-like structure is known as a precursor of "hsa-miR-3679-5p".

The term "hsa-miR-4792 gene" or "hsa-miR-4792" used herein includes the hsa-miR-4792 gene (miRBase Accession No. MIMAT0019964) shown in SEQ ID NO: 178, a homolog or an ortholog thereof of a different organism species, and the like. The gene can be obtained by a method described in Persson H et al., 2011, Cancer Res, Vol. 71, p. 78-86. Also, "hsa-mir-4792" (miRBase Accession No. MI0017439, SEQ ID NO: 437) having a hairpin-like structure is known as a precursor of "hsa-miR-4792".

The term "hsa-miR-3656 gene" or "hsa-miR-3656" used herein includes the hsa-miR-3656 gene (miRBase Accession No. MIMAT0018076) shown in SEQ ID NO: 179, a homolog or an ortholog thereof of a different organism species, and the like. The gene can be obtained by a method described in Meiri E et al., 2010, Nucleic Acids Res, Vol. 38, p. 6234-6246. Also, "hsa-mir-3656" (miRBase Accession No. MI0016056, SEQ ID NO: 438) having a hairpin-like structure is known as a precursor of "hsa-miR-3656".

The term "hsa-miR-92a-2-5p gene" or "hsa-miR-92a-2-5p" used herein includes the hsa-miR-92a-2-5p gene (miRBase Accession No. MIMAT0004508) shown in SEQ ID NO: 180, a homolog or an ortholog thereof of a different organism species, and the like. The gene can be obtained by a method described in Mourelatos Z et al., 2002, Genes Dev, Vol. 16, p. 720-728. Also, "hsa-mir-92a-2" (miRBase Accession No. MI0000094, SEQ ID NO: 439) having a hairpin-like structure is known as a precursor of "hsa-miR-92a-2-5p".

The term "hsa-miR-4466 gene" or "hsa-miR-4466" used herein includes the hsa-miR-4466 gene (miRBase Accession No. MIMAT0018993) shown in SEQ ID NO: 181, a homolog or an ortholog thereof of a different organism species, and the like. The gene can be obtained by a method described in Jima DD et al., 2010, Blood, Vol. 116, e118-e127. Also, "hsa-mir-4466" (miRBase Accession No. MI0016817, SEQ ID NO: 440) having a hairpin-like structure is known as a precursor of "hsa-miR-4466".

The term "hsa-miR-4513 gene" or "hsa-miR-4513" used herein includes the hsa-miR-4513 gene (miRBase Accession No. MIMAT0019050) shown in SEQ ID NO: 182, a homolog or an ortholog thereof of a different organism species, and the like. The gene can be obtained by a method described in Jima DD et al., 2010, Blood, Vol. 116, e118-e127. Also, "hsa-mir-4513" (miRBase Accession No. MI0016879, SEQ ID NO: 441) having a hairpin-like structure is known as a precursor of "hsa-miR-4513".

The term "hsa-miR-6781-5p gene" or "hsa-miR-6781-5p" used herein includes the hsa-miR-6781-5p gene (miRBase Accession No. MIMAT0027462) shown in SEQ ID NO: 183, a homolog or an ortholog thereof of a different organism species, and the like. The gene can be obtained by a method described in Ladewig E et al., 2012, Genome Res, Vol. 22, p. 1634-1645. Also, "hsa-mir-6781" (miRBase Accession No. MI0022626, SEQ ID NO: 442) having a hairpin-like structure is known as a precursor of "hsa-miR-6781-5p".

The term "hsa-miR-4649-5p gene" or "hsa-miR-4649-5p" used herein includes the hsa-miR-4649-5p gene (miRBase Accession No. MIMAT0019711) shown in SEQ ID NO: 184, a homolog or an ortholog thereof of a different organism species, and the like. The gene can be obtained by a method described in Persson H et al., 2011, Cancer Res, Vol. 71, p. 78-86. Also, "hsa-mir-4649" (miRBase Accession No. MI0017276, SEQ ID NO: 443) having a hairpin-like structure is known as a precursor of "hsa-miR-4649-5p".

The term "hsa-miR-6775-5p gene" or "hsa-miR-6775-5p" used herein includes the hsa-miR-6775-5p gene (miRBase Accession No. MIMAT0027450) shown in SEQ ID NO: 185, a homolog or an ortholog thereof of a different organism species, and the like. The gene can be obtained by a method described in Ladewig E et al., 2012, Genome Res, Vol. 22, p. 1634-1645. Also, "hsa-mir-6775" (miRBase Accession No. MI0022620, SEQ ID NO: 444) having a hairpin-like structure is known as a precursor of "hsa-miR-6775-5p".

The term "hsa-miR-4651 gene" or "hsa-miR-4651" used herein includes the hsa-miR-4651 gene (miRBase Accession No. MIMAT0019715) shown in SEQ ID NO: 186, a homolog or an ortholog thereof of a different organism species, and the like. The gene can be obtained by a method described in Persson H et al., 2011, Cancer Res, Vol. 71, p. 78-86. Also, "hsa-mir-4651" (miRBase Accession No. MI0017279, SEQ ID NO: 445) having a hairpin-like structure is known as a precursor of "hsa-miR-4651".

The term "hsa-miR-3195 gene" or "hsa-miR-3195" used herein includes the hsa-miR-3195 gene (miRBase Accession No. MIMAT0015079) shown in SEQ ID NO: 187, a homolog or an ortholog thereof of a different organism species, and the like. The gene can be obtained by a method described in Stark MS et al., 2010, PLoS One, Vol. 5, e9685. Also, "hsa-mir-3195" (miRBase Accession No. MI0014240, SEQ ID NO: 446) having a hairpin-like structure is known as a precursor of "hsa-miR-3195".

The term "hsa-miR-6726-5p gene" or "hsa-miR-6726-5p" used herein includes the hsa-miR-6726-5p gene (miRBase Accession No. MIMAT0027353) shown in SEQ ID NO: 188, a homolog or an ortholog thereof of a different organism species, and the like. The gene can be obtained by a method described in Ladewig E et al., 2012, Genome Res, Vol. 22, p. 1634-1645. Also, "hsa-mir-6726" (miRBase Accession No. MI0022571, SEQ ID NO: 447) having a hairpin-like structure is known as a precursor of "hsa-miR-6726-5p".

The term "hsa-miR-6872-3p gene" or "hsa-miR-6872-3p" used herein includes the hsa-miR-6872-3p gene (miRBase Accession No. MIMAT0027645) shown in SEQ ID NO: 189, a homolog or an ortholog thereof of a different organism species, and the like. The gene can be obtained by a method described in Ladewig E et al., 2012, Genome Res, Vol. 22, p. 1634-1645. Also, "hsa-mir-6872" (miRBase Accession No. MI0022719, SEQ ID NO: 448) having a hairpin-like structure is known as a precursor of "hsa-miR-6872-3p".

The term "hsa-miR-371a-5p gene" or "hsa-miR-371a-5p" used herein includes the hsa-miR-371a-5p gene (miRBase Accession No. MIMAT0004687) shown in SEQ ID NO: 190, a homolog or an ortholog thereof of a different organism species, and the like. The gene can be obtained by a method described in Suh MR et al., 2004, Dev Biol, Vol. 270, p. 488-498. Also, "hsa-mir-371a" (miRBase Accession No. MI0000779, SEQ ID NO: 449) having a hairpin-like structure is known as a precursor of "hsa-miR-371a-5p".

The term "hsa-miR-6777-5p gene" or "hsa-miR-6777-5p" used herein includes the hsa-miR-6777-5p gene (miRBase Accession No. MIMAT0027454) shown in SEQ ID NO: 191, a homolog or an ortholog thereof of a different organism species, and the like. The gene can be obtained by a method described in Ladewig E et al., 2012, Genome Res, Vol. 22, p. 1634-1645. Also, "hsa-mir-6777" (miRBase Accession No. MI0022622, SEQ ID NO: 450) having a hairpin-like structure is known as a precursor of "hsa-miR-6777-5p".

The term "hsa-miR-6789-5p gene" or "hsa-miR-6789-5p" used herein includes the hsa-miR-6789-5p gene (miRBase Accession No. MIMAT0027478) shown in SEQ ID NO: 192, a homolog or an ortholog thereof of a different organism species, and the like. The gene can be obtained by a method described in Ladewig E et al., 2012, Genome Res, Vol. 22, p. 1634-1645. Also, "hsa-mir-6789" (miRBase Accession No. MI0022634, SEQ ID NO: 451) having a hairpin-like structure is known as a precursor of "hsa-miR-6789-5p".

The term "hsa-miR-7975 gene" or "hsa-miR-7975" used herein includes the hsa-miR-7975 gene (miRBase Accession No. MIMAT0031178) shown in SEQ ID NO: 193, a homolog or an ortholog of a different organism species, and the like. The gene can be obtained by a method described in Velthut-Meikas A et al., 2013, Mol Endocrinol, Vol. 27, p. 1128-1141. Also, "hsa-mir-7975" (miRBase Accession No. MI0025751, SEQ ID NO: 452) having a hairpin-like structure is known as a precursor of "hsa-miR-7975".

The term "hsa-miR-6821-5p gene" or "hsa-miR-6821-5p" used herein includes the hsa-miR-6821-5p gene (miRBase Accession No. MIMAT0027542) shown in SEQ ID NO: 194, a homolog or an ortholog thereof of a different organism species, and the like. The gene can be obtained by a method described in Ladewig E et al., 2012, Genome Res, Vol. 22, p. 1634-1645. Also, "hsa-mir-6821" (miRBase Accession No. MI0022666, SEQ ID NO: 453) having a hairpin-like structure is known as a precursor of "hsa-miR-6821-5p".

The term "hsa-miR-4534 gene" or "hsa-miR-4534" used herein includes the hsa-miR-4534 gene (miRBase Accession No. MIMAT0019073) shown in SEQ ID NO: 195, a homolog or an ortholog thereof of a different organism species, and the like. The gene can be obtained by a method described in Jima DD et al., 2010, Blood, Vol. 116, e118-e127. Also, "hsa-mir-4534" (miRBase Accession No. MI0016901, SEQ ID NO: 454) having a hairpin-like structure is known as a precursor of "hsa-miR-4534".

The term "hsa-miR-619-5p gene" or "hsa-miR-619-5p" used herein includes the hsa-miR-619-5p gene (miRBase Accession No. MIMAT0026622) shown in SEQ ID NO: 196, a homolog or an ortholog thereof of a different organism species, and the like. The gene can be obtained by a method described in Cummins JM et al., 2006, Proc Natl Acad Sci U S A, Vol. 103, p. 3687-3692. Also, "hsa-mir-619" (miRBase Accession No. MI0003633, SEQ ID NO: 455) having a hairpin-like structure is known as a precursor of "hsa-miR-619-5p".

The term "hsa-miR-7107-5p gene" or "hsa-miR-7107-5p" used herein includes the hsa-miR-7107-5p gene (miRBase Accession No. MIMAT0028111) shown in SEQ ID NO: 197, a homolog or an ortholog thereof of a different organism species, and the like. The gene can be obtained by a method described in Ladewig E et al., 2012, Genome Res, Vol. 22, p. 1634-1645. Also, "hsa-mir-7107" (miRBase Accession No. MI0022958, SEQ ID NO: 456) having a hairpin-like structure is known as a precursor of "hsa-miR-7107-5p".

The term "hsa-miR-1228-3p gene" or "hsa-miR-1228-3p" used herein includes the hsa-miR-1228-3p gene (miRBase Accession No. MIMAT0005583) shown in SEQ ID NO: 198, a homolog or an ortholog thereof of a different organism species, and the like. The gene can be obtained by a method described in Berezikov E et al., 2007, Mol Cell, Vol. 28, p. 328-336. Also, "hsa-mir-1228" (miRBase Accession No. MI0006318, SEQ ID NO: 457) having a hairpin-like structure is known as a precursor of "hsa-miR-1228-3p".

The term "hsa-miR-6774-5p gene" or "hsa-miR-6774-5p" used herein includes the hsa-miR-6774-5p gene (miRBase Accession No. MIMAT0027448) shown in SEQ ID NO: 199, a homolog or an ortholog thereof of a different organism species, and the like. The gene can be obtained by a method described in Ladewig E et al., 2012, Genome Res, Vol. 22, p. 1634-1645. Also, "hsa-mir-6774" (miRBase Accession No. MI0022619, SEQ ID NO: 458) having a hairpin-like structure is known as a precursor of "hsa-miR-6774-5p".

The term "hsa-miR-6805-5p gene" or "hsa-miR-6805-5p" used herein includes the hsa-miR-6805-5p gene (miRBase Accession No. MIMAT0027510) shown in SEQ ID NO: 200, a homolog or an ortholog thereof of a different organism species, and the like. The gene can be obtained by a method described in Ladewig E et al., 2012, Genome Res, Vol. 22, p. 1634-1645. Also, "hsa-mir-6805" (miRBase Accession No. MI0022650, SEQ ID NO: 459) having a hairpin-like structure is known as a precursor of "hsa-miR-6805-5p".

The term "hsa-miR-23a-3p gene" or "hsa-miR-23a-3p" used herein includes the hsa-miR-23a-3p gene (miRBase Accession No. MIMAT0000078) shown in SEQ ID NO: 201, a homolog or an ortholog thereof of a different organism species, and the like. The gene can be obtained by a method described in Lagos-Quintana M et al., 2001, Science, Vol. 294, p. 853-858. Also, "hsa-mir-23a" (miRBase Accession No. MI0000079, SEQ ID NO: 460) having a hairpin-like structure is known as a precursor of "hsa-miR-23a-3p".

The term "hsa-miR-4665-5p gene" or "hsa-miR-4665-5p" used herein includes the hsa-miR-4665-5p gene (miRBase Accession No. MIMAT0019739) shown in SEQ ID NO: 202, a homolog or an ortholog thereof of a different organism species, and the like. The gene can be obtained by a method described in Persson H et al., 2011, Cancer Res, Vol. 71, p. 78-86. Also, "hsa-mir-4665" (miRBase Accession No. MI0017295, SEQ ID NO: 461) having a hairpin-like structure is known as a precursor of "hsa-miR-4665-5p".

The term "hsa-miR-4505 gene" or "hsa-miR-4505" used herein includes the hsa-miR-4505 gene (miRBase Accession No. MIMAT0019041) shown in SEQ ID NO: 203, a homolog or an ortholog thereof of a different organism species, and the like. The gene can be obtained by a method described in Jima DD et al., 2010, Blood, Vol. 116, e118-e127. Also, "hsa-mir-4505" (miRBase Accession No. MI0016868, SEQ ID NO: 462) having a hairpin-like structure is known as a precursor of "hsa-miR-4505".

The term "hsa-miR-4638-5p gene" or "hsa-miR-4638-5p" used herein includes the hsa-miR-4638-5p gene (miRBase Accession No. MIMAT0019695) shown in SEQ ID NO: 204, a homolog or an ortholog thereof of a different organism species, and the like. The gene can be obtained by a method described in Persson H et al., 2011, Cancer Res, Vol. 71, p. 78-86. Also, "hsa-mir-4638" (miRBase Accession No. MI0017265, SEQ ID NO: 463) having a hairpin-like structure is known as a precursor of "hsa-miR-4638-5p".

The term "hsa-miR-24-3p gene" or "hsa-miR-24-3p" used herein includes the hsa-miR-24-3p gene (miRBase Accession No. MIMAT0000080) shown in SEQ ID NO: 205, a homolog or an ortholog thereof of a different organism species, and the like. The gene can be obtained by a method described in Lagos-Quintana M et al., 2001, Science, Vol. 294, p. 853-858. Also, "hsa-mir-24-1 and hsa-mir-24-2" (miRBase Accession Nos. MI0000080 and MI0000081, SEQ ID NOs: 464 and 465) having a hairpin-like structure are known as a precursor of "hsa-miR-24-3p".

The term "hsa-miR-3135b gene" or "hsa-miR-3135b" used herein includes the hsa-miR-3135b gene (miRBase Accession No. MIMAT0018985) shown in SEQ ID NO: 206, a homolog or an ortholog thereof of a different organism species, and the like. The gene can be obtained by a method described in Jima DD et al., 2010, Blood, Vol. 116, e118-e127. Also, "hsa-mir-3135b" (miRBase Accession No. MI0016809, SEQ ID NO: 466) having a hairpin-like structure is known as a precursor of "hsa-miR-3135b".

The term "hsa-miR-4745-5p gene" or "hsa-miR-4745-5p" used herein includes the hsa-miR-4745-5p gene (miRBase Accession No. MIMAT0019878) shown in SEQ ID NO: 207, a homolog or an ortholog thereof of a different organism species, and the like. The gene can be obtained by a method described in Persson H et al., 2011, Cancer Res, Vol. 71, p. 78-86. Also, "hsa-mir-4745" (miRBase Accession No. MI0017384, SEQ ID NO: 467) having a hairpin-like structure is known as a precursor of "hsa-miR-4745-5p".

The term "hsa-miR-128-1-5p gene" or "hsa-miR-128-1-5p" used herein includes the hsa-miR-128-1-5p gene (miRBase Accession No. MIMAT0026477) shown in SEQ ID NO: 208, a homolog or an ortholog thereof of a different organism species, and the like. The gene can be obtained by a method described in Lagos-Quintana M et al., 2002, Curr Biol, Vol. 12, p. 735-739. Also, "hsa-mir-128-1" (miRBase Accession No. MI0000447, SEQ ID NO: 468) having a hairpin-like structure is known as a precursor of "hsa-miR-128-1-5p".

The term "hsa-miR-4476 gene" or "hsa-miR-4476" used herein includes the hsa-miR-4476 gene (miRBase Accession No. MIMAT0019003) shown in SEQ ID NO: 209, a homolog or an ortholog thereof of a different organism species, and the like. The gene can be obtained by a method described in Jima DD et al., 2010, Blood, Vol. 116, e118-e127. Also, "hsa-mir-4476" (miRBase Accession No. MI0016828, SEQ ID NO: 469) having a hairpin-like structure is known as a precursor of "hsa-miR-4476".

The term "hsa-miR-4687-3p gene" or "hsa-miR-4687-3p" used herein includes the hsa-miR-4687-3p gene (miRBase Accession No. MIMAT0019775) shown in SEQ ID NO: 210, a homolog or an ortholog thereof of a different organism species, and the like. The gene can be obtained by a method described in Persson H et al., 2011, Cancer Res, Vol. 71, p. 78-86. Also, "hsa-mir-4687" (miRBase Accession No. MI0017319, SEQ ID NO: 470) having a hairpin-like structure is known as a precursor of "hsa-miR-4687-3p".

The term "hsa-miR-3665 gene" or "hsa-miR-3665" used herein includes the hsa-miR-3665 gene (miRBase Accession No. MIMAT0018087) shown in SEQ ID NO: 211, a homolog or an ortholog thereof of a different organism species, and the like. The gene can be obtained by a method described in Xie X et al., 2005, Nature, Vol. 434, p. 338-345. Also, "hsa-mir-3665" (miRBase Accession No. MI0016066, SEQ ID NO: 471) having a hairpin-like structure is known as a precursor of "hsa-miR-3665".

The term "hsa-miR-6806-5p gene" or "hsa-miR-6806-5p" used herein includes the hsa-miR-6806-5p gene (miRBase Accession No. MIMAT0027512) shown in SEQ ID NO: 212, a homolog or an ortholog thereof of a different organism species, and the like. The gene can be obtained by a method described in Ladewig E et al., 2012, Genome Res, Vol. 22, p. 1634-1645. Also, "hsa-mir-6806" (miRBase Accession No. MI0022651, SEQ ID NO: 472) having a hairpin-like structure is known as a precursor of "hsa-miR-6806-5p".

The term "hsa-miR-3937 gene" or "hsa-miR-3937" used herein includes the hsa-miR-3937 gene (miRBase Accession No. MIMAT0018352) shown in SEQ ID NO: 213, a homolog or an ortholog thereof of a different organism species, and the like. The gene can be obtained by a method described in Liao JY et al., 2010, PLoS One, Vol. 5, e10563. Also, "hsa-mir-3937" (miRBase Accession No. MI0016593, SEQ ID NO: 473) having a hairpin-like structure is known as a precursor of "hsa-miR-3937".

The term "hsa-miR-711 gene" or "hsa-miR-711" used herein includes the hsa-miR-711 gene (miRBase Accession No. MIMAT0012734) shown in SEQ ID NO: 214, a homolog or an ortholog thereof of a different organism species, and the like. The gene can be obtained by a method described in Artzi S et al., 2008, BMC Bioinformatics, Vol. 9, p. 39. Also, "hsa-mir-711" (miRBase Accession No. MI0012488, SEQ ID NO: 474) having a hairpin-like structure is known as a precursor of "hsa-miR-711"

The term "hsa-miR-3141 gene" or "hsa-miR-3141" used herein includes the hsa-miR-3141 gene (miRBase Accession No. MIMAT0015010) shown in SEQ ID NO: 215, a homolog or an ortholog thereof of a different organism species, and the like. The gene can be obtained by a method described in Stark MS et al., 2010, PLoS One, Vol. 5, e9685. Also, "hsa-mir-3141" (miRBase Accession No. MI0014165, SEQ ID NO: 475) having a hairpin-like structure is known as a precursor of "hsa-miR-3141".

The term "hsa-miR-3188 gene" or "hsa-miR-3188" used herein includes the hsa-miR-3188 gene (miRBase Accession No. MIMAT0015070) shown in SEQ ID NO: 216, a homolog or an ortholog thereof of a different organism species, and the like. The gene can be obtained by a method described in Stark MS et al., 2010, PLoS One, Vol. 5, e9685. Also, "hsa-mir-3188" (miRBase Accession No. MI0014232, SEQ ID NO: 476) having a hairpin-like structure is known as a precursor of "hsa-miR-3188".

The term "hsa-miR-4281 gene" or "hsa-miR-4281" used herein includes the hsa-miR-4281 gene (miRBase Accession No. MIMAT0016907) shown in SEQ ID NO: 217, a homolog or an ortholog thereof of a different organism species, and the like. The gene can be obtained by a method described in Goff LA et al., 2009, PLoS One, Vol. 4, e7192. Also, "hsa-mir-4281" (miRBase Accession No. MI0015885, SEQ ID NO: 477) having a hairpin-like structure is known as a precursor of "hsa-miR-4281".

The term "hsa-miR-5196-5p gene" or "hsa-miR-5196-5p" used herein includes the hsa-miR-5196-5p gene (miRBase Accession No. MIMAT0021128) shown in SEQ ID NO: 218, a homolog or an ortholog thereof of a different organism species, and the like. The gene can be obtained by a method described in Schotte D et al., 2011, Leukemia, Vol. 25, p. 1389-1399. Also, "hsa-mir-5196" (miRBase Accession No. MI0018175, SEQ ID NO: 478) having a hairpin-like structure is known as a precursor of "hsa-miR-5196-5p".

The term "hsa-miR-6880-5p gene" or "hsa-miR-6880-5p" used herein includes the hsa-miR-6880-5p gene (miRBase Accession No. MIMAT0027660) shown in SEQ ID NO: 219, a homolog or an ortholog thereof of a different organism species, and the like. The gene can be obtained by a method described in Ladewig E et al., 2012, Genome Res, Vol. 22, p. 1634-1645. Also, "hsa-mir-6880" (miRBase Accession No. MI0022727, SEQ ID NO: 479) having a hairpin-like structure is known as a precursor of "hsa-miR-6880-5p".

The term "hsa-miR-3960 gene" or "hsa-miR-3960" used herein includes the hsa-miR-3960 gene (miRBase Accession No. MIMAT0019337) shown in SEQ ID NO: 220, a homolog or an ortholog thereof of a different organism species, and the like. The gene can be obtained by a method described in Hu R et al., 2011, J Biol Chem, Vol. 286, p. 12328-12339. Also, "hsa-mir-3960" (miRBase Accession No. MI0016964, SEQ ID NO: 480) having a hairpin-like structure is known as a precursor of "hsa-miR-3960".

The term "hsa-miR-3648 gene" or "hsa-miR-3648" used herein includes the hsa-miR-3648 gene (miRBase Accession No. MIMAT0018068) shown in SEQ ID NO: 221, a homolog or an ortholog thereof of a different organism species, and the like. The gene can be obtained by a method described in Meiri E et al., 2010, Nucleic Acids Res, Vol. 38, p. 6234-6246. Also, "hsa-mir-3648-1 and hsa-miR-3648-2" (miRBase Accession Nos. MI0016048 and MI0031512, SEQ ID NOs: 481 and 482) having a hairpin-like structure are known as a precursor of "hsa-miR-3648".

The term "hsa-miR-6721-5p gene" or "hsa-miR-6721-5p" used herein includes the hsa-miR-6721-5p gene (miRBase Accession No. MIMAT0025852) shown in SEQ ID NO: 222, a homolog or an ortholog thereof of a different organism species, and the like. The gene can be obtained by a method described in Li Y et al., 2012, Gene, Vol. 497, p. 330-335. Also, "hsa-mir-6721" (miRBase Accession No. MI0022556, SEQ ID NO: 483) having a hairpin-like structure is known as a precursor of "hsa-miR-6721-5p".

The term "hsa-miR-4492 gene" or "hsa-miR-4492" used herein includes the hsa-miR-4492 gene (miRBase Accession No. MIMAT0019027) shown in SEQ ID NO: 223, a homolog or an ortholog thereof of a different organism species, and the like. The gene can be obtained by a method described in Jima DD et al., 2010, Blood, Vol. 116, e118-e127. Also, "hsa-mir-4492" (miRBase Accession No. MI0016854, SEQ ID NO: 484) having a hairpin-like structure is known as a precursor of "hsa-miR-4492".

The term "hsa-miR-744-5p gene" or "hsa-miR-744-5p" used herein includes the hsa-miR-744-5p gene (miRBase Accession No. MIMAT0004945) shown in SEQ ID NO: 224, a homolog or an ortholog thereof of a different organism species, and the like. The gene can be obtained by a method described in Berezikov E et al., 2006, Genome Res, Vol. 16, p. 1289-1298. Also, "hsa-mir-744" (miRBase Accession No. MI0005559, SEQ ID NO: 485) having a hairpin-like structure is known as a precursor of "hsa-miR-744-5p".

The term "hsa-miR-7704 gene" or "hsa-miR-7704" used herein includes the hsa-miR-7704 gene (miRBase Accession No. MIMAT0030019) shown in SEQ ID NO: 225, a homolog or an ortholog thereof of a different organism species, and the like. The gene can be obtained by a method described in Swaminathan S et al., 2013, Biochem Biophys Res Commun, Vol. 434, p. 228-234. Also, "hsa-mir-7704" (miRBase Accession No. MI0025240, SEQ ID NO: 486) having a hairpin-like structure is known as a precursor of "hsa-miR-7704".

The term "hsa-miR-4749-5p gene" or "hsa-miR-4749-5p" used herein includes the hsa-miR-4749-5p gene (miRBase Accession No. MIMAT0019885) shown in SEQ ID NO: 226, a homolog or an ortholog thereof of a different organism species, and the like. The gene can be obtained by a method described in Persson H et al., 2011, Cancer Res, Vol. 71, p. 78-86. Also, "hsa-mir-4749" (miRBase Accession No. MI0017388, SEQ ID NO: 487) having a hairpin-like structure is known as a precursor of "hsa-miR-4749-5p".

The term "hsa-miR-6794-5p gene" or "hsa-miR-6794-5p" used herein includes the hsa-miR-6794-5p gene (miRBase Accession No. MIMAT0027488) shown in SEQ ID NO: 227, a homolog or an ortholog thereof of a different organism species, and the like. The gene can be obtained by a method described in Ladewig E et al., 2012, Genome Res, Vol. 22, p. 1634-1645. Also, "hsa-mir-6794" (miRBase Accession No. MI0022639, SEQ ID NO: 488) having a hairpin-like structure is known as a precursor of "hsa-miR-6794-5p".

The term "hsa-miR-6511a-5p gene" or "hsa-miR-6511a-5p" used herein includes the hsa-miR-6511a-5p gene (miRBase Accession No. MIMAT0025478) shown in SEQ ID NO: 228, a homolog or an ortholog thereof of a different organism species, and the like. The gene can be obtained by a method described in Joyce CE et al., 2011, Hum Mol Genet, Vol. 20, p. 4025-4040. Also, "hsa-mir-6511a-1, hsa-mir-6511a-2, hsa-mir-6511a-3 and hsa-mir-6511a-4" (miRBase Accession Nos. MI0022223, MI0023564, MI0023565 and MI0023566, SEQ ID NOs: 489, 490, 491 and 492) having a hairpin-like structure are known as a precursor of "hsa-miR-6511a-5p".

The term "hsa-miR-6824-5p gene" or "hsa-miR-6824-5p" used herein includes the hsa-miR-6824-5p gene (miRBase Accession No. MIMAT0027548) shown in SEQ ID NO: 229, a homolog or an ortholog thereof of a different organism species, and the like. The gene can be obtained by a method described in Ladewig E et al., 2012, Genome Res, Vol. 22, p. 1634-1645. Also, "hsa-mir-6824" (miRBase Accession No. MI0022669, SEQ ID NO: 493) having a hairpin-like structure is known as a precursor of "hsa-miR-6824-5p".

The term "hsa-miR-762 gene" or "hsa-miR-762" used herein includes the hsa-miR-762 gene (miRBase Accession No. MIMAT0010313) shown in SEQ ID NO: 230, a homolog or an ortholog thereof of a different organism species, and the like. The gene can be obtained by a method described in Berezikov E et al., 2006, Genome Res, Vol. 16, p. 1289-1298. Also, "hsa-mir-762" (miRBase Accession No. MI0003892, SEQ ID NO: 494) having a hairpin-like structure is known as a precursor of "hsa-miR-762".

The term "hsa-miR-6836-3p gene" or "hsa-miR-6836-3p" used herein includes the hsa-miR-6836-3p gene (miRBase Accession No. MIMAT0027575) shown in SEQ ID NO: 231, a homolog or an ortholog thereof of a different organism species, and the like. The gene can be obtained by a method described in Ladewig E et al., 2012, Genome Res, Vol. 22, p. 1634-1645. Also, "hsa-mir-6836" (miRBase Accession No. MI0022682, SEQ ID NO: 495) having a hairpin-like structure is known as a precursor of "hsa-miR-6836-3p".

The term "hsa-miR-6727-5p gene" or "hsa-miR-6727-5p" used herein includes the hsa-miR-6727-5p gene (miRBase Accession No. MIMAT0027355) shown in SEQ ID NO: 232, a homolog or an ortholog thereof of a different organism species, and the like. The gene can be obtained by a method described in Ladewig E et al., 2012, Genome Res, Vol. 22, p. 1634-1645. Also, "hsa-mir-6727" (miRBase Accession No. MI0022572, SEQ ID NO: 496) having a hairpin-like structure is known as a precursor of "hsa-miR-6727-5p".

The term "hsa-miR-4739 gene" or "hsa-miR-4739" used herein includes the hsa-miR-4739 gene (miRBase Accession No. MIMAT0019868) shown in SEQ ID NO: 233, a homolog or an ortholog thereof of a different organism species, and the like. The gene can be obtained by a method described in Persson H et al., 2011, Cancer Res, Vol. 71, p. 78-86. Also, "hsa-mir-4739" (miRBase Accession No. MI0017377, SEQ ID NO: 497) having a hairpin-like structure is known as a precursor of "hsa-miR-4739".

The term "hsa-miR-7977 gene" or "hsa-miR-7977" used herein includes the hsa-miR-7977 gene (miRBase Accession No. MIMAT0031180) shown in SEQ ID NO: 234, a homolog or an ortholog thereof of a different organism species, and the like. The gene can be obtained by a method described in Velthut-Meikas A et al., 2013, Mol Endocrinol, Vol. 27, p. 1128-1141. Also, "hsa-mir-7977" (miRBase Accession No. MI0025753, SEQ ID NO: 498) having a hairpin-like structure is known as a precursor of "hsa-miR-7977".

The term "hsa-miR-4484 gene" or "hsa-miR-4484" used herein includes the hsa-miR-4484 gene (miRBase Accession No. MIMAT0019018) shown in SEQ ID NO: 235, a homolog or an ortholog thereof of a different organism species, and the like. The gene can be obtained by a method described in Jima DD et al., 2010, Blood, Vol. 116, e118-e127. Also, "hsa-mir-4484" (miRBase Accession No. MI0016845, SEQ ID NO: 499) having a hairpin-like structure is known as a precursor of "hsa-miR-4484".

The term "hsa-miR-6515-3p gene" or "hsa-miR-6515-3p" used herein includes the hsa-miR-6515-3p gene (miRBase Accession No. MIMAT0025487) shown in SEQ ID NO: 236, a homolog or an ortholog thereof of a different organism species, and the like. The gene can be obtained by a method described in Joyce CE et al., 2011, Hum Mol Genet, Vol. 20, p. 4025-4040. Also, "hsa-mir-6515" (miRBase Accession No. MI0022227, SEQ ID NO: 500) having a hairpin-like structure is known as a precursor of "hsa-miR-6515-3p".

The term "hsa-miR-373-5p gene" or "hsa-miR-373-5p" used herein includes the hsa-miR-373-5p gene (miRBase Accession No. MIMAT0000725) shown in SEQ ID NO: 237, a homolog or an ortholog thereof of a different organism species, and the like. The gene can be obtained by a method described in Suh MR et al., 2004, Dev Biol, Vol. 270, p. 488-498. Also, "hsa-mir-373" (miRBase Accession No. MI0000781, SEQ ID NO: 501) having a hairpin-like structure is known as a precursor of "hsa-miR-373-5p".

The term "hsa-miR-4258 gene" or "hsa-miR-4258" used herein includes the hsa-miR-4258 gene (miRBase Accession No. MIMAT0016879) shown in SEQ ID NO: 238, a homolog or an ortholog thereof of a different organism species, and the like. The gene can be obtained by a method described in Goff LA et al., 2009, PLoS One, Vol. 4, e7192. Also, "hsa-mir-4258" (miRBase Accession No. MI0015857, SEQ ID NO: 502) having a hairpin-like structure is known as a precursor of "hsa-miR-4258".

The term "hsa-miR-4674 gene" or "hsa-miR-4674" used herein includes the hsa-miR-4674 gene (miRBase Accession No. MIMAT0019756) shown in SEQ ID NO: 239, a homolog or an ortholog thereof of a different organism species, and the like. The gene can be obtained by a method described in Persson H et al., 2011, Cancer Res, Vol. 71, p. 78-86. Also, "hsa-mir-4674" (miRBase Accession No. MI0017305, SEQ ID NO: 503) having a hairpin-like structure is known as a precursor of "hsa-miR-4674".

The term "hsa-miR-3180 gene" or "hsa-miR-3180" used herein includes the hsa-miR-3180 gene (miRBase Accession No. MIMAT0018178) shown in SEQ ID NO: 240, a homolog or an ortholog thereof of a different organism species, and the like. The gene can be obtained by a method described in Creighton CJ et al., 2010, PLoS One, Vol. 5, e9637. Also, "hsa-mir-3180-4 and hsa-mir-3180-5" (miRBase Accession Nos. MI0016408 and MI0016409, SEQ ID NOs: 504 and 505) having a hairpin-like structure are known as a precursor of "hsa-miR-3180".

The term "hsa-miR-6076 gene" or "hsa-miR-6076" used herein includes the hsa-miR-6076 gene (miRBase Accession No. MIMAT0023701) shown in SEQ ID NO: 241, a homolog or an ortholog thereof of a different organism species, and the like. The gene can be obtained by a method described in Voellenkle C et al., 2012, RNA, VOL. 18, p. 472-484. Also, "hsa-mir-6076" (miRBase Accession No. MI0020353, SEQ ID NO: 506) having a hairpin-like structure is known as a precursor of "hsa-miR-6076".

The term "hsa-miR-1238-5p gene" or "hsa-miR-1238-5p" used herein includes the hsa-miR-1238-5p gene (miRBase Accession No. MIMAT0022947) shown in SEQ ID NO: 242, a homolog or an ortholog thereof of a different organism species, and the like. The gene can be obtained by a method described in Berezikov E et al., 2007, Mol Cell, Vol. 28, p. 328-336. Also, "hsa-mir-1238" (miRBase Accession No. MI0006328, SEQ ID NO: 507) having a hairpin-like structure is known as a precursor of "hsa-miR-1238-5p".

The term "hsa-miR-4463 gene" or "hsa-miR-4463" used herein includes the hsa-miR-4463 gene (miRBase Accession No. MIMAT0018987) shown in SEQ ID NO: 243, a homolog or an ortholog thereof of a different organism species, and the like. The gene can be obtained by a method described in Jima DD et al., 2010, Blood, Vol. 116, e118-e127. Also, "hsa-mir-4463" (miRBase Accession No. MI0016811, SEQ ID NO: 508) having a hairpin-like structure is known as a precursor of "hsa-miR-4463".

The term "hsa-miR-4486 gene" or "hsa-miR-4486" used herein includes the hsa-miR-4486 gene (miRBase Accession No. MIMAT0019020) shown in SEQ ID NO: 244, a homolog or an ortholog thereof of a different organism species, and the like. The gene can be obtained by a method described in Jima DD et al., 2010, Blood, Vol. 116, e118-e127. Also, "hsa-mir-4486" (miRBase Accession No. MI0016847, SEQ ID NO: 509) having a hairpin-like structure is known as a precursor of "hsa-miR-4486".

The term "hsa-miR-4730 gene" or "hsa-miR-4730" used herein includes the hsa-miR-4730 gene (miRBase Accession No. MIMAT0019852) shown in SEQ ID NO: 245, a homolog or an ortholog thereof of a different organism species, and the like. The gene can be obtained by a method described in Persson H et al., 2011, Cancer Res, Vol. 71, p. 78-86. Also, "hsa-mir-4730" (miRBase Accession No. MI0017367, SEQ ID NO: 510) having a hairpin-like structure is known as a precursor of "hsa-miR-4730".

The term "hsa-miR-6766-3p gene" or "hsa-miR-6766-3p" used herein includes the hsa-miR-6766-3p gene (miRBase Accession No. MIMAT0027433) shown in SEQ ID NO: 246, a homolog or an ortholog thereof of a different organism species, and the like. The gene can be obtained by a method described in Ladewig E et al., 2012, Genome Res, Vol. 22, p. 1634-1645. Also, "hsa-mir-6766" (miRBase Accession No. MI0022611, SEQ ID NO: 511) having a hairpin-like structure is known as a precursor of "hsa-miR-6766-3p".

The term "hsa-miR-4286 gene" or "hsa-miR-4286" used herein includes the hsa-miR-4286 gene (miRBase Accession No. MIMAT0016916) shown in SEQ ID NO: 247, a homolog or an ortholog thereof of a different organism species, and the like. The gene can be obtained by a method described in Goff LA et al., 2009, PLoS One, Vol. 4, e7192. Also, "hsa-mir-4286" (miRBase Accession No. MI0015894, SEQ ID NO: 512) having a hairpin-like structure is known as a precursor of "hsa-miR-4286".

The term "hsa-miR-6511a-5p gene" or "hsa-miR-6511a-5p" used herein includes the hsa-miR-6511a-5p gene (miRBase Accession No. MIMAT0025478) shown in SEQ ID NO: 248, a homolog or an ortholog thereof of a different organism species, and the like. The gene can be obtained by a method described in Joyce CE et al., 2011, Hum Mol Genet, Vol. 20, p. 4025-4040. Also, "hsa-mir-6511a-1, hsa-mir-6511a-2, hsa-mir-6511a-3 and hsa-mir-6511a-4" (miRBase Accession Nos. MI0022223, MI0023564, MI0023565 and MI0023566, SEQ ID NOs: 513, 514, 515 and 516) having a hairpin-like structure are known as a precursor of "hsa-miR-6511a-5p".

The term "hsa-miR-4739 gene" or "hsa-miR-4739" used herein includes the hsa-miR-4739 gene (miRBase Accession No. MIMAT0019868) shown in SEQ ID NO: 249, a homolog or an ortholog thereof of a different organism species, and the like. The gene can be obtained by a method described in Persson H et al., 2011, Cancer Res, Vol. 71, p. 78-86. Also, "hsa-mir-4739" (miRBase Accession No. MI0017377, SEQ ID NO: 517) having a hairpin-like structure is known as a precursor of "hsa-miR-4739".

The term "hsa-miR-6749-5p gene" or "hsa-miR-6749-5p" used herein includes the hsa-miR-6749-5p gene (miRBase Accession No. MIMAT0027398) shown in SEQ ID NO: 250, a homolog or an ortholog thereof of a different organism species, and the like. The gene can be obtained by a method described in Ladewig E et al., 2012, Genome Res, Vol. 22, p. 1634-1645. Also, "hsa-mir-6749" (miRBase Accession No. MI0022594, SEQ ID NO: 518) having a hairpin-like structure is known as a precursor of "hsa-miR-6749-5p".

A mature miRNA may become a variant, due to a cleavage whereby the resulting sequence is shorter or longer by one to several flanking nucleotides, or due to substitution of nucleotides, when cut out as the mature miRNA from its RNA precursor having a hairpin-like structure. This variant is called isomiR (Morin RD. et al., 2008, Genome Res., Vol. 18, p. 610-621). The miRBase Release 20 shows the nucleotide sequences represented by SEQ ID NOs: 1 to 250 as well as a large number of the nucleotide sequence variants and fragments represented by SEQ ID NOs: 519 to 812, called isomiRs. These variants can also be obtained as miRNAs having a nucleotide sequence represented by any of SEQ ID NOs: 1 to 250. Specifically, among the variants of polynucleotides consisting of the nucleotide sequence represented by any of SEQ ID NOs: 2, 3, 6, 7, 8, 11, 12, 13, 15, 19, 20, 25, 26, 27, 29, 31, 32, 37, 44, 45, 46, 47, 48, 49, 51, 52, 53, 54, 55, 56, 57, 59, 60, 61, 62, 63, 71, 72, 73, 74, 76, 77, 78, 79, 83, 84, 86, 87, 88, 89, 90, 92, 94, 98, 102, 105, 106, 108, 111, 112, 113, 114, 116, 117, 118, 122, 123, 124, 128, 129, 133, 134, 135, 136, 137, 140, 141, 145, 146, 147, 148, 149, 150, 151, 153, 154, 155, 157, 158, 159, 160, 161, 162, 163, 166, 167, 168, 169, 176, 177, 178, 179, 180, 181, 182, 184, 186, 187, 190, 193, 196, 198, 201, 202, 203, 204, 205, 206, 207, 208, 209, 210, 211, 214, 215, 216, 217, 218, 220, 221, 222, 223, 224, 226, 228, 233, 235, 236, 237, 239, 240, 243, 244, 245, 247, 248, and 249 or a nucleotide sequence derived from the nucleotide sequence by the replacement of u with t according to the present invention, examples of the longest variants registered in miRBase Release 20 include polynucleotides represented by SEQ ID NOs: 519, 521, 523, 525, 527, 529, 531, 533, 535, 537, 539, 541, 543, 545, 547, 549, 551, 553, 555, 557, 559, 561, 563, 565, 567, 569, 571, 573, 575, 577, 579, 581, 583, 585, 587, 589, 591, 593, 595, 597, 599, 601, 603, 605, 607, 609, 611, 613, 615, 617, 619, 621, 623, 625, 627, 629, 631, 633, 635, 637, 639, 641, 643, 645, 647, 649, 651, 653, 655, 657, 659, 661, 663, 665, 667, 669, 671, 673, 675, 677, 679, 681, 683, 685, 687, 689, 691, 693, 695, 697, 699, 701, 703, 705, 707, 709, 711, 713, 715, 717, 719, 721, 723, 725, 727, 729, 731, 733, 735, 737, 739, 741, 743, 745, 747, 749, 751, 753, 755, 757, 759, 761, 763, 765, 767, 769, 771, 773, 775, 777, 779, 781, 783, 785, 787, 789, 791, 793, 795, 797, 799, 801, 803, 805, 807, 809, and 811, respectively. Also, among the variants of polynucleotides consisting of a nucleotide sequence represented by any of SEQ ID NOs: 2, 3, 6, 7, 8, 11, 12, 13, 15, 19, 20, 25, 26, 27, 29, 31, 32, 37, 44, 45, 46, 47, 48, 49, 51, 52, 53, 54, 55, 56, 57, 59, 60, 61, 62, 63, 71, 72, 73, 74, 76, 77, 78, 79, 83, 84, 86, 87, 88, 89, 90, 92, 94, 98, 102, 105, 106, 108, 111, 112, 113, 114, 116, 117, 118, 122, 123, 124, 128, 129, 133, 134, 135, 136, 137, 140, 141, 145, 146, 147, 148, 149, 150, 151, 153, 154, 155, 157, 158, 159, 160, 161, 162, 163, 166, 167, 168, 169, 176, 177, 178, 179, 180, 181, 182, 184, 186, 187, 190, 193, 196, 198, 201, 202, 203, 204, 205, 206, 207, 208, 209, 210, 211, 214, 215, 216, 217, 218, 220, 221, 222, 223, 224, 226, 228, 233, 235, 236, 237, 239, 240, 243, 244, 245, 247, 248, and 249 or a nucleotide sequence derived from the nucleotide sequence by the replacement of u with t according to the present invention, examples of shortest variants registered in the miRBase Release 20 include polynucleotides having sequences represented by SEQ ID NOs: 520, 522, 524, 526, 528, 530, 532, 534, 536, 538, 540, 542, 544, 546, 548, 550, 552, 554, 556, 558, 560, 562, 564, 566, 568, 570, 572, 574, 576, 578, 580, 582, 584, 586, 588, 590, 592, 594, 596, 598, 600, 602, 604, 606, 608, 610, 612, 614, 616, 618, 620, 622, 624, 626, 628, 630, 632, 634, 636, 638, 640, 642, 644, 646, 648, 650, 652, 654, 656, 658, 660, 662, 664, 666, 668, 670, 672, 674, 676, 678, 680, 682, 684, 686, 688, 690, 692, 694, 696, 698, 700, 702, 704, 706, 708, 710, 712, 714, 716, 718, 720, 722, 724, 726, 728, 730, 732, 734, 736, 738, 740, 742, 744, 746, 748, 750, 752, 754, 756, 758, 760, 762, 764, 766, 768, 770, 772, 774, 776, 778, 780, 782, 784, 786, 788, 790, 792, 794, 796, 798, 800, 802, 804, 806, 808, 810, and 812, respectively. In addition to these variants and fragments, examples thereof include a large number of isomiR polynucleotides of SEQ ID NOs: 2, 3, 6, 7, 8, 11, 12, 13, 15, 19, 20, 25, 26, 27, 29, 31, 32, 37, 44, 45, 46, 47, 48, 49, 51, 52, 53, 54, 55, 56, 57, 59, 60, 61, 62, 63, 71, 72, 73, 74, 76, 77, 78, 79, 83, 84, 86, 87, 88, 89, 90, 92, 94, 98, 102, 105, 106, 108, 111, 112, 113, 114, 116, 117, 118, 122, 123, 124, 128, 129, 133, 134, 135, 136, 137, 140, 141, 145, 146, 147, 148, 149, 150, 151, 153, 154, 155, 157, 158, 159, 160, 161, 162, 163, 166, 167, 168, 169, 176, 177, 178, 179, 180, 181, 182, 184, 186, 187, 190, 193, 196, 198, 201, 202, 203, 204, 205, 206, 207, 208, 209, 210, 211, 214, 215, 216, 217, 218, 220, 221, 222, 223, 224, 226, 228, 233, 235, 236, 237, 239, 240, 243, 244, 245, 247, 248, and 249 registered in the miRBase. Examples of the polynucleotide comprising a nucleotide sequence represented by any of SEQ ID NOs: 1 to 250 include a polynucleotide represented by any of SEQ ID NOs: 251 to 518, which are their respective precursors.

The names and miRBase Accession Nos. (registration numbers) of the genes represented by SEQ ID NOs: 1 to 812 are shown in Table 1.

As used herein, the term "capable of specifically binding" means that the nucleic acid probe or the primer used in the present invention binds to a particular target nucleic acid and cannot substantially bind to other nucleic acids.

**[Table 1]**

| SEQ ID NO: | Gene name | miRBase registration No. |
|---|---|---|
| 1 | hsa-miR-6784-5p | MIMAT0027468 |
| 2 | hsa-miR-1181 | MIMAT0005826 |
| 3 | hsa-miR-671-5p | MIMAT0003880 |
| 4 | hsa-miR-6857-5p | MIMAT0027614 |
| 5 | hsa-miR-4276 | MIMAT0016904 |
| 6 | hsa-miR-1914-3p | MIMAT0007890 |
| 7 | hsa-miR-149-3p | MIMAT0004609 |
| 8 | hsa-miR-937-5p | MIMAT0022938 |
| 9 | hsa-miR-4675 | MIMAT0019757 |
| 10 | hsa-miR-6795-5p | MIMAT0027490 |
| 11 | hsa-miR-4731-5p | MIMAT0019853 |
| 12 | hsa-miR-5090 | MIMAT0021082 |
| 13 | hsa-miR-3620-5p | MIMAT0022967 |
| 14 | hsa-miR-1343-5p | MIMAT0027038 |
| 15 | hsa-miR-6717-5p | MIMAT0025846 |
| 16 | hsa-miR-6825-5p | MIMAT0027550 |
| 17 | hsa-miR-6738-5p | MIMAT0027377 |
| 18 | hsa-miR-6769a-5p | MIMAT0027438 |
| 19 | hsa-miR-4728-5p | MIMAT0019849 |
| 20 | hsa-miR-652-5p | MIMAT0022709 |
| 21 | hsa-miR-4257 | MIMAT0016878 |
| 22 | hsa-miR-6785-5p | MIMAT0027470 |
| 23 | hsa-miR-7110-5p | MIMAT0028117 |
| 24 | hsa-miR-6887-5p | MIMAT0027674 |
| 25 | hsa-miR-887-3p | MIMAT0004951 |
| 26 | hsa-miR-1228-5p | MIMAT0005582 |
| 27 | hsa-miR-5572 | MIMAT0022260 |
| 28 | hsa-miR-6782-5p | MIMAT0027464 |
| 29 | hsa-miR-4298 | MIMAT0016852 |
| 30 | hsa-miR-6786-5p | MIMAT0027472 |
| 31 | hsa-miR-5010-5p | MIMAT0021043 |
| 32 | hsa-miR-6087 | MIMAT0023712 |
| 33 | hsa-miR-6765-5p | MIMAT0027430 |
| 34 | hsa-miR-6732-5p | MIMAT0027365 |
| 35 | hsa-miR-6787-5p | MIMAT0027474 |
| 36 | hsa-miR-6737-5p | MIMAT0027375 |
| 37 | hsa-miR-128-2-5p | MIMAT0031095 |
| 38 | hsa-miR-4270 | MIMAT0016900 |
| 39 | hsa-miR-6861-5p | MIMAT0027623 |
| 40 | hsa-miR-6756-5p | MIMAT0027412 |
| 41 | hsa-miR-1229-5p | MIMAT0022942 |
| 42 | hsa-miR-6891-5p | MIMAT0027682 |
| 43 | hsa-miR-6848-5p | MIMAT0027596 |
| 44 | hsa-miR-1237-5p | MIMAT0022946 |
| 45 | hsa-miR-30c-1-3p | MIMAT0004674 |
| 46 | hsa-miR-1233-5p | MIMAT0022943 |
| 47 | hsa-miR-211-3p | MIMAT0022694 |
| 48 | hsa-miR-4758-5p | MIMAT0019903 |
| 49 | hsa-miR-614 | MIMAT0003282 |
| 50 | hsa-miR-6746-5p | MIMAT0027392 |
| 51 | hsa-miR-1915-5p | MIMAT0007891 |
| 52 | hsa-miR-4688 | MIMAT0019777 |
| 53 | hsa-miR-3917 | MIMAT0018191 |
| 54 | hsa-miR-5787 | MIMAT0023252 |
| 55 | hsa-miR-4632-5p | MIMAT0022977 |
| 56 | hsa-miR-6126 | MIMAT0024599 |
| 57 | hsa-miR-135a-3p | MIMAT0004595 |
| 58 | hsa-miR-8063 | MIMAT0030990 |
| 59 | hsa-miR-5698 | MIMAT0022491 |
| 60 | hsa-miR-6089 | MIMAT0023714 |
| 61 | hsa-miR-498 | MIMAT0002824 |
| 62 | hsa-miR-296-3p | MIMAT0004679 |
| 63 | hsa-miR-4419b | MIMAT0019034 |
| 64 | hsa-miR-6802-5p | MIMAT0027504 |
| 65 | hsa-miR-6829-5p | MIMAT0027558 |
| 66 | hsa-miR-6803-5p | MIMAT0027506 |
| 67 | hsa-miR-1199-5p | MIMAT0031119 |
| 68 | hsa-miR-6840-3p | MIMAT0027583 |
| 69 | hsa-miR-6752-5p | MIMAT0027404 |
| 70 | hsa-miR-6798-5p | MIMAT0027496 |
| 71 | hsa-miR-6131 | MIMAT0024615 |
| 72 | hsa-miR-4667-5p | MIMAT0019743 |
| 73 | hsa-miR-6510-5p | MIMAT0025476 |
| 74 | hsa-miR-4690-5p | MIMAT0019779 |
| 75 | hsa-miR-920 | MIMAT0004970 |
| 76 | hsa-miR-23b-3p | MIMAT0000418 |
| 77 | hsa-miR-4448 | MIMAT0018967 |
| 78 | hsa-miR-2110 | MIMAT0010133 |
| 79 | hsa-miR-4706 | MIMAT0019806 |
| 80 | hsa-miR-7845-5p | MIMAT0030420 |
| 81 | hsa-miR-6808-5p | MIMAT0027516 |
| 82 | hsa-miR-4447 | MIMAT0018966 |
| 83 | hsa-miR-6869-5p | MIMAT0027638 |
| 84 | hsa-miR-1908-5p | MIMAT0007881 |
| 85 | hsa-miR-6729-5p | MIMAT0027359 |
| 86 | hsa-miR-5195-3p | MIMAT0021127 |
| 87 | hsa-miR-638 | MIMAT0003308 |
| 88 | hsa-miR-6125 | MIMAT0024598 |
| 89 | hsa-miR-3178 | MIMAT0015055 |
| 90 | hsa-miR-3196 | MIMAT0015080 |
| 91 | hsa-miR-8069 | MIMAT0030996 |
| 92 | hsa-miR-4723-5p | MIMAT0019838 |
| 93 | hsa-miR-4746-3p | MIMAT0019881 |
| 94 | hsa-miR-4689 | MIMAT0019778 |
| 95 | hsa-miR-6816-5p | MIMAT0027532 |
| 96 | hsa-miR-6757-5p | MIMAT0027414 |
| 97 | hsa-miR-7109-5p | MIMAT0028115 |
| 98 | hsa-miR-6724-5p | MIMAT0025856 |
| 99 | hsa-miR-1225-3p | MIMAT0005573 |
| 100 | hsa-miR-6875-5p | MIMAT0027650 |
| 101 | hsa-miR-7108-5p | MIMAT0028113 |
| 102 | hsa-miR-4508 | MIMAT0019045 |
| 103 | hsa-miR-6085 | MIMAT0023710 |
| 104 | hsa-miR-6779-5p | MIMAT0027458 |
| 105 | hsa-miR-642a-3p | MIMAT0020924 |
| 106 | hsa-miR-4695-5p | MIMAT0019788 |
| 107 | hsa-miR-7847-3p | MIMAT0030422 |
| 108 | hsa-miR-3197 | MIMAT0015082 |
| 109 | hsa-miR-6769b-5p | MIMAT0027620 |
| 110 | hsa-miR-7641 | MIMAT0029782 |
| 111 | hsa-miR-187-5p | MIMAT0004561 |
| 112 | hsa-miR-3185 | MIMAT0015065 |
| 113 | hsa-miR-2861 | MIMAT0013802 |
| 114 | hsa-miR-3940-5p | MIMAT0019229 |
| 115 | hsa-miR-1203 | MIMAT0005866 |
| 116 | hsa-miR-615-5p | MIMAT0004804 |
| 117 | hsa-miR-4787-5p | MIMAT0019956 |
| 118 | hsa-miR-1343-3p | MIMAT0019776 |
| 119 | hsa-miR-6813-5p | MIMAT0027526 |
| 120 | hsa-miR-1225-5p | MIMAT0005572 |
| 121 | hsa-miR-602 | MIMAT0003270 |
| 122 | hsa-miR-4488 | MIMAT0019022 |
| 123 | hsa-miR-125a-3p | MIMAT0004602 |
| 124 | hsa-miR-5100 | MIMAT0022259 |
| 125 | hsa-miR-4294 | MIMAT0016849 |
| 126 | hsa-miR-1231 | MIMAT0005586 |
| 127 | hsa-miR-6765-3p | MIMAT0027431 |
| 128 | hsa-miR-4442 | MIMAT0018960 |
| 129 | hsa-miR-718 | MIMAT0012735 |
| 130 | hsa-miR-6780b-5p | MIMAT0027572 |
| 131 | hsa-miR-6090 | MIMAT0023715 |
| 132 | hsa-miR-6845-5p | MIMAT0027590 |
| 133 | hsa-miR-4741 | MIMAT0019871 |
| 134 | hsa-miR-4467 | MIMAT0018994 |
| 135 | hsa-miR-4707-5p | MIMAT0019807 |
| 136 | hsa-miR-4271 | MIMAT0016901 |
| 137 | hsa-miR-4673 | MIMAT0019755 |
| 138 | hsa-miR-3184-5p | MIMAT0015064 |
| 139 | hsa-miR-1469 | MIMAT0007347 |
| 140 | hsa-miR-4640-5p | MIMAT0019699 |
| 141 | hsa-miR-663a | MIMAT0003326 |
| 142 | hsa-miR-6791-5p | MIMAT0027482 |
| 143 | hsa-miR-6826-5p | MIMAT0027552 |
| 144 | hsa-miR-4433b-3p | MIMAT0030414 |
| 145 | hsa-miR-1915-3p | MIMAT0007892 |
| 146 | hsa-miR-4417 | MIMAT0018929 |
| 147 | hsa-miR-4449 | MIMAT0018968 |
| 148 | hsa-miR-4707-3p | MIMAT0019808 |
| 149 | hsa-miR-3180-3p | MIMAT0015058 |
| 150 | hsa-miR-5585-3p | MIMAT0022286 |
| 151 | hsa-miR-1268a | MIMAT0005922 |
| 152 | hsa-miR-8072 | MIMAT0030999 |
| 153 | hsa-miR-296-5p | MIMAT0000690 |
| 154 | hsa-miR-204-3p | MIMAT0022693 |
| 155 | hsa-miR-4454 | MIMAT0018976 |
| 156 | hsa-miR-6722-3p | MIMAT0025854 |
| 157 | hsa-miR-1290 | MIMAT0005880 |
| 158 | hsa-miR-3622a-5p | MIMAT0018003 |
| 159 | hsa-miR-939-5p | MIMAT0004982 |
| 160 | hsa-miR-675-5p | MIMAT0004284 |
| 161 | hsa-miR-3131 | MIMAT0014996 |
| 162 | hsa-miR-4648 | MIMAT0019710 |
| 163 | hsa-miR-1268b | MIMAT0018925 |
| 164 | hsa-miR-6741-5p | MIMAT0027383 |
| 165 | hsa-miR-6893-5p | MIMAT0027686 |
| 166 | hsa-miR-3162-5p | MIMAT0015036 |
| 167 | hsa-miR-642b-3p | MIMAT0018444 |
| 168 | hsa-miR-4734 | MIMAT0019859 |
| 169 | hsa-miR-150-3p | MIMAT0004610 |
| 170 | hsa-miR-8089 | MIMAT0031016 |
| 171 | hsa-miR-6805-3p | MIMAT0027511 |
| 172 | hsa-miR-7113-3p | MIMAT0028124 |
| 173 | hsa-miR-6850-5p | MIMAT0027600 |
| 174 | hsa-miR-6799-5p | MIMAT0027498 |
| 175 | hsa-miR-6768-5p | MIMAT0027436 |
| 176 | hsa-miR-92b-5p | MIMAT0004792 |
| 177 | hsa-miR-3679-5p | MIMAT0018104 |
| 178 | hsa-miR-4792 | MIMAT0019964 |
| 179 | hsa-miR-3656 | MIMAT0018076 |
| 180 | hsa-miR-92a-2-5p | MIMAT0004508 |
| 181 | hsa-miR-4466 | MIMAT0018993 |
| 182 | hsa-miR-4513 | MIMAT0019050 |
| 183 | hsa-miR-6781-5p | MIMAT0027462 |
| 184 | hsa-miR-4649-5p | MIMAT0019711 |
| 185 | hsa-miR-6775-5p | MIMAT0027450 |
| 186 | hsa-miR-4651 | MIMAT0019715 |
| 187 | hsa-miR-3195 | MIMAT0015079 |
| 188 | hsa-miR-6726-5p | MIMAT0027353 |
| 189 | hsa-miR-6872-3p | MIMAT0027645 |
| 190 | hsa-miR-371a-5p | MIMAT0004687 |
| 191 | hsa-miR-6777-5p | MIMAT0027454 |
| 192 | hsa-miR-6789-5p | MIMAT0027478 |
| 193 | hsa-miR-7975 | MIMAT0031178 |
| 194 | hsa-miR-6821-5p | MIMAT0027542 |
| 195 | hsa-miR-4534 | MIMAT0019073 |
| 196 | hsa-miR-619-5p | MIMAT0026622 |
| 197 | hsa-miR-7107-5p | MIMAT0028111 |
| 198 | hsa-miR-1228-3p | MIMAT0005583 |
| 199 | hsa-miR-6774-5p | MIMAT0027448 |
| 200 | hsa-miR-6805-5p | MIMAT0027510 |
| 201 | hsa-miR-23a-3p | MIMAT0000078 |
| 202 | hsa-miR-4665-5p | MIMAT0019739 |
| 203 | hsa-miR-4505 | MIMAT0019041 |
| 204 | hsa-miR-4638-5p | MIMAT0019695 |
| 205 | hsa-miR-24-3p | MIMAT0000080 |
| 206 | hsa-miR-3135b | MIMAT0018985 |
| 207 | hsa-miR-4745-5p | MIMAT0019878 |
| 208 | hsa-miR-128-1-5p | MIMAT0026477 |
| 209 | hsa-miR-4476 | MIMAT0019003 |
| 210 | hsa-miR-4687-3p | MIMAT0019775 |
| 211 | hsa-miR-3665 | MIMAT0018087 |
| 212 | hsa-miR-6806-5p | MIMAT0027512 |
| 213 | hsa-miR-3937 | MIMAT0018352 |
| 214 | hsa-miR-711 | MIMAT0012734 |
| 215 | hsa-miR-3141 | MIMAT0015010 |
| 216 | hsa-miR-3188 | MIMAT0015070 |
| 217 | hsa-miR-4281 | MIMAT0016907 |
| 218 | hsa-miR-5196-5p | MIMAT0021128 |
| 219 | hsa-miR-6880-5p | MIMAT0027660 |
| 220 | hsa-miR-3960 | MIMAT0019337 |
| 221 | hsa-miR-3648 | MIMAT0018068 |
| 222 | hsa-miR-6721-5p | MIMAT0025852 |
| 223 | hsa-miR-4492 | MIMAT0019027 |
| 224 | hsa-miR-744-5p | MIMAT0004945 |
| 225 | hsa-miR-7704 | MIMAT0030019 |
| 226 | hsa-miR-4749-5p | MIMAT0019885 |
| 227 | hsa-miR-6794-5p | MIMAT0027488 |
| 228 | hsa-miR-6511a-5p | MIMAT0025478 |
| 229 | hsa-miR-6824-5p | MIMAT0027548 |
| 230 | hsa-miR-762 | MIMAT0010313 |
| 231 | hsa-miR-6836-3p | MIMAT0027575 |
| 232 | hsa-miR-6727-5p | MIMAT0027355 |
| 233 | hsa-miR-4739 | MIMAT0019868 |
| 234 | hsa-miR-7977 | MIMAT0031180 |
| 235 | hsa-miR-4484 | MIMAT0019018 |
| 236 | hsa-miR-6515-3p | MIMAT0025487 |
| 237 | hsa-miR-373-5p | MIMAT0000725 |
| 238 | hsa-miR-4258 | MIMAT0016879 |
| 239 | hsa-miR-4674 | MIMAT0019756 |
| 240 | hsa-miR-3180 | MIMAT0018178 |
| 241 | hsa-miR-6076 | MIMAT0023701 |
| 242 | hsa-miR-1238-5p | MIMAT0022947 |
| 243 | hsa-miR-4463 | MIMAT0018987 |
| 244 | hsa-miR-4486 | MIMAT0019020 |
| 245 | hsa-miR-4730 | MIMAT0019852 |
| 246 | hsa-miR-6766-3p | MIMAT0027433 |
| 247 | hsa-miR-4286 | MIMAT0016916 |
| 248 | hsa-miR-6511a-5p | MIMAT0025478 |
| 249 | hsa-miR-4739 | MIMAT0019868 |
| 250 | hsa-miR-6749-5p | MIMAT0027398 |
| 251 | hsa-mir-6784 | MI0022629 |
| 252 | hsa-mir-1181 | MI0006274 |
| 253 | hsa-mir-671 | MI0003760 |
| 254 | hsa-mir-6857 | MI0022703 |
| 255 | hsa-mir-4276 | MI0015882 |
| 256 | hsa-mir-1914 | MI0008335 |
| 257 | hsa-mir-149 | MI0000478 |
| 258 | hsa-mir-937 | MI0005759 |
| 259 | hsa-mir-4675 | MI0017306 |
| 260 | hsa-mir-6795 | MI0022640 |
| 261 | hsa-mir-4731 | MI0017368 |
| 262 | hsa-mir-5090 | MI0017979 |
| 263 | hsa-mir-3620 | MI0016011 |
| 264 | hsa-mir-1343 | MI0017320 |
| 265 | hsa-mir-6717 | MI0022551 |
| 266 | hsa-mir-6825 | MI0022670 |
| 267 | hsa-mir-6738 | MI0022583 |
| 268 | hsa-mir-6769a | MI0022614 |
| 269 | hsa-mir-4728 | MI0017365 |
| 270 | hsa-mir-652 | MI0003667 |
| 271 | hsa-mir-4257 | MI0015856 |
| 272 | hsa-mir-6785 | MI0022630 |
| 273 | hsa-mir-7110 | MI0022961 |
| 274 | hsa-mir-6887 | MI0022734 |
| 275 | hsa-mir-887 | MI0005562 |
| 276 | hsa-mir-1228 | MI0006318 |
| 277 | hsa-mir-5572 | MI0019117 |
| 278 | hsa-mir-6782 | MI0022627 |
| 279 | hsa-mir-4298 | MI0015830 |
| 280 | hsa-mir-6786 | MI0022631 |
| 281 | hsa-mir-5010 | MI0017878 |
| 282 | hsa-mir-6087 | MI0020364 |
| 283 | hsa-mir-6765 | MI0022610 |
| 284 | hsa-mir-6732 | MI0022577 |
| 285 | hsa-mir-6787 | MI0022632 |
| 286 | hsa-mir-6737 | MI0022582 |
| 287 | hsa-mir-128-2 | MI0000727 |
| 288 | hsa-mir-4270 | MI0015878 |
| 289 | hsa-mir-6861 | MI0022708 |
| 290 | hsa-mir-6756 | MI0022601 |
| 291 | hsa-mir-1229 | MI0006319 |
| 292 | hsa-mir-6891 | MI0022738 |
| 293 | hsa-mir-6848 | MI0022694 |
| 294 | hsa-mir-1237 | MI0006327 |
| 295 | hsa-mir-30c-1 | MI0000736 |
| 296 | hsa-mir-1233-1 | MI0006323 |
| 297 | hsa-mir-1233-2 | MI0015973 |
| 298 | hsa-mir-211 | MI0000287 |
| 299 | hsa-mir-4758 | MI0017399 |
| 300 | hsa-mir-614 | MI0003627 |
| 301 | hsa-mir-6746 | MI0022591 |
| 302 | hsa-mir-1915 | MI0008336 |
| 303 | hsa-mir-4688 | MI0017321 |
| 304 | hsa-mir-3917 | MI0016423 |
| 305 | hsa-mir-5787 | MI0019797 |
| 306 | hsa-mir-4632 | MI0017259 |
| 307 | hsa-mir-6126 | MI0021260 |
| 308 | hsa-mir-135a-1 | MI0000452 |
| 309 | hsa-mir-8063 | MI0025899 |
| 310 | hsa-mir-5698 | MI0019305 |
| 311 | hsa-mir-6089-1 | MI0020366 |
| 312 | hsa-mir-6089-2 | MI0023563 |
| 313 | hsa-mir-498 | MI0003142 |
| 314 | hsa-mir-296 | MI0000747 |
| 315 | hsa-mir-4419b | MI0016861 |
| 316 | hsa-mir-6802 | MI0022647 |
| 317 | hsa-mir-6829 | MI0022674 |
| 318 | hsa-mir-6803 | MI0022648 |
| 319 | hsa-mir-1199 | MI0020340 |
| 320 | hsa-mir-6840 | MI0022686 |
| 321 | hsa-mir-6752 | MI0022597 |
| 322 | hsa-mir-6798 | MI0022643 |
| 323 | hsa-mir-6131 | MI0021276 |
| 324 | hsa-mir-4667 | MI0017297 |
| 325 | hsa-mir-6510 | MI0022222 |
| 326 | hsa-mir-4690 | MI0017323 |
| 327 | hsa-mir-920 | MI0005712 |
| 328 | hsa-mir-23b | MI0000439 |
| 329 | hsa-mir-4448 | MI0016791 |
| 330 | hsa-mir-2110 | MI0010629 |
| 331 | hsa-mir-4706 | MI0017339 |
| 332 | hsa-mir-7845 | MI0025515 |
| 333 | hsa-mir-6808 | MI0022653 |
| 334 | hsa-mir-4447 | MI0016790 |
| 335 | hsa-mir-6869 | MI0022716 |
| 336 | hsa-mir-1908 | MI0008329 |
| 337 | hsa-mir-6729 | MI0022574 |
| 338 | hsa-mir-5195 | MI0018174 |
| 339 | hsa-mir-638 | MI0003653 |
| 340 | hsa-mir-6125 | MI0021259 |
| 341 | hsa-mir-3178 | MI0014212 |
| 342 | hsa-mir-3196 | MI0014241 |
| 343 | hsa-mir-8069-1 | MI0025905 |
| 344 | hsa-mir-8069-2 | MI0031519 |
| 345 | hsa-mir-4723 | MI0017359 |
| 346 | hsa-mir-4746 | MI0017385 |
| 347 | hsa-mir-4689 | MI0017322 |
| 348 | hsa-mir-6816 | MI0022661 |
| 349 | hsa-mir-6757 | MI0022602 |
| 350 | hsa-mir-7109 | MI0022960 |
| 351 | hsa-mir-6724-1 | MI0022559 |
| 352 | hsa-mir-6724-2 | MI0031516 |
| 353 | hsa-mir-6724-3 | MI0031517 |
| 354 | hsa-mir-6724-4 | MI0031518 |
| 355 | hsa-mir-1225 | MI0006311 |
| 356 | hsa-mir-6875 | MI0022722 |
| 357 | hsa-mir-7108 | MI0022959 |
| 358 | hsa-mir-4508 | MI0016872 |
| 359 | hsa-mir-6085 | MI0020362 |
| 360 | hsa-mir-6779 | MI0022624 |
| 361 | hsa-mir-642a | MI0003657 |
| 362 | hsa-mir-4695 | MI0017328 |
| 363 | hsa-mir-7847 | MI0025517 |
| 364 | hsa-mir-3197 | MI0014245 |
| 365 | hsa-mir-6769b | MI0022706 |
| 366 | hsa-mir-7641-1 | MI0024975 |
| 367 | hsa-mir-7641-2 | MI0024976 |
| 368 | hsa-mir-187 | MI0000274 |
| 369 | hsa-mir-3185 | MI0014227 |
| 370 | hsa-mir-2861 | MI0013006 |
| 371 | hsa-mir-3940 | MI0016597 |
| 372 | hsa-mir-1203 | MI0006335 |
| 373 | hsa-mir-615 | MI0003628 |
| 374 | hsa-mir-4787 | MI0017434 |
| 375 | hsa-mir-1343 | MI0017320 |
| 376 | hsa-mir-6813 | MI0022658 |
| 377 | hsa-mir-1225 | MI0006311 |
| 378 | hsa-mir-602 | MI0003615 |
| 379 | hsa-mir-4488 | MI0016849 |
| 380 | hsa-mir-125a | MI0000469 |
| 381 | hsa-mir-5100 | MI0019116 |
| 382 | hsa-mir-4294 | MI0015827 |
| 383 | hsa-mir-1231 | MI0006321 |
| 384 | hsa-mir-6765 | MI0022610 |
| 385 | hsa-mir-4442 | MI0016785 |
| 386 | hsa-mir-718 | MI0012489 |
| 387 | hsa-mir-6780b | MI0022681 |
| 388 | hsa-mir-6090 | MI0020367 |
| 389 | hsa-mir-6845 | MI0022691 |
| 390 | hsa-mir-4741 | MI0017379 |
| 391 | hsa-mir-4467 | MI0016818 |
| 392 | hsa-mir-4707 | MI0017340 |
| 393 | hsa-mir-4271 | MI0015879 |
| 394 | hsa-mir-4673 | MI0017304 |
| 395 | hsa-mir-3184 | MI0014226 |
| 396 | hsa-mir-1469 | MI0007074 |
| 397 | hsa-mir-4640 | MI0017267 |
| 398 | hsa-mir-663a | M10003672 |
| 399 | hsa-mir-6791 | MI0022636 |
| 400 | hsa-mir-6826 | MI0022671 |
| 401 | hsa-mir-4433b | MI0025511 |
| 402 | hsa-mir-1915 | MI0008336 |
| 403 | hsa-mir-4417 | MI0016753 |
| 404 | hsa-mir-4449 | MI0016792 |
| 405 | hsa-mir-4707 | MI0017340 |
| 406 | hsa-mir-3180-1 | MI0014214 |
| 407 | hsa-mir-3180-2 | MI0014215 |
| 408 | hsa-mir-3180-3 | MI0014217 |
| 409 | hsa-mir-5585 | MI0019142 |
| 410 | hsa-mir-1268a | MI0006405 |
| 411 | hsa-mir-8072 | MI0025908 |
| 412 | hsa-mir-296 | MI0000747 |
| 413 | hsa-mir-204 | MI0000284 |
| 414 | hsa-mir-4454 | MI0016800 |
| 415 | hsa-mir-6722 | MI0022557 |
| 416 | hsa-mir-1290 | MI0006352 |
| 417 | hsa-mir-3622a | MI0016013 |
| 418 | hsa-mir-939 | MI0005761 |
| 419 | hsa-mir-675 | MI0005416 |
| 420 | hsa-mir-3131 | MI0014151 |
| 421 | hsa-mir-4648 | MI0017275 |
| 422 | hsa-mir-1268b | MI0016748 |
| 423 | hsa-mir-6741 | MI0022586 |
| 424 | hsa-mir-6893 | MI0022740 |
| 425 | hsa-mir-3162 | MI0014192 |
| 426 | hsa-mir-642b | MI0016685 |
| 427 | hsa-mir-4734 | MI0017371 |
| 428 | hsa-mir-150 | MI0000479 |
| 429 | hsa-mir-8089 | MI0025925 |
| 430 | hsa-mir-6805 | MI0022650 |
| 431 | hsa-mir-7113 | MI0022964 |
| 432 | hsa-mir-6850 | MI0022696 |
| 433 | hsa-mir-6799 | MI0022644 |
| 434 | hsa-mir-6768 | MI0022613 |
| 435 | hsa-mir-92b | MI0003560 |
| 436 | hsa-mir-3679 | MI0016080 |
| 437 | hsa-mir-4792 | MI0017439 |
| 438 | hsa-mir-3656 | MI0016056 |
| 439 | hsa-mir-92a-2 | MI0000094 |
| 440 | hsa-mir-4466 | MI0016817 |
| 441 | hsa-mir-4513 | MI0016879 |
| 442 | hsa-mir-6781 | MI0022626 |
| 443 | hsa-mir-4649 | MI0017276 |
| 444 | hsa-mir-6775 | MI0022620 |
| 445 | hsa-mir-4651 | MI0017279 |
| 446 | hsa-mir-3195 | MI0014240 |
| 447 | hsa-mir-6726 | MI0022571 |
| 448 | hsa-mir-6872 | MI0022719 |
| 449 | hsa-mir-371a | MI0000779 |
| 450 | hsa-mir-6777 | MI0022622 |
| 451 | hsa-mir-6789 | MI0022634 |
| 452 | hsa-mir-7975 | MI0025751 |
| 453 | hsa-mir-6821 | MI0022666 |
| 454 | hsa-mir-4534 | MI0016901 |
| 455 | hsa-mir-619 | MI0003633 |
| 456 | hsa-mir-7107 | MI0022958 |
| 457 | hsa-mir-1228 | MI0006318 |
| 458 | hsa-mir-6774 | MI0022619 |
| 459 | hsa-mir-6805 | MI0022650 |
| 460 | hsa-mir-23a | MI0000079 |
| 461 | hsa-mir-4665 | M10017295 |
| 462 | hsa-mir-4505 | MI0016868 |
| 463 | hsa-mir-4638 | MI0017265 |
| 464 | hsa-mir-24-1 | MI0000080 |
| 465 | hsa-mir-24-2 | MI0000081 |
| 466 | hsa-mir-3135b | MI0016809 |
| 467 | hsa-mir-4745 | MI0017384 |
| 468 | hsa-mir-128-1 | MI0000447 |
| 469 | hsa-mir-4476 | MI0016828 |
| 470 | hsa-mir-4687 | MI0017319 |
| 471 | hsa-mir-3665 | MI0016066 |
| 472 | hsa-mir-6806 | MI0022651 |
| 473 | hsa-mir-3937 | MI0016593 |
| 474 | hsa-mir-711 | MI0012488 |
| 475 | hsa-mir-3141 | MI0014165 |
| 476 | hsa-mir-3188 | MI0014232 |
| 477 | hsa-mir-4281 | MI0015885 |
| 478 | hsa-mir-5196 | MI0018175 |
| 479 | hsa-mir-6880 | MI0022727 |
| 480 | hsa-mir-3960 | MI0016964 |
| 481 | hsa-mir-3648-1 | MI0016048 |
| 482 | hsa-mir-3648-2 | MI0031512 |
| 483 | hsa-mir-6721 | MI0022556 |
| 484 | hsa-mir-4492 | MI0016854 |
| 485 | hsa-mir-744 | MI0005559 |
| 486 | hsa-mir-7704 | MI0025240 |
| 487 | hsa-mir-4749 | MI0017388 |
| 488 | hsa-mir-6794 | MI0022639 |
| 489 | hsa-mir-6511a-1 | MI0022223 |
| 490 | hsa-mir-6511a-2 | MI0023564 |
| 491 | hsa-mir-6511a-3 | MI0023565 |
| 492 | hsa-mir-6511a-4 | MI0023566 |
| 493 | hsa-mir-6824 | MI0022669 |
| 494 | hsa-mir-762 | MI0003892 |
| 495 | hsa-mir-6836 | MI0022682 |
| 496 | hsa-mir-6727 | MI0022572 |
| 497 | hsa-mir-4739 | MI0017377 |
| 498 | hsa-mir-7977 | MI0025753 |
| 499 | hsa-mir-4484 | MI0016845 |
| 500 | hsa-mir-6515 | MI0022227 |
| 501 | hsa-mir-373 | MI0000781 |
| 502 | hsa-mir-4258 | MI0015857 |
| 503 | hsa-mir-4674 | MI0017305 |
| 504 | hsa-mir-3180-4 | MI0016408 |
| 505 | hsa-mir-3180-5 | MI0016409 |
| 506 | hsa-mir-6076 | MI0020353 |
| 507 | hsa-mir-1238 | MI0006328 |
| 508 | hsa-mir-4463 | MI0016811 |
| 509 | hsa-mir-4486 | MI0016847 |
| 510 | hsa-mir-4730 | MI0017367 |
| 511 | hsa-mir-6766 | MI0022611 |
| 512 | hsa-mir-4286 | MI0015894 |
| 513 | hsa-mir-6511a-1 | MI0022223 |
| 514 | hsa-mir-6511a-2 | MI0023564 |
| 515 | hsa-mir-6511a-3 | MI0023565 |
| 516 | hsa-mir-6511a-4 | MI0023566 |
| 517 | hsa-mir-4739 | MI0017377 |
| 518 | hsa-mir-6749 | MI0022594 |
| 519 | isomiR example 1 of SEQ ID NO: 2 | - |
| 520 | isomiR example 2 of SEQ ID NO: 2 | - |
| 521 | isomiR example 1 of SEQ ID NO: 3 | - |
| 522 | isomiR example 2 of SEQ ID NO: 3 | - |
| 523 | isomiR example 1 of SEQ ID NO: 6 | - |
| 524 | isomiR example 2 of SEQ ID NO: 6 | - |
| 525 | isomiR example 1 of SEQ ID NO: 7 | - |
| 526 | isomiR example 2 of SEQ ID NO: 7 | - |
| 527 | isomiR example 1 of SEQ ID NO: 8 | - |
| 528 | isomiR example 2 of SEQ ID NO: 8 | - |
| 529 | isomiR example 1 of SEQ ID NO: 11 | - |
| 530 | isomiR example 2 of SEQ ID NO: 11 | - |
| 531 | isomiR example 1 of SEQ ID NO: 12 | - |
| 532 | isomiR example 2 of SEQ ID NO: 12 | - |
| 533 | isomiR example 1 of SEQ ID NO: 13 | - |
| 534 | isomiR example 2 of SEQ ID NO: 13 | - |
| 535 | isomiR example 1 of SEQ ID NO: 15 | - |
| 536 | isomiR example 2 of SEQ ID NO: 15 | - |
| 537 | isomiR example 1 of SEQ ID NO: 19 | - |
| 538 | isomiR example 2 of SEQ ID NO: 19 | - |
| 539 | isomiR example 1 of SEQ ID NO: 20 | - |
| 540 | isomiR example 2 of SEQ ID NO: 20 | - |
| 541 | isomiR example 1 of SEQ ID NO: 25 | - |
| 542 | isomiR example 2 of SEQ ID NO: 25 | - |
| 543 | isomiR example 1 of SEQ ID NO: 26 | - |
| 544 | isomiR example 2 of SEQ ID NO: 26 | - |
| 545 | isomiR example 1 of SEQ ID NO: 27 | - |
| 546 | isomiR example 2 of SEQ ID NO: 27 | - |
| 547 | isomiR example 1 of SEQ ID NO: 29 | - |
| 548 | isomiR example 2 of SEQ ID NO: 29 | - |
| 549 | isomiR example 1 of SEQ ID NO: 31 | - |
| 550 | isomiR example 2 of SEQ ID NO: 31 | - |
| 551 | isomiR example 1 of SEQ ID NO: 32 | - |
| 552 | isomiR example 2 of SEQ ID NO: 32 | - |
| 553 | isomiR example 1 of SEQ ID NO: 37 | - |
| 554 | isomiR example 2 of SEQ ID NO: 37 | - |
| 555 | isomiR example 1 of SEQ ID NO: 44 | - |
| 556 | isomiR example 2 of SEQ ID NO: 44 | - |
| 557 | isomiR example 1 of SEQ ID NO: 45 | - |
| 558 | isomiR example 2 of SEQ ID NO: 45 | - |
| 559 | isomiR example 1 of SEQ ID NO: 46 | - |
| 560 | isomiR example 2 of SEQ ID NO: 46 | - |
| 561 | isomiR example 1 of SEQ ID NO: 47 | - |
| 562 | isomiR example 2 of SEQ ID NO: 47 | - |
| 563 | isomiR example 1 of SEQ ID NO: 48 | - |
| 564 | isomiR example 2 of SEQ ID NO: 48 | - |
| 565 | isomiR example 1 of SEQ ID NO: 49 | - |
| 566 | isomiR example 2 of SEQ ID NO: 49 | - |
| 567 | isomiR example 1 of SEQ ID NO: 51 | - |
| 568 | isomiR example 2 of SEQ ID NO: 51 | - |
| 569 | isomiR example 1 of SEQ ID NO: 52 | - |
| 570 | isomiR example 2 of SEQ ID NO: 52 | - |
| 571 | isomiR example 1 of SEQ ID NO: 53 | - |
| 572 | isomiR example 2 of SEQ ID NO: 53 | - |
| 573 | isomiR example 1 of SEQ ID NO: 54 | - |
| 574 | isomiR example 2 of SEQ ID NO: 54 | - |
| 575 | isomiR example 1 of SEQ ID NO: 55 | - |
| 576 | isomiR example 2 of SEQ ID NO: 55 | - |
| 577 | isomiR example 1 of SEQ ID NO: 56 | - |
| 578 | isomiR example 2 of SEQ ID NO: 56 | - |
| 579 | isomiR example 1 of SEQ ID NO: 57 | - |
| 580 | isomiR example 2 of SEQ ID NO: 57 | - |
| 581 | isomiR example 1 of SEQ ID NO: 59 | - |
| 582 | isomiR example 2 of SEQ ID NO: 59 | - |
| 583 | isomiR example 1 of SEQ ID NO: 60 | - |
| 584 | isomiR example 2 of SEQ ID NO: 60 | - |
| 585 | isomiR example 1 of SEQ ID NO: 61 | - |
| 586 | isomiR example 2 of SEQ ID NO: 61 | - |
| 587 | isomiR example 1 of SEQ ID NO: 62 | - |
| 588 | isomiR example 2 of SEQ ID NO: 62 | - |
| 589 | isomiR example 1 of SEQ ID NO: 63 | - |
| 590 | isomiR example 2 of SEQ ID NO: 63 | - |
| 591 | isomiR example 1 of SEQ ID NO: 71 | - |
| 592 | isomiR example 2 of SEQ ID NO: 71 | - |
| 593 | isomiR example 1 of SEQ ID NO: 72 | - |
| 594 | isomiR example 2 of SEQ ID NO: 72 | - |
| 595 | isomiR example 1 of SEQ ID NO: 73 | - |
| 596 | isomiR example 2 of SEQ ID NO: 73 | - |
| 597 | isomiR example 1 of SEQ ID NO: 74 | - |
| 598 | isomiR example 2 of SEQ ID NO: 74 | - |
| 599 | isomiR example 1 of SEQ ID NO: 76 | - |
| 600 | isomiR example 2 of SEQ ID NO: 76 | - |
| 601 | isomiR example 1 of SEQ ID NO: 77 | - |
| 602 | isomiR example 2 of SEQ ID NO: 77 | - |
| 603 | isomiR example 1 of SEQ ID NO: 78 | - |
| 604 | isomiR example 2 of SEQ ID NO: 78 | - |
| 605 | isomiR example 1 of SEQ ID NO: 79 | - |
| 606 | isomiR example 2 of SEQ ID NO: 79 | - |
| 607 | isomiR example 1 of SEQ ID NO: 83 | - |
| 608 | isomiR example 2 of SEQ ID NO: 83 | - |
| 609 | isomiR example 1 of SEQ ID NO: 84 | - |
| 610 | isomiR example 2 of SEQ ID NO: 84 | - |
| 611 | isomiR example 1 of SEQ ID NO: 86 | - |
| 612 | isomiR example 2 of SEQ ID NO: 86 | - |
| 613 | isomiR example 1 of SEQ ID NO: 87 | - |
| 614 | isomiR example 2 of SEQ ID NO: 87 | - |
| 615 | isomiR example 1 of SEQ ID NO: 88 | - |
| 616 | isomiR example 2 of SEQ ID NO: 88 | - |
| 617 | isomiR example 1 of SEQ ID NO: 89 | - |
| 618 | isomiR example 2 of SEQ ID NO: 89 | - |
| 619 | isomiR example 1 of SEQ ID NO: 90 | - |
| 620 | isomiR example 2 of SEQ ID NO: 90 | - |
| 621 | isomiR example 1 of SEQ ID NO: 92 | - |
| 622 | isomiR example 2 of SEQ ID NO: 92 | - |
| 623 | isomiR example 1 of SEQ ID NO: 94 | - |
| 624 | isomiR example 2 of SEQ ID NO: 94 | - |
| 625 | isomiR example 1 of SEQ ID NO: 98 | - |
| 626 | isomiR example 2 of SEQ ID NO: 98 | - |
| 627 | isomiR example 1 of SEQ ID NO: 102 | - |
| 628 | isomiR example 2 of SEQ ID NO: 102 | - |
| 629 | isomiR example 1 of SEQ ID NO: 105 | - |
| 630 | isomiR example 2 of SEQ ID NO: 105 | - |
| 631 | isomiR example 1 of SEQ ID NO: 106 | - |
| 632 | isomiR example 2 of SEQ ID NO: 106 | - |
| 633 | isomiR example 1 of SEQ ID NO: 108 | - |
| 634 | isomiR example 2 of SEQ ID NO: 108 | - |
| 635 | isomiR example 1 of SEQ ID NO: 111 | - |
| 636 | isomiR example 2 of SEQ ID NO: 111 | - |
| 637 | isomiR example 1 of SEQ ID NO: 112 | - |
| 638 | isomiR example 2 of SEQ ID NO: 112 | - |
| 639 | isomiR example 1 of SEQ ID NO: 113 | - |
| 640 | isomiR example 2 of SEQ ID NO: 113 | - |
| 641 | isomiR example 1 of SEQ ID NO: 114 | - |
| 642 | isomiR example 2 of SEQ ID NO: 114 | - |
| 643 | isomiR example 1 of SEQ ID NO: 116 | - |
| 644 | isomiR example 2 of SEQ ID NO: 116 | - |
| 645 | isomiR example 1 of SEQ ID NO: 117 | - |
| 646 | isomiR example 2 of SEQ ID NO: 117 | - |
| 647 | isomiR example 1 of SEQ ID NO: 118 | - |
| 648 | isomiR example 2 of SEQ ID NO: 118 | - |
| 649 | isomiR example 1 of SEQ ID NO: 122 | - |
| 650 | isomiR example 2 of SEQ ID NO: 122 | - |
| 651 | isomiR example 1 of SEQ ID NO: 123 | - |
| 652 | isomiR example 2 of SEQ ID NO: 123 | - |
| 653 | isomiR example 1 of SEQ ID NO: 124 | - |
| 654 | isomiR example 2 of SEQ ID NO: 124 | - |
| 655 | isomiR example 1 of SEQ ID NO: 128 | - |
| 656 | isomiR example 2 of SEQ ID NO: 128 | - |
| 657 | isomiR example 1 of SEQ ID NO: 129 | - |
| 658 | isomiR example 2 of SEQ ID NO: 129 | - |
| 659 | isomiR example 1 of SEQ ID NO: 133 | - |
| 660 | isomiR example 2 of SEQ ID NO: 133 | - |
| 661 | isomiR example 1 of SEQ ID NO: 134 | - |
| 662 | isomiR example 2 of SEQ ID NO: 134 | - |
| 663 | isomiR example 1 of SEQ ID NO: 135 | - |
| 664 | isomiR example 2 of SEQ ID NO: 135 | - |
| 665 | isomiR example 1 of SEQ ID NO: 136 | - |
| 666 | isomiR example 2 of SEQ ID NO: 136 | - |
| 667 | isomiR example 1 of SEQ ID NO: 137 | - |
| 668 | isomiR example 2 of SEQ ID NO: 137 | - |
| 669 | isomiR example 1 of SEQ ID NO: 140 | - |
| 670 | isomiR example 2 of SEQ ID NO: 140 | - |
| 671 | isomiR example 1 of SEQ ID NO: 141 | - |
| 672 | isomiR example 2 of SEQ ID NO: 141 | - |
| 673 | isomiR example 1 of SEQ ID NO: 145 | - |
| 674 | isomiR example 2 of SEQ ID NO: 145 | - |
| 675 | isomiR example 1 of SEQ ID NO: 146 | - |
| 676 | isomiR example 2 of SEQ ID NO: 146 | - |
| 677 | isomiR example 1 of SEQ ID NO: 147 | - |
| 678 | isomiR example 2 of SEQ ID NO: 147 | - |
| 679 | isomiR example 1 of SEQ ID NO: 148 | - |
| 680 | isomiR example 2 of SEQ ID NO: 148 | - |
| 681 | isomiR example 1 of SEQ ID NO: 149 | - |
| 682 | isomiR example 2 of SEQ ID NO: 149 | - |
| 683 | isomiR example 1 of SEQ ID NO: 150 | - |
| 684 | isomiR example 2 of SEQ ID NO: 150 | - |
| 685 | isomiR example 1 of SEQ ID NO: 151 | - |
| 686 | isomiR example 2 of SEQ ID NO: 151 | - |
| 687 | isomiR example 1 of SEQ ID NO: 153 | - |
| 688 | isomiR example 2 of SEQ ID NO: 153 | - |
| 689 | isomiR example 1 of SEQ ID NO: 154 | - |
| 690 | isomiR example 2 of SEQ ID NO: 154 | - |
| 691 | isomiR example 1 of SEQ ID NO: 155 | - |
| 692 | isomiR example 2 of SEQ ID NO: 155 | - |
| 693 | isomiR example 1 of SEQ ID NO: 157 | - |
| 694 | isomiR example 2 of SEQ ID NO: 157 | - |
| 695 | isomiR example 1 of SEQ ID NO: 158 | - |
| 696 | isomiR example 2 of SEQ ID NO: 158 | - |
| 697 | isomiR example 1 of SEQ ID NO: 159 | - |
| 698 | isomiR example 2 of SEQ ID NO: 159 | - |
| 699 | isomiR example 1 of SEQ ID NO: 160 | - |
| 700 | isomiR example 2 of SEQ ID NO: 160 | - |
| 701 | isomiR example 1 of SEQ ID NO: 161 | - |
| 702 | isomiR example 2 of SEQ ID NO: 161 | - |
| 703 | isomiR example 1 of SEQ ID NO: 162 | - |
| 704 | isomiR example 2 of SEQ ID NO: 162 | - |
| 705 | isomiR example 1 of SEQ ID NO: 163 | - |
| 706 | isomiR example 2 of SEQ ID NO: 163 | - |
| 707 | isomiR example 1 of SEQ ID NO: 166 | - |
| 708 | isomiR example 2 of SEQ ID NO: 166 | - |
| 709 | isomiR example 1 of SEQ ID NO: 167 | - |
| 710 | isomiR example 2 of SEQ ID NO: 167 | - |
| 711 | isomiR example 1 of SEQ ID NO: 168 | - |
| 712 | isomiR example 2 of SEQ ID NO: 168 | - |
| 713 | isomiR example 1 of SEQ ID NO: 169 | - |
| 714 | isomiR example 2 of SEQ ID NO: 169 | - |
| 715 | isomiR example 1 of SEQ ID NO: 176 | - |
| 716 | isomiR example 2 of SEQ ID NO: 176 | - |
| 717 | isomiR example 1 of SEQ ID NO: 177 | - |
| 718 | isomiR example 2 of SEQ ID NO: 177 | - |
| 719 | isomiR example 1 of SEQ ID NO: 178 | - |
| 720 | isomiR example 2 of SEQ ID NO: 178 | - |
| 721 | isomiR example 1 of SEQ ID NO: 179 | - |
| 722 | isomiR example 2 of SEQ ID NO: 179 | - |
| 723 | isomiR example 1 of SEQ ID NO: 180 | - |
| 724 | isomiR example 2 of SEQ ID NO: 180 | - |
| 725 | isomiR example 1 of SEQ ID NO: 181 | - |
| 726 | isomiR example 2 of SEQ ID NO: 181 | - |
| 727 | isomiR example 1 of SEQ ID NO: 182 | - |
| 728 | isomiR example 2 of SEQ ID NO: 182 | - |
| 729 | isomiR example 1 of SEQ ID NO: 184 | - |
| 730 | isomiR example 2 of SEQ ID NO: 184 | - |
| 731 | isomiR example 1 of SEQ ID NO: 186 | - |
| 732 | isomiR example 2 of SEQ ID NO: 186 | - |
| 733 | isomiR example 1 of SEQ ID NO: 187 | - |
| 734 | isomiR example 2 of SEQ ID NO: 187 | - |
| 735 | isomiR example 1 of SEQ ID NO: 190 | - |
| 736 | isomiR example 2 of SEQ ID NO: 190 | - |
| 737 | isomiR example 1 of SEQ ID NO: 193 | - |
| 738 | isomiR example 2 of SEQ ID NO: 193 | - |
| 739 | isomiR example 1 of SEQ ID NO: 196 | - |
| 740 | isomiR example 2 of SEQ ID NO: 196 | - |
| 741 | isomiR example 1 of SEQ ID NO: 198 | - |
| 742 | isomiR example 2 of SEQ ID NO: 198 | - |
| 743 | isomiR example 1 of SEQ ID NO: 201 | - |
| 744 | isomiR example 2 of SEQ ID NO: 201 | - |
| 745 | isomiR example 1 of SEQ ID NO: 202 | - |
| 746 | isomiR example 2 of SEQ ID NO: 202 | - |
| 747 | isomiR example 1 of SEQ ID NO: 203 | - |
| 748 | isomiR example 2 of SEQ ID NO: 203 | - |
| 749 | isomiR example 1 of SEQ ID NO: 204 | - |
| 750 | isomiR example 2 of SEQ ID NO: 204 | - |
| 751 | isomiR example 1 of SEQ ID NO: 205 | - |
| 752 | isomiR example 2 of SEQ ID NO: 205 | - |
| 753 | isomiR example 1 of SEQ ID NO: 206 | - |
| 754 | isomiR example 2 of SEQ ID NO: 206 | - |
| 755 | isomiR example 1 of SEQ ID NO: 207 | - |
| 756 | isomiR example 2 of SEQ ID NO: 207 | - |
| 757 | isomiR example 1 of SEQ ID NO: 208 | - |
| 758 | isomiR example 2 of SEQ ID NO: 208 | - |
| 759 | isomiR example 1 of SEQ ID NO: 209 | - |
| 760 | isomiR example 2 of SEQ ID NO: 209 | - |
| 761 | isomiR example 1 of SEQ ID NO: 210 | - |
| 762 | isomiR example 2 of SEQ ID NO: 210 | - |
| 763 | isomiR example 1 of SEQ ID NO: 211 | - |
| 764 | isomiR example 2 of SEQ ID NO: 211 | - |
| 765 | isomiR example 1 of SEQ ID NO: 214 | - |
| 766 | isomiR example 2 of SEQ ID NO: 214 | - |
| 767 | isomiR example 1 of SEQ ID NO: 215 | - |
| 768 | isomiR example 2 of SEQ ID NO: 215 | - |
| 769 | isomiR example 1 of SEQ ID NO: 216 | - |
| 770 | isomiR example 2 of SEQ ID NO: 216 | - |
| 771 | isomiR example 1 of SEQ ID NO: 217 | - |
| 772 | isomiR example 2 of SEQ ID NO: 217 | - |
| 773 | isomiR example 1 of SEQ ID NO: 218 | - |
| 774 | isomiR example 2 of SEQ ID NO: 218 | - |
| 775 | isomiR example 1 of SEQ ID NO: 220 | - |
| 776 | isomiR example 2 of SEQ ID NO: 220 | - |
| 777 | isomiR example 1 of SEQ ID NO: 221 | - |
| 778 | isomiR example 2 of SEQ ID NO: 221 | - |
| 779 | isomiR example 1 of SEQ ID NO: 222 | - |
| 780 | isomiR example 2 of SEQ ID NO: 222 | - |
| 781 | isomiR example 1 of SEQ ID NO: 223 | - |
| 782 | isomiR example 2 of SEQ ID NO: 223 | - |
| 783 | isomiR example 1 of SEQ ID NO: 224 | - |
| 784 | isomiR example 2 of SEQ ID NO: 224 | - |
| 785 | isomiR example 1 of SEQ ID NO: 226 | - |
| 786 | isomiR example 2 of SEQ ID NO: 226 | - |
| 787 | isomiR example 1 of SEQ ID NO: 228 | - |
| 788 | isomiR example 2 of SEQ ID NO: 228 | - |
| 789 | isomiR example 1 of SEQ ID NO: 233 | - |
| 790 | isomiR example 2 of SEQ ID NO: 233 | - |
| 791 | isomiR example 1 of SEQ ID NO: 235 | - |
| 792 | isomiR example 2 of SEQ ID NO: 235 | - |
| 793 | isomiR example 1 of SEQ ID NO: 236 | - |
| 794 | isomiR example 2 of SEQ ID NO: 236 | - |
| 795 | isomiR example 1 of SEQ ID NO: 237 | - |
| 796 | isomiR example 2 of SEQ ID NO: 237 | - |
| 797 | isomiR example 1 of SEQ ID NO: 239 | - |
| 798 | isomiR example 2 of SEQ ID NO: 239 | - |
| 799 | isomiR example 1 of SEQ ID NO: 240 | - |
| 800 | isomiR example 2 of SEQ ID NO: 240 | - |
| 801 | isomiR example 1 of SEQ ID NO: 243 | - |
| 802 | isomiR example 2 of SEQ ID NO: 243 | - |
| 803 | isomiR example 1 of SEQ ID NO: 244 | - |
| 804 | isomiR example 2 of SEQ ID NO: 244 | - |
| 805 | isomiR example 1 of SEQ ID NO: 245 | - |
| 806 | isomiR example 2 of SEQ ID NO: 245 | - |
| 807 | isomiR example 1 of SEQ ID NO: 247 | - |
| 808 | isomiR example 2 of SEQ ID NO: 247 | - |
| 809 | isomiR example 1 of SEQ ID NO: 248 | - |
| 810 | isomiR example 2 of SEQ ID NO: 248 | - |
| 811 | isomiR example 1 of SEQ ID NO: 249 | - |
| 812 | isomiR example 2 of SEQ ID NO: 249 | - |
| 813 | hsa-miR-145-5p | MIMAT0000437 |
| 814 | hsa-let-7f-5p | MIMAT0000067 |
| 815 | hsa-miR-146a-5p | MIMAT0000449 |
| 816 | hsa-let-7d-5p | MIMAT0000065 |
| 817 | hsa-let-7a-5p | MIMAT0000062 |

The present specification incorporates the contents disclosed in Japanese Patent Application No. 2016-073132 (filing date: March 31, 2016) to which the present application claims priorities.

### Advantageous Effect of Invention

According to the present invention, early pancreatic cancer or pancreatic cancer precursor lesion can be detected easily and in high accuracy.

For example, the presence or absence of early pancreatic cancer or a pancreatic cancer precursor lesion in patients can be easily detected by using, as indicators, the determined expression levels of several miRNAs in blood, serum, and/or plasma of the patients, which can be collected with minimal invasiveness.

### BRIEF DESCRIPTION OF THE DRAWINGS

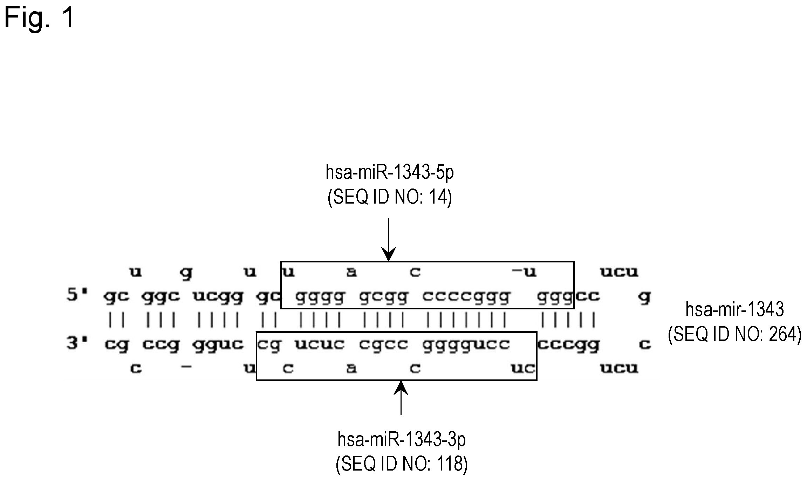

[Figure 1] This figure shows the relationship between the nucleotide sequences of hsa-miR-1343-3p represented by SEQ ID NO: 118 and hsa-miR-1343-5p represented by SEQ ID NO: 14, which are produced from hsa-mir-1343 represented by SEQ ID NO: 264 as a precursor.

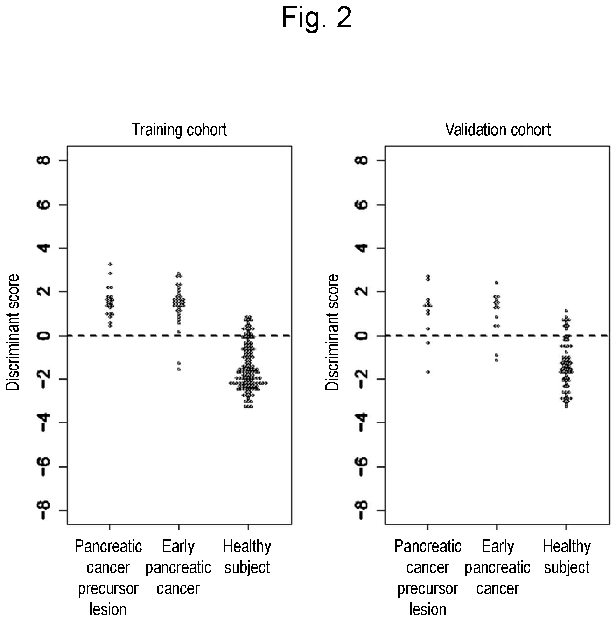

[Figure 2] In the left diagram of Figure 2, a discriminant (3.10 × hsa-miR-miR-6784-5p - 39.85) was prepared by use of Fisher's discriminant analysis from the expression level measurement values of hsa-miR-6784-5p (SEQ ID NO: 1) in pancreatic cancer precursor lesion patients (21 persons), early pancreatic cancer patients (31 persons), and healthy subjects (123 persons) selected as a training cohort, and discriminant scores obtained with the discriminant are plotted on the vertical axis with the cohort plotted on the horizontal axis. The dotted line in the diagram depicts a discriminant boundary on which the discriminant score is 0 and by which the two groups are discriminated. In the right diagram of Figure 2, the vertical axis plots discriminant scores for the expression level measurement values of hsa-miR-6784-5p (SEQ ID NO: 1) in pancreatic cancer precursor lesion patients (12 persons), early pancreatic cancer patients (13 persons), and healthy subjects (61 persons) selected as a validation cohort, wherein the discriminant scores are obtained with the discriminant prepared from the training cohort, and the horizontal axis plots the cohort. The dotted line in the diagram depicts the discriminant boundary on which the discriminant score is 0 and by which the two groups are discriminated.

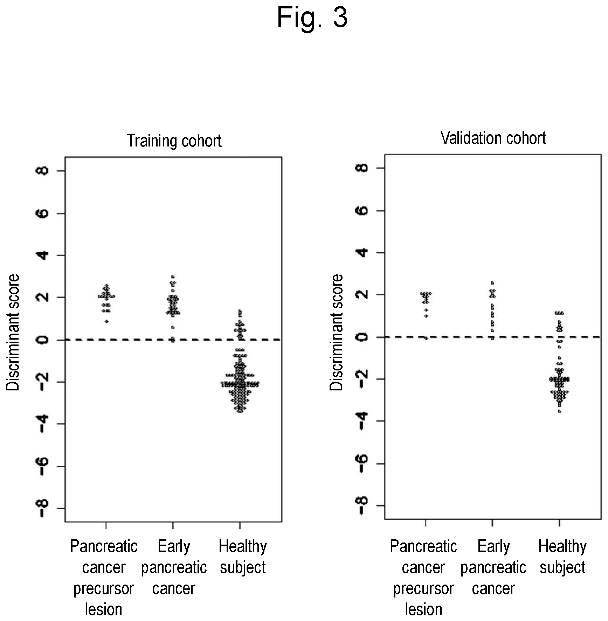

[Figure 3] In the left diagram of Figure 3, a discriminant (1.90 × hsa-miR-6784-5p + 1.72 × hsa-miR-1181 - 34.50) was prepared by use of Fisher's discriminant analysis from the expression level measurement values of hsa-miR-6784-5p (SEQ ID NO: 1) and hsa-miR-1181 (SEQ ID NO: 2) in pancreatic cancer precursor lesion patients (21 persons), early pancreatic cancer patients (31 persons), and healthy subjects (123 persons) selected as a training cohort, and discriminant scores obtained with the discriminant are plotted on the vertical axis with the cohort plotted on the horizontal axis. The dotted line in the diagram depicts a discriminant boundary on which the discriminant score is 0 and by which the two groups are discriminated. In the right diagram of Figure 3, the vertical axis plots discriminant scores for the expression level measurement values of hsa-miR-6784-5p (SEQ ID NO: 1) and hsa-miR-1181 (SEQ ID NO: 2) in pancreatic cancer precursor lesion patients (12 persons), early pancreatic cancer patients (13 persons), and healthy subjects (61 persons) selected as a validation cohort, wherein the discriminant scores are obtained with the discriminant prepared from the training cohort, and the horizontal axis plots the cohort. The dotted line in the diagram depicts a discriminant boundary on which the discriminant score is 0 and by which the two groups are discriminated.

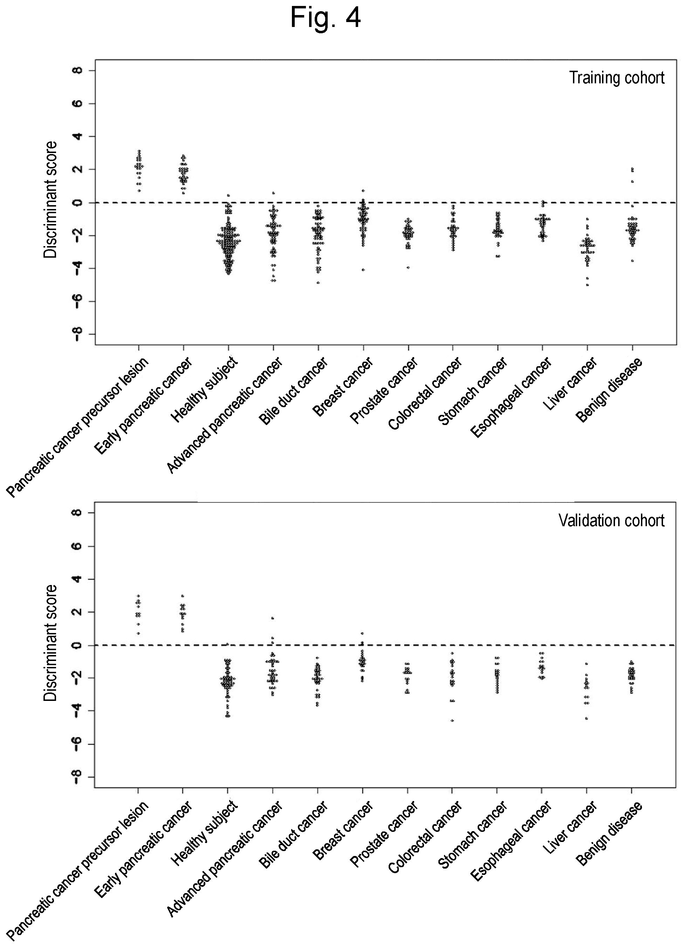

[Figure 4] In the top diagram of Figure 4, a discriminant (0.48 × hsa-miR-4695-5p - 1.75 × hsa-miR-5090 + 1.31 × hsa-miR-4673 - 0.98 × hsa-miR-6813-5p - 1.16 × hsa-miR-642a-3p + 15.39) was prepared by use of Fisher's discriminant analysis from the expression level measurement values of hsa-miR-4695-5p (SEQ ID NO: 106), hsa-miR-5090 (SEQ ID NO: 12), hsa-miR-4673 (SEQ ID NO: 137), hsa-miR-6813-5p (SEQ ID NO: 119), and hsa-miR-642a-3p (SEQ ID NO: 105) in 21 pancreatic cancer precursor lesion patients, 31 early pancreatic cancer patients, 128 healthy subjects, 61 advanced pancreatic cancer patients, 66 bile duct cancer patients, 51 breast cancer patients, 35 prostate cancer patients, 31 colorectal cancer patients, 32 stomach cancer patients, 34 esophageal cancer patients, 38 liver cancer patients, 15 benign pancreatic disease patients, and 26 benign prostatic disease patients selected as a training cohort, and discriminant scores obtained with the discriminant are plotted on the vertical axis with the cohort plotted on the horizontal axis. The dotted line in the diagram depicts a discriminant boundary on which the discriminant score is 0 and by which the two groups are discriminated. In the bottom diagram of Figure 4, the vertical axis plots discriminant scores for the expression level measurement values of hsa-miR-4695-5p (SEQ ID NO: 106), hsa-miR-5090 (SEQ ID NO: 12), hsa-miR-4673 (SEQ ID NO: 137), hsa-miR-6813-5p (SEQ ID NO: 119), and hsa-miR-642a-3p (SEQ ID NO: 105) in 12 pancreatic cancer precursor lesion patients, 13 early pancreatic cancer patients, 56 healthy subjects, 39 advanced pancreatic cancer patients, 32 bile duct cancer patients, 23 breast cancer patients, 17 prostate cancer patients, 19 colorectal cancer patients, 18 stomach cancer patients, 16 esophageal cancer patients, 14 liver cancer patients, and 24 benign pancreatic and prostatic disease patients selected as a validation cohort, wherein the discriminant scores are obtained with the discriminant prepared from the training cohort, and the horizontal axis plots the cohort. The dotted line in the diagram depicts a discriminant boundary on which the discriminant score is 0 and by which the two groups are discriminated.

### MODES FOR CARRYING OUT THE INVENTION

Hereinafter, the present invention will be further described in detail.

### 1. Target nucleic acid for early pancreatic cancer or pancreatic cancer precursor lesion

Primary target nucleic acids, as early pancreatic cancer or pancreatic cancer precursor lesion markers, for detecting the presence and/or absence of early pancreatic cancer or a pancreatic cancer precursor lesion or early pancreatic cancer or pancreatic cancer precursor lesion cells using the nucleic acid probes or the primers for the detection of early pancreatic cancer or a pancreatic cancer precursor lesion defined above according to the present invention comprise at least one miRNA selected from the group consisting of the following miRNAs: hsa-miR-6784-5p, hsa-miR-1181, hsa-miR-671-5p, hsa-miR-6857-5p, hsa-miR-4276, hsa-miR-1914-3p, hsa-miR-149-3p, hsa-miR-937-5p, hsa-miR-4675, hsa-miR-6795-5p, hsa-miR-4731-5p, hsa-miR-5090, hsa-miR-3620-5p, hsa-miR-1343-5p, hsa-miR-6717-5p, hsa-miR-6825-5p, hsa-miR-6738-5p, hsa-miR-6769a-5p, hsa-miR-4728-5p, hsa-miR-652-5p, hsa-miR-4257, hsa-miR-6785-5p, hsa-miR-7110-5p, hsa-miR-6887-5p, hsa-miR-887-3p, hsa-miR-1228-5p, hsa-miR-5572, hsa-miR-6782-5p, hsa-miR-4298, hsa-miR-6786-5p, hsa-miR-5010-5p, hsa-miR-6087, hsa-miR-6765-5p, hsa-miR-6732-5p, hsa-miR-6787-5p, hsa-miR-6737-5p, hsa-miR-128-2-5p, hsa-miR-4270, hsa-miR-6861-5p, hsa-miR-6756-5p, hsa-miR-1229-5p, hsa-miR-6891-5p, hsa-miR-6848-5p, hsa-miR-1237-5p, hsa-miR-30c-1-3p, hsa-miR-1233-5p, hsa-miR-211-3p, hsa-miR-4758-5p, hsa-miR-614, hsa-miR-6746-5p, hsa-miR-1915-5p, hsa-miR-4688, hsa-miR-3917, hsa-miR-5787, hsa-miR-4632-5p, hsa-miR-6126, hsa-miR-135a-3p, hsa-miR-8063, hsa-miR-5698, hsa-miR-6089, hsa-miR-498, hsa-miR-296-3p, hsa-miR-4419b, hsa-miR-6802-5p, hsa-miR-6829-5p, hsa-miR-6803-5p, hsa-miR-1199-5p, hsa-miR-6840-3p, hsa-miR-6752-5p, hsa-miR-6798-5p, hsa-miR-6131, hsa-miR-4667-5p, hsa-miR-6510-5p, hsa-miR-4690-5p, hsa-miR-920, hsa-miR-23b-3p, hsa-miR-4448, hsa-miR-2110, hsa-miR-4706, hsa-miR-7845-5p, hsa-miR-6808-5p, hsa-miR-4447, hsa-miR-6869-5p, hsa-miR-6794-5p, hsa-miR-6511a-5p, hsa-miR-6824-5p, hsa-miR-6766-3p, hsa-miR-6511a-5p, and hsa-miR-6749-5p.

Furthermore, at least one miRNAs selected from the group consisting of the following other early pancreatic cancer or pancreatic cancer precursor lesion markers that can be combined with these miRNAs, i.e., hsa-miR-1908-5p, hsa-miR-6729-5p, hsa-miR-5195-3p, hsa-miR-638, hsa-miR-6125, hsa-miR-3178, hsa-miR-3196, hsa-miR-8069, hsa-miR-4723-5p, hsa-miR-4746-3p, hsa-miR-4689, hsa-miR-6816-5p, hsa-miR-6757-5p, hsa-miR-7109-5p, hsa-miR-6724-5p, hsa-miR-1225-3p, hsa-miR-6875-5p, hsa-miR-7108-5p, hsa-miR-4508, hsa-miR-6085, hsa-miR-6779-5p, hsa-miR-642a-3p, hsa-miR-4695-5p, hsa-miR-7847-3p, hsa-miR-3197, hsa-miR-6769b-5p, hsa-miR-7641, hsa-miR-187-5p, hsa-miR-3185, hsa-miR-2861, hsa-miR-3940-5p, hsa-miR-1203, hsa-miR-615-5p, hsa-miR-4787-5p, hsa-miR-1343-3p, hsa-miR-6813-5p, hsa-miR-1225-5p, hsa-miR-602, hsa-miR-4488, hsa-miR-125a-3p, hsa-miR-5100, hsa-miR-4294, hsa-miR-1231, hsa-miR-6765-3p, hsa-miR-4442, hsa-miR-718, hsa-miR-6780b-5p, hsa-miR-6090, hsa-miR-6845-5p, hsa-miR-4741, hsa-miR-4467, hsa-miR-4707-5p, hsa-miR-4271, hsa-miR-4673, hsa-miR-3184-5p, hsa-miR-1469, hsa-miR-4640-5p, hsa-miR-663a, hsa-miR-6791-5p, hsa-miR-6826-5p, hsa-miR-4433b-3p, hsa-miR-1915-3p, hsa-miR-4417, hsa-miR-4449, hsa-miR-4707-3p, hsa-miR-3180-3p, hsa-miR-5585-3p, hsa-miR-1268a, hsa-miR-8072, hsa-miR-296-5p, hsa-miR-204-3p, hsa-miR-4454, hsa-miR-6722-3p, hsa-miR-1290, hsa-miR-3622a-5p, hsa-miR-939-5p, hsa-miR-675-5p, hsa-miR-3131, hsa-miR-4648, hsa-miR-1268b, hsa-miR-6741-5p, hsa-miR-6893-5p, hsa-miR-3162-5p, hsa-miR-642b-3p, hsa-miR-4734, hsa-miR-150-3p, hsa-miR-8089, hsa-miR-6805-3p, hsa-miR-7113-3p, hsa-miR-6850-5p, hsa-miR-6799-5p, hsa-miR-6768-5p, hsa-miR-92b-5p, hsa-miR-3679-5p, hsa-miR-4792, hsa-miR-3656, hsa-miR-92a-2-5p, hsa-miR-4466, hsa-miR-4513, hsa-miR-6781-5p, hsa-miR-4649-5p, hsa-miR-6775-5p, hsa-miR-4651, hsa-miR-3195, hsa-miR-6726-5p, hsa-miR-6872-3p, hsa-miR-371a-5p, hsa-miR-6777-5p, hsa-miR-6789-5p, hsa-miR-7975, hsa-miR-6821-5p, hsa-miR-4534, hsa-miR-619-5p, hsa-miR-7107-5p, hsa-miR-1228-3p, hsa-miR-6774-5p, hsa-miR-6805-5p, hsa-miR-23a-3p, hsa-miR-4665-5p, hsa-miR-4505, hsa-miR-4638-5p, hsa-miR-24-3p, hsa-miR-3135b, hsa-miR-4745-5p, hsa-miR-128-1-5p, hsa-miR-4476, hsa-miR-4687-3p, hsa-miR-3665, hsa-miR-6806-5p, hsa-miR-3937, hsa-miR-711, hsa-miR-3141, hsa-miR-3188, hsa-miR-4281, hsa-miR-5196-5p, hsa-miR-6880-5p, hsa-miR-3960, hsa-miR-3648, hsa-miR-6721-5p, hsa-miR-4492, hsa-miR-744-5p, hsa-miR-7704, hsa-miR-4749-5p, hsa-miR-762, hsa-miR-6836-3p, hsa-miR-6727-5p, hsa-miR-4739, hsa-miR-7977, hsa-miR-4484, hsa-miR-6515-3p, hsa-miR-373-5p, hsa-miR-4258, hsa-miR-4674, hsa-miR-3180, hsa-miR-6076, hsa-miR-1238-5p, hsa-miR-4463, hsa-miR-4486, hsa-miR-4730, hsa-miR-4286, and hsa-miR-4739 can also be preferably used as target nucleic acids.

These miRNAs include, for example, a human gene comprising a nucleotide sequence represented by any of SEQ ID NOs: 1 to 250 (i.e., hsa-miR-6784-5p, hsa-miR-1181, hsa-miR-671-5p, hsa-miR-6857-5p, hsa-miR-4276, hsa-miR-1914-3p, hsa-miR-149-3p, hsa-miR-937-5p, hsa-miR-4675, hsa-miR-6795-5p, hsa-miR-4731-5p, hsa-miR-5090, hsa-miR-3620-5p, hsa-miR-1343-5p, hsa-miR-6717-5p, hsa-miR-6825-5p, hsa-miR-6738-5p, hsa-miR-6769a-5p, hsa-miR-4728-5p, hsa-miR-652-5p, hsa-miR-4257, hsa-miR-6785-5p, hsa-miR-7110-5p, hsa-miR-6887-5p, hsa-miR-887-3p, hsa-miR-1228-5p, hsa-miR-5572, hsa-miR-6782-5p, hsa-miR-4298, hsa-miR-6786-5p, hsa-miR-5010-5p, hsa-miR-6087, hsa-miR-6765-5p, hsa-miR-6732-5p, hsa-miR-6787-5p, hsa-miR-6737-5p, hsa-miR-128-2-5p, hsa-miR-4270, hsa-miR-6861-5p, hsa-miR-6756-5p, hsa-miR-1229-5p, hsa-miR-6891-5p, hsa-miR-6848-5p, hsa-miR-1237-5p, hsa-miR-30c-1-3p, hsa-miR-1233-5p, hsa-miR-211-3p, hsa-miR-4758-5p, hsa-miR-614, hsa-miR-6746-5p, hsa-miR-1915-5p, hsa-miR-4688, hsa-miR-3917, hsa-miR-5787, hsa-miR-4632-5p, hsa-miR-6126, hsa-miR-135a-3p, hsa-miR-8063, hsa-miR-5698, hsa-miR-6089, hsa-miR-498, hsa-miR-296-3p, hsa-miR-4419b, hsa-miR-6802-5p, hsa-miR-6829-5p, hsa-miR-6803-5p, hsa-miR-1199-5p, hsa-miR-6840-3p, hsa-miR-6752-5p, hsa-miR-6798-5p, hsa-miR-6131, hsa-miR-4667-5p, hsa-miR-6510-5p, hsa-miR-4690-5p, hsa-miR-920, hsa-miR-23b-3p, hsa-miR-4448, hsa-miR-2110, hsa-miR-4706, hsa-miR-7845-5p, hsa-miR-6808-5p, hsa-miR-4447, hsa-miR-6869-5p, hsa-miR-1908-5p, hsa-miR-6729-5p, hsa-miR-5195-3p, hsa-miR-638, hsa-miR-6125, hsa-miR-3178, hsa-miR-3196, hsa-miR-8069, hsa-miR-4723-5p, hsa-miR-4746-3p, hsa-miR-4689, hsa-miR-6816-5p, hsa-miR-6757-5p, hsa-miR-7109-5p, hsa-miR-6724-5p, hsa-miR-1225-3p, hsa-miR-6875-5p, hsa-miR-7108-5p, hsa-miR-4508, hsa-miR-6085, hsa-miR-6779-5p, hsa-miR-642a-3p, hsa-miR-4695-5p, hsa-miR-7847-3p, hsa-miR-3197, hsa-miR-6769b-5p, hsa-miR-7641, hsa-miR-187-5p, hsa-miR-3185, hsa-miR-2861, hsa-miR-3940-5p, hsa-miR-1203, hsa-miR-615-5p, hsa-miR-4787-5p, hsa-miR-1343-3p, hsa-miR-6813-5p, hsa-miR-1225-5p, hsa-miR-602, hsa-miR-4488, hsa-miR-125a-3p, hsa-miR-5100, hsa-miR-4294, hsa-miR-1231, hsa-miR-6765-3p, hsa-miR-4442, hsa-miR-718, hsa-miR-6780b-5p, hsa-miR-6090, hsa-miR-6845-5p, hsa-miR-4741, hsa-miR-4467, hsa-miR-4707-5p, hsa-miR-4271, hsa-miR-4673, hsa-miR-3184-5p, hsa-miR-1469, hsa-miR-4640-5p, hsa-miR-663a, hsa-miR-6791-5p, hsa-miR-6826-5p, hsa-miR-4433b-3p, hsa-miR-1915-3p, hsa-miR-4417, hsa-miR-4449, hsa-miR-4707-3p, hsa-miR-3180-3p, hsa-miR-5585-3p, hsa-miR-1268a, hsa-miR-8072, hsa-miR-296-5p, hsa-miR-204-3p, hsa-miR-4454, hsa-miR-6722-3p, hsa-miR-1290, hsa-miR-3622a-5p, hsa-miR-939-5p, hsa-miR-675-5p, hsa-miR-3131, hsa-miR-4648, hsa-miR-1268b, hsa-miR-6741-5p, hsa-miR-6893-5p, hsa-miR-3162-5p, hsa-miR-642b-3p, hsa-miR-4734, hsa-miR-150-3p, hsa-miR-8089, hsa-miR-6805-3p, hsa-miR-7113-3p, hsa-miR-6850-5p, hsa-miR-6799-5p, hsa-miR-6768-5p, hsa-miR-92b-5p, hsa-miR-3679-5p, hsa-miR-4792, hsa-miR-3656, hsa-miR-92a-2-5p, hsa-miR-4466, hsa-miR-4513, hsa-miR-6781-5p, hsa-miR-4649-5p, hsa-miR-6775-5p, hsa-miR-4651, hsa-miR-3195, hsa-miR-6726-5p, hsa-miR-6872-3p, hsa-miR-371a-5p, hsa-miR-6777-5p, hsa-miR-6789-5p, hsa-miR-7975, hsa-miR-6821-5p, hsa-miR-4534, hsa-miR-619-5p, hsa-miR-7107-5p, hsa-miR-1228-3p, hsa-miR-6774-5p, hsa-miR-6805-5p, hsa-miR-23a-3p, hsa-miR-4665-5p, hsa-miR-4505, hsa-miR-4638-5p, hsa-miR-24-3p, hsa-miR-3135b, hsa-miR-4745-5p, hsa-miR-128-1-5p, hsa-miR-4476, hsa-miR-4687-3p, hsa-miR-3665, hsa-miR-6806-5p, hsa-miR-3937, hsa-miR-711, hsa-miR-3141, hsa-miR-3188, hsa-miR-4281, hsa-miR-5196-5p, hsa-miR-6880-5p, hsa-miR-3960, hsa-miR-3648, hsa-miR-6721-5p, hsa-miR-4492, hsa-miR-744-5p, hsa-miR-7704, hsa-miR-4749-5p, hsa-miR-6794-5p, hsa-miR-6511a-5p, hsa-miR-6824-5p, hsa-miR-762, hsa-miR-6836-3p, hsa-miR-6727-5p, hsa-miR-4739, hsa-miR-7977, hsa-miR-4484, hsa-miR-6515-3p, hsa-miR-373-5p, hsa-miR-4258, hsa-miR-4674, hsa-miR-3180, hsa-miR-6076, hsa-miR-1238-5p, hsa-miR-4463, hsa-miR-4486, hsa-miR-4730, hsa-miR-6766-3p, hsa-miR-4286, hsa-miR-6511a-5p, hsa-miR-4739, and hsa-miR-6749-5p, respectively), a congener, a transcript thereof, or/and a variant or a derivative thereof. In this context, the gene, the congener, the transcript, the variant, and the derivative are as defined above.

The target nucleic acid is preferably a human gene comprising a nucleotide sequence represented by any of SEQ ID NOs: 1 to 812 or a transcript thereof, more preferably the transcript, i.e., a miRNA or its precursor RNA (pri-miRNA or pre-miRNA).

The first target gene is the hsa-miR-6784-5p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The second target gene is the hsa-miR-1181 gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The third target gene is the hsa-miR-671-5p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The fourth target gene is the hsa-miR-6857-5p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The fifth target gene is the hsa-miR-4276 gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The sixth target gene is the hsa-miR-1914-3p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The seventh target gene is the hsa-miR-149-3p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The eighth target gene is the hsa-miR-937-5p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The ninth target gene is the hsa-miR-4675 gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 10th target gene is the hsa-miR-6795-5p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 11th target gene is the hsa-miR-4731-5p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 12th target gene is the hsa-miR-5090 gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 13th target gene is the hsa-miR-3620-5p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 14th target gene is the hsa-miR-1343-5p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 15th target gene is the hsa-miR-6717-5p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 16th target gene is the hsa-miR-6825-5p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 17th target gene is the hsa-miR-6738-5p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 18th target gene is the hsa-miR-6769a-5p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 19th target gene is the hsa-miR-4728-5p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 20th target gene is the hsa-miR-652-5p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 21st target gene is the hsa-miR-4257 gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 22nd target gene is the hsa-miR-6785-5p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 23rd target gene is the hsa-miR-7110-5p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 24th target gene is the hsa-miR-6887-5p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 25th target gene is the hsa-miR-887-3p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 26th target gene is the hsa-miR-1228-5p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 27th target gene is the hsa-miR-5572 gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 28th target gene is the hsa-miR-6782-5p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 29th target gene is the hsa-miR-4298 gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 30th target gene is the hsa-miR-6786-5p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 31st target gene is the hsa-miR-5010-5p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 32nd target gene is the hsa-miR-6087 gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 33rd target gene is the hsa-miR-6765-5p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 34th target gene is the hsa-miR-6732-5p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 35th target gene is the hsa-miR-6787-5p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 36th target gene is the hsa-miR-6737-5p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 37th target gene is the hsa-miR-128-2-5p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 38th target gene is the hsa-miR-4270 gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 39th target gene is the hsa-miR-6861-5p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 40th target gene is the hsa-miR-6756-5p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 41st target gene is the hsa-miR-1229-5p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 42nd target gene is the hsa-miR-6891-5p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 43rd target gene is the hsa-miR-6848-5p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 44th target gene is the hsa-miR-1237-5p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 45th target gene is the hsa-miR-30c-1-3p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 46th target gene is the hsa-miR-1233-5p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 47th target gene is the hsa-miR-211-3p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 48th target gene is the hsa-miR-4758-5p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 49th target gene is the hsa-miR-614 gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 50th target gene is the hsa-miR-6746-5p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 51st target gene is the hsa-miR-1915-5p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 52nd target gene is the hsa-miR-4688 gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 53rd target gene is the hsa-miR-3917 gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 54th target gene is the hsa-miR-5787 gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 55th target gene is the hsa-miR-4632-5p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 56th target gene is the hsa-miR-6126 gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 57th target gene is the hsa-miR-135a-3p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 58th target gene is the hsa-miR-8063 gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 59th target gene is the hsa-miR-5698 gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 60th target gene is the hsa-miR-6089 gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 61st target gene is the hsa-miR-498 gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 62nd target gene is the hsa-miR-296-3p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 63rd target gene is the hsa-miR-4419b gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 64th target gene is the hsa-miR-6802-5p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 65th target gene is the hsa-miR-6829-5p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 66th target gene is the hsa-miR-6803-5p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 67th target gene is the hsa-miR-1199-5p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 68th target gene is the hsa-miR-6840-3p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 69th target gene is the hsa-miR-6752-5p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 70th target gene is the hsa-miR-6798-5p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 71st target gene is the hsa-miR-6131 gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 72nd target gene is the hsa-miR-4667-5p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 73rd target gene is the hsa-miR-6510-5p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 74th target gene is the hsa-miR-4690-5p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 75th target gene is the hsa-miR-920 gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 76th target gene is the hsa-miR-23b-3p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 77th target gene is the hsa-miR-4448 gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 78th target gene is the hsa-miR-2110 gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 79th target gene is the hsa-miR-4706 gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 80th target gene is the hsa-miR-7845-5p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 81st target gene is the hsa-miR-6808-5p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 82nd target gene is the hsa-miR-4447 gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 83rd target gene is the hsa-miR-6869-5p gene, a congener thereof, a transcript thereof, or a variant or a derivative thereof. None of the previously known reports show that change in the expression of the gene or the transcript thereof can serve as a marker for early pancreatic cancer or a pancreatic cancer precursor lesion.

The 84th target gene is the hsa-miR-1908-5p gene, a congener thereof, a transcript thereof, or a variant or a derivative thereof. The previously known report shows that change in the expression of the gene or the transcript thereof can serve as a marker for pancreatic cancer (Patent Literature 2 described above).

The 85th target gene is the hsa-miR-6729-5p gene, a congener thereof, a transcript thereof, or a variant or a derivative thereof. The previously known report shows that change in the expression of the gene or the transcript thereof can serve as a marker for pancreatic cancer (Patent Literature 2 described above).

The 86th target gene is the hsa-miR-5195-3p gene, a congener thereof, a transcript thereof, or a variant or a derivative thereof. The previously known report shows that change in the expression of the gene or the transcript thereof can serve as a marker for pancreatic cancer (Patent Literature 2 described above).

The 87th target gene is the hsa-miR-638 gene, a congener thereof, a transcript thereof, or a variant or a derivative thereof. The previously known report shows that change in the expression of the gene or the transcript thereof can serve as a marker for pancreatic cancer (Non-Patent Literature 5 described above).

The 88th target gene is the hsa-miR-6125 gene, a congener thereof, a transcript thereof, or a variant or a derivative thereof. The previously known report shows that change in the expression of the gene or the transcript thereof can serve as a marker for pancreatic cancer (Patent Literature 2 described above).

The 89th target gene is the hsa-miR-3178 gene, a congener thereof, a transcript thereof, or a variant or a derivative thereof. The previously known report shows that change in the expression of the gene or the transcript thereof can serve as a marker for pancreatic cancer (Patent Literature 2 described above).

The 90th target gene is the hsa-miR-3196 gene, a congener thereof, a transcript thereof, or a variant or a derivative thereof. The previously known report shows that change in the expression of the gene or the transcript thereof can serve as a marker for pancreatic cancer (Non-Patent Literature 5 described above).

The 91st target gene is the hsa-miR-8069 gene, a congener thereof, a transcript thereof, or a variant or a derivative thereof. The previously known report shows that change in the expression of the gene or the transcript thereof can serve as a marker for pancreatic cancer (Patent Literature 2 described above).

The 92nd target gene is the hsa-miR-4723-5p gene, a congener thereof, a transcript thereof, or a variant or a derivative thereof. The previously known report shows that change in the expression of the gene or the transcript thereof can serve as a marker for pancreatic cancer (Patent Literature 2 described above).

The 93rd target gene is the hsa-miR-4746-3p gene, a congener thereof, a transcript thereof, or a variant or a derivative thereof. The previously known report shows that change in the expression of the gene or the transcript thereof can serve as a marker for pancreatic cancer (Patent Literature 2 described above).

The 94th target gene is the hsa-miR-4689 gene, a congener thereof, a transcript thereof, or a variant or a derivative thereof. The previously known report shows that change in the expression of the gene or the transcript thereof can serve as a marker for pancreatic cancer (Patent Literature 2 described above).

The 95th target gene is the hsa-miR-6816-5p gene, a congener thereof, a transcript thereof, or a variant or a derivative thereof. The previously known report shows that change in the expression of the gene or the transcript thereof can serve as a marker for pancreatic cancer (Patent Literature 2 described above).

The 96th target gene is the hsa-miR-6757-5p gene, a congener thereof, a transcript thereof, or a variant or a derivative thereof. The previously known report shows that change in the expression of the gene or the transcript thereof can serve as a marker for pancreatic cancer (Patent Literature 2 described above).

The 97th target gene is the hsa-miR-7109-5p gene, a congener thereof, a transcript thereof, or a variant or a derivative thereof. The previously known report shows that change in the expression of the gene or the transcript thereof can serve as a marker for pancreatic cancer (Patent Literature 2 described above).

The 98th target gene is the hsa-miR-6724-5p gene, a congener thereof, a transcript thereof, or a variant or a derivative thereof. The previously known report shows that change in the expression of the gene or the transcript thereof can serve as a marker for pancreatic cancer (Patent Literature 2 described above).

The 99th target gene is the hsa-miR-1225-3p gene, a congener thereof, a transcript thereof, or a variant or a derivative thereof. The previously known report shows that change in the expression of the gene or the transcript thereof can serve as a marker for pancreatic cancer (Non-Patent Literature 5 described above).

The 100th target gene is the hsa-miR-6875-5p gene, a congener thereof, a transcript thereof, or a variant or a derivative thereof. The previously known report shows that change in the expression of the gene or the transcript thereof can serve as a marker for pancreatic cancer (Patent Literature 2 described above).

The 101st target gene is the hsa-miR-7108-5p gene, a congener thereof, a transcript thereof, or a variant or a derivative thereof. The previously known report shows that change in the expression of the gene or the transcript thereof can serve as a marker for pancreatic cancer (Patent Literature 2 described above).

The 102nd target gene is the hsa-miR-4508 gene, a congener thereof, a transcript thereof, or a variant or a derivative thereof. The previously known report shows that change in the expression of the gene or the transcript thereof can serve as a marker for pancreatic cancer (Patent Literature 2 described above).

The 103rd target gene is the hsa-miR-6085 gene, a congener thereof, a transcript thereof, or a variant or a derivative thereof. The previously known report shows that change in the expression of the gene or the transcript thereof can serve as a marker for pancreatic cancer (Patent Literature 2 described above).

The 104th target gene is the hsa-miR-6779-5p gene, a congener thereof, a transcript thereof, or a variant or a derivative thereof. The previously known report shows that change in the expression of the gene or the transcript thereof can serve as a marker for pancreatic cancer (Patent Literature 2 described above).

The 105th target gene is the hsa-miR-642a-3p gene, a congener thereof, a transcript thereof, or a variant or a derivative thereof. The previously known report shows that change in the expression of the gene or the transcript thereof can serve as a marker for pancreatic cancer (Patent Literature 2 described above).

The 106th target gene is the hsa-miR-4695-5p gene, a congener thereof, a transcript thereof, or a variant or a derivative thereof. The previously known report shows that change in the expression of the gene or the transcript thereof can serve as a marker for pancreatic cancer (Non-Patent Literature 5 described above).

The 107th target gene is the hsa-miR-7847-3p gene, a congener thereof, a transcript thereof, or a variant or a derivative thereof. The previously known report shows that change in the expression of the gene or the transcript thereof can serve as a marker for pancreatic cancer (Patent Literature 2 described above).

The 108th target gene is the hsa-miR-3197 gene, a congener thereof, a transcript thereof, or a variant or a derivative thereof. The previously known report shows that change in the expression of the gene or the transcript thereof can serve as a marker for pancreatic cancer (Non-Patent Literature 5 described above).

The 109th target gene is the hsa-miR-6769b-5p gene, a congener thereof, a transcript thereof, or a variant or a derivative thereof. The previously known report shows that change in the expression of the gene or the transcript thereof can serve as a marker for pancreatic cancer (Patent Literature 2 described above).

The 110th target gene is the hsa-miR-7641 gene, a congener thereof, a transcript thereof, or a variant or a derivative thereof. The previously known report shows that change in the expression of the gene or the transcript thereof can serve as a marker for pancreatic cancer (Patent Literature 2 described above).

The 111th target gene is the hsa-miR-187-5p gene, a congener thereof, a transcript thereof, or a variant or a derivative thereof. The previously known report shows that change in the expression of the gene or the transcript thereof can serve as a marker for pancreatic cancer (Patent Literature 2 described above).

The 112th target gene is the hsa-miR-3185 gene, a congener thereof, a transcript thereof, or a variant or a derivative thereof. The previously known report shows that change in the expression of the gene or the transcript thereof can serve as a marker for pancreatic cancer (Patent Literature 2 described above).

The 113th target gene is the hsa-miR-2861 gene, a congener thereof, a transcript thereof, or a variant or a derivative thereof. The previously known report shows that change in the expression of the gene or the transcript thereof can serve as a marker for pancreatic cancer (Patent Literature 2 described above).

The 114th target gene is the hsa-miR-3940-5p gene, a congener thereof, a transcript thereof, or a variant or a derivative thereof. The previously known report shows that change in the expression of the gene or the transcript thereof can serve as a marker for pancreatic cancer (Patent Literature 2 described above).

The 115th target gene is the hsa-miR-1203 gene, a congener thereof, a transcript thereof, or a variant or a derivative thereof. The previously known report shows that change in the expression of the gene or the transcript thereof can serve as a marker for pancreatic cancer (Patent Literature 2 described above).

The 116th target gene is the hsa-miR-615-5p gene, a congener thereof, a transcript thereof, or a variant or a derivative thereof. The previously known report shows that change in the expression of the gene or the transcript thereof can serve as a marker for pancreatic cancer (Patent Literature 2 described above).

The 117th target gene is the hsa-miR-4787-5p gene, a congener thereof, a transcript thereof, or a variant or a derivative thereof. The previously known report shows that change in the expression of the gene or the transcript thereof can serve as a marker for pancreatic cancer (Non-Patent Literature 5 described above).

The 118th target gene is the hsa-miR-1343-3p gene, a congener thereof, a transcript thereof, or a variant or a derivative thereof. The previously known report shows that change in the expression of the gene or the transcript thereof can serve as a marker for pancreatic cancer (Patent Literature 2 described above).

The 119th target gene is the hsa-miR-6813-5p gene, a congener thereof, a transcript thereof, or a variant or a derivative thereof. The previously known report shows that change in the expression of the gene or the transcript thereof can serve as a marker for pancreatic cancer (Patent Literature 2 described above).

The 120th target gene is the hsa-miR-1225-5p gene, a congener thereof, a transcript thereof, or a variant or a derivative thereof. The previously known report shows that change in the expression of the gene or the transcript thereof can serve as a marker for pancreatic cancer (Non-Patent Literature 5 described above).

The 121st target gene is the hsa-miR-602 gene, a congener thereof, a transcript thereof, or a variant or a derivative thereof. The previously known report shows that change in the expression of the gene or the transcript thereof can serve as a marker for pancreatic cancer (Patent Literature 2 described above).

The 122nd target gene is the hsa-miR-4488 gene, a congener thereof, a transcript thereof, or a variant or a derivative thereof. The previously known report shows that change in the expression of the gene or the transcript thereof can serve as a marker for pancreatic cancer (Non-Patent Literature 5 described above).

The 123rd target gene is the hsa-miR-125a-3p gene, a congener thereof, a transcript thereof, or a variant or a derivative thereof. The previously known report shows that change in the expression of the gene or the transcript thereof can serve as a marker for pancreatic cancer (Patent Literature 2 described above).

The 124th target gene is the hsa-miR-5100 gene, a congener thereof, a transcript thereof, or a variant or a derivative thereof. The previously known report shows that change in the expression of the gene or the transcript thereof can serve as a marker for pancreatic cancer (Patent Literature 2 described above).

The 125th target gene is the hsa-miR-4294 gene, a congener thereof, a transcript thereof, or a variant or a derivative thereof. The previously known report shows that change in the expression of the gene or the transcript thereof can serve as a marker for pancreatic cancer (Patent Literature 2 described above).

The 126th target gene is the hsa-miR-1231 gene, a congener thereof, a transcript thereof, or a variant or a derivative thereof. The previously known report shows that change in the expression of the gene or the transcript thereof can serve as a marker for pancreatic cancer (Patent Literature 2 described above).

The 127th target gene is the hsa-miR-6765-3p gene, a congener thereof, a transcript thereof, or a variant or a derivative thereof. The previously known report shows that change in the expression of the gene or the transcript thereof can serve as a marker for pancreatic cancer (Patent Literature 2 described above).

The 128th target gene is the hsa-miR-4442 gene, a congener thereof, a transcript thereof, or a variant or a derivative thereof. The previously known report shows that change in the expression of the gene or the transcript thereof can serve as a marker for pancreatic cancer (Patent Literature 2 described above).

The 129th target gene is the hsa-miR-718 gene, a congener thereof, a transcript thereof, or a variant or a derivative thereof. The previously known report shows that change in the expression of the gene or the transcript thereof can serve as a marker for pancreatic cancer (Patent Literature 2 described above).

The 130th target gene is the hsa-miR-6780b-5p gene, a congener thereof, a transcript thereof, or a variant or a derivative thereof. The previously known report shows that change in the expression of the gene or the transcript thereof can serve as a marker for pancreatic cancer (Patent Literature 2 described above).

The 131st target gene is the hsa-miR-6090 gene, a congener thereof, a transcript thereof, or a variant or a derivative thereof. The previously known report shows that change in the expression of the gene or the transcript thereof can serve as a marker for pancreatic cancer (Patent Literature 2 described above).

The 132nd target gene is the hsa-miR-6845-5p gene, a congener thereof, a transcript thereof, or a variant or a derivative thereof. The previously known report shows that change in the expression of the gene or the transcript thereof can serve as a marker for pancreatic cancer (Patent Literature 2 described above).

The 133rd target gene is the hsa-miR-4741 gene, a congener thereof, a transcript thereof, or a variant or a derivative thereof. The previously known report shows that change in the expression of the gene or the transcript thereof can serve as a marker for pancreatic cancer (Patent Literature 2 described above).

The 134th target gene is the hsa-miR-4467 gene, a congener thereof, a transcript thereof, or a variant or a derivative thereof. The previously known report shows that change in the expression of the gene or the transcript thereof can serve as a marker for pancreatic cancer (Patent Literature 2 described above).

The 135th target gene is the hsa-miR-4707-5p gene, a congener thereof, a transcript thereof, or a variant or a derivative thereof. The previously known report shows that change in the expression of the gene or the transcript thereof can serve as a marker for pancreatic cancer (Patent Literature 2 described above).

The 136th target gene is the hsa-miR-4271 gene, a congener thereof, a transcript thereof, or a variant or a derivative thereof. The previously known report shows that change in the expression of the gene or the transcript thereof can serve as a marker for pancreatic cancer (Patent Literature 2 described above).

The 137th target gene is the hsa-miR-4673 gene, a congener thereof, a transcript thereof, or a variant or a derivative thereof. The previously known report shows that change in the expression of the gene or the transcript thereof can serve as a marker for pancreatic cancer (Patent Literature 2 described above).

The 138th target gene is the hsa-miR-3184-5p gene, a congener thereof, a transcript thereof, or a variant or a derivative thereof. The previously known report shows that change in the expression of the gene or the transcript thereof can serve as a marker for pancreatic cancer (Patent Literature 2 described above).

The 139th target gene is the hsa-miR-1469 gene, a congener thereof, a transcript thereof, or a variant or a derivative thereof. The previously known report shows that change in the expression of the gene or the transcript thereof can serve as a marker for pancreatic cancer (Patent Literature 2 described above).

The 140th target gene is the hsa-miR-4640-5p gene, a congener thereof, a transcript thereof, or a variant or a derivative thereof. The previously known report shows that change in the expression of the gene or the transcript thereof can serve as a marker for pancreatic cancer (Non-Patent Literature 5 described above).

The 141st target gene is the hsa-miR-663a gene, a congener thereof, a transcript thereof, or a variant or a derivative thereof. The previously known report shows that change in the expression of the gene or the transcript thereof can serve as a marker for pancreatic cancer (Patent Literature 2 described above).

The 142nd target gene is the hsa-miR-6791-5p gene, a congener thereof, a transcript thereof, or a variant or a derivative thereof. The previously known report shows that change in the expression of the gene or the transcript thereof can serve as a marker for pancreatic cancer (Patent Literature 2 described above).

The 143rd target gene is the hsa-miR-6826-5p gene, a congener thereof, a transcript thereof, or a variant or a derivative thereof. The previously known report shows that change in the expression of the gene or the transcript thereof can serve as a marker for pancreatic cancer (Patent Literature 2 described above).

The 144th target gene is the hsa-miR-4433b-3p gene, a congener thereof, a transcript thereof, or a variant or a derivative thereof. The previously known report shows that change in the expression of the gene or the transcript thereof can serve as a marker for pancreatic cancer (Patent Literature 2 described above).

The 145th target gene is the hsa-miR-1915-3p gene, a congener thereof, a transcript thereof, or a variant or a derivative thereof. The previously known report shows that change in the expression of the gene or the transcript thereof can serve as a marker for pancreatic cancer (Patent Literature 2 described above).

The 146th target gene is the hsa-miR-4417 gene, a congener thereof, a transcript thereof, or a variant or a derivative thereof. The previously known report shows that change in the expression of the gene or the transcript thereof can serve as a marker for pancreatic cancer (Patent Literature 2 described above).

The 147th target gene is the hsa-miR-4449 gene, a congener thereof, a transcript thereof, or a variant or a derivative thereof. The previously known report shows that change in the expression of the gene or the transcript thereof can serve as a marker for pancreatic cancer (Patent Literature 2 described above).

The 148th target gene is the hsa-miR-4707-3p gene, a congener thereof, a transcript thereof, or a variant or a derivative thereof. The previously known report shows that change in the expression of the gene or the transcript thereof can serve as a marker for pancreatic cancer (Non-Patent Literature 5 described above).

The 149th target gene is the hsa-miR-3180-3p gene, a congener thereof, a transcript thereof, or a variant or a derivative thereof. The previously known report shows that change in the expression of the gene or the transcript thereof can serve as a marker for pancreatic cancer (Non-Patent Literature 5 described above).

The 150th target gene is the hsa-miR-5585-3p gene, a congener thereof, a transcript thereof, or a variant or a derivative thereof. The previously known report shows that change in the expression of the gene or the transcript thereof can serve as a marker for pancreatic cancer (Patent Literature 2 described above).

The 151st target gene is the hsa-miR-1268a gene, a congener thereof, a transcript thereof, or a variant or a derivative thereof. The previously known report shows that change in the expression of the gene or the transcript thereof can serve as a marker for early pancreatic cancer or a pancreatic cancer precursor lesion (Patent Literature 4 described above).

The 152nd target gene is the hsa-miR-8072 gene, a congener thereof, a transcript thereof, or a variant or a derivative thereof. The previously known report shows that change in the expression of the gene or the transcript thereof can serve as a marker for pancreatic cancer (Patent Literature 2 described above).

The 153rd target gene is the hsa-miR-296-5p gene, a congener thereof, a transcript thereof, or a variant or a derivative thereof. The previously known report shows that change in the expression of the gene or the transcript thereof can serve as a marker for early pancreatic cancer or a pancreatic cancer precursor lesion (Patent Literature 5 described above).

The 154th target gene is the hsa-miR-204-3p gene, a congener thereof, a transcript thereof, or a variant or a derivative thereof. The previously known report shows that change in the expression of the gene or the transcript thereof can serve as a marker for pancreatic cancer (Patent Literature 2 described above).

The 155th target gene is the hsa-miR-4454 gene, a congener thereof, a transcript thereof, or a variant or a derivative thereof. The previously known report shows that change in the expression of the gene or the transcript thereof can serve as a marker for pancreatic cancer (Patent Literature 2 described above).

The 156th target gene is the hsa-miR-6722-3p gene, a congener thereof, a transcript thereof, or a variant or a derivative thereof. The previously known report shows that change in the expression of the gene or the transcript thereof can serve as a marker for pancreatic cancer (Patent Literature 2 described above).

The 157th target gene is the hsa-miR-1290 gene, a congener thereof, a transcript thereof, or a variant or a derivative thereof. The previously known report shows that change in the expression of the gene or the transcript thereof can serve as a marker for pancreatic cancer (Patent Literature 2 described above).

The 158th target gene is the hsa-miR-3622a-5p gene, a congener thereof, a transcript thereof, or a variant or a derivative thereof. The previously known report shows that change in the expression of the gene or the transcript thereof can serve as a marker for pancreatic cancer (Patent Literature 2 described above).

The 159th target gene is the hsa-miR-939-5p gene, a congener thereof, a transcript thereof, or a variant or a derivative thereof. The previously known report shows that change in the expression of the gene or the transcript thereof can serve as a marker for early pancreatic cancer or a pancreatic cancer precursor lesion (Patent Literature 4 described above).

The 160th target gene is the hsa-miR-675-5p gene, a congener thereof, a transcript thereof, or a variant or a derivative thereof. The previously known report shows that change in the expression of the gene or the transcript thereof can serve as a marker for pancreatic cancer (Non-Patent Literature 5 described above).

The 161st target gene is the hsa-miR-3131 gene, a congener thereof, a transcript thereof, or a variant or a derivative thereof. The previously known report shows that change in the expression of the gene or the transcript thereof can serve as a marker for pancreatic cancer (Patent Literature 2 described above).

The 162nd target gene is the hsa-miR-4648 gene, a congener thereof, a transcript thereof, or a variant or a derivative thereof. The previously known report shows that change in the expression of the gene or the transcript thereof can serve as a marker for pancreatic cancer (Patent Literature 2 described above).

The 163rd target gene is the hsa-miR-1268b gene, a congener thereof, a transcript thereof, or a variant or a derivative thereof. The previously known report shows that change in the expression of the gene or the transcript thereof can serve as a marker for pancreatic cancer (Non-Patent Literature 5 described above).

The 164th target gene is the hsa-miR-6741-5p gene, a congener thereof, a transcript thereof, or a variant or a derivative thereof. The previously known report shows that change in the expression of the gene or the transcript thereof can serve as a marker for pancreatic cancer (Patent Literature 2 described above).

The 165th target gene is the hsa-miR-6893-5p gene, a congener thereof, a transcript thereof, or a variant or a derivative thereof. The previously known report shows that change in the expression of the gene or the transcript thereof can serve as a marker for pancreatic cancer (Patent Literature 2 described above).

The 166th target gene is the hsa-miR-3162-5p gene, a congener thereof, a transcript thereof, or a variant or a derivative thereof. The previously known report shows that change in the expression of the gene or the transcript thereof can serve as a marker for pancreatic cancer (Patent Literature 2 described above).

The 167th target gene is the hsa-miR-642b-3p gene, a congener thereof, a transcript thereof, or a variant or a derivative thereof. The previously known report shows that change in the expression of the gene or the transcript thereof can serve as a marker for early pancreatic cancer or a pancreatic cancer precursor lesion (Patent Literature 4 described above).

The 168th target gene is the hsa-miR-4734 gene, a congener thereof, a transcript thereof, or a variant or a derivative thereof. The previously known report shows that change in the expression of the gene or the transcript thereof can serve as a marker for pancreatic cancer (Patent Literature 2 described above).

The 169th target gene is the hsa-miR-150-3p gene, a congener thereof, a transcript thereof, or a variant or a derivative thereof. The previously known report shows that change in the expression of the gene or the transcript thereof can serve as a marker for pancreatic cancer (Patent Literature 2 described above).

The 170th target gene is the hsa-miR-8089 gene, a congener thereof, a transcript thereof, or a variant or a derivative thereof. The previously known report shows that change in the expression of the gene or the transcript thereof can serve as a marker for pancreatic cancer (Patent Literature 2 described above).

The 171st target gene is the hsa-miR-6805-3p gene, a congener thereof, a transcript thereof, or a variant or a derivative thereof. The previously known report shows that change in the expression of the gene or the transcript thereof can serve as a marker for pancreatic cancer (Patent Literature 2 described above).

The 172nd target gene is the hsa-miR-7113-3p gene, a congener thereof, a transcript thereof, or a variant or a derivative thereof. The previously known report shows that change in the expression of the gene or the transcript thereof can serve as a marker for pancreatic cancer (Patent Literature 2 described above).

The 173rd target gene is the hsa-miR-6850-5p gene, a congener thereof, a transcript thereof, or a variant or a derivative thereof. The previously known report shows that change in the expression of the gene or the transcript thereof can serve as a marker for pancreatic cancer (Patent Literature 2 described above).

The 174th target gene is the hsa-miR-6799-5p gene, a congener thereof, a transcript thereof, or a variant or a derivative thereof. The previously known report shows that change in the expression of the gene or the transcript thereof can serve as a marker for pancreatic cancer (Patent Literature 2 described above).

The 175th target gene is the hsa-miR-6768-5p gene, a congener thereof, a transcript thereof, or a variant or a derivative thereof. The previously known report shows that change in the expression of the gene or the transcript thereof can serve as a marker for pancreatic cancer (Patent Literature 2 described above).

The 176th target gene is the hsa-miR-92b-5p gene, a congener thereof, a transcript thereof, or a variant or a derivative thereof. The previously known report shows that change in the expression of the gene or the transcript thereof can serve as a marker for pancreatic cancer (Patent Literature 2 described above).

The 177th target gene is the hsa-miR-3679-5p gene, a congener thereof, a transcript thereof, or a variant or a derivative thereof. The previously known report shows that change in the expression of the gene or the transcript thereof can serve as a marker for pancreatic cancer (Non-Patent Literature 5 described above).

The 178th target gene is the hsa-miR-4792 gene, a congener thereof, a transcript thereof, or a variant or a derivative thereof. The previously known report shows that change in the expression of the gene or the transcript thereof can serve as a marker for pancreatic cancer (Patent Literature 2 described above).

The 179th target gene is the hsa-miR-3656 gene, a congener thereof, a transcript thereof, or a variant or a derivative thereof. The previously known report shows that change in the expression of the gene or the transcript thereof can serve as a marker for pancreatic cancer (Patent Literature 2 described above).

The 180th target gene is the hsa-miR-92a-2-5p gene, a congener thereof, a transcript thereof, or a variant or a derivative thereof. The previously known report shows that change in the expression of the gene or the transcript thereof can serve as a marker for pancreatic cancer (Patent Literature 2 described above).

The 181st target gene is the hsa-miR-4466 gene, a congener thereof, a transcript thereof, or a variant or a derivative thereof. The previously known report shows that change in the expression of the gene or the transcript thereof can serve as a marker for pancreatic cancer (Non-Patent Literature 5 described above).

The 182nd target gene is the hsa-miR-4513 gene, a congener thereof, a transcript thereof, or a variant or a derivative thereof. The previously known report shows that change in the expression of the gene or the transcript thereof can serve as a marker for pancreatic cancer (Patent Literature 2 described above).

The 183rd target gene is the hsa-miR-6781-5p gene, a congener thereof, a transcript thereof, or a variant or a derivative thereof. The previously known report shows that change in the expression of the gene or the transcript thereof can serve as a marker for pancreatic cancer (Patent Literature 2 described above).

The 184th target gene is the hsa-miR-4649-5p gene, a congener thereof, a transcript thereof, or a variant or a derivative thereof. The previously known report shows that change in the expression of the gene or the transcript thereof can serve as a marker for pancreatic cancer (Patent Literature 2 described above).

The 185th target gene is the hsa-miR-6775-5p gene, a congener thereof, a transcript thereof, or a variant or a derivative thereof. The previously known report shows that change in the expression of the gene or the transcript thereof can serve as a marker for pancreatic cancer (Patent Literature 2 described above).

The 186th target gene is the hsa-miR-4651 gene, a congener thereof, a transcript thereof, or a variant or a derivative thereof. The previously known report shows that change in the expression of the gene or the transcript thereof can serve as a marker for pancreatic cancer (Patent Literature 2 described above).

The 187th target gene is the hsa-miR-3195 gene, a congener thereof, a transcript thereof, or a variant or a derivative thereof. The previously known report shows that change in the expression of the gene or the transcript thereof can serve as a marker for pancreatic cancer (Non-Patent Literature 5 described above).

The 188th target gene is the hsa-miR-6726-5p gene, a congener thereof, a transcript thereof, or a variant or a derivative thereof. The previously known report shows that change in the expression of the gene or the transcript thereof can serve as a marker for pancreatic cancer (Patent Literature 2 described above).

The 189th target gene is the hsa-miR-6872-3p gene, a congener thereof, a transcript thereof, or a variant or a derivative thereof. The previously known report shows that change in the expression of the gene or the transcript thereof can serve as a marker for pancreatic cancer (Patent Literature 2 described above).

The 190th target gene is the hsa-miR-371a-5p gene, a congener thereof, a transcript thereof, or a variant or a derivative thereof. The previously known report shows that change in the expression of the gene or the transcript thereof can serve as a marker for pancreatic cancer (Patent Literature 2 described above).

The 191st target gene is the hsa-miR-6777-5p gene, a congener thereof, a transcript thereof, or a variant or a derivative thereof. The previously known report shows that change in the expression of the gene or the transcript thereof can serve as a marker for pancreatic cancer (Patent Literature 2 described above).

The 192nd target gene is the hsa-miR-6789-5p gene, a congener thereof, a transcript thereof, or a variant or a derivative thereof. The previously known report shows that change in the expression of the gene or the transcript thereof can serve as a marker for pancreatic cancer (Patent Literature 2 described above).

The 193rd target gene is the hsa-miR-7975 gene, a congener thereof, a transcript thereof, or a variant or a derivative thereof. The previously known report shows that change in the expression of the gene or the transcript thereof can serve as a marker for pancreatic cancer (Patent Literature 2 described above).

The 194th target gene is the hsa-miR-6821-5p gene, a congener thereof, a transcript thereof, or a variant or a derivative thereof. The previously known report shows that change in the expression of the gene or the transcript thereof can serve as a marker for pancreatic cancer (Patent Literature 2 described above).

The 195th target gene is the hsa-miR-4534 gene, a congener thereof, a transcript thereof, or a variant or a derivative thereof. The previously known report shows that change in the expression of the gene or the transcript thereof can serve as a marker for pancreatic cancer (Patent Literature 2 described above).

The 196th target gene is the hsa-miR-619-5p gene, a congener thereof, a transcript thereof, or a variant or a derivative thereof. The previously known report shows that change in the expression of the gene or the transcript thereof can serve as a marker for pancreatic cancer (Patent Literature 2 described above).

The 197th target gene is the hsa-miR-7107-5p gene, a congener thereof, a transcript thereof, or a variant or a derivative thereof. The previously known report shows that change in the expression of the gene or the transcript thereof can serve as a marker for pancreatic cancer (Patent Literature 2 described above).

The 198th target gene is the hsa-miR-1228-3p gene, a congener thereof, a transcript thereof, or a variant or a derivative thereof. The previously known report shows that change in the expression of the gene or the transcript thereof can serve as a marker for pancreatic cancer (Patent Literature 2 described above).

The 199th target gene is the hsa-miR-6774-5p gene, a congener thereof, a transcript thereof, or a variant or a derivative thereof. The previously known report shows that change in the expression of the gene or the transcript thereof can serve as a marker for pancreatic cancer (Patent Literature 2 described above).

The 200th target gene is the hsa-miR-6805-5p gene, a congener thereof, a transcript thereof, or a variant or a derivative thereof. The previously known report shows that change in the expression of the gene or the transcript thereof can serve as a marker for pancreatic cancer (Patent Literature 2 described above).

The 201st target gene is the hsa-miR-23a-3p gene, a congener thereof, a transcript thereof, or a variant or a derivative thereof. The previously known report shows that change in the expression of the gene or the transcript thereof can serve as a marker for pancreatic cancer (Patent Literature 3 described above).

The 202nd target gene is the hsa-miR-4665-5p gene, a congener thereof, a transcript thereof, or a variant or a derivative thereof. The previously known report shows that change in the expression of the gene or the transcript thereof can serve as a marker for pancreatic cancer (Patent Literature 2 described above).

The 203rd target gene is the hsa-miR-4505 gene, a congener thereof, a transcript thereof, or a variant or a derivative thereof. The previously known report shows that change in the expression of the gene or the transcript thereof can serve as a marker for pancreatic cancer (Patent Literature 2 described above).

The 204th target gene is the hsa-miR-4638-5p gene, a congener thereof, a transcript thereof, or a variant or a derivative thereof. The previously known report shows that change in the expression of the gene or the transcript thereof can serve as a marker for pancreatic cancer (Patent Literature 2 described above).

The 205th target gene is the hsa-miR-24-3p gene, a congener thereof, a transcript thereof, or a variant or a derivative thereof. The previously known report shows that change in the expression of the gene or the transcript thereof can serve as a marker for pancreatic cancer (Patent Literature 2 described above).

The 206th target gene is the hsa-miR-3135b gene, a congener thereof, a transcript thereof, or a variant or a derivative thereof. The previously known report shows that change in the expression of the gene or the transcript thereof can serve as a marker for pancreatic cancer (Patent Literature 2 described above).

The 207th target gene is the hsa-miR-4745-5p gene, a congener thereof, a transcript thereof, or a variant or a derivative thereof. The previously known report shows that change in the expression of the gene or the transcript thereof can serve as a marker for pancreatic cancer (Non-Patent Literature 5 described above).

The 208th target gene is the hsa-miR-128-1-5p gene, a congener thereof, a transcript thereof, or a variant or a derivative thereof. The previously known report shows that change in the expression of the gene or the transcript thereof can serve as a marker for pancreatic cancer (Patent Literature 2 described above).

The 209th target gene is the hsa-miR-4476 gene, a congener thereof, a transcript thereof, or a variant or a derivative thereof. The previously known report shows that change in the expression of the gene or the transcript thereof can serve as a marker for pancreatic cancer (Patent Literature 2 described above).

The 210th target gene is the hsa-miR-4687-3p gene, a congener thereof, a transcript thereof, or a variant or a derivative thereof. The previously known report shows that change in the expression of the gene or the transcript thereof can serve as a marker for pancreatic cancer (Patent Literature 2 described above).

The 211th target gene is the hsa-miR-3665 gene, a congener thereof, a transcript thereof, or a variant or a derivative thereof. The previously known report shows that change in the expression of the gene or the transcript thereof can serve as a marker for pancreatic cancer (Non-Patent Literature 5 described above).

The 212th target gene is the hsa-miR-6806-5p gene, a congener thereof, a transcript thereof, or a variant or a derivative thereof. The previously known report shows that change in the expression of the gene or the transcript thereof can serve as a marker for pancreatic cancer (Patent Literature 2 described above).

The 213th target gene is the hsa-miR-3937 gene, a congener thereof, a transcript thereof, or a variant or a derivative thereof. The previously known report shows that change in the expression of the gene or the transcript thereof can serve as a marker for pancreatic cancer (Non-Patent Literature 5 described above).

The 214th target gene is the hsa-miR-711 gene, a congener thereof, a transcript thereof, or a variant or a derivative thereof. The previously known report shows that change in the expression of the gene or the transcript thereof can serve as a marker for pancreatic cancer (Non-Patent Literature 5 described above).

The 215th target gene is the hsa-miR-3141 gene, a congener thereof, a transcript thereof, or a variant or a derivative thereof. The previously known report shows that change in the expression of the gene or the transcript thereof can serve as a marker for pancreatic cancer (Non-Patent Literature 5 described above).

The 216th target gene is the hsa-miR-3188 gene, a congener thereof, a transcript thereof, or a variant or a derivative thereof. The previously known report shows that change in the expression of the gene or the transcript thereof can serve as a marker for pancreatic cancer (Patent Literature 2 described above).

The 217th target gene is the hsa-miR-4281 gene, a congener thereof, a transcript thereof, or a variant or a derivative thereof. The previously known report shows that change in the expression of the gene or the transcript thereof can serve as a marker for pancreatic cancer (Patent Literature 2 described above).

The 218th target gene is the hsa-miR-5196-5p gene, a congener thereof, a transcript thereof, or a variant or a derivative thereof. The previously known report shows that change in the expression of the gene or the transcript thereof can serve as a marker for pancreatic cancer (Patent Literature 2 described above).

The 219th target gene is the hsa-miR-6880-5p gene, a congener thereof, a transcript thereof, or a variant or a derivative thereof. The previously known report shows that change in the expression of the gene or the transcript thereof can serve as a marker for pancreatic cancer (Patent Literature 2 described above).

The 220th target gene is the hsa-miR-3960 gene, a congener thereof, a transcript thereof, or a variant or a derivative thereof. The previously known report shows that change in the expression of the gene or the transcript thereof can serve as a marker for pancreatic cancer (Non-Patent Literature 5 described above).

The 221st target gene is the hsa-miR-3648 gene, a congener thereof, a transcript thereof, or a variant or a derivative thereof. The previously known report shows that change in the expression of the gene or the transcript thereof can serve as a marker for pancreatic cancer (Patent Literature 1 described above).

The 222nd target gene is the hsa-miR-6721-5p gene, a congener thereof, a transcript thereof, or a variant or a derivative thereof. The previously known report shows that change in the expression of the gene or the transcript thereof can serve as a marker for pancreatic cancer (Patent Literature 2 described above).

The 223rd target gene is the hsa-miR-4492 gene, a congener thereof, a transcript thereof, or a variant or a derivative thereof. The previously known report shows that change in the expression of the gene or the transcript thereof can serve as a marker for pancreatic cancer (Non-Patent Literature 5 described above).

The 224th target gene is the hsa-miR-744-5p gene, a congener thereof, a transcript thereof, or a variant or a derivative thereof. The previously known report shows that change in the expression of the gene or the transcript thereof can serve as a marker for pancreatic cancer (Non-Patent Literature 5 described above).

The 225th target gene is the hsa-miR-7704 gene, a congener thereof, a transcript thereof, or a variant or a derivative thereof. The previously known report shows that change in the expression of the gene or the transcript thereof can serve as a marker for pancreatic cancer (Patent Literature 2 described above).

The 226th target gene is the hsa-miR-4749-5p gene, a congener thereof, a transcript thereof, or a variant or a derivative thereof. The previously known report shows that change in the expression of the gene or the transcript thereof can serve as a marker for pancreatic cancer (Non-Patent Literature 5 described above).

The 227th target gene is the hsa-miR-6794-5p gene, a congener thereof, a transcript thereof, or a variant or a derivative thereof. None of the previously known report shows that change in the expression of the gene or the transcript thereof can serve as a marker for pancreatic cancer.

The 228th target gene is the hsa-miR-6511a-5p gene, a congener thereof, a transcript thereof, or a variant or a derivative thereof. None of the previously known report shows that change in the expression of the gene or the transcript thereof can serve as a marker for pancreatic cancer.

The 229th target gene is the hsa-miR-6824-5p gene, a congener thereof, a transcript thereof, or a variant or a derivative thereof. None of the previously known report shows that change in the expression of the gene or the transcript thereof can serve as a marker for pancreatic cancer.

The 230th target gene is the hsa-miR-762 gene, a congener thereof, a transcript thereof, or a variant or a derivative thereof. The previously known report shows that change in the expression of the gene or the transcript thereof can serve as a marker for pancreatic cancer (Non-Patent Literature 5 described above).

The 231st target gene is the hsa-miR-6836-3p gene, a congener thereof, a transcript thereof, or a variant or a derivative thereof. The previously known report shows that change in the expression of the gene or the transcript thereof can serve as a marker for pancreatic cancer (Patent Literature 2 described above).

The 232nd target gene is the hsa-miR-6727-5p gene, a congener thereof, a transcript thereof, or a variant or a derivative thereof. The previously known report shows that change in the expression of the gene or the transcript thereof can serve as a marker for pancreatic cancer (Patent Literature 2 described above).

The 233rd target gene is the hsa-miR-4739 gene, a congener thereof, a transcript thereof, or a variant or a derivative thereof. The previously known report shows that change in the expression of the gene or the transcript thereof can serve as a marker for pancreatic cancer (Non-Patent Literature 5 described above).

The 234th target gene is the hsa-miR-7977 gene, a congener thereof, a transcript thereof, or a variant or a derivative thereof. The previously known report shows that change in the expression of the gene or the transcript thereof can serve as a marker for pancreatic cancer (Patent Literature 2 described above).

The 235th target gene is the hsa-miR-4484 gene, a congener thereof, a transcript thereof, or a variant or a derivative thereof. The previously known report shows that change in the expression of the gene or the transcript thereof can serve as a marker for pancreatic cancer (Patent Literature 2 described above).

The 236th target gene is the hsa-miR-6515-3p gene, a congener thereof, a transcript thereof, or a variant or a derivative thereof. The previously known report shows that change in the expression of the gene or the transcript thereof can serve as a marker for pancreatic cancer (Patent Literature 2 described above).

The 237th target gene is the hsa-miR-373-5p gene, a congener thereof, a transcript thereof, or a variant or a derivative thereof. The previously known report shows that change in the expression of the gene or the transcript thereof can serve as a marker for pancreatic cancer (Non-Patent Literature 5 described above).

The 238th target gene is the hsa-miR-4258 gene, a congener thereof, a transcript thereof, or a variant or a derivative thereof. The previously known report shows that change in the expression of the gene or the transcript thereof can serve as a marker for pancreatic cancer (Patent Literature 2 described above).

The 239th target gene is the hsa-miR-4674 gene, a congener thereof, a transcript thereof, or a variant or a derivative thereof. The previously known report shows that change in the expression of the gene or the transcript thereof can serve as a marker for pancreatic cancer (Patent Literature 2 described above).

The 240th target gene is the hsa-miR-3180 gene, a congener thereof, a transcript thereof, or a variant or a derivative thereof. The previously known report shows that change in the expression of the gene or the transcript thereof can serve as a marker for pancreatic cancer (Non-Patent Literature 5 described above).

The 241st target gene is the hsa-miR-6076 gene, a congener thereof, a transcript thereof, or a variant or a derivative thereof. The previously known report shows that change in the expression of the gene or the transcript thereof can serve as a marker for pancreatic cancer (Patent Literature 2 described above).

The 242nd target gene is the hsa-miR-1238-5p gene, a congener thereof, a transcript thereof, or a variant or a derivative thereof. The previously known report shows that change in the expression of the gene or the transcript thereof can serve as a marker for pancreatic cancer (Patent Literature 2 described above).

The 243rd target gene is the hsa-miR-4463 gene, a congener thereof, a transcript thereof, or a variant or a derivative thereof. The previously known report shows that change in the expression of the gene or the transcript thereof can serve as a marker for pancreatic cancer (Non-Patent Literature 5 described above).

The 244th target gene is the hsa-miR-4486 gene, a congener thereof, a transcript thereof, or a variant or a derivative thereof. The previously known report shows that change in the expression of the gene or the transcript thereof can serve as a marker for pancreatic cancer (Patent Literature 2 described above).

The 245th target gene is the hsa-miR-4730 gene, a congener thereof, a transcript thereof, or a variant or a derivative thereof. The previously known report shows that change in the expression of the gene or the transcript thereof can serve as a marker for pancreatic cancer (Non-Patent Literature 5 described above).

The 246th target gene is the hsa-miR-6766-3p gene, a congener thereof, a transcript thereof, or a variant or a derivative thereof. None of the previously known report shows that change in the expression of the gene or the transcript thereof can serve as a marker for pancreatic cancer.

The 247th target gene is the hsa-miR-4286 gene, a congener thereof, a transcript thereof, or a variant or a derivative thereof. The previously known report shows that change in the expression of the gene or the transcript thereof can serve as a marker for pancreatic cancer (Patent Literature 2 described above).

The 248th target gene is the hsa-miR-6511a-5p gene, a congener thereof, a transcript thereof, or a variant or a derivative thereof. None of the previously known report shows that change in the expression of the gene or the transcript thereof can serve as a marker for pancreatic cancer.

The 249th target gene is the hsa-miR-4739 gene, a congener thereof, a transcript thereof, or a variant or a derivative thereof. The previously known report shows that change in the expression of the gene or the transcript thereof can serve as a marker for pancreatic cancer (Non-Patent Literature 5 described above).

The 250th target gene is the hsa-miR-6749-5p gene, a congener thereof, a transcript thereof, or a variant or a derivative thereof. None of the previously known report shows that change in the expression of the gene or the transcript thereof can serve as a marker for pancreatic cancer.

### 2. Nucleic acid probe or primer for detection of early pancreatic cancer or a pancreatic cancer precursor lesion

In the present invention, a nucleic acid capable of specifically binding to any of the target nucleic acids as the early pancreatic cancer or pancreatic cancer precursor lesion markers described above can be used as a nucleic acid, for example, a nucleic acid probe or a primer, for the detection or diagnosis of early pancreatic cancer or a pancreatic cancer precursor lesion.

In the present invention, the nucleic acid probes or the primers that can be used for detecting early pancreatic cancer or a pancreatic cancer precursor lesion or for diagnosing early pancreatic cancer or a pancreatic cancer precursor lesion enable qualitative and/or quantitative measurement of the presence, expression level, or existing amount (abundance) of: any of human-derived hsa-miR-6784-5p, hsa-miR-1181, hsa-miR-671-5p, hsa-miR-6857-5p, hsa-miR-4276, hsa-miR-1914-3p, hsa-miR-149-3p, hsa-miR-937-5p, hsa-miR-4675, hsa-miR-6795-5p, hsa-miR-4731-5p, hsa-miR-5090, hsa-miR-3620-5p, hsa-miR-1343-5p, hsa-miR-6717-5p, hsa-miR-6825-5p, hsa-miR-6738-5p, hsa-miR-6769a-5p, hsa-miR-4728-5p, hsa-miR-652-5p, hsa-miR-4257, hsa-miR-6785-5p, hsa-miR-7110-5p, hsa-miR-6887-5p, hsa-miR-887-3p, hsa-miR-1228-5p, hsa-miR-5572, hsa-miR-6782-5p, hsa-miR-4298, hsa-miR-6786-5p, hsa-miR-5010-5p, hsa-miR-6087, hsa-miR-6765-5p, hsa-miR-6732-5p, hsa-miR-6787-5p, hsa-miR-6737-5p, hsa-miR-128-2-5p, hsa-miR-4270, hsa-miR-6861-5p, hsa-miR-6756-5p, hsa-miR-1229-5p, hsa-miR-6891-5p, hsa-miR-6848-5p, hsa-miR-1237-5p, hsa-miR-30c-1-3p, hsa-miR-1233-5p, hsa-miR-211-3p, hsa-miR-4758-5p, hsa-miR-614, hsa-miR-6746-5p, hsa-miR-1915-5p, hsa-miR-4688, hsa-miR-3917, hsa-miR-5787, hsa-miR-4632-5p, hsa-miR-6126, hsa-miR-135a-3p, hsa-miR-8063, hsa-miR-5698, hsa-miR-6089, hsa-miR-498, hsa-miR-296-3p, hsa-miR-4419b, hsa-miR-6802-5p, hsa-miR-6829-5p, hsa-miR-6803-5p, hsa-miR-1199-5p, hsa-miR-6840-3p, hsa-miR-6752-5p, hsa-miR-6798-5p, hsa-miR-6131, hsa-miR-4667-5p, hsa-miR-6510-5p, hsa-miR-4690-5p, hsa-miR-920, hsa-miR-23b-3p, hsa-miR-4448, hsa-miR-2110, hsa-miR-4706, hsa-miR-7845-5p, hsa-miR-6808-5p, hsa-miR-4447, hsa-miR-6869-5p, hsa-miR-6794-5p, hsa-miR-6511a-5p, hsa-miR-6824-5p, hsa-miR-6766-3p, hsa-miR-6511a-5p, and hsa-miR-6749-5p, as target nucleic acids for early pancreatic cancer or a pancreatic cancer precursor lesion, or a combination thereof; and hsa-miR-1908-5p, hsa-miR-6729-5p, hsa-miR-5195-3p, hsa-miR-638, hsa-miR-6125, hsa-miR-3178, hsa-miR-3196, hsa-miR-8069, hsa-miR-4723-5p, hsa-miR-4746-3p, hsa-miR-4689, hsa-miR-6816-5p, hsa-miR-6757-5p, hsa-miR-7109-5p, hsa-miR-6724-5p, hsa-miR-1225-3p, hsa-miR-6875-5p, hsa-miR-7108-5p, hsa-miR-4508, hsa-miR-6085, hsa-miR-6779-5p, hsa-miR-642a-3p, hsa-miR-4695-5p, hsa-miR-7847-3p, hsa-miR-3197, hsa-miR-6769b-5p, hsa-miR-7641, hsa-miR-187-5p, hsa-miR-3185, hsa-miR-2861, hsa-miR-3940-5p, hsa-miR-1203, hsa-miR-615-5p, hsa-miR-4787-5p, hsa-miR-1343-3p, hsa-miR-6813-5p, hsa-miR-1225-5p, hsa-miR-602, hsa-miR-4488, hsa-miR-125a-3p, hsa-miR-5100, hsa-miR-4294, hsa-miR-1231, hsa-miR-6765-3p, hsa-miR-4442, hsa-miR-718, hsa-miR-6780b-5p, hsa-miR-6090, hsa-miR-6845-5p, hsa-miR-4741, hsa-miR-4467, hsa-miR-4707-5p, hsa-miR-4271, hsa-miR-4673, hsa-miR-3184-5p, hsa-miR-1469, hsa-miR-4640-5p, hsa-miR-663a, hsa-miR-6791-5p, hsa-miR-6826-5p, hsa-miR-4433b-3p, hsa-miR-1915-3p, hsa-miR-4417, hsa-miR-4449, hsa-miR-4707-3p, hsa-miR-3180-3p, hsa-miR-5585-3p, hsa-miR-1268a, hsa-miR-8072, hsa-miR-296-5p, hsa-miR-204-3p, hsa-miR-4454, hsa-miR-6722-3p, hsa-miR-1290, hsa-miR-3622a-5p, hsa-miR-939-5p, hsa-miR-675-5p, hsa-miR-3131, hsa-miR-4648, hsa-miR-1268b, hsa-miR-6741-5p, hsa-miR-6893-5p, hsa-miR-3162-5p, hsa-miR-642b-3p, hsa-miR-4734, hsa-miR-150-3p, hsa-miR-8089, hsa-miR-6805-3p, hsa-miR-7113-3p, hsa-miR-6850-5p, hsa-miR-6799-5p, hsa-miR-6768-5p, hsa-miR-92b-5p, hsa-miR-3679-5p, hsa-miR-4792, hsa-miR-3656, hsa-miR-92a-2-5p, hsa-miR-4466, hsa-miR-4513, hsa-miR-6781-5p, hsa-miR-4649-5p, hsa-miR-6775-5p, hsa-miR-4651, hsa-miR-3195, hsa-miR-6726-5p, hsa-miR-6872-3p, hsa-miR-371a-5p, hsa-miR-6777-5p, hsa-miR-6789-5p, hsa-miR-7975, hsa-miR-6821-5p, hsa-miR-4534, hsa-miR-619-5p, hsa-miR-7107-5p, hsa-miR-1228-3p, hsa-miR-6774-5p, hsa-miR-6805-5p, hsa-miR-23a-3p, hsa-miR-4665-5p, hsa-miR-4505, hsa-miR-4638-5p, hsa-miR-24-3p, hsa-miR-3135b, hsa-miR-4745-5p, hsa-miR-128-1-5p, hsa-miR-4476, hsa-miR-4687-3p, hsa-miR-3665, hsa-miR-6806-5p, hsa-miR-3937, hsa-miR-711, hsa-miR-3141, hsa-miR-3188, hsa-miR-4281, hsa-miR-5196-5p, hsa-miR-6880-5p, hsa-miR-3960, hsa-miR-3648, hsa-miR-6721-5p, hsa-miR-4492, hsa-miR-744-5p, hsa-miR-7704, hsa-miR-4749-5p, hsa-miR-762, hsa-miR-6836-3p, hsa-miR-6727-5p, hsa-miR-4739, hsa-miR-7977, hsa-miR-4484, hsa-miR-6515-3p, hsa-miR-373-5p, hsa-miR-4258, hsa-miR-4674, hsa-miR-3180, hsa-miR-6076, hsa-miR-1238-5p, hsa-miR-4463, hsa-miR-4486, hsa-miR-4730, hsa-miR-4286, and hsa-miR-4739, which can be further optionally combined therewith or a combination thereof; congeners thereof: transcripts thereof: or variants or derivatives thereof.

The expression levels of the target nucleic acids described above are increased or decreased (hereinafter, referred to as "increased/decreased") depending on the identities of the target nucleic acids in subjects having early pancreatic cancer or a pancreatic cancer precursor lesion as compared with healthy subjects. For example, Table 2 illustrates change in the expression levels of target miRNAs corresponding to SEQ ID NOs: 1 to 226 in the blood (serum) of pancreatic cancer precursor lesion patients (humans) relative to healthy subjects. As shown in Table 2, the expression levels of the target miRNAs are increased or decreased depending on the identities of the target miRNAs. In the present invention, any of the target miRNAs selected this time and described herein can be used for the detection and determination of early pancreatic cancer or a pancreatic cancer precursor lesion in a subject.

Accordingly, the present invention can be effectively used for measuring expression levels of the target nucleic acids in body fluids from subjects (e.g., humans) suspected of having early pancreatic cancer or a pancreatic cancer precursor lesion and body fluids from healthy subjects and thereby detecting early pancreatic cancer or a pancreatic cancer precursor lesion with high accuracy through the comparison thereof. The present invention can also be effectively used for measuring expression levels of the target nucleic acids in body fluids from subjects (e.g., humans) suspected of having early pancreatic cancer or a pancreatic cancer precursor lesion and body fluids from advanced pancreatic cancer patients, bile duct cancer patients, breast cancer patients, prostate cancer patients, colorectal cancer patients, stomach cancer patients, esophageal cancer patients, liver cancer patients, benign pancreatic disease patients, or benign prostatic disease patients, or a combination thereof and thereby specifically discriminating early pancreatic cancer or a pancreatic cancer precursor lesion from other cancers, benign diseases or the like, with high accuracy through the comparison thereof.

The nucleic acid probe or the primer that can be used in the present invention is a nucleic acid probe(s) capable of specifically binding to a polynucleotide(s) consisting of a nucleotide sequence(s) represented by at least one, at least two, at least three, at least four, or at least five of SEQ ID NOs: 1 to 83, 227 to 229, 246, 248, and 250, or a primer(s) for amplifying a polynucleotide(s) consisting of a nucleotide sequence(s) represented by at least one, at least two, at least three, at least four, or at least five of SEQ ID NOs: 1 to 83, 227 to 229, 246, 248, and 250.

The nucleic acid probe or the primer that can be used in the present invention may further comprise a nucleic acid probe(s) capable of specifically binding to a polynucleotide(s) consisting of a nucleotide sequence(s) represented by at least one, at least two, at least three, at least four, or at least five of SEQ ID NOs: 84 to 226, 230 to 245, 247, and 249, or a primer(s) for amplifying a polynucleotide(s) consisting of a nucleotide sequence(s) represented by at least one, at least two, at least three, at least four, or at least five of SEQ ID NOs: 84 to 226, 230 to 245, 247, and 249.

Specifically, these nucleic acid probes or primers comprise a combination of one or more polynucleotides selected from: a group of polynucleotides comprising nucleotide sequences represented by any of SEQ ID NOs: 1 to 250 or nucleotide sequences derived from the nucleotide sequences by the replacement of u with t, and a group of complementary polynucleotides thereof; a group of polynucleotides respectively hybridizing under stringent conditions (mentioned later) to DNAs consisting of nucleotide sequences complementary to these nucleotide sequences, and a group of complementary polynucleotides thereof; and a group of polynucleotides comprising 15 or more, preferably 17 or more consecutive nucleotides from the nucleotide sequences of these polynucleotide groups. In this respect, the target miRNA used in the present invention also includes, for example, precursor miRNAs as shown in SEQ ID NOs: 251 to 518 and isomiRNAs as shown in SEQ ID NOs: 519 to 812 in Table 1. The isomiRNAs include those having the number of nucleotides as short as approximately 15, those having the number of nucleotides as long as approximately 29, those having mutation such as substitution, and the like. Hence, in the present invention, the nucleic acid probes or the primers also include nucleic acid probes or primers for enabling measurement of the expression of precursor miRNAs and target isomiRNAs. These polynucleotides can be used as nucleic acid probes and primers for detecting the early pancreatic cancer or pancreatic cancer precursor lesion markers as target nucleic acids.

More specifically, examples of the nucleic acid probes or the primers that can be used in the present invention include at least one (i.e., one or more) polynucleotide selected from the group consisting of the following polynucleotides (a) to (e):
(a) a polynucleotide consisting of a nucleotide sequence represented by any of SEQ ID NOs: 1 to 83, 227 to 229, 246, 248, and 250 or a nucleotide sequence derived from the nucleotide sequence by the replacement of u with t, a variant thereof, a derivative thereof, or a fragment thereof comprising 15 or more consecutive nucleotides;
(b) a polynucleotide comprising a nucleotide sequence represented by any of SEQ ID NOs: 1 to 83, 227 to 229, 246, 248, and 250;
(c) a polynucleotide consisting of a nucleotide sequence complementary to a nucleotide sequence represented by any of SEQ ID NOs: 1 to 83, 227 to 229, 246, 248, and 250 or a nucleotide sequence derived from the nucleotide sequence by the replacement of u with t, a variant thereof, a derivative thereof, or a fragment thereof comprising 15 or more consecutive nucleotides;
(d) a polynucleotide comprising a nucleotide sequence complementary to a nucleotide sequence represented by any of SEQ ID NOs: 1 to 83, 227 to 229, 246, 248, and 250 or a nucleotide sequence derived from the nucleotide sequence by the replacement of u with t; and
(e) a polynucleotide hybridizing under stringent conditions to any of the polynucleotides (a) to (d).

In addition to at least one (i.e., one or more) polynucleotides selected from any of the polynucleotides (a) to (e), the nucleic acid probes or the primers that can be used in the present invention may further comprise at least one (i.e., one or more) polynucleotides of the following polynucleotides (f) to (j):
(f) a polynucleotide consisting of a nucleotide sequence represented by any of SEQ ID NOs: 84 to 226, 230 to 245, 247, and 249 or a nucleotide sequence derived from the nucleotide sequence by the replacement of u with t, a variant thereof, a derivative thereof, or a fragment thereof comprising 15 or more consecutive nucleotides;
(g) a polynucleotide comprising a nucleotide sequence represented by any of SEQ ID NOs: 84 to 226, 230 to 245, 247, and 249;
(h) a polynucleotide consisting of a nucleotide sequence complementary to a nucleotide sequence represented by any of SEQ ID NOs: 84 to 226, 230 to 245, 247, and 249 or a nucleotide sequence derived from the nucleotide sequence by the replacement of u with t, a variant thereof, a derivative thereof, or a fragment thereof comprising 15 or more consecutive nucleotides;
(i) a polynucleotide comprising a nucleotide sequence complementary to a nucleotide sequence represented by any of SEQ ID NOs: 84 to 226, 230 to 245, 247, and 249 or a nucleotide sequence derived from the nucleotide sequence by the replacement of u with t; and
(j) a polynucleotide hybridizing under stringent conditions to any of the polynucleotides (f) to (i).

For the above-mentioned polynucleotides, the "fragment thereof comprising 15 or more consecutive nucleotides" is derived from the nucleotide sequence of each polynucleotide, and can comprise, but is not limited to, the number of nucleotides in the range of, for example, from 15 consecutive nucleotides to less than the total number of nucleotides of the sequence, from 17 consecutive nucleotides to less than the total number of nucleotides of the sequence, from 19 consecutive nucleotides to less than the total number of nucleotides of the sequence, or the like.

These polynucleotides or fragments thereof used in the present invention may each be DNA or RNA.

The polynucleotides that can be used in the present invention can be prepared by use of a general technique such as a DNA recombination technique, a PCR method, or a method using an automatic DNA/RNA synthesizer.

The DNA recombination technique and the PCR method may employ techniques described in, for example, Ausubel et al., Current Protocols in Molecular Biology, John Willey & Sons, US (1993); and Sambrook et al., Molecular Cloning - A Laboratory Manual, Cold Spring Harbor Laboratory Press, US (1989).

The human-derived hsa-miR-6784-5p, hsa-miR-1181, hsa-miR-671-5p, hsa-miR-6857-5p, hsa-miR-4276, hsa-miR-1914-3p, hsa-miR-149-3p, hsa-miR-937-5p, hsa-miR-4675, hsa-miR-6795-5p, hsa-miR-4731-5p, hsa-miR-5090, hsa-miR-3620-5p, hsa-miR-1343-5p, hsa-miR-6717-5p, hsa-miR-6825-5p, hsa-miR-6738-5p, hsa-miR-6769a-5p, hsa-miR-4728-5p, hsa-miR-652-5p, hsa-miR-4257, hsa-miR-6785-5p, hsa-miR-7110-5p, hsa-miR-6887-5p, hsa-miR-887-3p, hsa-miR-1228-5p, hsa-miR-5572, hsa-miR-6782-5p, hsa-miR-4298, hsa-miR-6786-5p, hsa-miR-5010-5p, hsa-miR-6087, hsa-miR-6765-5p, hsa-miR-6732-5p, hsa-miR-6787-5p, hsa-miR-6737-5p, hsa-miR-128-2-5p, hsa-miR-4270, hsa-miR-6861-5p, hsa-miR-6756-5p, hsa-miR-1229-5p, hsa-miR-6891-5p, hsa-miR-6848-5p, hsa-miR-1237-5p, hsa-miR-30c-1-3p, hsa-miR-1233-5p, hsa-miR-211-3p, hsa-miR-4758-5p, hsa-miR-614, hsa-miR-6746-5p, hsa-miR-1915-5p, hsa-miR-4688, hsa-miR-3917, hsa-miR-5787, hsa-miR-4632-5p, hsa-miR-6126, hsa-miR-135a-3p, hsa-miR-8063, hsa-miR-5698, hsa-miR-6089, hsa-miR-498, hsa-miR-296-3p, hsa-miR-4419b, hsa-miR-6802-5p, hsa-miR-6829-5p, hsa-miR-6803-5p, hsa-miR-1199-5p, hsa-miR-6840-3p, hsa-miR-6752-5p, hsa-miR-6798-5p, hsa-miR-6131, hsa-miR-4667-5p, hsa-miR-6510-5p, hsa-miR-4690-5p, hsa-miR-920, hsa-miR-23b-3p, hsa-miR-4448, hsa-miR-2110, hsa-miR-4706, hsa-miR-7845-5p, hsa-miR-6808-5p, hsa-miR-4447, hsa-miR-6869-5p, hsa-miR-1908-5p, hsa-miR-6729-5p, hsa-miR-5195-3p, hsa-miR-638, hsa-miR-6125, hsa-miR-3178, hsa-miR-3196, hsa-miR-8069, hsa-miR-4723-5p, hsa-miR-4746-3p, hsa-miR-4689, hsa-miR-6816-5p, hsa-miR-6757-5p, hsa-miR-7109-5p, hsa-miR-6724-5p, hsa-miR-1225-3p, hsa-miR-6875-5p, hsa-miR-7108-5p, hsa-miR-4508, hsa-miR-6085, hsa-miR-6779-5p, hsa-miR-642a-3p, hsa-miR-4695-5p, hsa-miR-7847-3p, hsa-miR-3197, hsa-miR-6769b-5p, hsa-miR-7641, hsa-miR-187-5p, hsa-miR-3185, hsa-miR-2861, hsa-miR-3940-5p, hsa-miR-1203, hsa-miR-615-5p, hsa-miR-4787-5p, hsa-miR-1343-3p, hsa-miR-6813-5p, hsa-miR-1225-5p, hsa-miR-602, hsa-miR-4488, hsa-miR-125a-3p, hsa-miR-5100, hsa-miR-4294, hsa-miR-1231, hsa-miR-6765-3p, hsa-miR-4442, hsa-miR-718, hsa-miR-6780b-5p, hsa-miR-6090, hsa-miR-6845-5p, hsa-miR-4741, hsa-miR-4467, hsa-miR-4707-5p, hsa-miR-4271, hsa-miR-4673, hsa-miR-3184-5p, hsa-miR-1469, hsa-miR-4640-5p, hsa-miR-663a, hsa-miR-6791-5p, hsa-miR-6826-5p, hsa-miR-4433b-3p, hsa-miR-1915-3p, hsa-miR-4417, hsa-miR-4449, hsa-miR-4707-3p, hsa-miR-3180-3p, hsa-miR-5585-3p, hsa-miR-1268a, hsa-miR-8072, hsa-miR-296-5p, hsa-miR-204-3p, hsa-miR-4454, hsa-miR-6722-3p, hsa-miR-1290, hsa-miR-3622a-5p, hsa-miR-939-5p, hsa-miR-675-5p, hsa-miR-3131, hsa-miR-4648, hsa-miR-1268b, hsa-miR-6741-5p, hsa-miR-6893-5p, hsa-miR-3162-5p, hsa-miR-642b-3p, hsa-miR-4734, hsa-miR-150-3p, hsa-miR-8089, hsa-miR-6805-3p, hsa-miR-7113-3p, hsa-miR-6850-5p, hsa-miR-6799-5p, hsa-miR-6768-5p, hsa-miR-92b-5p, hsa-miR-3679-5p, hsa-miR-4792, hsa-miR-3656, hsa-miR-92a-2-5p, hsa-miR-4466, hsa-miR-4513, hsa-miR-6781-5p, hsa-miR-4649-5p, hsa-miR-6775-5p, hsa-miR-4651, hsa-miR-3195, hsa-miR-6726-5p, hsa-miR-6872-3p, hsa-miR-371a-5p, hsa-miR-6777-5p, hsa-miR-6789-5p, hsa-miR-7975, hsa-miR-6821-5p, hsa-miR-4534, hsa-miR-619-5p, hsa-miR-7107-5p, hsa-miR-1228-3p, hsa-miR-6774-5p, hsa-miR-6805-5p, hsa-miR-23a-3p, hsa-miR-4665-5p, hsa-miR-4505, hsa-miR-4638-5p, hsa-miR-24-3p, hsa-miR-3135b, hsa-miR-4745-5p, hsa-miR-128-1-5p, hsa-miR-4476, hsa-miR-4687-3p, hsa-miR-3665, hsa-miR-6806-5p, hsa-miR-3937, hsa-miR-711, hsa-miR-3141, hsa-miR-3188, hsa-miR-4281, hsa-miR-5196-5p, hsa-miR-6880-5p, hsa-miR-3960, hsa-miR-3648, hsa-miR-6721-5p, hsa-miR-4492, hsa-miR-744-5p, hsa-miR-7704, hsa-miR-4749-5p, hsa-miR-6794-5p, hsa-miR-6511a-5p, hsa-miR-6824-5p, hsa-miR-762, hsa-miR-6836-3p, hsa-miR-6727-5p, hsa-miR-4739, hsa-miR-7977, hsa-miR-4484, hsa-miR-6515-3p, hsa-miR-373-5p, hsa-miR-4258, hsa-miR-4674, hsa-miR-3180, hsa-miR-6076, hsa-miR-1238-5p, hsa-miR-4463, hsa-miR-4486, hsa-miR-4730, hsa-miR-6766-3p, hsa-miR-4286, hsa-miR-6511a-5p, hsa-miR-4739, and hsa-miR-6749-5p represented by SEQ ID NOs: 1 to 250 are known in the art, and their obtainment methods are also known as mentioned above. Therefore, each polynucleotide that can be used as a nucleic acid probe or a primer in the present invention can be prepared by cloning the gene.

Such nucleic acid probes or primers can be chemically synthesized using an automatic DNA synthesizer. In general, the phosphoramidite method is used in this synthesis, and single-stranded DNA up to approximately 100 nucleotides can be automatically synthesized by this method. The automatic DNA synthesizer is commercially available from, for example, Polygen GmbH, ABI, or Applied Biosystems, Inc.

Alternatively, the polynucleotides of the present invention can also be prepared by cDNA cloning methods. The cDNA cloning technique may employ, for example, microRNA Cloning Kit Wako.

In this context, the sequences of the nucleic acid probes and the primers for detecting the polynucleotide consisting of a nucleotide sequence represented by any of SEQ ID NOs: 1 to 250 do not exist as miRNAs or precursors thereof in the living body or *in vivo*. For example, the nucleotide sequences represented by SEQ ID NO: 14 and SEQ ID NO: 118 are produced from the precursor represented by SEQ ID NO: 264. This precursor has a hairpin-like structure as shown in Figure 1, and the nucleotide sequences represented by SEQ ID NO: 14 and SEQ ID NO: 118 have mismatch sequences with each other. As such, a nucleotide sequence completely complementary to the nucleotide sequence represented by SEQ ID NO: 14 or SEQ ID NO: 118 does not naturally occur *in vivo*. Therefore, the nucleic acid probes and the primers for detecting the nucleotide sequence represented by any of SEQ ID NOs: 1 to 250 have artificial nucleotide sequences that do not exist in the living body or *in vivo*.

### 3. Kit or device for detection of early pancreatic cancer or a pancreatic cancer precursor lesion

The present invention also provides a kit or a device for the detection of early pancreatic cancer or a pancreatic cancer precursor lesion, comprising one or more polynucleotides (which may include a variant, a fragment, or a derivative thereof) that can be used as nucleic acid probes or primers in the present invention for measuring target nucleic acids as early pancreatic cancer or pancreatic cancer precursor lesion markers.

The target nucleic acids as early pancreatic cancer or pancreatic cancer precursor lesion markers according to the present invention are at least one nucleic acid selected from the following group A:
Group A:
hsa-miR-6784-5p, hsa-miR-1181, hsa-miR-671-5p, hsa-miR-6857-5p, hsa-miR-4276, hsa-miR-1914-3p, hsa-miR-149-3p, hsa-miR-937-5p, hsa-miR-4675, hsa-miR-6795-5p, hsa-miR-4731-5p, hsa-miR-5090, hsa-miR-3620-5p, hsa-miR-1343-5p, hsa-miR-6717-5p, hsa-miR-6825-5p, hsa-miR-6738-5p, hsa-miR-6769a-5p, hsa-miR-4728-5p, hsa-miR-652-5p, hsa-miR-4257, hsa-miR-6785-5p, hsa-miR-7110-5p, hsa-miR-6887-5p, hsa-miR-887-3p, hsa-miR-1228-5p, hsa-miR-5572, hsa-miR-6782-5p, hsa-miR-4298, hsa-miR-6786-5p, hsa-miR-5010-5p, hsa-miR-6087, hsa-miR-6765-5p, hsa-miR-6732-5p, hsa-miR-6787-5p, hsa-miR-6737-5p, hsa-miR-128-2-5p, hsa-miR-4270, hsa-miR-6861-5p, hsa-miR-6756-5p, hsa-miR-1229-5p, hsa-miR-6891-5p, hsa-miR-6848-5p, hsa-miR-1237-5p, hsa-miR-30c-1-3p, hsa-miR-1233-5p, hsa-miR-211-3p, hsa-miR-4758-5p, hsa-miR-614, hsa-miR-6746-5p, hsa-miR-1915-5p, hsa-miR-4688, hsa-miR-3917, hsa-miR-5787, hsa-miR-4632-5p, hsa-miR-6126, hsa-miR-135a-3p, hsa-miR-8063, hsa-miR-5698, hsa-miR-6089, hsa-miR-498, hsa-miR-296-3p, hsa-miR-4419b, hsa-miR-6802-5p, hsa-miR-6829-5p, hsa-miR-6803-5p, hsa-miR-1199-5p, hsa-miR-6840-3p, hsa-miR-6752-5p, hsa-miR-6798-5p, hsa-miR-6131, hsa-miR-4667-5p, hsa-miR-6510-5p, hsa-miR-4690-5p, hsa-miR-920, hsa-miR-23b-3p, hsa-miR-4448, hsa-miR-2110, hsa-miR-4706, hsa-miR-7845-5p, hsa-miR-6808-5p, hsa-miR-4447, hsa-miR-6869-5p, hsa-miR-6794-5p, hsa-miR-6511a-5p, hsa-miR-6824-5p, hsa-miR-6766-3p, hsa-miR-6511a-5p, and hsa-miR-6749-5p.

Additional target nucleic acids that may be optionally used in the measurement are at least one nucleic acid selected from the following group B:
Group B:
hsa-miR-1908-5p, hsa-miR-6729-5p, hsa-miR-5195-3p, hsa-miR-638, hsa-miR-6125, hsa-miR-3178, hsa-miR-3196, hsa-miR-8069, hsa-miR-4723-5p, hsa-miR-4746-3p, hsa-miR-4689, hsa-miR-6816-5p, hsa-miR-6757-5p, hsa-miR-7109-5p, hsa-miR-6724-5p, hsa-miR-1225-3p, hsa-miR-6875-5p, hsa-miR-7108-5p, hsa-miR-4508, hsa-miR-6085, hsa-miR-6779-5p, hsa-miR-642a-3p, hsa-miR-4695-5p, hsa-miR-7847-3p, hsa-miR-3197, hsa-miR-6769b-5p, hsa-miR-7641, hsa-miR-187-5p, hsa-miR-3185, hsa-miR-2861, hsa-miR-3940-5p, hsa-miR-1203, hsa-miR-615-5p, hsa-miR-4787-5p, hsa-miR-1343-3p, hsa-miR-6813-5p, hsa-miR-1225-5p, hsa-miR-602, hsa-miR-4488, hsa-miR-125a-3p, hsa-miR-5100, hsa-miR-4294, hsa-miR-1231, hsa-miR-6765-3p, hsa-miR-4442, hsa-miR-718, hsa-miR-6780b-5p, hsa-miR-6090, hsa-miR-6845-5p, hsa-miR-4741, hsa-miR-4467, hsa-miR-4707-5p, hsa-miR-4271, hsa-miR-4673, hsa-miR-3184-5p, hsa-miR-1469, hsa-miR-4640-5p, hsa-miR-663a, hsa-miR-6791-5p, hsa-miR-6826-5p, hsa-miR-4433b-3p, hsa-miR-1915-3p, hsa-miR-4417, hsa-miR-4449, hsa-miR-4707-3p, hsa-miR-3180-3p, hsa-miR-5585-3p, hsa-miR-1268a, hsa-miR-8072, hsa-miR-296-5p, hsa-miR-204-3p, hsa-miR-4454, hsa-miR-6722-3p, hsa-miR-1290, hsa-miR-3622a-5p, hsa-miR-939-5p, hsa-miR-675-5p, hsa-miR-3131, hsa-miR-4648, hsa-miR-1268b, hsa-miR-6741-5p, hsa-miR-6893-5p, hsa-miR-3162-5p, hsa-miR-642b-3p, hsa-miR-4734, hsa-miR-150-3p, hsa-miR-8089, hsa-miR-6805-3p, hsa-miR-7113-3p, hsa-miR-6850-5p, hsa-miR-6799-5p, hsa-miR-6768-5p, hsa-miR-92b-5p, hsa-miR-3679-5p, hsa-miR-4792, hsa-miR-3656, hsa-miR-92a-2-5p, hsa-miR-4466, hsa-miR-4513, hsa-miR-6781-5p, hsa-miR-4649-5p, hsa-miR-6775-5p, hsa-miR-4651, hsa-miR-3195, hsa-miR-6726-5p, hsa-miR-6872-3p, hsa-miR-371a-5p, hsa-miR-6777-5p, hsa-miR-6789-5p, hsa-miR-7975, hsa-miR-6821-5p, hsa-miR-4534, hsa-miR-619-5p, hsa-miR-7107-5p, hsa-miR-1228-3p, hsa-miR-6774-5p, hsa-miR-6805-5p, hsa-miR-23a-3p, hsa-miR-4665-5p, hsa-miR-4505, hsa-miR-4638-5p, hsa-miR-24-3p, hsa-miR-3135b, hsa-miR-4745-5p, hsa-miR-128-1-5p, hsa-miR-4476, hsa-miR-4687-3p, hsa-miR-3665, hsa-miR-6806-5p, hsa-miR-3937, hsa-miR-711, hsa-miR-3141, hsa-miR-3188, hsa-miR-4281, hsa-miR-5196-5p, hsa-miR-6880-5p, hsa-miR-3960, hsa-miR-3648, hsa-miR-6721-5p, hsa-miR-4492, hsa-miR-744-5p, hsa-miR-7704, hsa-miR-4749-5p, hsa-miR-762, hsa-miR-6836-3p, hsa-miR-6727-5p, hsa-miR-4739, hsa-miR-7977, hsa-miR-4484, hsa-miR-6515-3p, hsa-miR-373-5p, hsa-miR-4258, hsa-miR-4674, hsa-miR-3180, hsa-miR-6076, hsa-miR-1238-5p, hsa-miR-4463, hsa-miR-4486, hsa-miR-4730, hsa-miR-4286, and hsa-miR-4739.

The kit or the device of the present invention comprises nucleic acid capable of specifically binding to any of the target nucleic acids as the early pancreatic cancer or pancreatic cancer precursor lesion markers described above, preferably at least one (or one or more) polynucleotide selected from the polynucleotides described in the preceding Section 2, or a variant thereof.

Specifically, the kit or the device of the present invention can comprise at least one (or one or more) polynucleotides comprising (or consisting of) a nucleotide sequence represented by any of SEQ ID NOs: 1 to 83, 227 to 229, 246, 248, and 250 or a nucleotide sequence derived from the nucleotide sequence by the replacement of u with t, polynucleotide(s) comprising (or consisting of) a complementary sequence thereof, a polynucleotide(s) hybridizing under stringent conditions to any of these polynucleotides, or a variant(s) or a fragment(s) comprising 15 or more consecutive nucleotides of any of these polynucleotide sequences.

The kit or the device of the present invention can further comprise one or more, two or more, three or more, four or more, or five or more polynucleotides comprising (or consisting of) a nucleotide sequence represented by any of SEQ ID NOs: 84 to 226, 230 to 245, 247, and 249 or a nucleotide sequence derived from the nucleotide sequence by the replacement of u with t, a polynucleotide(s) comprising (or consisting of) a complementary sequence thereof, a polynucleotide(s) hybridizing under stringent conditions to any of these polynucleotides, a variant(s) or a fragment(s) comprising 15 or more, 17 or more, or 19 or more consecutive nucleotides of any of these polynucleotide sequences.

The fragment or fragments that can be comprised in the kit or the device of the present invention is/are, for example, one or more, two or more, three or more, four or more, or five or more polynucleotides selected from the group consisting of the following polynucleotides (1) and (2):
(1) a polynucleotide comprising 15 or more, 17 or more, or 19 or more consecutive nucleotides that are from a nucleotide sequence derived from a nucleotide sequence represented by any of SEQ ID NOs: 1 to 83, 227 to 229, 246, 248, and 250 by the replacement of u with t, or a complementary sequence thereof; and
(2) a polynucleotide comprising 15 or more, 17 or more, or 19 or more consecutive nucleotides that are from a nucleotide sequence derived from a nucleotide sequence represented by any of SEQ ID NOs: 84 to 226, 230 to 245, 247, and 249 by the replacement of u with t, or a complementary sequence thereof.

In a preferred embodiment, the polynucleotide is a polynucleotide consisting of a nucleotide sequence represented by any of SEQ ID NOs: 1 to 83, 227 to 229, 246, 248, and 250 or a nucleotide sequence derived from the nucleotide sequence by the replacement of u with t, a polynucleotide consisting of a complementary sequence thereof, a polynucleotide hybridizing under stringent conditions to any of these polynucleotides, or a variant thereof comprising 15 or more, 17 or more, or 19 or more consecutive nucleotides.

In a preferred embodiment, the polynucleotide is a polynucleotide consisting of a nucleotide sequence represented by any of SEQ ID NOs: 84 to 226, 230 to 245, 247, and 249 or a nucleotide sequence derived from the nucleotide sequence by the replacement of u with t, a polynucleotide consisting of a complementary sequence thereof, a polynucleotide hybridizing under stringent conditions to any of these polynucleotides, or a variant thereof comprising 15 or more, 17 or more, or 19 or more consecutive nucleotides.

In a preferred embodiment, the fragment can be a polynucleotide comprising 15 or more, 17 or more, or 19 or more consecutive nucleotides.

In the present invention, the size of the polynucleotide fragment is the number of nucleotides in the range from, for example, 15 consecutive nucleotides to less than the total number of nucleotides of the sequence, from 17 consecutive nucleotides to less than the total number of nucleotides of the sequence, or from 19 consecutive nucleotides to less than the total number of nucleotides of the sequence, in the nucleotide sequence of each polynucleotide.

Specific examples of the aforementioned combination constituting the kit or the device of the present invention can include the above-mentioned polynucleotides relating to the combinations of SEQ ID NOs shown in Table 1 (i.e., SEQ ID NOs: 1 to 250 corresponding to the miRNA markers in Table 1). However, these are given merely for illustrative purposes, and all of various possible combinations with polynucleotides capable of specifically binding to other miRNA markers in Table 1 (corresponding to SEQ ID NOs: 251 to 812) are included in the present invention.

The combination constituting the kit or the device for discriminating an early pancreatic cancer or pancreatic cancer precursor lesion patient from a healthy subject according to the present invention may be, for example, a combination of two or more, three or more, four or more, or five or more polynucleotides consisting of the nucleotide sequences represented by SEQ ID NOs shown in Table 1. Usually, a combination of even two of these polynucleotides can produce adequate performance.

The specific combination of two polynucleotides that consist of the above-mentioned nucleotide sequences or the complementary sequences thereof for discriminating an early pancreatic cancer or pancreatic cancer precursor lesion patient from a healthy subject is preferably a combination comprising at least one (one or more) polynucleotides of the newly found polynucleotides consisting of the nucleotide sequences represented by SEQ ID NOs: 1 to 83, 227 to 229, 246, 248, and 250, among the combinations constituted by two polynucleotides of the polynucleotides consisting of the nucleotide sequences represented by SEQ ID NOs: 1 to 250.

The combination of two polynucleotides that consist of the above-mentioned nucleotide sequences or the complementary sequences thereof for discriminating an early pancreatic cancer or pancreatic cancer precursor lesion patient from a healthy subject is preferably a combination of a plurality of polynucleotides comprising at least one polynucleotide selected from the group consisting of polynucleotides consisting of the nucleotide sequences represented unlimitedly, for example, by SEQ ID NOs: 2, 3, 18, 12, 20, 1, 15, 50, 63, 72, 5, 24, 10, 52, 9, 11, 19, 39, 61, 7, 17, 22, 26, 74, 21, and 28 or complementary sequences thereof, with any of the polynucleotides of the other SEQ ID NOs.

Non-limiting examples of the combination comprising a polynucleotide consisting of the nucleotide sequence represented by SEQ ID NO: 2 or a complementary sequence thereof among the combinations constituted by two polynucleotides of the polynucleotides consisting of the nucleotide sequences represented by SEQ ID NOs: 1 to 250 for discriminating an early pancreatic cancer or pancreatic cancer precursor lesion patient from a healthy subject are listed below:
(1) a combination of SEQ ID NOs: 2 and 18 (markers: hsa-miR-1181 and hsa-miR-6769a-5p);
(2) a combination of SEQ ID NOs: 2 and 53 (markers: hsa-miR-1181 and hsa-miR-3917);
(3) a combination of SEQ ID NOs: 2 and 20 (markers: hsa-miR-1181 and hsa-miR-652-5p);
(4) a combination of SEQ ID NOs: 2 and 3 (markers: hsa-miR-1181 and hsa-miR-671-5p); and
(5) a combination of SEQ ID NOs: 2 and 50 (markers: hsa-miR-1181 and hsa-miR-6746-5p).

Non-limiting examples of the combination comprising a polynucleotide consisting of the nucleotide sequence represented by SEQ ID NO: 3 or a complementary sequence thereof among the combinations constituted by two polynucleotides of the polynucleotides consisting of the nucleotide sequences represented by SEQ ID NOs: 1 to 250 for discriminating an early pancreatic cancer or pancreatic cancer precursor lesion patient from a healthy subject are listed below:
(1) a combination of SEQ ID NOs: 85 and 3 (markers: hsa-miR-6729-5p and hsa-miR-671-5p);
(2) a combination of SEQ ID NOs: 84 and 3 (markers: hsa-miR-1908-5p and hsa-miR-671-5p);
(3) a combination of SEQ ID NOs: 90 and 3 (markers: hsa-miR-3196 and hsa-miR-671-5p);
(4) a combination of SEQ ID NOs: 87 and 3 (markers: hsa-miR-638 and hsa-miR-671-5p); and
(5) a combination of SEQ ID NOs: 3 and 137 (markers: hsa-miR-671-5p and hsa-miR-4673).

Non-limiting examples of the combination comprising a polynucleotide consisting of the nucleotide sequence represented by SEQ ID NO: 18 or a complementary sequence thereof among the combinations constituted by two polynucleotides of the polynucleotides consisting of the nucleotide sequences represented by SEQ ID NOs: 1 to 250 for discriminating an early pancreatic cancer or pancreatic cancer precursor lesion patient from a healthy subject are listed below:
(1) a combination of SEQ ID NOs: 90 and 18 (markers: hsa-miR-3196 and hsa-miR-6769a-5p);
(2) a combination of SEQ ID NOs: 87 and 18 (markers: hsa-miR-638 and hsa-miR-6769a-5p);
(3) a combination of SEQ ID NOs: 89 and 18 (markers: hsa-miR-3178 and hsa-miR-6769a-5p);
(4) a combination of SEQ ID NOs: 18 and 137 (markers: hsa-miR-6769a-5p and hsa-miR-4673); and
(5) a combination of SEQ ID NOs: 84 and 18 (markers: hsa-miR-1908-5p and hsa-miR-6769a-5p).

Non-limiting examples of the combination comprising a polynucleotide consisting of the nucleotide sequence represented by SEQ ID NO: 12 or a complementary sequence thereof among the combinations constituted by two polynucleotides of the polynucleotides consisting of the nucleotide sequences represented by SEQ ID NOs: 1 to 250 for discriminating an early pancreatic cancer or pancreatic cancer precursor lesion patient from a healthy subject are listed below:
(1) a combination of SEQ ID NOs: 86 and 12 (markers: hsa-miR-5195-3p and hsa-miR-5090);
(2) a combination of SEQ ID NOs: 109 and 12 (markers: hsa-miR-6769b-5p and hsa-miR-5090);
(3) a combination of SEQ ID NOs: 85 and 12 (markers: hsa-miR-6729-5p and hsa-miR-5090);
(4) a combination of SEQ ID NOs: 88 and 12 (markers: hsa-miR-6125 and hsa-miR-5090); and
(5) a combination of SEQ ID NOs: 105 and 12 (markers: hsa-miR-642a-3p and hsa-miR-5090).

Non-limiting examples of the combination comprising a polynucleotide consisting of the nucleotide sequence represented by SEQ ID NO: 20 or a complementary sequence thereof among the combinations constituted by two polynucleotides of the polynucleotides consisting of the nucleotide sequences represented by SEQ ID NOs: 1 to 250 for discriminating an early pancreatic cancer or pancreatic cancer precursor lesion patient from a healthy subject are listed below:
(1) a combination of SEQ ID NOs: 85 and 20 (markers: hsa-miR-6729-5p and hsa-miR-652-5p);
(2) a combination of SEQ ID NOs: 84 and 20 (markers: hsa-miR-1908-5p and hsa-miR-652-5p);
(3) a combination of SEQ ID NOs: 106 and 20 (markers: hsa-miR-4695-5p and hsa-miR-652-5p);
(4) a combination of SEQ ID NOs: 90 and 20 (markers: hsa-miR-3196 and hsa-miR-652-5p); and
(5) a combination of SEQ ID NOs: 87 and 20 (markers: hsa-miR-638 and hsa-miR-652-5p).

Non-limiting examples of the combination comprising a polynucleotide consisting of the nucleotide sequence represented by SEQ ID NO: 1 or a complementary sequence thereof among the combinations constituted by two polynucleotides of the polynucleotides consisting of the nucleotide sequences represented by SEQ ID NOs: 1 to 250 for discriminating an early pancreatic cancer or pancreatic cancer precursor lesion patient from a healthy subject are listed below:
(1) a combination of SEQ ID NOs: 85 and 1 (markers: hsa-miR-6729-5p and hsa-miR-6784-5p);
(2) a combination of SEQ ID NOs: 87 and 1 (markers: hsa-miR-638 and hsa-miR-6784-5p);
(3) a combination of SEQ ID NOs: 88 and 1 (markers: hsa-miR-6125 and hsa-miR-6784-5p);
(4) a combination of SEQ ID NOs: 86 and 1 (markers: hsa-miR-5195-3p and hsa-miR-6784-5p); and
(5) a combination of SEQ ID NOs: 1 and 3(markers: hsa-miR-6784-5p and hsa-miR-671-5p).

Non-limiting examples of the combination comprising a polynucleotide consisting of the nucleotide sequence represented by SEQ ID NO: 15 or a complementary sequence thereof among the combinations constituted by two polynucleotides of the polynucleotides consisting of the nucleotide sequences represented by SEQ ID NOs: 1 to 250 for discriminating an early pancreatic cancer or pancreatic cancer precursor lesion patient from a healthy subject are listed below:
(1) a combination of SEQ ID NOs: 87 and 15 (markers: hsa-miR-638 and hsa-miR-6717-5p);
(2) a combination of SEQ ID NOs: 85 and 15 (markers: hsa-miR-6729-5p and hsa-miR-6717-5p);
(3) a combination of SEQ ID NOs: 2 and 15 (markers: hsa-miR-1181 and hsa-miR-6717-5p);
(4) a combination of SEQ ID NOs: 88 and 15 (markers: hsa-miR-6125 and hsa-miR-6717-5p); and
(5) a combination of SEQ ID NOs: 15 and 137 (markers: hsa-miR-6717-5p and hsa-miR-4673).

Non-limiting examples of the combination comprising a polynucleotide consisting of the nucleotide sequence represented by SEQ ID NO: 50 or a complementary sequence thereof among the combinations constituted by two polynucleotides of the polynucleotides consisting of the nucleotide sequences represented by SEQ ID NOs: 1 to 250 for discriminating an early pancreatic cancer or pancreatic cancer precursor lesion patient from a healthy subject are listed below:
(1) a combination of SEQ ID NOs: 85 and 50 (markers: hsa-miR-6729-5p and hsa-miR-6746-5p);
(2) a combination of SEQ ID NOs: 87 and 50 (markers: hsa-miR-638 and hsa-miR-6746-5p);
(3) a combination of SEQ ID NOs: 84 and 50 (markers: hsa-miR-1908-5p and hsa-miR-6746-5p);
(4) a combination of SEQ ID NOs: 106 and 50 (markers: hsa-miR-4695-5p and hsa-miR-6746-5p); and
(5) a combination of SEQ ID NOs: 90 and 50 (markers: hsa-miR-3196 and hsa-miR-6746-5p).

Non-limiting examples of the combination comprising a polynucleotide consisting of the nucleotide sequence represented by SEQ ID NO: 63 or a complementary sequence thereof among the combinations constituted by two polynucleotides of the polynucleotides consisting of the nucleotide sequences represented by SEQ ID NOs: 1 to 250 for discriminating an early pancreatic cancer or pancreatic cancer precursor lesion patient from a healthy subject are listed below:
(1) a combination of SEQ ID NOs: 2 and 63 (markers: hsa-miR-1181 and hsa-miR-4419b);
(2) a combination of SEQ ID NOs: 85 and 63 (markers: hsa-miR-6729-5p and hsa-miR-4419b);
(3) a combination of SEQ ID NOs: 90 and 63 (markers: hsa-miR-3196 and hsa-miR-4419b);
(4) a combination of SEQ ID NOs: 84 and 63 (markers: hsa-miR-1908-5p and hsa-miR-4419b); and
(5) a combination of SEQ ID NOs: 87 and 63 (markers: hsa-miR-638 and hsa-miR-4419b).

Non-limiting examples of the combination comprising a polynucleotide consisting of the nucleotide sequence represented by SEQ ID NO: 72 or a complementary sequence thereof among the combinations constituted by two polynucleotides of the polynucleotides consisting of the nucleotide sequences represented by SEQ ID NOs: 1 to 250 for discriminating an early pancreatic cancer or pancreatic cancer precursor lesion patient from a healthy subject are listed below:
(1) a combination of SEQ ID NOs: 84 and 72 (markers: hsa-miR-1908-5p and hsa-miR-4667-5p);
(2) a combination of SEQ ID NOs: 85 and 72 (markers: hsa-miR-6729-5p and hsa-miR-4667-5p);
(3) a combination of SEQ ID NOs: 88 and 72 (markers: hsa-miR-6125 and hsa-miR-4667-5p);
(4) a combination of SEQ ID NOs: 87 and 72 (markers: hsa-miR-638 and hsa-miR-4667-5p); and
(5) a combination of SEQ ID NOs: 93 and 72 (markers: hsa-miR-4746-3p and hsa-miR-4667-5p).

Non-limiting examples of the combination comprising a polynucleotide consisting of the nucleotide sequence represented by SEQ ID NO: 5 or a complementary sequence thereof among the combinations constituted by two polynucleotides of the polynucleotides consisting of the nucleotide sequences represented by SEQ ID NOs: 1 to 250 for discriminating an early pancreatic cancer or pancreatic cancer precursor lesion patient from a healthy subject are listed below:
(1) a combination of SEQ ID NOs: 94 and 5 (markers: hsa-miR-4689 and hsa-miR-4276);
(2) a combination of SEQ ID NOs: 85 and 5 (markers: hsa-miR-6729-5p and hsa-miR-4276);
(3) a combination of SEQ ID NOs: 87 and 5 (markers: hsa-miR-638 and hsa-miR-4276);
(4) a combination of SEQ ID NOs: 5 and 107 (markers: hsa-miR-4276 and hsa-miR-7847-3p); and
(5) a combination of SEQ ID NOs: 84 and 5 (markers: hsa-miR-1908-5p and hsa-miR-4276).

Non-limiting examples of the combination comprising a polynucleotide consisting of the nucleotide sequence represented by SEQ ID NO: 24 or a complementary sequence thereof among the combinations constituted by two polynucleotides of the polynucleotides consisting of the nucleotide sequences represented by SEQ ID NOs: 1 to 250 for discriminating an early pancreatic cancer or pancreatic cancer precursor lesion patient from a healthy subject are listed below:
(1) a combination of SEQ ID NOs: 85 and 24 (markers: hsa-miR-6729-5p and hsa-miR-6887-5p);
(2) a combination of SEQ ID NOs: 87 and 24 (markers: hsa-miR-638 and hsa-miR-6887-5p);
(3) a combination of SEQ ID NOs: 89 and 24 (markers: hsa-miR-3178 and hsa-miR-6887-5p);
(4) a combination of SEQ ID NOs: 90 and 24 (markers: hsa-miR-3196 and hsa-miR-6887-5p); and
(5) a combination of SEQ ID NOs: 102 and 24 (markers: hsa-miR-4508 and hsa-miR-6887-5p).

Non-limiting examples of the combination comprising a polynucleotide consisting of the nucleotide sequence represented by SEQ ID NO: 10 or a complementary sequence thereof among the combinations constituted by two polynucleotides of the polynucleotides consisting of the nucleotide sequences represented by SEQ ID NOs: 1 to 250 for discriminating an early pancreatic cancer or pancreatic cancer precursor lesion patient from a healthy subject are listed below:
(1) a combination of SEQ ID NOs: 85 and 10 (markers: hsa-miR-6729-5p and hsa-miR-6795-5p);
(2) a combination of SEQ ID NOs: 87 and 10 (markers: hsa-miR-638 and hsa-miR-6795-5p);
(3) a combination of SEQ ID NOs: 90 and 10 (markers: hsa-miR-3196 and hsa-miR-6795-5p);
(4) a combination of SEQ ID NOs: 88 and 10 (markers: hsa-miR-6125 and hsa-miR-6795-5p); and
(5) a combination of SEQ ID NOs: 2 and 10 (markers: hsa-miR-1181 and hsa-miR-6795-5p).

Non-limiting examples of the combination comprising a polynucleotide consisting of the nucleotide sequence represented by SEQ ID NO: 52 or a complementary sequence thereof among the combinations constituted by two polynucleotides of the polynucleotides consisting of the nucleotide sequences represented by SEQ ID NOs: 1 to 250 for discriminating an early pancreatic cancer or pancreatic cancer precursor lesion patient from a healthy subject are listed below:
(1) a combination of SEQ ID NOs: 85 and 52 (markers: hsa-miR-6729-5p and hsa-miR-4688);
(2) a combination of SEQ ID NOs: 88 and 52 (markers: hsa-miR-6125 and hsa-miR-4688);
(3) a combination of SEQ ID NOs: 87 and 52 (markers: hsa-miR-638 and hsa-miR-4688);
(4) a combination of SEQ ID NOs: 98 and 52 (markers: hsa-miR-6724-5p and hsa-miR-4688); and
(5) a combination of SEQ ID NOs: 84 and 52 (markers: hsa-miR-1908-5p and hsa-miR-4688).

Non-limiting examples of the combination comprising a polynucleotide consisting of the nucleotide sequence represented by SEQ ID NO: 9 or a complementary sequence thereof among the combinations constituted by two polynucleotides of the polynucleotides consisting of the nucleotide sequences represented by SEQ ID NOs: 1 to 250 for discriminating an early pancreatic cancer or pancreatic cancer precursor lesion patient from a healthy subject are listed below:
(1) a combination of SEQ ID NOs: 87 and 9 (markers: hsa-miR-638 and hsa-miR-4675);
(2) a combination of SEQ ID NOs: 89 and 9 (markers: hsa-miR-3178 and hsa-miR-4675);
(3) a combination of SEQ ID NOs: 85 and 9 (markers: hsa-miR-6729-5p and hsa-miR-4675);
(4) a combination of SEQ ID NOs: 117 and 9 (markers: hsa-miR-4787-5p and hsa-miR-4675); and
(5) a combination of SEQ ID NOs: 88 and 9 (markers: hsa-miR-6125 and hsa-miR-4675).

Non-limiting examples of the combination comprising a polynucleotide consisting of the nucleotide sequence represented by SEQ ID NO: 11 or a complementary sequence thereof among the combinations constituted by two polynucleotides of the polynucleotides consisting of the nucleotide sequences represented by SEQ ID NOs: 1 to 250 for discriminating an early pancreatic cancer or pancreatic cancer precursor lesion patient from a healthy subject are listed below:
(1) a combination of SEQ ID NOs: 87 and 11 (markers: hsa-miR-638 and hsa-miR-4731-5p);
(2) a combination of SEQ ID NOs: 85 and 11 (markers: hsa-miR-6729-5p and hsa-miR-4731-5p);
(3) a combination of SEQ ID NOs: 89 and 11 (markers: hsa-miR-3178 and hsa-miR-4731-5p);
(4) a combination of SEQ ID NOs: 102 and 11 (markers: hsa-miR-4508 and hsa-miR-4731-5p); and
(5) a combination of SEQ ID NOs: 84 and 11 (markers: hsa-miR-1908-5p and hsa-miR-4731-5p).

Non-limiting examples of the combination comprising a polynucleotide consisting of the nucleotide sequence represented by SEQ ID NO: 19 or a complementary sequence thereof among the combinations constituted by two polynucleotides of the polynucleotides consisting of the nucleotide sequences represented by SEQ ID NOs: 1 to 250 for discriminating an early pancreatic cancer or pancreatic cancer precursor lesion patient from a healthy subject are listed below:
(1) a combination of SEQ ID NOs: 85 and 19 (markers: hsa-miR-6729-5p and hsa-miR-4728-5p);
(2) a combination of SEQ ID NOs: 87 and 19 (markers: hsa-miR-638 and hsa-miR-4728-5p);
(3) a combination of SEQ ID NOs: 88 and 19 (markers: hsa-miR-6125 and hsa-miR-4728-5p);
(4) a combination of SEQ ID NOs: 89 and 19 (markers: hsa-miR-3178 and hsa-miR-4728-5p); and
(5) a combination of SEQ ID NOs: 106 and 19 (markers: hsa-miR-4695-5p and hsa-miR-4728-5p).

Non-limiting examples of the combination comprising a polynucleotide consisting of the nucleotide sequence represented by SEQ ID NO: 39 or a complementary sequence thereof among the combinations constituted by two polynucleotides of the polynucleotides consisting of the nucleotide sequences represented by SEQ ID NOs: 1 to 250 for discriminating an early pancreatic cancer or pancreatic cancer precursor lesion patient from a healthy subject are listed below:
(1) a combination of SEQ ID NOs: 87 and 39 (markers: hsa-miR-638 and hsa-miR-6861-5p);
(2) a combination of SEQ ID NOs: 85 and 39 (markers: hsa-miR-6729-5p and hsa-miR-6861-5p);
(3) a combination of SEQ ID NOs: 88 and 39 (markers: hsa-miR-6125 and hsa-miR-6861-5p);
(4) a combination of SEQ ID NOs: 84 and 39 (markers: hsa-miR-1908-5p and hsa-miR-6861-5p); and
(5) a combination of SEQ ID NOs: 2 and 39 (markers: hsa-miR-1181 and hsa-miR-6861-5p).

Non-limiting examples of the combination comprising a polynucleotide consisting of the nucleotide sequence represented by SEQ ID NO: 61 or a complementary sequence thereof among the combinations constituted by two polynucleotides of the polynucleotides consisting of the nucleotide sequences represented by SEQ ID NOs: 1 to 250 for discriminating an early pancreatic cancer or pancreatic cancer precursor lesion patient from a healthy subject are listed below:
(1) a combination of SEQ ID NOs: 87 and 61 (markers: hsa-miR-638 and hsa-miR-498);
(2) a combination of SEQ ID NOs: 85 and 61 (markers: hsa-miR-6729-5p and hsa-miR-498);
(3) a combination of SEQ ID NOs: 88 and 61 (markers: hsa-miR-6125 and hsa-miR-498);
(4) a combination of SEQ ID NOs: 108 and 61 (markers: hsa-miR-3197 and hsa-miR-498); and
(5) a combination of SEQ ID NOs: 93 and 61 (markers: hsa-miR-4746-3p and hsa-miR-498).

Non-limiting examples of the combination comprising a polynucleotide consisting of the nucleotide sequence represented by SEQ ID NO: 7 or a complementary sequence thereof among the combinations constituted by two polynucleotides of the polynucleotides consisting of the nucleotide sequences represented by SEQ ID NOs: 1 to 250 for discriminating an early pancreatic cancer or pancreatic cancer precursor lesion patient from a healthy subject are listed below:
(1) a combination of SEQ ID NOs: 88 and 7 (markers: hsa-miR-6125 and hsa-miR-149-3p);
(2) a combination of SEQ ID NOs: 85 and 7 (markers: hsa-miR-6729-5p and hsa-miR-149-3p);
(3) a combination of SEQ ID NOs: 87 and 7 (markers: hsa-miR-638 and hsa-miR-149-3p);
(4) a combination of SEQ ID NOs: 86 and 7 (markers: hsa-miR-5195-3p and hsa-miR-149-3p); and
(5) a combination of SEQ ID NOs: 91 and 7 (markers: hsa-miR-8069 and hsa-miR-149-3p).

Non-limiting examples of the combination comprising a polynucleotide consisting of the nucleotide sequence represented by SEQ ID NO: 17 or a complementary sequence thereof among the combinations constituted by two polynucleotides of the polynucleotides consisting of the nucleotide sequences represented by SEQ ID NOs: 1 to 250 for discriminating an early pancreatic cancer or pancreatic cancer precursor lesion patient from a healthy subject are listed below:
(1) a combination of SEQ ID NOs: 85 and 17 (markers: hsa-miR-6729-5p and hsa-miR-6738-5p);
(2) a combination of SEQ ID NOs: 87 and 17 (markers: hsa-miR-638 and hsa-miR-6738-5p);
(3) a combination of SEQ ID NOs: 89 and 17 (markers: hsa-miR-3178 and hsa-miR-6738-5p);
(4) a combination of SEQ ID NOs: 102 and 17 (markers: hsa-miR-4508 and hsa-miR-6738-5p); and
(5) a combination of SEQ ID NOs: 84 and 17 (markers: hsa-miR-1908-5p and hsa-miR-6738-5p).

Non-limiting examples of the combination comprising a polynucleotide consisting of the nucleotide sequence represented by SEQ ID NO: 22 or a complementary sequence thereof among the combinations constituted by two polynucleotides of the polynucleotides consisting of the nucleotide sequences represented by SEQ ID NOs: 1 to 250 for discriminating an early pancreatic cancer or pancreatic cancer precursor lesion patient from a healthy subject are listed below:
(1) a combination of SEQ ID NOs: 85 and 22 (markers: hsa-miR-6729-5p and hsa-miR-6785-5p);
(2) a combination of SEQ ID NOs: 87 and 22 (markers: hsa-miR-638 and hsa-miR-6785-5p);
(3) a combination of SEQ ID NOs: 102 and 22 (markers: hsa-miR-4508 and hsa-miR-6785-5p);
(4) a combination of SEQ ID NOs: 89 and 22 (markers: hsa-miR-3178 and hsa-miR-6785-5p); and
(5) a combination of SEQ ID NOs: 117 and 22 (markers: hsa-miR-4787-5p and hsa-miR-6785-5p).

Non-limiting examples of the combination comprising a polynucleotide consisting of the nucleotide sequence represented by SEQ ID NO: 26 or a complementary sequence thereof among the combinations constituted by two polynucleotides of the polynucleotides consisting of the nucleotide sequences represented by SEQ ID NOs: 1 to 250 for discriminating an early pancreatic cancer or pancreatic cancer precursor lesion patient from a healthy subject are listed below:
(1) a combination of SEQ ID NOs: 85 and 26 (markers: hsa-miR-6729-5p and hsa-miR-1228-5p);
(2) a combination of SEQ ID NOs: 87 and 26 (markers: hsa-miR-638 and hsa-miR-1228-5p);
(3) a combination of SEQ ID NOs: 88 and 26 (markers: hsa-miR-6125 and hsa-miR-1228-5p);
(4) a combination of SEQ ID NOs: 84 and 26 (markers: hsa-miR-1908-5p and hsa-miR-1228-5p); and
(5) a combination of SEQ ID NOs: 94 and 26 (markers: hsa-miR-4689 and hsa-miR-1228-5p).

Non-limiting examples of the combination comprising a polynucleotide consisting of the nucleotide sequence represented by SEQ ID NO: 74 or a complementary sequence thereof among the combinations constituted by two polynucleotides of the polynucleotides consisting of the nucleotide sequences represented by SEQ ID NOs: 1 to 250 for discriminating an early pancreatic cancer or pancreatic cancer precursor lesion patient from a healthy subject are listed below:
(1) a combination of SEQ ID NOs: 85 and 74 (markers: hsa-miR-6729-5p and hsa-miR-4690-5p);
(2) a combination of SEQ ID NOs: 2 and 74 (markers: hsa-miR-1181 and hsa-miR-4690-5p);
(3) a combination of SEQ ID NOs: 87 and 74 (markers: hsa-miR-638 and hsa-miR-4690-5p);
(4) a combination of SEQ ID NOs: 84 and 74 (markers: hsa-miR-1908-5p and hsa-miR-4690-5p); and
(5) a combination of SEQ ID NOs: 88 and 74 (markers: hsa-miR-6125 and hsa-miR-4690-5p).

Non-limiting examples of the combination comprising a polynucleotide consisting of the nucleotide sequence represented by SEQ ID NO: 21 or a complementary sequence thereof among the combinations constituted by two polynucleotides of the polynucleotides consisting of the nucleotide sequences represented by SEQ ID NOs: 1 to 250 for discriminating an early pancreatic cancer or pancreatic cancer precursor lesion patient from a healthy subject are listed below:
(1) a combination of SEQ ID NOs: 90 and 21 (markers: hsa-miR-3196 and hsa-miR-4257);
(2) a combination of SEQ ID NOs: 2 and 21 (markers: hsa-miR-1181 and hsa-miR-4257);
(3) a combination of SEQ ID NOs: 106 and 21 (markers: hsa-miR-4695-5p and hsa-miR-4257);
(4) a combination of SEQ ID NOs: 84 and 21 (markers: hsa-miR-1908-5p and hsa-miR-4257); and
(5) a combination of SEQ ID NOs: 85 and 21 (markers: hsa-miR-6729-5p and hsa-miR-4257).

Non-limiting examples of the combination comprising a polynucleotide consisting of the nucleotide sequence represented by SEQ ID NO: 28 or a complementary sequence thereof among the combinations constituted by two polynucleotides of the polynucleotides consisting of the nucleotide sequences represented by SEQ ID NOs: 1 to 250 for discriminating an early pancreatic cancer or pancreatic cancer precursor lesion patient from a healthy subject are listed below:
(1) a combination of SEQ ID NOs: 85 and 28 (markers: hsa-miR-6729-5p and hsa-miR-6782-5p);
(2) a combination of SEQ ID NOs: 84 and 28 (markers: hsa-miR-1908-5p and hsa-miR-6782-5p);
(3) a combination of SEQ ID NOs: 86 and 28 (markers: hsa-miR-5195-3p and hsa-miR-6782-5p);
(4) a combination of SEQ ID NOs: 87 and 28 (markers: hsa-miR-638 and hsa-miR-6782-5p); and
(5) a combination of SEQ ID NOs: 93 and 28 (markers: hsa-miR-4746-3p and hsa-miR-6782-5p).

The combination of polynucleotides with cancer type specificity capable of discriminating an early pancreatic cancer or pancreatic cancer precursor lesion patient not only from a healthy subject but also from patients with other cancers is preferably unlimitedly, for example, a combination of multiple polynucleotides comprising: at least one polynucleotide selected from the group consisting of polynucleotides consisting of the nucleotide sequences represented by SEQ ID NOs: 119, 12, 28, 105, 137, 121, 109, 87, 5, 140, 106, 2, 175, 90, 237, 247, 103, 97, 124, 92, 100, 32, 1, 246, 84, 13, 85, 153, 111, 86, 141, 54, and 24 or complementary sequences thereof (hereinafter, this group is referred to as "cancer type-specific polynucleotide group 1"); and any of the polynucleotides of the other SEQ ID NOs.

The combination of polynucleotides with cancer type specificity capable of discriminating an early pancreatic cancer or pancreatic cancer precursor lesion patient not only from a healthy subject but also from patients with other cancers is more preferably a combination of multiple polynucleotides selected from the cancer type-specific polynucleotide group 1.

The combination of polynucleotides with cancer type specificity capable of discriminating an early pancreatic cancer or pancreatic cancer precursor lesion patient not only from a healthy subject but also from patients with other cancers is more preferably, for example, a combination of polynucleotides comprising at least one polynucleotides selected from the group consisting of polynucleotides consisting of the nucleotide sequences represented by, for example, SEQ ID NOs: 119, 12, 28, 105, 137, 121, 109, 87, 5, 140, 106, 2, 175, 90, 237, and 247 or complementary sequences thereof (hereinafter, this group is referred to as "cancer type-specific polynucleotide group 2") included in the cancer type-specific polynucleotide group 1, among the combinations of multiple polynucleotides selected from the cancer type-specific polynucleotide group 1. The number of the polynucleotides with cancer type specificity may be 1, 2, 3, 4, 5, 6, 7, 8, 9, 10 or more in the combination and is more preferably 5 or more in the combination. Usually, the combination of 5 polynucleotides of these polynucleotides can produce sufficient performance (such as accuracy, sensitivity, or specificity).

Non-limiting examples of the combination of the polynucleotides consisting of the nucleotide sequences represented by SEQ ID NO: 12 or complementary sequences thereof, with polynucleotides consisting of nucleotide sequences represented by SEQ ID NOs of five polynucleotides selected from the cancer type-specific polynucleotide group 1 or complementary sequences thereof are further listed below:
(1) a combination of SEQ ID NOs: 12, 137, 119, 105, and 237 (markers: hsa-miR-5090, hsa-miR-4673, hsa-miR-6813-5p, hsa-miR-642a-3p, and hsa-miR-373-5p);
(2) a combination of SEQ ID NOs: 87, 12, 137, 119, and 105 (markers: hsa-miR-638, hsa-miR-5090, hsa-miR-4673, hsa-miR-6813-5p, and hsa-miR-642a-3p);
(3) a combination of SEQ ID NOs: 12, 103, 137, 105, and 247 (markers: hsa-miR-5090, hsa-miR-6085, hsa-miR-4673, hsa-miR-642a-3p, and hsa-miR-4286);
(4) a combination of SEQ ID NOs: 12, 137, 119, 92, and 105 (markers: hsa-miR-5090, hsa-miR-4673, hsa-miR-6813-5p, hsa-miR-4723-5p, and hsa-miR-642a-3p);
(5) a combination of SEQ ID NOs: 12, 137, 119, 105, and 121 (markers: hsa-miR-5090, hsa-miR-4673, hsa-miR-6813-5p, hsa-miR-642a-3p, and hsa-miR-602);
(6) a combination of SEQ ID NOs: 12, 137, 1, 119, and 105 (markers: hsa-miR-5090, hsa-miR-4673, hsa-miR-6784-5p, hsa-miR-6813-5p, and hsa-miR-642a-3p);
(7) a combination of SEQ ID NOs: 12, 137, 119, 124, and 105 (markers: hsa-miR-5090, hsa-miR-4673, hsa-miR-6813-5p, hsa-miR-5100, and hsa-miR-642a-3p);
(8) a combination of SEQ ID NOs: 12, 137, 119, 105, and 32 (markers: hsa-miR-5090, hsa-miR-4673, hsa-miR-6813-5p, hsa-miR-642a-3p, and hsa-miR-6087);
(9) a combination of SEQ ID NOs: 12, 137, 119, 105, and 100 (markers: hsa-miR-5090, hsa-miR-4673, hsa-miR-6813-5p, hsa-miR-642a-3p, and hsa-miR-6875-5p);
(10) a combination of SEQ ID NOs: 12, 137, 119, 105, and 86 (markers: hsa-miR-5090, hsa-miR-4673, hsa-miR-6813-5p, hsa-miR-642a-3p, and hsa-miR-5195-3p);
(11) a combination of SEQ ID NOs: 12, 137, 119, 105, and 153 (markers: hsa-miR-5090, hsa-miR-4673, hsa-miR-6813-5p, hsa-miR-642a-3p, and hsa-miR-296-5p);
(12) a combination of SEQ ID NOs: 12, 137, 119, 105, and 141 (markers: hsa-miR-5090, hsa-miR-4673, hsa-miR-6813-5p, hsa-miR-642a-3p, and hsa-miR-663a);
(13) a combination of SEQ ID NOs: 12, 137, 105, 246, and 153 (markers: hsa-miR-5090, hsa-miR-4673, hsa-miR-642a-3p, hsa-miR-6766-3p, and hsa-miR-296-5p);
(14) a combination of SEQ ID NOs: 12, 97, 137, 105, and 153 (markers: hsa-miR-5090, hsa-miR-7109-5p, hsa-miR-4673, hsa-miR-642a-3p, and hsa-miR-296-5p);
(15) a combination of SEQ ID NOs: 12, 103, 137, 119, and 105 (markers: hsa-miR-5090, hsa-miR-6085, hsa-miR-4673, hsa-miR-6813-5p, and hsa-miR-642a-3p);
(16) a combination of SEQ ID NOs: 12, 137, 119, 105, and 246 (markers: hsa-miR-5090, hsa-miR-4673, hsa-miR-6813-5p, hsa-miR-642a-3p, and hsa-miR-6766-3p);
(17) a combination of SEQ ID NOs: 12, 137, 92, 105, and 247 (markers: hsa-miR-5090, hsa-miR-4673, hsa-miR-4723-5p, hsa-miR-642a-3p, and hsa-miR-4286);
(18) a combination of SEQ ID NOs: 12, 137, 124, 105, and 153 (markers: hsa-miR-5090, hsa-miR-4673, hsa-miR-5100, hsa-miR-642a-3p, and hsa-miR-296-5p);
(19) a combination of SEQ ID NOs: 12, 137, 105, 32, and 153 (markers: hsa-miR-5090, hsa-miR-4673, hsa-miR-642a-3p, hsa-miR-6087, and hsa-miR-296-5p);
(20) a combination of SEQ ID NOs: 12, 137, 105, 13, and 121 (markers: hsa-miR-5090, hsa-miR-4673, hsa-miR-642a-3p, hsa-miR-3620-5p, and hsa-miR-602);
(21) a combination of SEQ ID NOs: 106, 12, 137, 119, and 105 (markers: hsa-miR-4695-5p, hsa-miR-5090, hsa-miR-4673, hsa-miR-6813-5p, and hsa-miR-642a-3p);
(22) a combination of SEQ ID NOs: 12, 137, 119, 105, and 13 (markers: hsa-miR-5090, hsa-miR-4673, hsa-miR-6813-5p, hsa-miR-642a-3p, and hsa-miR-3620-5p);
(23) a combination of SEQ ID NOs: 12, 137, 119, 105, and 140 (markers: hsa-miR-5090, hsa-miR-4673, hsa-miR-6813-5p, hsa-miR-642a-3p, and hsa-miR-4640-5p);
(24) a combination of SEQ ID NOs: 12, 137, 119, 105, and 247 (markers: hsa-miR-5090, hsa-miR-4673, hsa-miR-6813-5p, hsa-miR-642a-3p, and hsa-miR-4286);
(25) a combination of SEQ ID NOs: 12, 137, 119, 105, and 109 (markers: hsa-miR-5090, hsa-miR-4673, hsa-miR-6813-5p, hsa-miR-642a-3p, and hsa-miR-6769b-5p);
(26) a combination of SEQ ID NOs: 12, 137, 105, 109, and 121 (markers: hsa-miR-5090, hsa-miR-4673, hsa-miR-642a-3p, hsa-miR-6769b-5p, and hsa-miR-602);
(27) a combination of SEQ ID NOs: 12, 103, 137, 105, and 121 (markers: hsa-miR-5090, hsa-miR-6085, hsa-miR-4673, hsa-miR-642a-3p, and hsa-miR-602);
(28) a combination of SEQ ID NOs: 12, 137, 105, 32, and 121 (markers: hsa-miR-5090, hsa-miR-4673, hsa-miR-642a-3p, hsa-miR-6087, and hsa-miR-602);
(29) a combination of SEQ ID NOs: 12, 137, 124, 105, and 247 (markers: hsa-miR-5090, hsa-miR-4673, hsa-miR-5100, hsa-miR-642a-3p, and hsa-miR-4286);
(30) a combination of SEQ ID NOs: 12, 137, 105, 246, and 247 (markers: hsa-miR-5090, hsa-miR-4673, hsa-miR-642a-3p, hsa-miR-6766-3p, and hsa-miR-4286);
(31) a combination of SEQ ID NOs: 12, 137, 105, 153, and 247 (markers: hsa-miR-5090, hsa-miR-4673, hsa-miR-642a-3p, hsa-miR-296-5p, and hsa-miR-4286);
(32) a combination of SEQ ID NOs: 12, 137, 105, 247, and 141 (markers: hsa-miR-5090, hsa-miR-4673, hsa-miR-642a-3p, hsa-miR-4286, and hsa-miR-663a);
(33) a combination of SEQ ID NOs: 12, 137, 105, and 247 (markers: hsa-miR-5090, hsa-miR-4673, hsa-miR-642a-3p, and hsa-miR-4286);
(34) a combination of SEQ ID NOs: 12, 137, 105, 140, and 247 (markers: hsa-miR-5090, hsa-miR-4673, hsa-miR-642a-3p, hsa-miR-4640-5p, and hsa-miR-4286);
(35) a combination of SEQ ID NOs: 12, 119, 124, 105, and 140 (markers: hsa-miR-5090, hsa-miR-6813-5p, hsa-miR-5100, hsa-miR-642a-3p, and hsa-miR-4640-5p);
(36) a combination of SEQ ID NOs: 12, 119, 105, 100, and 140 (markers: hsa-miR-5090, hsa-miR-6813-5p, hsa-miR-642a-3p, hsa-miR-6875-5p, and hsa-miR-4640-5p);
(37) a combination of SEQ ID NOs: 90, 12, 119, 105, and 140 (markers: hsa-miR-3196, hsa-miR-5090, hsa-miR-6813-5p, hsa-miR-642a-3p, and hsa-miR-4640-5p);
(38) a combination of SEQ ID NOs: 90, 12, 137, 119, and 105 (markers: hsa-miR-3196, hsa-miR-5090, hsa-miR-4673, hsa-miR-6813-5p, and hsa-miR-642a-3p);
(39) a combination of SEQ ID NOs: 90, 12, 137, 105, and 32 (markers: hsa-miR-3196, hsa-miR-5090, hsa-miR-4673, hsa-miR-642a-3p, and hsa-miR-6087);
(40) a combination of SEQ ID NOs: 90, 12, 137, 105, and 153 (markers: hsa-miR-3196, hsa-miR-5090, hsa-miR-4673, hsa-miR-642a-3p, and hsa-miR-296-5p);
(41) a combination of SEQ ID NOs: 90, 12, 119, 105, and 100 (markers: hsa-miR-3196, hsa-miR-5090, hsa-miR-6813-5p, hsa-miR-642a-3p, and hsa-miR-6875-5p);
(42) a combination of SEQ ID NOs: 90, 12, 119, 109, and 140 (markers: hsa-miR-3196, hsa-miR-5090, hsa-miR-6813-5p, hsa-miR-6769b-5p, and hsa-miR-4640-5p);
(43) a combination of SEQ ID NOs: 87, 12, 137, 105, and 247 (markers: hsa-miR-638, hsa-miR-5090, hsa-miR-4673, hsa-miR-642a-3p, and hsa-miR-4286);
(44) a combination of SEQ ID NOs: 90, 12, 109, 140, and 237 (markers: hsa-miR-3196, hsa-miR-5090, hsa-miR-6769b-5p, hsa-miR-4640-5p, and hsa-miR-373-5p);
(45) a combination of SEQ ID NOs: 12, 137, 105, 109, and 153 (markers: hsa-miR-5090, hsa-miR-4673, hsa-miR-642a-3p, hsa-miR-6769b-5p, and hsa-miR-296-5p);
(46) a combination of SEQ ID NOs: 12, 137, 105, 109, and 247 (markers: hsa-miR-5090, hsa-miR-4673, hsa-miR-642a-3p, hsa-miR-6769b-5p, and hsa-miR-4286);
(47) a combination of SEQ ID NOs: 12, 137, 109, 140, and 247 (markers: hsa-miR-5090, hsa-miR-4673, hsa-miR-6769b-5p, hsa-miR-4640-5p, and hsa-miR-4286);
(48) a combination of SEQ ID NOs: 12, 137, 109, 121, and 237 (markers: hsa-miR-5090, hsa-miR-4673, hsa-miR-6769b-5p, hsa-miR-602, and hsa-miR-373-5p);
(49) a combination of SEQ ID NOs: 12, 137, 119, 105, and 175 (markers: hsa-miR-5090, hsa-miR-4673, hsa-miR-6813-5p, hsa-miR-642a-3p, and hsa-miR-6768-5p);
(50) a combination of SEQ ID NOs: 12, 137, 109, 175, and 121 (markers: hsa-miR-5090, hsa-miR-4673, hsa-miR-6769b-5p, hsa-miR-6768-5p, and hsa-miR-602);
(51) a combination of SEQ ID NOs: 87, 12, 119, 105, and 175 (markers: hsa-miR-638, hsa-miR-5090, hsa-miR-6813-5p, hsa-miR-642a-3p, and hsa-miR-6768-5p);
(52) a combination of SEQ ID NOs: 12, 137, 119, 105, and 111 (markers: hsa-miR-5090, hsa-miR-4673, hsa-miR-6813-5p, hsa-miR-642a-3p, and hsa-miR-187-5p);
(53) a combination of SEQ ID NOs: 12, 137, 119, 105, and 24 (markers: hsa-miR-5090, hsa-miR-4673, hsa-miR-6813-5p, hsa-miR-642a-3p, and hsa-miR-6887-5p);
(54) a combination of SEQ ID NOs: 12, 137, 105, 32, and 247 (markers: hsa-miR-5090, hsa-miR-4673, hsa-miR-642a-3p, hsa-miR-6087, and hsa-miR-4286);
(55) a combination of SEQ ID NOs: 90, 12, 137, 105, and 237 (markers: hsa-miR-3196, hsa-miR-5090, hsa-miR-4673, hsa-miR-642a-3p, and hsa-miR-373-5p);
(56) a combination of SEQ ID NOs: 12, 84, 137, 119, and 105 (markers: hsa-miR-5090, hsa-miR-1908-5p, hsa-miR-4673, hsa-miR-6813-5p, and hsa-miR-642a-3p);
(57) a combination of SEQ ID NOs: 12, 97, 137, 105, and 247 (markers: hsa-miR-5090, hsa-miR-7109-5p, hsa-miR-4673, hsa-miR-642a-3p, and hsa-miR-4286);
(58) a combination of SEQ ID NOs: 87, 12, 137, 105, and 237 (markers: hsa-miR-638, hsa-miR-5090, hsa-miR-4673, hsa-miR-642a-3p, and hsa-miR-373-5p);
(59) a combination of SEQ ID NOs: 87, 12, 100, 109, and 237 (markers: hsa-miR-638, hsa-miR-5090, hsa-miR-6875-5p, hsa-miR-6769b-5p, and hsa-miR-373-5p);
(60) a combination of SEQ ID NOs: 87, 12, 100, 109, and 237 (markers: hsa-miR-638, hsa-miR-5090, hsa-miR-6875-5p, hsa-miR-6769b-5p, and hsa-miR-373-5p);
(61) a combination of SEQ ID NOs: 106, 12, 137, 105, and 86 (markers: hsa-miR-4695-5p, hsa-miR-5090, hsa-miR-4673, hsa-miR-642a-3p, and hsa-miR-5195-3p);
(62) a combination of SEQ ID NOs: 106, 12, 137, 105, and 247 (markers: hsa-miR-4695-5p, hsa-miR-5090, hsa-miR-4673, hsa-miR-642a-3p, and hsa-miR-4286);
(63) a combination of SEQ ID NOs: 106, 12, 119, 105, and 100 (markers: hsa-miR-4695-5p, hsa-miR-5090, hsa-miR-6813-5p, hsa-miR-642a-3p, and hsa-miR-6875-5p); and
(64) a combination of SEQ ID NOs: 106, 12, 137, 105, and 121 (markers: hsa-miR-4695-5p, hsa-miR-5090, hsa-miR-4673, hsa-miR-642a-3p, and hsa-miR-602).

Non-limiting examples of the combination of the polynucleotides consisting of the nucleotide sequences represented by SEQ ID NO: 28 or complementary sequences thereof, with polynucleotides consisting of nucleotide sequences represented by SEQ ID NOs of five polynucleotides selected from the cancer type-specific polynucleotide group 1 or complementary sequences thereof are further listed below:
(1) a combination of SEQ ID NOs: 137, 119, 105, 28, and 237 (markers: hsa-miR-4673, hsa-miR-6813-5p, hsa-miR-642a-3p, hsa-miR-6782-5p, and hsa-miR-373-5p);
(2) a combination of SEQ ID NOs: 87, 106, 119, 28, and 121 (markers: hsa-miR-638, hsa-miR-4695-5p, hsa-miR-6813-5p, hsa-miR-6782-5p, and hsa-miR-602);
(3) a combination of SEQ ID NOs: 106, 137, 119, 28, and 121 (markers: hsa-miR-4695-5p, hsa-miR-4673, hsa-miR-6813-5p, hsa-miR-6782-5p, and hsa-miR-602); and
(4) a combination of SEQ ID NOs: 90, 119, 105, 28, and 237 (markers: hsa-miR-3196, hsa-miR-6813-5p, hsa-miR-642a-3p, hsa-miR-6782-5p, and hsa-miR-373-5p).

Non-limiting examples of the combination of the polynucleotides consisting of the nucleotide sequences represented by SEQ ID NO: 5 or complementary sequences thereof, with polynucleotides consisting of nucleotide sequences represented by SEQ ID NOs of five polynucleotides selected from the cancer type-specific polynucleotide group 1 or complementary sequences thereof are further listed below:
(1) a combination of SEQ ID NOs: 90, 5, 137, 119, and 105 (markers: hsa-miR-3196, hsa-miR-4276, hsa-miR-4673, hsa-miR-6813-5p, and hsa-miR-642a-3p);
(2) a combination of SEQ ID NOs: 5, 137, 119, 105, and 237 (markers: hsa-miR-4276, hsa-miR-4673, hsa-miR-6813-5p, hsa-miR-642a-3p, and hsa-miR-373-5p); and
(3) a combination of SEQ ID NOs: 5, 137, 119, 105, and 32 (markers: hsa-miR-4276, hsa-miR-4673, hsa-miR-6813-5p, hsa-miR-642a-3p, and hsa-miR-6087).

Non-limiting examples of the combination of the polynucleotides consisting of the nucleotide sequences represented by SEQ ID NO: 2 or complementary sequences thereof, with polynucleotides consisting of nucleotide sequences represented by SEQ ID NOs of five polynucleotides selected from the cancer type-specific polynucleotide group 1 or complementary sequences thereof are further listed below:
(1) a combination of SEQ ID NOs: 2, 137, 119, 105, and 237 (markers: hsa-miR-1181, hsa-miR-4673, hsa-miR-6813-5p, hsa-miR-642a-3p, and hsa-miR-373-5p);
(2) a combination of SEQ ID NOs: 2, 87, 137, 119, and 105 (markers: hsa-miR-1181, hsa-miR-638, hsa-miR-4673, hsa-miR-6813-5p, and hsa-miR-642a-3p);
(3) a combination of SEQ ID NOs: 2, 137, 119, 105, and 13 (markers: hsa-miR-1181, hsa-miR-4673, hsa-miR-6813-5p, hsa-miR-642a-3p, and hsa-miR-3620-5p);
(4) a combination of SEQ ID NOs: 2, 137, 119, 105, and 121 (markers: hsa-miR-1181, hsa-miR-4673, hsa-miR-6813-5p, hsa-miR-642a-3p, and hsa-miR-602);
(5) a combination of SEQ ID NOs: 2, 137, 119, 105, and 247 (markers: hsa-miR-1181, hsa-miR-4673, hsa-miR-6813-5p, hsa-miR-642a-3p, and hsa-miR-4286);
(6) a combination of SEQ ID NOs: 2, 87, 119, 109, and 247 (markers: hsa-miR-1181, hsa-miR-638, hsa-miR-6813-5p, hsa-miR-6769b-5p, and hsa-miR-4286);
(7) a combination of SEQ ID NOs: 2, 90, 137, 119, and 105 (markers: hsa-miR-1181, hsa-miR-3196, hsa-miR-4673, hsa-miR-6813-5p, and hsa-miR-642a-3p);
(8) a combination of SEQ ID NOs: 2, 137, 119, 105, and 140 (markers: hsa-miR-1181, hsa-miR-4673, hsa-miR-6813-5p, hsa-miR-642a-3p, and hsa-miR-4640-5p); and
(9) a combination of SEQ ID NOs: 2, 87, 119, 105, and 237 (markers: hsa-miR-1181, hsa-miR-638, hsa-miR-6813-5p, hsa-miR-642a-3p, and hsa-miR-373-5p).

Non-limiting examples of the combination of the polynucleotides consisting of the nucleotide sequences represented by SEQ ID NOs: 12 and 28 or complementary sequences thereof, with polynucleotides consisting of nucleotide sequences represented by SEQ ID NOs of five polynucleotides selected from the cancer type-specific polynucleotide group 1 or complementary sequences thereof are further listed below:
(1) a combination of SEQ ID NOs: 12, 137, 105, 28, and 247 (markers: hsa-miR-5090, hsa-miR-4673, hsa-miR-642a-3p, hsa-miR-6782-5p, and hsa-miR-4286);
(2) a combination of SEQ ID NOs: 12, 137, 119, 105, and 28 (markers: hsa-miR-5090, hsa-miR-4673, hsa-miR-6813-5p, hsa-miR-642a-3p, and hsa-miR-6782-5p);
(3) a combination of SEQ ID NOs: 12, 103, 137, 105, and 28 (markers: hsa-miR-5090, hsa-miR-6085, hsa-miR-4673, hsa-miR-642a-3p, and hsa-miR-6782-5p);
(4) a combination of SEQ ID NOs: 12, 84, 28, 140, and 121 (markers: hsa-miR-5090, hsa-miR-1908-5p, hsa-miR-6782-5p, hsa-miR-4640-5p, and hsa-miR-602);
(5) a combination of SEQ ID NOs: 12, 137, 28, 109, and 121 (markers: hsa-miR-5090, hsa-miR-4673, hsa-miR-6782-5p, hsa-miR-6769b-5p, and hsa-miR-602);
(6) a combination of SEQ ID NOs: 12, 1, 28, 121, and 247 (markers: hsa-miR-5090, hsa-miR-6784-5p, hsa-miR-6782-5p, hsa-miR-602, and hsa-miR-4286);
(7) a combination of SEQ ID NOs: 12, 119, 28, 100, and 121 (markers: hsa-miR-5090, hsa-miR-6813-5p, hsa-miR-6782-5p, hsa-miR-6875-5p, and hsa-miR-602);
(8) a combination of SEQ ID NOs: 12, 92, 28, 100, and 247 (markers: hsa-miR-5090, hsa-miR-4723-5p, hsa-miR-6782-5p, hsa-miR-6875-5p, and hsa-miR-4286);
(9) a combination of SEQ ID NOs: 12, 28, 100, 140, and 247 (markers: hsa-miR-5090, hsa-miR-6782-5p, hsa-miR-6875-5p, hsa-miR-4640-5p, and hsa-miR-4286);
(10) a combination of SEQ ID NOs: 12, 137, 28, 109, and 247 (markers: hsa-miR-5090, hsa-miR-4673, hsa-miR-6782-5p, hsa-miR-6769b-5p, and hsa-miR-4286);
(11) a combination of SEQ ID NOs: 12, 84, 28, 109, and 121 (markers: hsa-miR-5090, hsa-miR-1908-5p, hsa-miR-6782-5p, hsa-miR-6769b-5p, and hsa-miR-602);
(12) a combination of SEQ ID NOs: 12, 103, 1, 28, and 121 (markers: hsa-miR-5090, hsa-miR-6085, hsa-miR-6784-5p, hsa-miR-6782-5p, and hsa-miR-602);
(13) a combination of SEQ ID NOs: 12, 1, 28, 32, and 121 (markers: hsa-miR-5090, hsa-miR-6784-5p, hsa-miR-6782-5p, hsa-miR-6087, and hsa-miR-602);
(14) a combination of SEQ ID NOs: 12, 1, 28, 100, and 121 (markers: hsa-miR-5090, hsa-miR-6784-5p, hsa-miR-6782-5p, hsa-miR-6875-5p, and hsa-miR-602); and
(15) a combination of SEQ ID NOs: 12, 1, 28, 175, and 121 (markers: hsa-miR-5090, hsa-miR-6784-5p, hsa-miR-6782-5p, hsa-miR-6768-5p, and hsa-miR-602).

Non-limiting examples of the combination of the polynucleotides consisting of the nucleotide sequences represented by SEQ ID NOs: 12 and 5 or complementary sequences thereof, with polynucleotides consisting of nucleotide sequences represented by SEQ ID NOs of five polynucleotides selected from the cancer type-specific polynucleotide group 1 or complementary sequences thereof are further listed below:
(1) a combination of SEQ ID NOs: 5, 12, 137, 119, and 105 (markers: hsa-miR-4276, hsa-miR-5090, hsa-miR-4673, hsa-miR-6813-5p, and hsa-miR-642a-3p);
(2) a combination of SEQ ID NOs: 90, 5, 12, 119, and 105 (markers: hsa-miR-3196, hsa-miR-4276, hsa-miR-5090, hsa-miR-6813-5p, and hsa-miR-642a-3p);
(3) a combination of SEQ ID NOs: 5, 12, 137, 105, and 121 (markers: hsa-miR-4276, hsa-miR-5090, hsa-miR-4673, hsa-miR-642a-3p, and hsa-miR-602);
(4) a combination of SEQ ID NOs: 5, 12, 119, 92, and 121 (markers: hsa-miR-4276, hsa-miR-5090, hsa-miR-6813-5p, hsa-miR-4723-5p, and hsa-miR-602);
(5) a combination of SEQ ID NOs: 90, 5, 12, 109, and 247 (markers: hsa-miR-3196, hsa-miR-4276, hsa-miR-5090, hsa-miR-6769b-5p, and hsa-miR-4286);
(6) a combination of SEQ ID NOs: 5, 12, 137, 109, and 121 (markers: hsa-miR-4276, hsa-miR-5090, hsa-miR-4673, hsa-miR-6769b-5p, and hsa-miR-602);
(7) a combination of SEQ ID NOs: 5, 12, 137, 109, and 247 (markers: hsa-miR-4276, hsa-miR-5090, hsa-miR-4673, hsa-miR-6769b-5p, and hsa-miR-4286);
(8) a combination of SEQ ID NOs: 90, 5, 106, 12, and 109 (markers: hsa-miR-3196, hsa-miR-4276, hsa-miR-4695-5p, hsa-miR-5090, and hsa-miR-6769b-5p);
(9) a combination of SEQ ID NOs: 90, 5, 12, 137, and 105 (markers: hsa-miR-3196, hsa-miR-4276, hsa-miR-5090, hsa-miR-4673, and hsa-miR-642a-3p);
(10) a combination of SEQ ID NOs: 90, 5, 12, 119, and 109 (markers: hsa-miR-3196, hsa-miR-4276, hsa-miR-5090, hsa-miR-6813-5p, and hsa-miR-6769b-5p);
(11) a combination of SEQ ID NOs: 90, 5, 12, 105, and 109 (markers: hsa-miR-3196, hsa-miR-4276, hsa-miR-5090, hsa-miR-642a-3p, and hsa-miR-6769b-5p);
(12) a combination of SEQ ID NOs: 5, 12, 137, 105, and 153 (markers: hsa-miR-4276, hsa-miR-5090, hsa-miR-4673, hsa-miR-642a-3p, and hsa-miR-296-5p);
(13) a combination of SEQ ID NOs: 5, 12, 119, 105, and 54 (markers: hsa-miR-4276, hsa-miR-5090, hsa-miR-6813-5p, hsa-miR-642a-3p, and hsa-miR-5787);
(14) a combination of SEQ ID NOs: 87, 5, 106, 12, and 109 (markers: hsa-miR-638, hsa-miR-4276, hsa-miR-4695-5p, hsa-miR-5090, and hsa-miR-6769b-5p);
(15) a combination of SEQ ID NOs: 87, 5, 12, 137, and 105 (markers: hsa-miR-638, hsa-miR-4276, hsa-miR-5090, hsa-miR-4673, and hsa-miR-642a-3p);
(16) a combination of SEQ ID NOs: 90, 5, 12, 1, and 105 (markers: hsa-miR-3196, hsa-miR-4276, hsa-miR-5090, hsa-miR-6784-5p, and hsa-miR-642a-3p);
(17) a combination of SEQ ID NOs: 90, 5, 12, 109, and 86 (markers: hsa-miR-3196, hsa-miR-4276, hsa-miR-5090, hsa-miR-6769b-5p, and hsa-miR-5195-3p);
(18) a combination of SEQ ID NOs: 5, 12, 137, 105, and 247 (markers: hsa-miR-4276, hsa-miR-5090, hsa-miR-4673, hsa-miR-642a-3p, and hsa-miR-4286);
(19) a combination of SEQ ID NOs: 90, 5, 12, 109, and 175 (markers: hsa-miR-3196, hsa-miR-4276, hsa-miR-5090, hsa-miR-6769b-5p, and hsa-miR-6768-5p); and
(20) a combination of SEQ ID NOs: 5, 12, 100, 109, and 121 (markers: hsa-miR-4276, hsa-miR-5090, hsa-miR-6875-5p, hsa-miR-6769b-5p, and hsa-miR-602).

Non-limiting examples of the combination of the polynucleotides consisting of the nucleotide sequences represented by SEQ ID NOs: 12 and 2 or complementary sequences thereof, with polynucleotides consisting of nucleotide sequences represented by SEQ ID NOs of five polynucleotides selected from the cancer type-specific polynucleotide group 1 or complementary sequences thereof are further listed below:
(1) a combination of SEQ ID NOs: 2, 12, 137, 119, and 105 (markers: hsa-miR-1181, hsa-miR-5090, hsa-miR-4673, hsa-miR-6813-5p, and hsa-miR-642a-3p);
(2) a combination of SEQ ID NOs: 2, 12, 137, 105, and 153 (markers: hsa-miR-1181, hsa-miR-5090, hsa-miR-4673, hsa-miR-642a-3p, and hsa-miR-296-5p);
(3) a combination of SEQ ID NOs: 2, 12, 137, 105, and 121 (markers: hsa-miR-1181, hsa-miR-5090, hsa-miR-4673, hsa-miR-642a-3p, and hsa-miR-602);
(4) a combination of SEQ ID NOs: 2, 12, 109, 121, and 247 (markers: hsa-miR-1181, hsa-miR-5090, hsa-miR-6769b-5p, hsa-miR-602, and hsa-miR-4286);
(5) a combination of SEQ ID NOs: 2, 90, 12, 119, and 105 (markers: hsa-miR-1181, hsa-miR-3196, hsa-miR-5090, hsa-miR-6813-5p, and hsa-miR-642a-3p);
(6) a combination of SEQ ID NOs: 2, 90, 12, 109, and 140 (markers: hsa-miR-1181, hsa-miR-3196, hsa-miR-5090, hsa-miR-6769b-5p, and hsa-miR-4640-5p);
(7) a combination of SEQ ID NOs: 2, 12, 100, 109, and 121 (markers: hsa-miR-1181, hsa-miR-5090, hsa-miR-6875-5p, hsa-miR-6769b-5p, and hsa-miR-602);
(8) a combination of SEQ ID NOs: 2, 12, 109, 175, and 121 (markers: hsa-miR-1181, hsa-miR-5090, hsa-miR-6769b-5p, hsa-miR-6768-5p, and hsa-miR-602);
(9) a combination of SEQ ID NOs: 2, 12, 97, 105, and 247 (markers: hsa-miR-1181, hsa-miR-5090, hsa-miR-7109-5p, hsa-miR-642a-3p, and hsa-miR-4286);
(10) a combination of SEQ ID NOs: 2, 12, 137, 105, and 247 (markers: hsa-miR-1181, hsa-miR-5090, hsa-miR-4673, hsa-miR-642a-3p, and hsa-miR-4286); and
(11) a combination of SEQ ID NOs: 2, 12, 137, 109, and 121 (markers: hsa-miR-1181, hsa-miR-5090, hsa-miR-4673, hsa-miR-6769b-5p, and hsa-miR-602).

Non-limiting examples of the combination of the polynucleotides consisting of the nucleotide sequences represented by SEQ ID NOs: 5 and 2 or complementary sequences thereof, with polynucleotides consisting of nucleotide sequences represented by SEQ ID NOs of five polynucleotides selected from the cancer type-specific polynucleotide group 1 or complementary sequences thereof are further listed below:
(1) a combination of SEQ ID NOs: 2, 90, 5, 119, and 105 (markers: hsa-miR-1181, hsa-miR-3196, hsa-miR-4276, hsa-miR-6813-5p, and hsa-miR-642a-3p);
(2) a combination of SEQ ID NOs: 2, 5, 119, 109, and 121 (markers: hsa-miR-1181, hsa-miR-4276, hsa-miR-6813-5p, hsa-miR-6769b-5p, and hsa-miR-602); and
(3) a combination of SEQ ID NOs: 2, 5, 119, 86, and 121 (markers: hsa-miR-1181, hsa-miR-4276, hsa-miR-6813-5p, hsa-miR-5195-3p, and hsa-miR-602).

Non-limiting examples of the combination of the polynucleotides consisting of the nucleotide sequences represented by SEQ ID NOs: 12, 28, and 5 or complementary sequences thereof, with polynucleotides consisting of nucleotide sequences represented by SEQ ID NOs of five polynucleotides selected from the cancer type-specific polynucleotide group 1 or complementary sequences thereof are further listed below:
(1) a combination of SEQ ID NOs: 5, 12, 1, 28, and 121 (markers: hsa-miR-4276, hsa-miR-5090, hsa-miR-6784-5p, hsa-miR-6782-5p, and hsa-miR-602).

The kit or the device of the present invention can also comprise a known polynucleotide(s) that enables detection of early pancreatic cancer or a pancreatic cancer precursor lesion, or a polynucleotide(s) that will be found in the future, in addition to the polynucleotide(s) (which may include a variant(s), a fragment(s), or a derivative(s)) as described above according to the present invention.

The kit of the present invention can also comprise an antibody for measuring a marker or markers for examination of early pancreatic cancer or pancreatic cancer precursor lesion known in the art, such as CEA, CA19-9, SPan-1, DUPAN-2, CA50, CA242, TAG-72, urinary fucose, POA, and TPS, in addition to the polynucleotide(s) according to the present invention as described above, and a variant or variants thereof or a fragment or fragments thereof.

These polynucleotides and variants thereof or fragments thereof contained in the kit of the present invention may be packaged in different containers either individually or in any combination.

The kit of the present invention may comprise a kit for extracting nucleic acids (e.g., total RNA) from body fluids, cells, or tissues, a fluorescent material for labeling, an enzyme and a medium for nucleic acid amplification, an instruction manual, etc.

The device of the present invention is a device for cancer marker measurement in which nucleic acids such as the polynucleotides according to the present invention described above, variants thereof, derivatives thereof, or fragments thereof are linked or attached to, for example, a solid phase. Examples of the material for the solid phase include plastics, paper, glass, and silicon. The material for the solid phase is preferably a plastic from the viewpoint of easy processability. The solid phase has any shape and is, for example, square, round, reed-shaped, or film-shaped. The device of the present invention includes, for example, a device for measurement by a hybridization technique. Specific examples thereof include blotting devices and nucleic acid arrays (e.g., microarrays, DNA chips, and RNA chips).

The nucleic acid array technique is a technique which involves linking or attaching the nucleic acids one by one by use of a method [e.g., a method of spotting the nucleic acids using a high-density dispenser called spotter or arrayer onto the surface of the solid phase surface-treated, if necessary, by L-lysine coating or the introduction of a functional group such as an amino group or a carboxyl group, a method of spraying the nucleic acids onto the solid phase using an inkjet which injects very small liquid droplets by a piezoelectric element or the like from a nozzle, or a method of sequentially synthesizing nucleotides on the solid phase] to prepare an array such as a chip and measuring target nucleic acids through the use of hybridization using this array.

The kit or the device of the present invention comprises nucleic acids capable of specifically binding to the polynucleotides of at least one, preferably at least two, more preferably at least three, most preferably at least five to all of the early pancreatic cancer or pancreatic cancer precursor lesion marker miRNAs, respectively, of the group 1 described above. The kit or the device of the present invention can optionally further comprise nucleic acids capable of specifically binding to the polynucleotides of at least one, preferably at least two, more preferably at least three, most preferably at least five to all of the early pancreatic cancer or pancreatic cancer precursor lesion marker miRNAs, respectively, of the group 2 described above.

The kit or the device of the present invention can be used for detecting early pancreatic cancer or a pancreatic cancer precursor lesion as described in Section 4 below.

### 4. Method for detecting early pancreatic cancer or pancreatic cancer precursor lesion

The present invention further provides a method for detecting early pancreatic cancer or a pancreatic cancer precursor lesion in a subject, comprising using the kit or the device of the present invention (comprising the above-mentioned nucleic acid(s) that can be used in the present invention) as described in Section 3 above to measure an expression level of at least one early pancreatic cancer or pancreatic cancer precursor lesion-derived gene selected from the following group of miRNAs, i.e., miR-6784-5p, miR-1181, miR-671-5p, miR-6857-5p, miR-4276, miR-1914-3p, miR-149-3p, miR-937-5p, miR-4675, miR-6795-5p, miR-4731-5p, miR-5090, miR-3620-5p, miR-1343-5p, miR-6717-5p, miR-6825-5p, miR-6738-5p, miR-6769a-5p, miR-4728-5p, miR-652-5p, miR-4257, miR-6785-5p, miR-7110-5p, miR-6887-5p, miR-887-3p, miR-1228-5p, miR-5572, miR-6782-5p, miR-4298, miR-6786-5p, miR-5010-5p, miR-6087, miR-6765-5p, miR-6732-5p, miR-6787-5p, miR-6737-5p, miR-128-2-5p, miR-4270, miR-6861-5p, miR-6756-5p, miR-1229-5p, miR-6891-5p, miR-6848-5p, miR-1237-5p, miR-30c-1-3p, miR-1233-5p, miR-211-3p, miR-4758-5p, miR-614, miR-6746-5p, miR-1915-5p, miR-4688, miR-3917, miR-5787, miR-4632-5p, miR-6126, miR-135a-3p, miR-8063, miR-5698, miR-6089, miR-498, miR-296-3p, miR-4419b, miR-6802-5p, miR-6829-5p, miR-6803-5p, miR-1199-5p, miR-6840-3p, miR-6752-5p, miR-6798-5p, miR-6131, miR-4667-5p, miR-6510-5p, miR-4690-5p, miR-920, miR-23b-3p, miR-4448, miR-2110, miR-4706, miR-7845-5p, miR-6808-5p, miR-4447, miR-6869-5p, miR-6794-5p, miR-6511a-5p, miR-6824-5p, miR-6766-3p, miR-6511a-5p, and miR-6749-5p, and optionally an expression level of at least one early pancreatic cancer or pancreatic cancer precursor lesion-derived gene selected from the following group of miRNAs, i.e., miR-1908-5p, miR-6729-5p, miR-5195-3p, miR-638, miR-6125, miR-3178, miR-3196, miR-8069, miR-4723-5p, miR-4746-3p, miR-4689, miR-6816-5p, miR-6757-5p, miR-7109-5p, miR-6724-5p, miR-1225-3p, miR-6875-5p, miR-7108-5p, miR-4508, miR-6085, miR-6779-5p, miR-642a-3p, miR-4695-5p, miR-7847-3p, miR-3197, miR-6769b-5p, miR-7641, miR-187-5p, miR-3185, miR-2861, miR-3940-5p, miR-1203, miR-615-5p, miR-4787-5p, miR-1343-3p, miR-6813-5p, miR-1225-5p, miR-602, miR-4488, miR-125a-3p, miR-5100, miR-4294, miR-1231, miR-6765-3p, miR-4442, miR-718, miR-6780b-5p, miR-6090, miR-6845-5p, miR-4741, miR-4467, miR-4707-5p, miR-4271, miR-4673, miR-3184-5p, miR-1469, miR-4640-5p, miR-663a, miR-6791-5p, miR-6826-5p, miR-4433b-3p, miR-1915-3p, miR-4417, miR-4449, miR-4707-3p, miR-3180-3p, miR-5585-3p, miR-1268a, miR-8072, miR-296-5p, miR-204-3p, miR-4454, miR-6722-3p, miR-1290, miR-3622a-5p, miR-939-5p, miR-675-5p, miR-3131, miR-4648, miR-1268b, miR-6741-5p, miR-6893-5p, miR-3162-5p, miR-642b-3p, miR-4734, miR-150-3p, miR-8089, miR-6805-3p, miR-7113-3p, miR-6850-5p, miR-6799-5p, miR-6768-5p, miR-92b-5p, miR-3679-5p, miR-4792, miR-3656, miR-92a-2-5p, miR-4466, miR-4513, miR-6781-5p, miR-4649-5p, miR-6775-5p, miR-4651, miR-3195, miR-6726-5p, miR-6872-3p, miR-371a-5p, miR-6777-5p, miR-6789-5p, miR-7975, miR-6821-5p, miR-4534, miR-619-5p, miR-7107-5p, miR-1228-3p, miR-6774-5p, miR-6805-5p, miR-23a-3p, miR-4665-5p, miR-4505, miR-4638-5p, miR-24-3p, miR-3135b, miR-4745-5p, miR-128-1-5p, miR-4476, miR-4687-3p, miR-3665, miR-6806-5p, miR-3937, miR-711, miR-3141, miR-3188, miR-4281, miR-5196-5p, miR-6880-5p, miR-3960, miR-3648, miR-6721-5p, miR-4492, miR-744-5p, miR-7704, miR-4749-5p, miR-762, miR-6836-3p, miR-6727-5p, miR-4739, miR-7977, miR-4484, miR-6515-3p, miR-373-5p, miR-4258, miR-4674, miR-3180, miR-6076, miR-1238-5p, miR-4463, miR-4486, miR-4730, miR-4286, and miR-4739 in a sample such as blood, serum, or plasma *in vitro*; and comparing, for example, the expression levels having statistically significant difference using the expression level(s) thus measured and a control expression level(s) in a healthy subject(s) (including a non-pancreatic cancer patient(s) and a non-pancreatic cancer precursor lesion patient(s)) measured in the same way, or evaluating *in vitro* whether the subject has early pancreatic cancer or a pancreatic cancer precursor lesion on the basis of a discriminant score determined from the expression level(s) of the gene(s) in the sample and a discriminant (see below), to detect the presence or absence of early pancreatic cancer or a pancreatic cancer precursor lesion in the subject.

This method of the present invention enables a minimally invasive, early diagnosis of the cancer with high sensitivity and high specificity and thereby brings about early treatment and improved prognosis. In addition, exacerbation of the disease or the effectiveness of surgical, radiotherapeutic, and chemotherapeutic treatments can be monitored.

The method for extracting the early pancreatic cancer or pancreatic cancer precursor lesion-derived gene(s) from the sample such as blood, serum, or plasma according to the present invention is particularly preferably prepared by the addition of a reagent for RNA extraction in 3D-Gene^{(R)} RNA extraction reagent from liquid sample kit (Toray Industries, Inc.). A general acidic phenol method (acid guanidinium-phenol-chloroform (AGPC) method) may be used, or Trizol^{(R)} (Life Technologies Corp.) may be used. The pancreatic cancer or pancreatic cancer precursor lesion-derived gene(s) may be prepared by the addition of a reagent for RNA extraction containing acidic phenol, such as Trizol (Life Technologies Corp.) or Isogen (Nippon Gene Co., Ltd., Japan). Alternatively, a kit such as miRNeasy^{(R)} Mini Kit (Qiagen N.V.) may be used, though the method is not limited thereto.

The present invention also provides use of the kit or the device of the present invention for detecting *in vitro* an expression product(s) of an early pancreatic cancer or pancreatic cancer precursor lesion-derived miRNA gene(s) in a sample derived from a subject.

In the method of the present invention, the kit or the device described above comprises a single polynucleotide or any possible combination of polynucleotides that can be used in the present invention as described above.

In the detection or (genetic) diagnosis of early pancreatic cancer or a pancreatic cancer precursor lesion according to the present invention, each polynucleotide contained in the kit or the device of the present invention can be used as a probe or a primer. In the case of using the polynucleotide as a primer, TaqMan^{(R)} MicroRNA Assays from Life Technologies Corp., miScript PCR System from Qiagen N.V., or the like can be used, though the method is not limited thereto.

The polynucleotide contained in the kit or the device of the present invention can be used as a primer or a probe according to a routine method in a method known in the art for specifically detecting a particular gene, for example, a hybridization technique such as Northern blot, Southern blot, *in situ* hybridization, Northern hybridization, or Southern hybridization, a polynucleotide sequencing technique using a sequencer or the like, or a quantitative amplification technique such as quantitative RT-PCR. A body fluid such as blood, serum, plasma, or urine from a subject is collected as a sample to be assayed according to the type of the detection method used. Alternatively, total RNA prepared from such a body fluid by the method described above may be used, and various polynucleotides including cDNA prepared from the RNA may be used.

The kit or the device of the present invention is useful for the diagnosis of early pancreatic cancer or a pancreatic cancer precursor lesion or the detection of the presence or absence of early pancreatic cancer or a pancreatic cancer precursor lesion. Specifically, the detection of early pancreatic cancer or a pancreatic cancer precursor lesion using the kit or the device can be performed by detecting *in vitro* an expression level(s) of a gene(s) using the nucleic acid probe(s) or the primer(s) contained in the kit or the device, in a sample such as blood, serum, plasma, or urine from a subject suspected of having early pancreatic cancer or a pancreatic cancer precursor lesion. The subject suspected of having early pancreatic cancer or a pancreatic cancer precursor lesion can be evaluated as having early pancreatic cancer or a pancreatic cancer precursor lesion when the expression level(s) of a target miRNA marker(s) measured using polynucleotide(s) (including a variant(s), a fragment(s), and a derivative(s) thereof) consisting of a nucleotide sequence(s) represented by at least one (one or more) of SEQ ID NOs: 1 to 83, 227 to 229, 246, 248, and 250 or a complementary sequence(s) thereof, and optionally a nucleotide sequence(s) represented by one or more of SEQ ID NOs: 84 to 226, 230 to 245, 247, and 249 or a complementary sequence(s) thereof, in the sample such as blood, serum, plasma, or urine of the subject, has a statistically significant difference compared to an expression level(s) thereof in the sample such as blood, serum, or plasma, or urine of a healthy subject.

The method of the present invention can be combined with a diagnostic imaging method such as abdominal ultrasonography, CT scanning, endoscopic retrograde cholangiopancreatography, or endoscopic ultrasonography. The method of the present invention is capable of specifically detecting early pancreatic cancer or a pancreatic cancer precursor lesion and can substantially discriminate early pancreatic cancer or a pancreatic cancer precursor lesion from the other cancers. Alternatively, these cancers can be discriminated therefrom by combination with an additional diagnostic method such as the diagnostic imaging method as described above.

The method for detecting the absence of early pancreatic cancer or a pancreatic cancer precursor lesion or the presence of early pancreatic cancer or a pancreatic cancer precursor lesion in a sample from a subject using the kit or the device of the present invention comprises collecting a body fluid such as blood, serum, plasma, or urine of the subject, and measuring the expression level(s) of the target gene(s) (or the target nucleic acid(s)) contained therein using one or more polynucleotides (including a variant(s), a fragment(s), or a derivative(s)) selected from the groups of polynucleotides of the present invention, to evaluate the presence or absence of early pancreatic cancer or a pancreatic cancer precursor lesion or to detect early pancreatic cancer or a pancreatic cancer precursor lesion. The method for detecting early pancreatic cancer or a pancreatic cancer precursor lesion according to the present invention can also be used for evaluating or diagnosing, for example, the presence or absence of amelioration of the disease or the degree of amelioration thereof in an early pancreatic cancer or pancreatic cancer precursor lesion patient in the case that a pancreatic cancer-related therapeutic drug known or under development (non-limiting examples thereof include TS-1 (three-component combination drug of tegafur/gimeracil/oteracil potassium), Gemzar (gemcitabine hydrochloride), Tarceva (erlotinib hydrochloride), 5-FU (fluorouracil), levofolinate, irinotecan, oxaliplatin, Abraxane (nab-paclitaxel), and combinations thereof) is administered to the patient for the purpose of treating or ameliorating the disease.

The method of the present invention can comprise, for example, the following steps (a), (b), and (c):
(a) a step of contacting *in vitro* a sample from a subject with a polynucleotide(s) contained in the kit or the device of the present invention;
(b) a step of measuring an expression level(s) of the target nucleic acid(s) in the sample using the polynucleotide(s) as a nucleic acid probe(s) or primer(s); and
(c) a step of evaluating whether or not the subject has early pancreatic cancer or a pancreatic cancer precursor lesion (cells) on the basis of the measurement results in the step (b) to detect the presence or absence of early pancreatic cancer or a pancreatic cancer precursor lesion (cells) in the subject.

In the step (a), blood, serum, or plasma can be used as a preferred sample.

In the step (b), the measurement of the expression level(s) can be performed by a technique, for example, a hybridization technique such as a nucleic acid array method, a polynucleotide sequencing technique using a sequencer or the like, or a quantitative amplification technique such as quantitative RT-PCR.

In the step (c), the subject can be evaluated as having early pancreatic cancer or a pancreatic cancer precursor lesion on the basis of a discriminant score prepared from the expression level(s) of the target nucleic acid(s) in the sample from the subject and a discriminant (mentioned later) in the case that the expression level(s) of the target nucleic acid(s) in the sample from the subject is statistically significantly different from that in a sample(s) derived from a healthy subject(s) or benign pancreatic disease subject(s) (this expression level(s) is also referred to as "reference" or "control").

Specifically, the present invention provides a method for detecting early pancreatic cancer or a pancreatic cancer precursor lesion in a subject, comprising: measuring an expression level(s) of a target nucleic acid(s) in a sample of the subject using a nucleic acid(s) capable of specifically binding to at least one (one or more), preferably at least two, at least three, at least four, or at least five polynucleotides selected from the following miRNAs: miR-6784-5p, miR-1181, miR-671-5p, miR-6857-5p, miR-4276, miR-1914-3p, miR-149-3p, miR-937-5p, miR-4675, miR-6795-5p, miR-4731-5p, miR-5090, miR-3620-5p, miR-1343-5p, miR-6717-5p, miR-6825-5p, miR-6738-5p, miR-6769a-5p, miR-4728-5p, miR-652-5p, miR-4257, miR-6785-5p, miR-7110-5p, miR-6887-5p, miR-887-3p, miR-1228-5p, miR-5572, miR-6782-5p, miR-4298, miR-6786-5p, miR-5010-5p, miR-6087, miR-6765-5p, miR-6732-5p, miR-6787-5p, miR-6737-5p, miR-128-2-5p, miR-4270, miR-6861-5p, miR-6756-5p, miR-1229-5p, miR-6891-5p, miR-6848-5p, miR-1237-5p, miR-30c-1-3p, miR-1233-5p, miR-211-3p, miR-4758-5p, miR-614, miR-6746-5p, miR-1915-5p, miR-4688, miR-3917, miR-5787, miR-4632-5p, miR-6126, miR-135a-3p, miR-8063, miR-5698, miR-6089, miR-498, miR-296-3p, miR-4419b, miR-6802-5p, miR-6829-5p, miR-6803-5p, miR-1199-5p, miR-6840-3p, miR-6752-5p, miR-6798-5p, miR-6131, miR-4667-5p, miR-6510-5p, miR-4690-5p, miR-920, miR-23b-3p, miR-4448, miR-2110, miR-4706, miR-7845-5p, miR-6808-5p, miR-4447, miR-6869-5p, miR-6794-5p, miR-6511a-5p, miR-6824-5p, miR-6766-3p, miR-6511a-5p, and miR-6749-5p; and evaluating *in vitro* whether or not the subject has early pancreatic cancer or a pancreatic cancer precursor lesion using the above-measured expression levels and control expression levels of a healthy subject(s) measured in the same way as above, to detect the presence or absence of early pancreatic cancer or a pancreatic cancer precursor lesion in the subject.

As used herein, the term "evaluation" may be physician's judgement or is evaluation support based on results of *in vitro* examination without physician's judgment.

As described above, in the method of the present invention, specifically, miR-6784-5p is hsa-miR-6784-5p, miR-1181 is hsa-miR-1181, miR-671-5p is hsa-miR-671-5p, miR-6857-5p is hsa-miR-6857-5p, miR-4276 is hsa-miR-4276, miR-1914-3p is hsa-miR-1914-3p, miR-149-3p is hsa-miR-149-3p, miR-937-5p is hsa-miR-937-5p, miR-4675 is hsa-miR-4675, miR-6795-5p is hsa-miR-6795-5p, miR-4731-5p is hsa-miR-4731-5p, miR-5090 is hsa-miR-5090, miR-3620-5p is hsa-miR-3620-5p, miR-1343-5p is hsa-miR-1343-5p, miR-6717-5p is hsa-miR-6717-5p, miR-6825-5p is hsa-miR-6825-5p, miR-6738-5p is hsa-miR-6738-5p, miR-6769a-5p is hsa-miR-6769a-5p, miR-4728-5p is hsa-miR-4728-5p, miR-652-5p is hsa-miR-652-5p, miR-4257 is hsa-miR-4257, miR-6785-5p is hsa-miR-6785-5p, miR-7110-5p is hsa-miR-7110-5p, miR-6887-5p is hsa-miR-6887-5p, miR-887-3p is hsa-miR-887-3p, miR-1228-5p is hsa-miR-1228-5p, miR-5572 is hsa-miR-5572, miR-6782-5p is hsa-miR-6782-5p, miR-4298 is hsa-miR-4298, miR-6786-5p is hsa-miR-6786-5p, miR-5010-5p is hsa-miR-5010-5p, miR-6087 is hsa-miR-6087, miR-6765-5p is hsa-miR-6765-5p, miR-6732-5p is hsa-miR-6732-5p, miR-6787-5p is hsa-miR-6787-5p, miR-6737-5p is hsa-miR-6737-5p, miR-128-2-5p is hsa-miR-128-2-5p, miR-4270 is hsa-miR-4270, miR-6861-5p is hsa-miR-6861-5p, miR-6756-5p is hsa-miR-6756-5p, miR-1229-5p is hsa-miR-1229-5p, miR-6891-5p is hsa-miR-6891-5p, miR-6848-5p is hsa-miR-6848-5p, miR-1237-5p is hsa-miR-1237-5p, miR-30c-1-3p is hsa-miR-30c-1-3p, miR-1233-5p is hsa-miR-1233-5p, miR-211-3p is hsa-miR-211-3p, miR-4758-5p is hsa-miR-4758-5p, miR-614 is hsa-miR-614, miR-6746-5p is hsa-miR-6746-5p, miR-1915-5p is hsa-miR-1915-5p, miR-4688 is hsa-miR-4688, miR-3917 is hsa-miR-3917, miR-5787 is hsa-miR-5787, miR-4632-5p is hsa-miR-4632-5p, miR-6126 is hsa-miR-6126, miR-135a-3p is hsa-miR-135a-3p, miR-8063 is hsa-miR-8063, miR-5698 is hsa-miR-5698, miR-6089 is hsa-miR-6089, miR-498 is hsa-miR-498, miR-296-3p is hsa-miR-296-3p, miR-4419b is hsa-miR-4419b, miR-6802-5p is hsa-miR-6802-5p, miR-6829-5p is hsa-miR-6829-5p, miR-6803-5p is hsa-miR-6803-5p, miR-1199-5p is hsa-miR-1199-5p, miR-6840-3p is hsa-miR-6840-3p, miR-6752-5p is hsa-miR-6752-5p, miR-6798-5p is hsa-miR-6798-5p, miR-6131 is hsa-miR-6131, miR-4667-5p is hsa-miR-4667-5p, miR-6510-5p is hsa-miR-6510-5p, miR-4690-5p is hsa-miR-4690-5p, miR-920 is hsa-miR-920, miR-23b-3p is hsa-miR-23b-3p, miR-4448 is hsa-miR-4448, miR-2110 is hsa-miR-2110, miR-4706 is hsa-miR-4706, miR-7845-5p is hsa-miR-7845-5p, miR-6808-5p is hsa-miR-6808-5p, miR-4447 is hsa-miR-4447, miR-6869-5p is hsa-miR-6869-5p, miR-6794-5p is hsa-miR-6794-5p, miR-6511a-5p is hsa-miR-6511a-5p, miR-6824-5p is hsa-miR-6824-5p, miR-6766-3p is hsa-miR-6766-3p, miR-6511a-5p is hsa-miR-6511a-5p, and miR-6749-5p is hsa-miR-6749-5p.

In the method of the present invention, specifically, the nucleic acid(s) (specifically, probe(s) or primer(s)) is selected from the group consisting of the following polynucleotides (a) to (e):
(a) a polynucleotide consisting of a nucleotide sequence represented by any of SEQ ID NOs: 1 to 83, 227 to 229, 246, 248, and 250 or a nucleotide sequence derived from the nucleotide sequence by the replacement of u with t, a variant thereof, a derivative thereof, or a fragment thereof comprising 15 or more consecutive nucleotides;
(b) a polynucleotide comprising a nucleotide sequence represented by any of SEQ ID NOs: 1 to 83, 227 to 229, 246, 248, and 250;
(c) a polynucleotide consisting of a nucleotide sequence complementary to a nucleotide sequence represented by any of SEQ ID NOs: 1 to 83, 227 to 229, 246, 248, and 250 or a nucleotide sequence derived from the nucleotide sequence by the replacement of u with t, a variant thereof, a derivative thereof, or a fragment thereof comprising 15 or more consecutive nucleotides;
(d) a polynucleotide comprising a nucleotide sequence complementary to a nucleotide sequence represented by any of SEQ ID NOs: 1 to 83, 227 to 229, 246, 248, and 250 or a nucleotide sequence derived from the nucleotide sequence by the replacement of u with t; and
(e) a polynucleotide hybridizing under stringent conditions to any of the polynucleotides (a) to (d).

The nucleic acid(s) used in the method of the present invention can further comprise a nucleic acid(s) capable of specifically binding to at least one (one or more) polynucleotides selected from the following miRNAs: miR-1908-5p, miR-6729-5p, miR-5195-3p, miR-638, miR-6125, miR-3178, miR-3196, miR-8069, miR-4723-5p, miR-4746-3p, miR-4689, miR-6816-5p, miR-6757-5p, miR-7109-5p, miR-6724-5p, miR-1225-3p, miR-6875-5p, miR-7108-5p, miR-4508, miR-6085, miR-6779-5p, miR-642a-3p, miR-4695-5p, miR-7847-3p, miR-3197, miR-6769b-5p, miR-7641, miR-187-5p, miR-3185, miR-2861, miR-3940-5p, miR-1203, miR-615-5p, miR-4787-5p, miR-1343-3p, miR-6813-5p, miR-1225-5p, miR-602, miR-4488, miR-125a-3p, miR-5100, miR-4294, miR-1231, miR-6765-3p, miR-4442, miR-718, miR-6780b-5p, miR-6090, miR-6845-5p, miR-4741, miR-4467, miR-4707-5p, miR-4271, miR-4673, miR-3184-5p, miR-1469, miR-4640-5p, miR-663a, miR-6791-5p, miR-6826-5p, miR-4433b-3p, miR-1915-3p, miR-4417, miR-4449, miR-4707-3p, miR-3180-3p, miR-5585-3p, miR-1268a, miR-8072, miR-296-5p, miR-204-3p, miR-4454, miR-6722-3p, miR-1290, miR-3622a-5p, miR-939-5p, miR-675-5p, miR-3131, miR-4648, miR-1268b, miR-6741-5p, miR-6893-5p, miR-3162-5p, miR-642b-3p, miR-4734, miR-150-3p, miR-8089, miR-6805-3p, miR-7113-3p, miR-6850-5p, miR-6799-5p, miR-6768-5p, miR-92b-5p, miR-3679-5p, miR-4792, miR-3656, miR-92a-2-5p, miR-4466, miR-4513, miR-6781-5p, miR-4649-5p, miR-6775-5p, miR-4651, miR-3195, miR-6726-5p, miR-6872-3p, miR-371a-5p, miR-6777-5p, miR-6789-5p, miR-7975, miR-6821-5p, miR-4534, miR-619-5p, miR-7107-5p, miR-1228-3p, miR-6774-5p, miR-6805-5p, miR-23a-3p, miR-4665-5p, miR-4505, miR-4638-5p, miR-24-3p, miR-3135b, miR-4745-5p, miR-128-1-5p, miR-4476, miR-4687-3p, miR-3665, miR-6806-5p, miR-3937, miR-711, miR-3141, miR-3188, miR-4281, miR-5196-5p, miR-6880-5p, miR-3960, miR-3648, miR-6721-5p, miR-4492, miR-744-5p, miR-7704, miR-4749-5p, miR-762, miR-6836-3p, miR-6727-5p, miR-4739, miR-7977, miR-4484, miR-6515-3p, miR-373-5p, miR-4258, miR-4674, miR-3180, miR-6076, miR-1238-5p, miR-4463, miR-4486, miR-4730, miR-4286, and miR-4739.

Specifically, miR-1908-5p is hsa-miR-1908-5p, miR-6729-5p is hsa-miR-6729-5p, miR-5195-3p is hsa-miR-5195-3p, miR-638 is hsa-miR-638, miR-6125 is hsa-miR-6125, miR-3178 is hsa-miR-3178, miR-3196 is hsa-miR-3196, miR-8069 is hsa-miR-8069, miR-4723-5p is hsa-miR-4723-5p, miR-4746-3p is hsa-miR-4746-3p, miR-4689 is hsa-miR-4689, miR-6816-5p is hsa-miR-6816-5p, miR-6757-5p is hsa-miR-6757-5p, miR-7109-5p is hsa-miR-7109-5p, miR-6724-5p is hsa-miR-6724-5p, miR-1225-3p is hsa-miR-1225-3p, miR-6875-5p is hsa-miR-6875-5p, miR-7108-5p is hsa-miR-7108-5p, miR-4508 is hsa-miR-4508, miR-6085 is hsa-miR-6085, miR-6779-5p is hsa-miR-6779-5p, miR-642a-3p is hsa-miR-642a-3p, miR-4695-5p is hsa-miR-4695-5p, miR-7847-3p is hsa-miR-7847-3p, miR-3197 is hsa-miR-3197, miR-6769b-5p is hsa-miR-6769b-5p, miR-7641 is hsa-miR-7641, miR-187-5p is hsa-miR-187-5p, miR-3185 is hsa-miR-3185, miR-2861 is hsa-miR-2861, miR-3940-5p is hsa-miR-3940-5p, miR-1203 is hsa-miR-1203, miR-615-5p is hsa-miR-615-5p, miR-4787-5p is hsa-miR-4787-5p, miR-1343-3p is hsa-miR-1343-3p, miR-6813-5p is hsa-miR-6813-5p, miR-1225-5p is hsa-miR-1225-5p, miR-602 is hsa-miR-602, miR-4488 is hsa-miR-4488, miR-125a-3p is hsa-miR-125a-3p, miR-5100 is hsa-miR-5100, miR-4294 is hsa-miR-4294, miR-1231 is hsa-miR-1231, miR-6765-3p is hsa-miR-6765-3p, miR-4442 is hsa-miR-4442, miR-718 is hsa-miR-718, miR-6780b-5p is hsa-miR-6780b-5p, miR-6090 is hsa-miR-6090, miR-6845-5p is hsa-miR-6845-5p, miR-4741 is hsa-miR-4741, miR-4467 is hsa-miR-4467, miR-4707-5p is hsa-miR-4707-5p, miR-4271 is hsa-miR-4271, miR-4673 is hsa-miR-4673, miR-3184-5p is hsa-miR-3184-5p, miR-1469 is hsa-miR-1469, miR-4640-5p is hsa-miR-4640-5p, miR-663a is hsa-miR-663a, miR-6791-5p is hsa-miR-6791-5p, miR-6826-5p is hsa-miR-6826-5p, miR-4433b-3p is hsa-miR-4433b-3p, miR-1915-3p is hsa-miR-1915-3p, miR-4417 is hsa-miR-4417, miR-4449 is hsa-miR-4449, miR-4707-3p is hsa-miR-4707-3p, miR-3180-3p is hsa-miR-3180-3p, miR-5585-3p is hsa-miR-5585-3p, miR-1268a is hsa-miR-1268a, miR-8072 is hsa-miR-8072, miR-296-5p is hsa-miR-296-5p, miR-204-3p is hsa-miR-204-3p, miR-4454 is hsa-miR-4454, miR-6722-3p is hsa-miR-6722-3p, miR-1290 is hsa-miR-1290, miR-3622a-5p is hsa-miR-3622a-5p, miR-939-5p is hsa-miR-939-5p, miR-675-5p is hsa-miR-675-5p, miR-3131 is hsa-miR-3131, miR-4648 is hsa-miR-4648, miR-1268b is hsa-miR-1268b, miR-6741-5p is hsa-miR-6741-5p, miR-6893-5p is hsa-miR-6893-5p, miR-3162-5p is hsa-miR-3162-5p, miR-642b-3p is hsa-miR-642b-3p, miR-4734 is hsa-miR-4734, miR-150-3p is hsa-miR-150-3p, miR-8089 is hsa-miR-8089, miR-6805-3p is hsa-miR-6805-3p, miR-7113-3p is hsa-miR-7113-3p, miR-6850-5p is hsa-miR-6850-5p, miR-6799-5p is hsa-miR-6799-5p, miR-6768-5p is hsa-miR-6768-5p, miR-92b-5p is hsa-miR-92b-5p, miR-3679-5p is hsa-miR-3679-5p, miR-4792 is hsa-miR-4792, miR-3656 is hsa-miR-3656, miR-92a-2-5p is hsa-miR-92a-2-5p, miR-4466 is hsa-miR-4466, miR-4513 is hsa-miR-4513, miR-6781-5p is hsa-miR-6781-5p, miR-4649-5p is hsa-miR-4649-5p, miR-6775-5p is hsa-miR-6775-5p, miR-4651 is hsa-miR-4651, miR-3195 is hsa-miR-3195, miR-6726-5p is hsa-miR-6726-5p, miR-6872-3p is hsa-miR-6872-3p, miR-371a-5p is hsa-miR-371a-5p, miR-6777-5p is hsa-miR-6777-5p, miR-6789-5p is hsa-miR-6789-5p, miR-7975 is hsa-miR-7975, miR-6821-5p is hsa-miR-6821-5p, miR-4534 is hsa-miR-4534, miR-619-5p is hsa-miR-619-5p, miR-7107-5p is hsa-miR-7107-5p, miR-1228-3p is hsa-miR-1228-3p, miR-6774-5p is hsa-miR-6774-5p, miR-6805-5p is hsa-miR-6805-5p, miR-23a-3p is hsa-miR-23a-3p, miR-4665-5p is hsa-miR-4665-5p, miR-4505 is hsa-miR-4505, miR-4638-5p is hsa-miR-4638-5p, miR-24-3p is hsa-miR-24-3p, miR-3135b is hsa-miR-3135b, miR-4745-5p is hsa-miR-4745-5p, miR-128-1-5p is hsa-miR-128-1-5p, miR-4476 is hsa-miR-4476, miR-4687-3p is hsa-miR-4687-3p, miR-3665 is hsa-miR-3665, miR-6806-5p is hsa-miR-6806-5p, miR-3937 is hsa-miR-3937, miR-711 is hsa-miR-711, miR-3141 is hsa-miR-3141, miR-3188 is hsa-miR-3188, miR-4281 is hsa-miR-4281, miR-5196-5p is hsa-miR-5196-5p, miR-6880-5p is hsa-miR-6880-5p, miR-3960 is hsa-miR-3960, miR-3648 is hsa-miR-3648, miR-6721-5p is hsa-miR-6721-5p, miR-4492 is hsa-miR-4492, miR-744-5p is hsa-miR-744-5p, miR-7704 is hsa-miR-7704, miR-4749-5p is hsa-miR-4749-5p, miR-762 is hsa-miR-762, miR-6836-3p is hsa-miR-6836-3p, miR-6727-5p is hsa-miR-6727-5p, miR-4739 is hsa-miR-4739, miR-7977 is hsa-miR-7977, miR-4484 is hsa-miR-4484, miR-6515-3p is hsa-miR-6515-3p, miR-373-5p is hsa-miR-373-5p, miR-4258 is hsa-miR-4258, miR-4674 is hsa-miR-4674, miR-3180 is hsa-miR-3180, miR-6076 is hsa-miR-6076, miR-1238-5p is hsa-miR-1238-5p, miR-4463 is hsa-miR-4463, miR-4486 is hsa-miR-4486, miR-4730 is hsa-miR-4730, miR-4286 is hsa-miR-4286, and miR-4739 is hsa-miR-4739.

Specifically, the nucleic acid(s) is further selected from the group consisting of the following polynucleotides (f) to (j):
(f) a polynucleotide consisting of a nucleotide sequence represented by any of SEQ ID NOs: 84 to 226, 230 to 245, 247, and 249 or a nucleotide sequence derived from the nucleotide sequence by the replacement of u with t, a variant thereof, a derivative thereof, or a fragment thereof comprising 15 or more consecutive nucleotides;
(g) a polynucleotide comprising a nucleotide sequence represented by any of SEQ ID NOs: 84 to 226, 230 to 245, 247, and 249;
(h) a polynucleotide consisting of a nucleotide sequence complementary to a nucleotide sequence represented by any of SEQ ID NOs: 84 to 226, 230 to 245, 247, and 249 or a nucleotide sequence derived from the nucleotide sequence by the replacement of u with t, a variant thereof, a derivative thereof, or a fragment thereof comprising 15 or more consecutive nucleotides;
(i) a polynucleotide comprising a nucleotide sequence complementary to a nucleotide sequence represented by any of SEQ ID NOs: 84 to 226, 230 to 245, 247, and 249 or a nucleotide sequence derived from the nucleotide sequence by the replacement of u with t; and
(j) a polynucleotide hybridizing under stringent conditions to any of the polynucleotides (f) to (i).

Examples of the sample used in the method of the present invention can include samples prepared from living tissues (preferably pancreatic tissues) or body fluids such as blood, serum, plasma, and urine from subjects. Specifically, for example, an RNA-containing sample prepared from the tissue, a polynucleotide-containing sample further prepared therefrom, a body fluid such as blood, serum, plasma, or urine, a portion or the whole of a living tissue collected from the subject by biopsy or the like, or a living tissue excised by surgery can be used, and the sample for measurement can be prepared therefrom.

The "subject" used herein refers to a mammal, for example, a primate such as a human or a monkey, a rodent such as a mouse or a rat, a pet animal such as a dog or a cat, an athletic animal such as a horse, and an animal that is kept in a zoo without any limitation, and is preferably a human.

The steps of the method of the present invention can be changed according to the type of the sample to be assayed.

In the case of using RNA as an analyte, the detection of early pancreatic cancer or a pancreatic cancer precursor lesion (cells) in a subject can comprise, for example, the following steps (a), (b), and (c):
(a) a step of binding RNA prepared from a sample from the subject or complementary polynucleotides (cDNAs) transcribed from the RNA to a polynucleotide(s) in the kit or the device of the present invention;
(b) a step of quantitatively or qualitatively measuring the sample-derived RNA or the cDNAs synthesized from the RNA, which is/are bound to the polynucleotide(s), by hybridization using the polynucleotide(s) as a nucleic acid probe(s), by use of a sequencer for polynucleotide sequencing, or by quantitative RT-PCR using the polynucleotide(s) as a primer(s); and
(c) a step of evaluating *in vitro* whether or not the subject has early pancreatic cancer or pancreatic cancer precursor lesion through comparison with a control on the basis of the measurement results of the step (b), to detect the presence or absence of early pancreatic cancer or a pancreatic cancer precursor lesion (or early pancreatic cancer- or pancreatic cancer precursor lesion-derived gene expression) in the subject.

For example, various hybridization methods can be used for detecting, examining, evaluating, or diagnosing early pancreatic cancer or a pancreatic cancer precursor lesion (or pancreatic cancer-derived gene expression) *in vitro* according to the present invention. For example, Northern blot, Southern blot, RT-PCR, DNA chip analysis, *in situ* hybridization, Northern hybridization, Southern hybridization, or a polynucleotide sequencing technique using a sequencer or the like can be used as such a hybridization method.

In the case of using the Northern blot, the presence or absence of expression of each gene or the expression level thereof in the RNA can be detected or measured by use of the nucleic acid probe(s) that can be used in the present invention. Specific examples thereof can include a method which comprises labeling the nucleic acid probe (or a complementary strand) with a radioisotope (³²P, ³³P, ³⁵S, etc.), a fluorescent material, or the like, hybridizing the labeled product with the living tissue-derived RNA from a subject, which is transferred to a nylon membrane or the like according to a routine method, and then detecting and measuring a signal derived from the label (radioisotope or fluorescent material) on the formed DNA/RNA duplex using a radiation detector (examples thereof can include BAS-1800 II (Fujifilm Corp., Japan)) or a fluorescence detector (examples thereof can include STORM 865 (GE Healthcare Japan Corp.)).

In the case of using the quantitative RT-PCR, the presence or absence of expression of each gene or the expression level thereof in the RNA can be detected or measured by use of the primer that can be used in the present invention. Specific examples thereof can include a method which comprises preparing cDNAs from the living tissue-derived RNA of a subject according to a routine method, hybridizing a pair of primers (consisting of a plus strand and a reverse strand binding to the cDNA) prepared from the composition for detection of the present invention with the cDNA and performing PCR according to a routine method such that the region of each target gene can be amplified with the cDNA as a template, and thereby detecting the obtained double-stranded DNA. The method for detecting the double-stranded DNA can include a method of performing the PCR using the primers labeled in advance with a radioisotope or a fluorescent material, a method of electrophoresing the PCR product on an agarose gel and staining the double-stranded DNA with ethidium bromide or the like for detection, and a method of transferring the produced double-stranded DNA to a nylon membrane or the like according to a routine method and hybridizing the double-stranded DNA to a labeled nucleic acid probe for detection.

In the case of using the sequencer, the presence or absence of expression of each gene or the expression level thereof in the RNA can be detected or measured from the number of reads by use of the primer that can be used in the present invention. Specific examples thereof can include a method which comprises preparing cDNAs from the living tissue-derived RNA of a subject according to a routine method, hybridizing a pair of primers (consisting of a plus strand and a reverse strand binding to the cDNA) prepared from the composition for detection of the present invention with the cDNA and performing PCR according to a routine method such that the region of each target gene can be amplified with the cDNA as a template, and detecting or measuring the amplified DNA using a sequencer such as HiSeq 2500 (Illumina, Inc.) or Ion Proton^{(R)} System (Thermo Fisher Scientific Inc.). Another specific example thereof can include a method which comprises detecting or measuring the living tissue-derived RNA of a subject using PacBio RS II (Pacific Biosciences of California, Inc.) without PCR amplification.

In the case of using the nucleic acid array technique (or analysis), an RNA chip or a DNA chip on which the composition for detection of the present invention is attached as nucleic acid probes (single-stranded or double-stranded) to a substrate (solid phase) is used. Regions having the attached nucleic acid probes are referred to as probe spots, and regions having no attached nucleic acid probe are referred to as blank spots. A substrate on which a group of genes are immobilized is generally called a nucleic acid chip, a nucleic acid array, a microarray, or the like. The DNA or RNA array includes a DNA or RNA macroarray and a DNA or RNA microarray. In the present specification, the term "chip" includes these arrays. 3D-Gene^{(R)} Human miRNA Oligo chip (Toray Industries, Inc.) can be used as the DNA chip, though the DNA chip is not limited thereto.

Examples of the measurement using the DNA chip can include, but are not limited to, a method of detecting and measuring a signal derived from the label of the composition for detection using an image detector (examples thereof can include Typhoon 9410 (GE Healthcare) and 3D-Gene^{(R)} scanner (Toray Industries, Inc.)).

The "stringent conditions" used herein are, as mentioned above, conditions under which a nucleic acid probe hybridizes to its target sequence to a detectably larger extent (e.g., a measurement value equal to or larger than "(a mean of background measurement values) + (a standard error of the background measurement values) × 2") than that for other sequences.

The stringent conditions are defined by hybridization and subsequent washing. Examples of the hybridization conditions include, but not limited to, 30°C to 60°C for 1 to 24 hours in a solution containing SSC, a surfactant, formamide, dextran sulfate, a blocking agent(s), etc. In this context, 1 × SSC is an aqueous solution (pH 7.0) containing 150 mM sodium chloride and 15 mM sodium citrate. The surfactant includes, for example, SDS (sodium dodecyl sulfate), Triton, or Tween. The hybridization conditions more preferably comprise 3-10 × SSC and 0.1-1% SDS. Furthermore, examples of the conditions for the washing, following the hybridization, which is another condition to define the stringent conditions, can include conditions comprising sequential washing at 30°C in a solution containing 0.5 × SSC and 0.1% SDS, at 30°C in a solution containing 0.2 × SSC and 0.1% SDS, and at 30°C in a 0.05 × SSC solution. It is desirable that the complementary strand should maintain its hybridized state with a target plus strand even during washing under such conditions. Specifically, examples of such a complementary strand can include a strand consisting of a nucleotide sequence in a completely complementary relationship with the nucleotide sequence of the target plus strand, and a strand consisting of a nucleotide sequence having at least 80%, preferably at least 85%, more preferably at least 90% or at least 95%, for example, at least 98% or at least 99% identity to the strand.

Other examples of the "stringent conditions" for the hybridization are described in, for example, Sambrook, J. & Russel, D., Molecular Cloning, A LABORATORY MANUAL, Cold Spring Harbor Laboratory Press, published on January 15, 2001, Vol. 1, 7.42 to 7.45 and Vol. 2, 8.9 to 8.17, and can be used in the present invention.

Examples of the conditions for carrying out PCR using polynucleotide fragments in the kit of the present invention as primers include treatment for approximately 15 seconds to 1 minute at a Tm value + 5-10°C, wherein the Tm value is calculated from the sequences of the primers, using a PCR buffer having composition such as 10 mM Tris-HCL (pH 8.3), 50 mM KCL, and 1 to 2 mM MgCl₂. Examples of the method for calculating such a Tm value include Tm value = 2 × (the number of adenine residues + the number of thymine residues) + 4 × (the number of guanine residues + the number of cytosine residues).

In the case of using the quantitative RT-PCR, a commercially available kit for measurement specially designed for quantitatively measuring miRNA, such as TaqMan^{(R)} MicroRNA Assays (Life Technologies Corp.), LNA^{(R)}-based MicroRNA PCR (Exiqon), or Ncode^{(R)} miRNA qRT-PCT kit (Invitrogen Corp.) may be used.

For the calculation of gene expression levels, statistical processing described in, for example, Statistical analysis of gene expression microarray data (Speed T., Chapman and Hall/CRC), and A beginner's guide Microarray gene expression data analysis (Causton H.C. et al., Blackwell publishing) can be used in the present invention, though the calculation method is not limited thereto. For example, twice, preferably 3 times, more preferably 6 times the standard deviation of the measurement values of the blank spots are added to the average measurement value of the blank spots on the DNA chip, and probe spots having a signal value equal to or larger than the resulting value can be defined as detection spots. Furthermore, the average measurement value of the blank spots can be considered as a background and can be subtracted from the measurement values of the probe spots to determine the resulting value as gene expression levels. A missing value for a gene expression level can be excluded from the analyte, preferably replaced with the smallest value of the gene expression level in each DNA chip, or more preferably replaced with a value obtained by subtracting 0.1 from a logarithmic value of the smallest value of the gene expression level. In order to exclude low-signal genes, only a gene having a gene expression level of 2⁶, preferably 2⁸, more preferably 2¹⁰ or larger in 20% or more, preferably 50% or more, more preferably 80% or more of the number of measurement samples can be selected as the analyte. Examples of the normalization of the gene expression level include, but are not limited to, global normalization and quantile normalization (Bolstad, B. M. et al., 2003, Bioinformatics, Vol. 19, p. 185-193).

The present invention also provides a method for detecting (or assisting in the detection of) early pancreatic cancer or a pancreatic cancer precursor lesion in a subject, comprising measuring expression levels of target genes in a sample from the subject using the polynucleotides, the kit, or the device (e.g., chip) for diagnosis of the present invention, or a combination thereof, and assigning the expression levels of the target genes in the sample from the subject to a discriminant (discriminant function) to evaluate the presence or absence of early pancreatic cancer or a pancreatic cancer precursor lesion, wherein the discriminant is prepared with gene expression levels in a sample from a subject (or a patient) known to have early pancreatic cancer or a pancreatic cancer precursor lesion and gene expression levels in a sample from a healthy subject as supervising samples and is capable of discriminating an early pancreatic cancer or pancreatic cancer precursor lesion patient from a healthy subject.

The present invention further provides the method comprising: a first step of measuring *in vitro* expression levels of target genes in a plurality of samples from subjects known to have early pancreatic cancer or a pancreatic cancer precursor lesion and/or to have neither early pancreatic cancer nor a pancreatic cancer precursor lesion, using the polynucleotides, the kit, or the device (e.g., chip) for diagnosis of the present invention, or a combination thereof; a second step of preparing a discriminant with the measurement values of the expression levels of the target genes obtained in the first step as supervising samples; a third step of measuring *in vitro* expression levels of the target genes in a sample derived from a subject in the same way as in the first step; and a fourth step of assigning the measurement values of the expression levels of the target genes obtained in the third step to the discriminant obtained in the second step, and determining or evaluating the presence or absence of early pancreatic cancer or a pancreatic cancer precursor lesion in the subject on the basis of the results obtained from the discriminant, wherein the target genes can be detected using the polynucleotides, the polynucleotides contained in the kit or the device (e.g., chip), variants thereof, or fragments thereof.

As used herein, the discriminant can be prepared by use of any discriminant analysis method that can prepare a discriminant for discriminating an early pancreatic cancer or pancreatic cancer precursor lesion patient from a healthy subject, for example, Fisher's discriminant analysis, nonlinear discriminant analysis based on Mahalanobis' distance, neural network, or Support Vector Machine (SVM), though the method is not limited thereto.

When a clustering boundary is a straight line or a hyperplane, the linear discriminant analysis is a method for determining the belonging of a cluster using Formula 1 as a discriminant. In Formula 1, x represents an explanatory variable, w represents a coefficient of the explanatory variable, and w₀ represents a constant term. $f \left(x\right) = {w}_{0} + {\sum }_{i = 1}^{n} {w}_{i} {x}_{i}$ Formula 1

Values obtained from the discriminant are referred to as discriminant scores. The measurement values of a newly offered dataset can be assigned as explanatory variables to the discriminant to determine clusters by the signs (+ or -) of the discriminant scores.

The Fisher's discriminant analysis, one type of linear discriminant analysis, is a dimensionality reduction method for selecting a dimension suitable for discriminating classes, and constructs a highly discriminating synthetic variable by focusing on the variance of the synthetic variables and minimizing the variance of data having the same label (Venables, W.N. et al., Modern Applied Statistics with S. Fourth edition. Springer, 2002). In the Fisher's discriminant analysis, projection direction w is determined so as to maximize Formula 2. In this formula, µ represents an average input, n_{g} represents the number of data belonging to class g, and µ_{g} represents an average input of the data belonging to class g. The numerator and the denominator are the interclass variance and the intraclass variance, respectively, when each data is projected in the direction of the vector w. Discriminant coefficient wᵢ is determined by maximizing this ratio (Takafumi Kanamori et al., "Pattern Recognition", Kyoritsu Shuppan Co., Ltd., (Tokyo, Japan) (2009); and Richard O. et al., Pattern Classification Second Edition., Wiley-Interscience, 2000). $J \left(w\right) = \frac{{\sum }_{g = 1}^{G} {n}_{g} \left({w}^{T}{μ}_{g}-{w}^{T}μ\right) {\left({w}^{T}{μ}_{g}-{w}^{T}μ\right)}^{T}}{{\sum }_{g = 1}^{G} {\sum }_{i : {y}_{i} = g} \left({w}^{T}{x}_{i}-{w}^{T}{μ}_{g}\right) \left({w}^{T}{x}_{i}-{w}^{T}{μ}_{g}\right)}$ Formula 2 *subject to* $μ = {\sum }_{i = 1}^{n} \frac{{x}_{i}}{n} , {μ}_{g} = {\sum }_{i : {u}_{i} = g}^{n} \frac{{x}_{i}}{{n}_{g}}$

The Mahalanobis' distance is calculated according to Formula 3 in consideration of data correlation and can be used as nonlinear discriminant analysis for determining a cluster to which a data point belongs, based on the smallest Mahalanobis' distance between the data point and each cluster. In Formula 3, µ represents a central vector of each cluster, and S⁻¹ represents an inverse matrix of the variance-covariance matrix of the cluster. The central vector is calculated from explanatory variable x, and an average vector, a median value vector, or the like can be used. $D \left(x, μ\right) = {\left\{{\left(x-μ\right)}^{t}{S}^{- 1}\left(x-μ\right)\right\}}^{\frac{1}{2}}$ Formula 3

SVM is a discriminant analysis method devised by V. Vapnik (The Nature of Statistical Leaning Theory, Springer, 1995). Particular data points of a dataset having known classes are defined as explanatory variables, and classes are defined as objective variables. A boundary plane called hyperplane for correctly classifying the dataset into the known classes is determined, and a discriminant for data classification is determined using the boundary plane. Then, the measurement values of a newly offered dataset can be assigned as explanatory variables to the discriminant to determine classes. In this respect, the result of the discriminant analysis may be classes, may be a probability of being classified into correct classes, or may be the distance from the hyperplane. In SVM, a method of nonlinearly converting a feature vector to a high dimension and performing linear discriminant analysis in the space is known as a method for tackling nonlinear problems. An expression in which an inner product of two factors in a nonlinearly mapped space is expressed only by inputs in their original spaces is called kernel. Examples of the kernel can include a linear kernel, a RBF (Radial Basis Function) kernel, and a Gaussian kernel. While highly dimensional mapping is performed according to the kernel, the optimum discriminant, i.e., a discriminant, can be actually constructed by mere calculation according to the kernel, which avoids calculating features in the mapped space (e.g., Hideki Aso et al., Frontier of Statistical Science 6 "Statistics of pattern recognition and learning - New concepts and approaches", Iwanami Shoten, Publishers, (Tokyo, Japan) (2004); Nello Cristianini et al., Introduction to SVM, Kyoritsu Shuppan Co., Ltd., (Tokyo, Japan) (2008)).

C-support vector classification (C-SVC), one type of SVM, comprises preparing a hyperplane by supervision according to a dataset with the explanatory variables of two groups and classifying an unknown dataset into either of the groups (C. Cortes et al., 1995, Machine Learning, Vol. 20, p. 273-297).

Exemplary calculation of the C-SVC discriminant that can be used in the method of the present invention will be given below. First, all subjects are divided into two groups, i.e., an early pancreatic cancer or pancreatic cancer precursor lesion patient group and a healthy subject group. For example, pancreatic tissue examination can be used for a reference under which each subject is confirmed either as an early pancreatic cancer or pancreatic cancer precursor lesion patient or as a healthy subject.

Next, a dataset consisting of comprehensive gene expression levels of serum-derived samples of the two divided groups (hereinafter, this dataset is referred to as a training cohort) is prepared, and a C-SVC discriminant is determined by using genes found to differ clearly in their gene expression levels between the two groups as explanatory variables and this grouping as objective variables (e.g., -1 and +1). An optimizing objective function is represented by Formula 4 wherein e represents all input vectors, y represents an objective variable, a represents a Lagrange multiplier vector, Q represents a positive definite matrix, and C represents a parameter for adjusting constrained conditions. ${min}_{a} \frac{1}{2} {a}^{T} Qa - {e}^{T} a$ Formula 4 *subject to y^{T}a* = 0, 0 ≤ *aᵢ* ≤ *C*, *i* = 1,..., *l*,

Formula 5 is a finally obtained discriminant, and a group to which the data point belongs to can be determined on the basis of the sign of a value obtained according to the discriminant. In this formula, x represents a support vector, y represents a label indicating the belonging of a group, a represents the corresponding coefficient, b represents a constant term, and K represents a kernel function. $f \left(x\right) = sgn \left({\sum }_{i = 1}^{l} {y}_{i} {a}_{i} K \left({x}_{i}, x\right) + b\right)$ Formula 5

For example, a RBF kernel defined by Formula 6 can be used as the kernel function. In this formula, x represents a support vector, and γ represents a kernel parameter for adjusting the complexity of the hyperplane. $K \left({x}_{i}, {x}_{j}\right) = exp \left(-r{\left‖{x}_{i}-{x}_{j}\right‖}^{2}\right) , r < 0$ Formula 6

In addition, an approach such as neural network, k-nearest neighbor algorithms, decision trees, or logistic regression analysis can be selected as a method for determining or evaluating the presence and/or absence of an early pancreatic cancer or pancreatic cancer precursor lesion in a subject, or for evaluating the expression level thereof by comparison with a control derived from a healthy subject.

The method of the present invention can comprise, for example, the following steps (a), (b), and (c):
(a) a step of measuring an expression level(s) of a target gene(s) in samples already known to be derived from early pancreatic cancer or pancreatic cancer precursor lesion patients and to be derived from healthy subjects or subjects having neither early pancreatic cancer nor a pancreatic cancer precursor lesion, using the polynucleotide(s), the kit, or the device (e.g., DNA chip) for detection according to the present invention;
(b) a step of preparing the discriminants of Formulas 1 to 3, 5, and 6 described above from the measurement values of the expression level measured in the step (a); and
(c) a step of measuring an expression level(s) of the target gene(s) in a sample derived from a subject using the polynucleotide(s), the kit, or the device (e.g., DNA chip) for diagnosis (detection) according to the present invention, assigning the obtained measurement value(s) to the discriminants prepared in the step (b), and determining or evaluating the presence or absence of early pancreatic cancer or a pancreatic cancer precursor lesion in the subject, or evaluating early pancreatic cancer- or pancreatic cancer precursor lesion-derived expression levels by comparison with a healthy subject-derived control, on the basis of the obtained results.

In this context, in the discriminants of Formulas 1 to 3, 5, and 6, x represents an explanatory variable and includes a value obtained by measuring a polynucleotide(s) selected from the polynucleotides or the like described in Section 2 above, or any fragment thereof. Specifically, the explanatory variable for discriminating an early pancreatic cancer or pancreatic cancer precursor lesion patient from a healthy subject according to the present invention is a gene expression level(s) selected from, for example, the following expression levels (1) to (2):
(1) a gene expression level(s) in the serum of an early pancreatic cancer or pancreatic cancer precursor lesion patient and a healthy subject measured by any of RNAs or DNAs comprising 15 or more consecutive nucleotides in a nucleotide sequence represented by any of SEQ ID NOs: 1 to 83, 227 to 229, 246, 248, and 250 or a complementary sequence thereof, or nucleotides derived from the nucleotides by the replacement of u with t;
(2) a gene expression level(s) in the serum of an early pancreatic cancer or pancreatic cancer precursor lesion patient and a healthy subject measured by any of RNAs or DNAs comprising 15 or more consecutive nucleotides in a nucleotide sequence represented by any of SEQ ID NOs: 84 to 226, 230 to 245, 247, and 249 or a complementary sequence thereof, or nucleotides derived from the nucleotides by the replacement of u with t; and

As described above, for the method for determining or evaluating whether or not a subject has early pancreatic cancer or a pancreatic cancer precursor lesion as to a sample derived from the subject, the preparation of a discriminant requires a discriminant prepared from a training cohort. For enhancing the discrimination accuracyof the discriminant, it is necessary to use genes having clear difference in their expression level between two groups consisting of an early pancreatic cancer or pancreatic cancer precursor lesion patient group and a healthy subject group in the training cohort when preparing the discriminant.

Each gene that is used for an explanatory variable in a discriminant is preferably determined as follows. First, comprehensive gene expression levels of an early pancreatic cancer or pancreatic cancer precursor lesion patient group and comprehensive gene expression levels of a healthy subject group, both of which are in a training cohort, are used as a dataset, and the degree of difference in the expression level of each gene between the two groups is determined through the use of, for example, the P value of t test, which is parametric analysis, or the P value of Mann-Whitney's U test or Wilcoxon test, which is nonparametric analysis.

The degree of difference in the expression level can be considered as being statistically significant when the critical rate (significance level) as the P value obtained by the test is smaller than, for example, 5%, 1%, or 0.01%.

In order to correct an increased probability of type I error attributed to the repetition of a test, a method known in the art, for example, Bonferroni or Holm method, can be used for the correction (e.g., Yasushi Nagata et al., "Basics of statistical multiple comparison methods", Scientist Press Co., Ltd. (Tokyo, Japan) (2007)). As an example of the Bonferroni correction, for example, the P value obtained by a test is multiplied by the number of repetitions of the test, i.e., the number of genes used in the analysis, and the obtained value can be compared with a desired significance level to reduce a probability of causing type I error in the whole test.

Instead of the test, the absolute value (fold change) of an expression ratio of a median value of each gene expression level between gene expression levels of an early pancreatic cancer or pancreatic cancer precursor lesion patient group and gene expression levels of a healthy subject group may be calculated to select a gene that is used for an explanatory variable in a discriminant. Alternatively, ROC curves may be prepared using gene expression levels of an early pancreatic cancer or pancreatic cancer precursor lesion patient group and a healthy subject group, and a gene that is used for an explanatory variable in a discriminant can be selected on the basis of an AUROC value.

Next, a discriminant that can be calculated by various methods described above is prepared using any number of genes having large difference in their gene expression levels determined here. Examples of the method for constructing a discriminant that produces the largest discrimination accuracy include a method of constructing a discriminant in every combination of genes that satisfy the significance level for P value, and a method of repetitively evaluating the genes for use in the preparation of a discriminant while increasing the number of genes one by one in a descending order of difference in gene expression level (Furey TS. et al., 2000, Bioinformatics., Vol. 16, p. 906-14). A gene expression level of another independent early pancreatic cancer or pancreatic cancer precursor lesion patient or healthy subject is assigned as an explanatory variable to this discriminant to calculate discrimination results of the group to which this independent early pancreatic cancer or pancreatic cancer precursor lesion patient or healthy subject belongs. Specifically, the identified gene set for diagnosis and the discriminant constructed using the gene set for diagnosis can be evaluated with an independent sample cohort to identify a more universal gene set for diagnosis capable of detecting early pancreatic cancer or a pancreatic cancer precursor lesion and a more universal method for discriminating early pancreatic cancer or a pancreatic cancer precursor lesion.

Split-sample method is preferably used for evaluating the discrimination performance (generalization) of the discriminant. Specifically, a dataset is divided into a training cohort and a validation cohort, and gene selection by a statistical test and discriminant preparation are performed using the training cohort. Accuracy, sensitivity, and specificity are calculated using a result of discriminating a validation cohort according to the discriminant, and a true group to which the validation cohort belongs, to evaluate the discrimination performance of the discriminant. On the other hand, instead of dividing a dataset, the gene selection by a statistical test and discriminant preparation may be performed using all of samples, and accuracy, sensitivity, and specificity can be calculated by discriminatig a newly prepared sample with the discriminant for evaluation of the discrimination performance of the discriminant.

The present invention provides polynucleotides for disease diagnosis useful in the diagnosis and treatment of early pancreatic cancer or a pancreatic cancer precursor lesion, a method for detecting early pancreatic cancer or a pancreatic cancer precursor lesion using the polynucleotide(s), and a kit and a device for the detection of early pancreatic cancer or a pancreatic cancer precursor lesion, comprising the polynucleotide(s). Particularly, in order to select a gene(s) for diagnosis and prepare a discriminant so as to exhibit accuracy beyond the early pancreatic cancer or pancreatic cancer precursor lesion diagnosis methods using the existing tumor markers CEA and CA19-9, a gene set for diagnosis and a discriminant for the method of the present invention can be constructed, which exhibit accuracy beyond CEA and CA19-9, for example, by comparing expressed genes in serum from a patient confirmed to be negative using CEA and CA19-9 but finally found to have early pancreatic cancer or a pancreatic cancer precursor lesion by detailed examination such as computed tomography using a contrast medium, with genes expressed in serum from a patient having no early pancreatic cancer or pancreatic cancer precursor lesion.

For example, the gene set for diagnosis is determined as any combination selected from one or two or more of the polynucleotides based on a nucleotide sequence represented by any of SEQ ID NOs: 1 to 83, 227 to 229, 246, 248, and 250 or a nucleotide sequence derived from the nucleotide sequence by the replacement of u with t, or a complementary sequence thereof as described above; and optionally one or two or more of the polynucleotides based on a nucleotide sequence represented by any of SEQ ID NOs: 84 to 226, 230 to 245, 247, and 249 or a nucleotide sequence derived from the nucleotide sequence by the replacement of u with t, or a complementary sequence thereof; and optionally one or two or more of the polynucleotides based on a nucleotide sequence represented by any of SEQ ID NOs: 227 to 245 or a nucleotide sequence derived from the nucleotide sequence by the replacement of u with t, or a complementary sequence thereof. Further, a discriminant is constructed using expression levels of the gene set for diagnosis in samples from class I early pancreatic cancer or pancreatic cancer precursor lesion patients as a result of tissue diagnosis and samples from class II healthy subjects and/or other cancers and/or benign diseases as a result of tissue diagnosis. As a result, the presence or absence of early pancreatic cancer or a pancreatic cancer precursor lesion in a subject from which an unknown sample is derived can be determined with 100% accuracy at the maximum by measuring expression levels of the gene set for diagnosis in the unknown sample.

### EXAMPLES

Hereinafter, the present invention will be described further specifically with reference to Examples below. However, the scope of the present invention is not intended to be limited by these Examples.

### [Reference Example 1]

### <Collection of samples of early pancreatic cancer or pancreatic cancer precursor lesion patient and healthy subject>

Sera were collected using VENOJECT II vacuum blood collecting tube VP-AS109K60 (Terumo Corp., Japan) from each of 21 pancreatic cancer precursor lesion patients (4 cases with IPMA low grade, 5 cases with IPMA high grade, and 12 cases with IPMC) confirmed to have no cancer in organs, 31 early pancreatic cancer patients (9 cases with stage IIA and 22 cases with stage IIB) confirmed to have no cancer in organs other than the pancreas, and 123 healthy subjects after obtainment of informed consent, and used as a training cohort. Likewise, sera were collected using VENOJECT II vacuum blood collecting tube VP-AS109K60 (Terumo Corp.) from each of 12 pancreatic cancer precursor lesion patients (3 cases with IPMA low grade, 3 cases with IPMA high grade, and 6 cases with IPMC) confirmed to have no cancer in organs, 13 early pancreatic cancer patients (3 cases with stage IIA and 10 cases with stage IIB) confirmed to have no cancer in organs other than the pancreas, and 61 healthy subjects after obtainment of informed consent, and used as a validation cohort.

### <Extraction of total RNA>

Total RNA was obtained using a reagent for RNA extraction in 3D-Gene^{(R)} RNA extraction reagent from liquid sample kit (Toray Industries, Inc., Japan) according to the protocol provided by the manufacturer from 300 µL of the serum sample obtained from each of 261 persons in total of 33 pancreatic cancer precursor lesion patients, 184 healthy subjects, and 44 early pancreatic cancer patients included in the training cohort and the validation cohort.

### <Measurement of gene expression level>

miRNAs in the total RNA obtained from sera of each of the 261 persons in total of the 33 pancreatic cancer precursor lesion patients, the 184 healthy subjects, and the 44 early pancreatic cancer patients included in the training cohort and the validation cohort were fluorescently labeled using 3D-Gene^{(R)} miRNA Labeling kit (Toray Industries, Inc.) according to the protocol (ver 2.20) provided by the manufacturer. The oligo DNA chip used was 3D-Gene^{(R)} Human miRNA Oligo chip (Toray Industries, Inc.) with attached probes having sequences complementary to 2,555 miRNAs among the miRNAs registered in miRBase Release 20. Hybridization under stringent conditions and washing following the hybridization were performed according to the protocol provided by the manufacturer. The DNA chip was scanned using 3D-Gene^{(R)} scanner (Toray Industries, Inc.) to obtain images. Fluorescence intensity was digitized using 3D-Gene^{(R)} Extraction (Toray Industries, Inc.). The digitized fluorescence intensity was converted to a logarithmic value having a base of 2 and used as a gene expression level, from which a blank value was subtracted. A missing value was replaced with a value obtained by subtracting 0.1 from a logarithmic value of the smallest value of the gene expression level in each DNA chip. As a result, the comprehensive gene expression levels of the miRNAs in the sera were obtained from each of the 261 persons in total of the 33 pancreatic cancer precursor lesion patients, the 184 healthy subjects, and the 46 early pancreatic cancer patients. Calculation and statistical analysis using the digitized gene expression levels of the miRNAs were carried out using R language 3.0.2 (R Development Core Team (2013). R: A language and environment for statistical computing. R Foundation for Statistical Computing, URL http://www.R-project.org/.) and MASS package 7.3-30 (Venables, W. N. & Ripley, B. D. (2002) Modern Applied Statistics with S. Fourth Edition. Springer, New York. ISBN 0-387-95457-0).

### [Reference Example 2]

### <Collection of samples of other cancers and benign diseases>

Sera were collected using VENOJECT II vacuum blood collecting tube VP-AS109K60 (Terumo Corp.) from each of 61 advanced pancreatic cancer patients, 66 bile duct cancer patients, 31 colorectal cancer patients, 32 stomach cancer patients, 34 esophageal cancer patients, 38 liver cancer patients, and 15 benign pancreatic disease patients confirmed to have no cancer in other organs after obtainment of informed consent. Also, data on 51 breast cancer patients, 35 prostate cancer patients, and 26 benign prostatic disease patients was extracted from the data set under Accession No. GSE73002 of a gene expression information database Gene Expression Omnibus (http://www.ncbi.nlm.nih.gov/geo/). These samples were used as a training cohort together with the samples of 21 pancreatic cancer precursor lesion patients (4 cases with IPMA low grade, 5 cases with IPMA high grade, and 12 cases with IPMC), 31 early pancreatic cancer patients (8 cases with stage IIA and 22 cases with stage IIB), and 128 healthy subjects of Reference Example 1. Likewise, sera were collected using VENOJECT II vacuum blood collecting tube VP-AS109K60 (Terumo Corp.) from each of 39 advanced pancreatic cancer patients, 32 bile duct cancer patients, 19 colorectal cancer patients, 18 stomach cancer patients, 16 esophageal cancer patients, 14 liver cancer patients, and 9 benign pancreatic disease patients confirmed to have no cancer in other organs after obtainment of informed consent. Also, data on 23 breast cancer patients, 17 prostate cancer patients, and 15 benign prostatic disease patients was extracted from the dataset under Accession No. GSE73002 of a gene expression information database Gene Expression Omnibus (http://www.ncbi.nlm.nih.gov/geo/). These samples were used as a validation cohort together with the samples of 12 pancreatic cancer precursor lesion patients (3 cases with IPMA low grade, 3 cases with IPMA high grade, and 6 cases with IPMC), 13 early pancreatic cancer patients (3 cases with stage IIA and 10 cases with stage IIB), and 56 healthy subjects of Reference Example 1. Subsequent extraction of total RNA and measurement and analysis of gene expression levels were conducted in the same way as in Reference Example 1.

### [Example 1]

### <Selection of gene markers using samples of the training cohort, and method for evaluating early pancreatic cancer or pancreatic cancer precursor lesion discriminant performance of the single gene marker using the validation cohort>

In this Example, a gene marker for discriminating an early pancreatic cancer or pancreatic cancer precursor lesion patient as a positive control group from a healthy subject as a negative control group was selected from the training cohort and studied in samples of the validation cohort independent of the training cohort.

Specifically, first, the miRNA expression levels of the training cohort and the validation cohort obtained in the preceding Reference Examples were combined and normalized by global normalization.

Next, genes for diagnosis were selected using the training cohort. Here, in order to acquire diagnostic markers with higher reliability, only genes having the expression level of 2⁶ or higher in 50% or more of the samples in either of the early pancreatic cancer or pancreatic cancer precursor lesion patient group of the training cohort or the healthy subject group of the training cohort were selected. In order to further acquire statistically significant genes for discriminating an early pancreatic cancer or pancreatic cancer precursor lesion patient group from a healthy subject group, the P value obtained by two-tailed t-test assuming equal variance as to each gene expression level was corrected by the Bonferroni method, and genes that satisfied p < 0.01 were acquired as gene markers for use in explanatory variables of a discriminant and described in Table 2.

In this way, hsa-miR-6784-5p, hsa-miR-1181, hsa-miR-671-5p, hsa-miR-6857-5p, hsa-miR-4276, hsa-miR-1914-3p, hsa-miR-149-3p, hsa-miR-937-5p, hsa-miR-4675, hsa-miR-6795-5p, hsa-miR-4731-5p, hsa-miR-5090, hsa-miR-3620-5p, hsa-miR-1343-5p, hsa-miR-6717-5p, hsa-miR-6825-5p, hsa-miR-6738-5p, hsa-miR-6769a-5p, hsa-miR-4728-5p, hsa-miR-652-5p, hsa-miR-4257, hsa-miR-6785-5p, hsa-miR-7110-5p, hsa-miR-6887-5p, hsa-miR-887-3p, hsa-miR-1228-5p, hsa-miR-5572, hsa-miR-6782-5p, hsa-miR-4298, hsa-miR-6786-5p, hsa-miR-5010-5p, hsa-miR-6087, hsa-miR-6765-5p, hsa-miR-6732-5p, hsa-miR-6787-5p, hsa-miR-6737-5p, hsa-miR-128-2-5p, hsa-miR-4270, hsa-miR-6861-5p, hsa-miR-6756-5p, hsa-miR-1229-5p, hsa-miR-6891-5p, hsa-miR-6848-5p, hsa-miR-1237-5p, hsa-miR-30c-1-3p, hsa-miR-1233-5p, hsa-miR-211-3p, hsa-miR-4758-5p, hsa-miR-614, hsa-miR-6746-5p, hsa-miR-1915-5p, hsa-miR-4688, hsa-miR-3917, hsa-miR-5787, hsa-miR-4632-5p, hsa-miR-6126, hsa-miR-135a-3p, hsa-miR-8063, hsa-miR-5698, hsa-miR-6089, hsa-miR-498, hsa-miR-296-3p, hsa-miR-4419b, hsa-miR-6802-5p, hsa-miR-6829-5p, hsa-miR-6803-5p, hsa-miR-1199-5p, hsa-miR-6840-3p, hsa-miR-6752-5p, hsa-miR-6798-5p, hsa-miR-6131, hsa-miR-4667-5p, hsa-miR-6510-5p, hsa-miR-4690-5p, hsa-miR-920, hsa-miR-23b-3p, hsa-miR-4448, hsa-miR-2110, hsa-miR-4706, hsa-miR-7845-5p, hsa-miR-6808-5p, hsa-miR-4447, hsa-miR-6869-5p, hsa-miR-1908-5p, hsa-miR-6729-5p, hsa-miR-5195-3p, hsa-miR-638, hsa-miR-6125, hsa-miR-3178, hsa-miR-3196, hsa-miR-8069, hsa-miR-4723-5p, hsa-miR-4746-3p, hsa-miR-4689, hsa-miR-6816-5p, hsa-miR-6757-5p, hsa-miR-7109-5p, hsa-miR-6724-5p, hsa-miR-1225-3p, hsa-miR-6875-5p, hsa-miR-7108-5p, hsa-miR-4508, hsa-miR-6085, hsa-miR-6779-5p, hsa-miR-642a-3p, hsa-miR-4695-5p, hsa-miR-7847-3p, hsa-miR-3197, hsa-miR-6769b-5p, hsa-miR-7641, hsa-miR-187-5p, hsa-miR-3185, hsa-miR-2861, hsa-miR-3940-5p, hsa-miR-1203, hsa-miR-615-5p, hsa-miR-4787-5p, hsa-miR-1343-3p, hsa-miR-6813-5p, hsa-miR-1225-5p, hsa-miR-602, hsa-miR-4488, hsa-miR-125a-3p, hsa-miR-5100, hsa-miR-4294, hsa-miR-1231, hsa-miR-6765-3p, hsa-miR-4442, hsa-miR-718, hsa-miR-6780b-5p, hsa-miR-6090, hsa-miR-6845-5p, hsa-miR-4741, hsa-miR-4467, hsa-miR-4707-5p, hsa-miR-4271, hsa-miR-4673, hsa-miR-3184-5p, hsa-miR-1469, hsa-miR-4640-5p, hsa-miR-663a, hsa-miR-6791-5p, hsa-miR-6826-5p, hsa-miR-4433b-3p, hsa-miR-1915-3p, hsa-miR-4417, hsa-miR-4449, hsa-miR-4707-3p, hsa-miR-3180-3p, hsa-miR-5585-3p, hsa-miR-1268a, hsa-miR-8072, hsa-miR-296-5p, hsa-miR-204-3p, hsa-miR-4454, hsa-miR-6722-3p, hsa-miR-1290, hsa-miR-3622a-5p, hsa-miR-939-5p, hsa-miR-675-5p, hsa-miR-3131, hsa-miR-4648, hsa-miR-1268b, hsa-miR-6741-5p, hsa-miR-6893-5p, hsa-miR-3162-5p, hsa-miR-642b-3p, hsa-miR-4734, hsa-miR-150-3p, hsa-miR-8089, hsa-miR-6805-3p, hsa-miR-7113-3p, hsa-miR-6850-5p, hsa-miR-6799-5p, hsa-miR-6768-5p, hsa-miR-92b-5p, hsa-miR-3679-5p, hsa-miR-4792, hsa-miR-3656, hsa-miR-92a-2-5p, hsa-miR-4466, hsa-miR-4513, hsa-miR-6781-5p, hsa-miR-4649-5p, hsa-miR-6775-5p, hsa-miR-4651, hsa-miR-3195, hsa-miR-6726-5p, hsa-miR-6872-3p, hsa-miR-371a-5p, hsa-miR-6777-5p, hsa-miR-6789-5p, hsa-miR-7975, hsa-miR-6821-5p, hsa-miR-4534, hsa-miR-619-5p, hsa-miR-7107-5p, hsa-miR-1228-3p, hsa-miR-6774-5p, hsa-miR-6805-5p, hsa-miR-23a-3p, hsa-miR-4665-5p, hsa-miR-4505, hsa-miR-4638-5p, hsa-miR-24-3p, hsa-miR-3135b, hsa-miR-4745-5p, hsa-miR-128-1-5p, hsa-miR-4476, hsa-miR-4687-3p, hsa-miR-3665, hsa-miR-6806-5p, hsa-miR-3937, hsa-miR-711, hsa-miR-3141, hsa-miR-3188, hsa-miR-4281, hsa-miR-5196-5p, hsa-miR-6880-5p, hsa-miR-3960, hsa-miR-3648, hsa-miR-6721-5p, hsa-miR-4492, hsa-miR-744-5p, hsa-miR-7704, and hsa-miR-4749-5p genes, and the nucleotide sequences of SEQ ID NOs: 1 to 226 related thereto were identified.

A discriminant for determining the presence or absence of early pancreatic cancer or a pancreatic cancer precursor lesion was further prepared by Fisher's discriminant analysis with the expression levels of these genes as indicators. Specifically, any newly found expression level measurement values of polynucleotide consisting of a nucleotide sequence represented by any of SEQ ID NOs: 1 to 83 among the 226 genes selected in the training cohort was input to Formula 2 above to prepare a discriminant. Calculated accuracy, sensitivity, and specificity are shown in Table 3. In this respect, a discriminant coefficient and a constant term are shown in Table 4.

Next, accuracy, sensitivity, and specificity in the validation cohort were calculated using the discriminant thus prepared, and the discriminant performance of the selected polynucleotides was validated using independent samples (Table 3). For example, the discriminant score obtained by use of Fisher's discriminant analysis from the expression level measurement value of the nucleotide sequence represented by SEQ ID NO: 1 was compared between the pancreatic cancer precursor lesion patients (21 persons) or the early pancreatic cancer patients (31 persons) and the healthy subjects in the training cohort. As a result, the discriminant score in the training cohort was found to be significantly higher in the early pancreatic cancer or pancreatic cancer precursor lesion group than in the healthy subject group (see the left diagram of Figure 2). These results were also reproducible in the validation cohort (see the right diagram of Figure 2). Likewise, the results obtained about the other polynucleotides shown in SEQ ID NOs: 1 to 226 showed that the gene expression level measurement values were significantly lower (decrease) or higher (increase) in the early pancreatic cancer or pancreatic cancer precursor lesion patient group than in the healthy subject group (Table 2). These results were able to be validated in the validation cohort. For example, as for this nucleotide sequence represented by SEQ ID NO: 1, the number of correctly or incorrectly identified samples in the detection of early pancreatic cancer or a pancreatic cancer precursor lesion in the validation cohort was calculated using the threshold (0) that was set in the training cohort for discriminating the two groups. As a result, 22 true positives, 52 true negatives, 9 false positives, and 4 false negatives were obtained. From these values, 85.1% accuracy, 84.6% sensitivity, and 85.2% specificity were obtained as the detection performance. In this way, the detection performance was calculated as to all of the polynucleotides shown in SEQ ID NOs: 1 to 83, and described in Table 3. Among the polynucleotides consisting of the nucleotide sequences represented by SEQ ID NOs: 1 to 83 shown in Table 2, for example, 26 polynucleotides consisting of the nucleotide sequences represented by SEQ ID NOs: 2, 3, 18, 12, 20, 1, 15, 50, 63, 72, 5, 24, 10, 52, 9, 11, 19, 39, 61, 7, 17, 22, 26, 74, 21, and 28 exhibited sensitivity of 100%, 92.3%, 92.3%, 76.9%, 80.8%, 84.6%, 76.9%, 84.6%, 73.1%, 80.8%, 88.5%, 88.5%, 88.5%, 73.1%, 73.1%, 76.9%, 61.5%, 65.4%, 84.6%, 92.3%, 73.1%, 61.5%, 76.9%, 73.1%, 80.8%, and 92.3%, respectively, in the validation cohort (Table 3). As seen from Comparative Examples mentioned later, the existing markers CEA and CA19-9 had sensitivity of 20% and 68%, respectively, in the validation cohort (Table 5-2), demonstrating that, for example, the 26 polynucleotides consisting of the nucleotide sequences represented by SEQ ID NOs: 2, 3, 18, 12, 20, 1, 15, 50, 63, 72, 5, 24, 10, 52, 9, 11, 19, 39, 61, 7, 17, 22, 26, 74, 21, and 28 can discriminate, each alone, early pancreatic cancer or a pancreatic cancer precursor lesion in the validation cohort with sensitivity beyond or equivalent to CA19-9.

### [Example 2]

### <Method A for evaluating early pancreatic cancer or pancreatic cancer precursor lesion discriminant performance by combination of plurality of gene markers using samples in the validation cohort>

In this Example, a method for evaluating early pancreatic cancer or a pancreatic cancer precursor lesion discriminant performance by a combination of the gene markers selected in Example 1 was studied.

Specifically, Fisher's discriminant analysis was conducted as to 15,272 combinations of two expression level measurement values comprising at least one or more of the expression level measurement values of the newly found polynucleotides consisting of the nucleotide sequences represented by SEQ ID NOs: 1 to 83 among the polynucleotides consisting of the nucleotide sequences represented by SEQ ID NOs: 1 to 226 selected in Example 1, to construct a discriminant for determining the presence or absence of early pancreatic cancer or a pancreatic cancer precursor lesion. Next, accuracy, sensitivity, and specificity in the validation cohort were calculated using the discriminant thus prepared, and the discriminant performance of the selected polynucleotides was validated using independent samples. For example, the expression level measurement values of the nucleotide sequences represented by SEQ ID NO: 1 and SEQ ID NO: 2 were compared between the pancreatic cancer precursor lesion patients (21 persons) or the early pancreatic cancer patients (31 persons) and the healthy subjects (123 persons) in the training cohort. As a result, a scatter diagram that significantly separated the expression level measurement values of the early pancreatic cancer or pancreatic cancer precursor lesion patient group from those of the healthy subject group was obtained in the training cohort. These results were also reproducible in the validation cohort. Likewise, a scatter diagram that significantly separated the gene expression level measurement values of the early pancreatic cancer or pancreatic cancer precursor lesion patient group from those of the healthy subject group was also obtained as to the other combinations of two expression level measurement values comprising at least one of the expression level measurement values of the newly found polynucleotides consisting of the nucleotide sequences represented by SEQ ID NOs: 1 to 83 among the polynucleotides consisting of the nucleotide sequences represented by SEQ ID NOs: 1 to 226. These results were able to be validated in the validation cohort. For example, as for these nucleotide sequences represented by SEQ ID NO: 1 and SEQ ID NO: 2, the number of correctly or incorrectly identified samples in the detection of early pancreatic cancer or a pancreatic cancer precursor lesion was calculated using the threshold (0 = 1.90 × hsa-miR-6784-5p + 1.72 × hsa-miR-1181 - 34.50) that was set in the training cohort for discriminating the two groups. As a result, 52 true positives, 107 true negatives, 16 false positives, and 1 false negative were obtained in the training cohort. From these values, 90.3% accuracy, 98.1% sensitivity, and 87% specificity were obtained as the detection performance (see the left diagram of Figure 3). These results were also reproducible in the validation cohort (see the right diagram of Figure 3). In this way, the detection performance was calculated for all of the combinations of two expression level measurement values comprising at least one of the expression level measurement values of the newly found polynucleotides consisting of the nucleotide sequences represented by SEQ ID NOs: 1 to 83 among the polynucleotides consisting of the nucleotide sequences represented by SEQ ID NOs: 1 to 226. Among them, 225 combinations comprising the expression level measurement value of the polynucleotide consisting of the nucleotide sequence represented by SEQ ID NO: 1, and the detection performance thereof are described in Table 6 as an example. In Table 6, "SEQ ID NO" indicates the combinations of a plurality of polynucleotides used by SEQ ID NO (the same applies to the tables mentioned later herein). For example, the combinations of the expression level measurement values of the polynucleotides consisting of the nucleotide sequences represented by SEQ ID NO: 1 and SEQ ID NO: 2, SEQ ID NO: 1 and SEQ ID NO: 3, SEQ ID NO: 1 and SEQ ID NO: 4, and SEQ ID NO: 1 and SEQ ID NO: 5 showed sensitivity of 92.3%, 88.5%, 84.6%, 88.5%, and 84.6%, respectively, in the validation cohort (Table 6). Further, the combinations of two polynucleotides consisting of nucleotide sequences other than SEQ ID NO: 1 are shown in Table 7 as an example. For example, the combinations of SEQ ID NOs: 2 and 18, SEQ ID NOs: 2 and 53, SEQ ID NOs: 2 and 20, SEQ ID NOs: 2 and 3, SEQ ID NOs: 2 and 50, SEQ ID NOs: 85 and 3, SEQ ID NOs: 84 and 3, SEQ ID NOs: 90 and 3, SEQ ID NOs: 87 and 3, SEQ ID NOs: 90 and 18, SEQ ID NOs: 87 and 18, SEQ ID NOs: 89 and 18, SEQ ID NOs: 84 and 18, SEQ ID NOs: 85 and 12, SEQ ID NOs: 85 and 20, SEQ ID NOs: 84 and 20, SEQ ID NOs: 90 and 20, SEQ ID NOs: 87 and 20, SEQ ID NOs: 87 and 15, SEQ ID NOs: 85 and 15, SEQ ID NOs: 2 and 15, SEQ ID NOs: 85 and 50, SEQ ID NOs: 87 and 50, SEQ ID NOs: 84 and 50, SEQ ID NOs: 106 and 50, SEQ ID NOs: 90 and 50, SEQ ID NOs: 2 and 63, SEQ ID NOs: 85 and 63, SEQ ID NOs: 90 and 63, SEQ ID NOs: 87 and 63, SEQ ID NOs: 84 and 72, SEQ ID NOs: 85 and 72, SEQ ID NOs: 88 and 72, SEQ ID NOs: 87 and 72, SEQ ID NOs: 85 and 5, SEQ ID NOs: 87 and 5, SEQ ID NOs: 84 and 5, SEQ ID NOs: 85 and 10, SEQ ID NOs: 90 and 10, SEQ ID NOs: 85 and 52, SEQ ID NOs: 88 and 52, SEQ ID NOs: 87 and 52, SEQ ID NOs: 98 and 52, SEQ ID NOs: 84 and 52, SEQ ID NOs: 87 and 9, SEQ ID NOs: 85 and 9, SEQ ID NOs: 117 and 9, SEQ ID NOs: 88 and 9, SEQ ID NOs: 87 and 11, SEQ ID NOs: 85 and 11, SEQ ID NOs: 102 and 11, SEQ ID NOs: 84 and 11, SEQ ID NOs: 85 and 19, SEQ ID NOs: 87 and 19, SEQ ID NOs: 88 and 19, SEQ ID NOs: 89 and 19, SEQ ID NOs: 87 and 39, SEQ ID NOs: 85 and 39, SEQ ID NOs: 2 and 39, SEQ ID NOs: 87 and 61, SEQ ID NOs: 85 and 61, SEQ ID NOs: 88 and 61, SEQ ID NOs: 88 and 7, SEQ ID NOs: 85 and 7, SEQ ID NOs: 87 and 7, SEQ ID NOs: 91 and 7, SEQ ID NOs: 85 and 17, SEQ ID NOs: 87 and 17, SEQ ID NOs: 85 and 22, SEQ ID NOs: 87 and 22, SEQ ID NOs: 117 and 22, SEQ ID NOs: 85 and 26, SEQ ID NOs: 87 and 26, SEQ ID NOs: 84 and 26, SEQ ID NOs: 85 and 74, SEQ ID NOs: 2 and 74, SEQ ID NOs: 87 and 74, SEQ ID NOs: 84 and 74, SEQ ID NOs: 88 and 74, SEQ ID NOs: 85 and 28, and SEQ ID NOs: 84 and 28 as specific combinations of two polynucleotides showed sensitivity of 95% or higher to discriminate early pancreatic cancer or pancreatic cancer precursor lesion patients from healthy subjects in both of the training cohort and the validation cohort. In this way, the 14,079 combinations of the expression level measurement values of the polynucleotides having sensitivity beyond the existing marker CA19-9 (68% from Table 5-2) were obtained in the validation cohort. All of the nucleotide sequences 1 to 226 described in Table 2 obtained in Example 1 were employed at least once in these combinations. These results demonstrated that the combinations of two expression level measurement values comprising at least one of the expression level measurement values of the polynucleotides consisting of the nucleotide sequences represented by SEQ ID NOs: 1 to 226 detect early pancreatic cancer or a pancreatic cancer precursor lesion with sensitivity beyond CA19-9 in the validation cohort.

Thus, markers capable of detecting early pancreatic cancer or a pancreatic cancer precursor lesion with excellent sensitivity are obtained even if 3, 4, 5, 6, 7, 8, 9, 10 or more of the expression level measurement values of the polynucleotides consisting of the nucleotide sequences represented by SEQ ID NOs: 1 to 226 are combined. For example, the polynucleotides consisting of the nucleotide sequences represented by SEQ ID NOs: 1 to 226 selected in Example 1 were ranked in the descending order of their P values which indicate statistical significance, and detection performance was calculated using combinations of one or more miRNAs to which the miRNAs were added one by one from the top to the bottom according to the rank. As a result, the sensitivity in the validation cohort was 69.2% for 2 miRNAs, 80.8% for 5 miRNAs, 92.3% for 10 miRNAs, 96.2% for 20 miRNAs, 100% for 100 miRNAs, and 100% for 226 miRNAs. These values of the sensitivity were higher than the sensitivity of the existing tumor marker in blood, demonstrating that even combinations of a plurality of the miRNAs can serve as excellent markers for the detection of early pancreatic cancer or a pancreatic cancer precursor lesion. In this context, the combinations of a plurality of the miRNAs are not limited to the combinations of the miRNAs added in the order of statistically significant difference as described above, and any combination of a plurality of the miRNAs can be used in the detection of early pancreatic cancer or a pancreatic cancer precursor lesion.

From these results, it can be concluded that all of the polynucleotides consisting of the nucleotide sequences represented by SEQ ID NOs: 1 to 226 serve as excellent diagnostic markers.

Tables 2, 3, 4, 5, 6, and 7 mentioned above are as follows.

**[Table 2]**

| SEQ ID NO: | Gene name | P value after Bonferroni correction | Change in expression level in early pancreatic cancer and pancreatic cancer precursor lesion patient relative to healthy subject |
|---|---|---|---|
| 1 | hsa-miR-6784-5p | 8.01E-38 | Increase |
| 2 | hsa-miR-1181 | 1.56E-37 | Increase |
| 3 | hsa-miR-671-5p | 4.06E-32 | Decrease |
| 4 | hsa-miR-6857-5p | 1.70E-31 | Increase |
| 5 | hsa-miR-4276 | 2.59E-29 | Increase |
| 6 | hsa-miR-1914-3p | 5.13E-28 | Decrease |
| 7 | hsa-miR-149-3p | 6.76E-25 | Decrease |
| 8 | hsa-miR-937-5p | 1.08E-24 | Decrease |
| 9 | hsa-miR-4675 | 8.93E-24 | Decrease |
| 10 | hsa-miR-6795-5p | 3.80E-23 | Decrease |
| 11 | hsa-miR-4731-5p | 5.78E-23 | Decrease |
| 12 | hsa-miR-5090 | 9.07E-23 | Decrease |
| 13 | hsa-miR-3620-5p | 5.97E-22 | Increase |
| 14 | hsa-miR-1343-5p | 3.10E-21 | Increase |
| 15 | hsa-miR-6717-5p | 1.26E-20 | Decrease |
| 16 | hsa-miR-6825-5p | 3.53E-20 | Increase |
| 17 | hsa-miR-6738-5p | 5.73E-20 | Decrease |
| 18 | hsa-miR-6769a-5p | 6.03E-20 | Decrease |
| 19 | hsa-miR-4728-5p | 6.45E-20 | Decrease |
| 20 | hsa-miR-652-5p | 6.72E-20 | Decrease |
| 21 | hsa-miR-4257 | 9.04E-20 | Decrease |
| 22 | hsa-miR-6785-5p | 1.42E-19 | Decrease |
| 23 | hsa-miR-7110-5p | 2.12E-19 | Increase |
| 24 | hsa-miR-6887-5p | 2.20E-19 | Decrease |
| 25 | hsa-miR-887-3p | 3.36E-19 | Increase |
| 26 | hsa-miR-1228-5p | 8.56E-19 | Increase |
| 27 | hsa-miR-5572 | 3.16E-18 | Increase |
| 28 | hsa-miR-6782-5p | 2.24E-17 | Decrease |
| 29 | hsa-miR-4298 | 2.85E-17 | Decrease |
| 30 | hsa-miR-6786-5p | 1.52E-16 | Increase |
| 31 | hsa-miR-5010-5p | 2.17E-16 | Decrease |
| 32 | hsa-miR-6087 | 9.02E-16 | Increase |
| 33 | hsa-miR-6765-5p | 2.35E-15 | Increase |
| 34 | hsa-miR-6732-5p | 4.15E-15 | Increase |
| 35 | hsa-miR-6787-5p | 9.05E-15 | Decrease |
| 36 | hsa-miR-6737-5p | 9.97E-15 | Increase |
| 37 | hsa-miR-128-2-5p | 1.76E-14 | Decrease |
| 38 | hsa-miR-4270 | 2.27E-14 | Decrease |
| 39 | hsa-miR-6861-5p | 2.90E-14 | Decrease |
| 40 | hsa-miR-6756-5p | 8.32E-14 | Decrease |
| 41 | hsa-miR-1229-5p | 4.69E-13 | Decrease |
| 42 | hsa-miR-6891-5p | 7.35E-13 | Decrease |
| 43 | hsa-miR-6848-5p | 1.26E-12 | Increase |
| 44 | hsa-miR-1237-5p | 9.08E-12 | Increase |
| 45 | hsa-miR-30c-1-3p | 1.04E-11 | Decrease |
| 46 | hsa-miR-1233-5p | 2.45E-11 | Decrease |
| 47 | hsa-miR-211-3p | 3.35E-11 | Decrease |
| 48 | hsa-miR-4758-5p | 4.39E-11 | Decrease |
| 49 | hsa-miR-614 | 5.53E-11 | Decrease |
| 50 | hsa-miR-6746-5p | 9.72E-11 | Decrease |
| 51 | hsa-miR-1915-5p | 1.53E-10 | Decrease |
| 52 | hsa-miR-4688 | 1.67E-10 | Decrease |
| 53 | hsa-miR-3917 | 2.52E-10 | Decrease |
| 54 | hsa-miR-5787 | 2.64E-10 | Increase |
| 55 | hsa-miR-4632-5p | 2.94E-10 | Increase |
| 56 | hsa-miR-6126 | 3.79E-10 | Increase |
| 57 | hsa-miR-135a-3p | 7.42E-10 | Increase |
| 58 | hsa-miR-8063 | 1.29E-09 | Decrease |
| 59 | hsa-miR-5698 | 3.21E-09 | Decrease |
| 60 | hsa-miR-6089 | 2.31E-08 | Increase |
| 61 | hsa-miR-498 | 2.38E-08 | Decrease |
| 62 | hsa-miR-296-3p | 5.37E-08 | Decrease |
| 63 | hsa-miR-4419b | 1.02E-07 | Decrease |
| 64 | hsa-miR-6802-5p | 1.69E-07 | Decrease |
| 65 | hsa-miR-6829-5p | 2.31E-07 | Decrease |
| 66 | hsa-miR-6803-5p | 3.54E-07 | Increase |
| 67 | hsa-miR-1199-5p | 5.49E-07 | Decrease |
| 68 | hsa-miR-6840-3p | 7.30E-07 | Decrease |
| 69 | hsa-miR-6752-5p | 3.97E-06 | Increase |
| 70 | hsa-miR-6798-5p | 1.85E-05 | Increase |
| 71 | hsa-miR-6131 | 4.80E-05 | Decrease |
| 72 | hsa-miR-4667-5p | 9.09E-05 | Decrease |
| 73 | hsa-miR-6510-5p | 1.04E-04 | Decrease |
| 74 | hsa-miR-4690-5p | 1.22E-04 | Decrease |
| 75 | hsa-miR-920 | 1.49E-04 | Decrease |
| 76 | hsa-miR-23b-3p | 1.85E-04 | Decrease |
| 77 | hsa-miR-4448 | 2.38E-04 | Increase |
| 78 | hsa-miR-2110 | 3.99E-04 | Decrease |
| 79 | hsa-miR-4706 | 5.83E-04 | Decrease |
| 80 | hsa-miR-7845-5p | 2.34E-03 | Increase |
| 81 | hsa-miR-6808-5p | 4.82E-03 | Decrease |
| 82 | hsa-miR-4447 | 5.38E-03 | Decrease |
| 83 | hsa-miR-6869-5p | 6.71 E-03 | Increase |
| 84 | hsa-miR-1908-5p | 3.48E-50 | Increase |
| 85 | hsa-miR-6729-5p | 1.20E-47 | Increase |
| 86 | hsa-miR-5195-3p | 4.85E-45 | Decrease |
| 87 | hsa-miR-638 | 6.56E-44 | Increase |
| 88 | hsa-miR-6125 | 2.97E-42 | Increase |
| 89 | hsa-miR-3178 | 3.76E-41 | Increase |
| 90 | hsa-miR-3196 | 2.70E-39 | Increase |
| 91 | hsa-miR-8069 | 3.80E-36 | Increase |
| 92 | hsa-miR-4723-5p | 3.97E-36 | Decrease |
| 93 | hsa-miR-4746-3p | 1.24E-35 | Increase |
| 94 | hsa-miR-4689 | 2.84E-35 | Decrease |
| 95 | hsa-miR-6816-5p | 3.48E-35 | Increase |
| 96 | hsa-miR-6757-5p | 1.20E-34 | Decrease |
| 97 | hsa-miR-7109-5p | 1.21E-34 | Decrease |
| 98 | hsa-miR-6724-5p | 2.96E-34 | Increase |
| 99 | hsa-miR-1225-3p | 2.03E-33 | Increase |
| 100 | hsa-miR-6875-5p | 5.61E-33 | Increase |
| 101 | hsa-miR-7108-5p | 4.48E-32 | Increase |
| 102 | hsa-miR-4508 | 1.03E-31 | Increase |
| 103 | hsa-miR-6085 | 7.30E-30 | Decrease |
| 104 | hsa-miR-6779-5p | 7.94E-30 | Decrease |
| 105 | hsa-miR-642a-3p | 1.28E-28 | Decrease |
| 106 | hsa-miR-4695-5p | 1.37E-28 | Increase |
| 107 | hsa-miR-7847-3p | 1.49E-28 | Decrease |
| 108 | hsa-miR-3197 | 1.08E-27 | Increase |
| 109 | hsa-miR-6769b-5p | 1.82E-27 | Decrease |
| 110 | hsa-miR-7641 | 3.73E-27 | Decrease |
| 111 | hsa-miR-187-5p | 1.45E-26 | Decrease |
| 112 | hsa-miR-3185 | 1.98E-26 | Increase |
| 113 | hsa-miR-2861 | 4.70E-26 | Increase |
| 114 | hsa-miR-3940-5p | 2.68E-25 | Increase |
| 115 | hsa miR 1203 | 3.13E 25 | Increase |
| 116 | hsa-miR-615-5p | 9.78E-25 | Decrease |
| 117 | hsa-miR-4787-5p | 4.84E-24 | Increase |
| 118 | hsa-miR-1343-3p | 7.76E-24 | Decrease |
| 119 | hsa-miR-6813-5p | 9.50E-24 | Decrease |
| 120 | hsa-miR-1225-5p | 2.32E-22 | Increase |
| 121 | hsa-miR-602 | 3.00E-22 | Increase |
| 122 | hsa-miR-4488 | 5.30E-22 | Increase |
| 123 | hsa-miR-125a-3p | 1.08E-21 | Decrease |
| 124 | hsa-miR-5100 | 1.58E-21 | Increase |
| 125 | hsa-miR-4294 | 2.49E-21 | Decrease |
| 126 | hsa-miR-1231 | 3.00E-21 | Increase |
| 127 | hsa-miR-6765-3p | 5.05E-21 | Decrease |
| 128 | hsa-miR-4442 | 8.24E-21 | Decrease |
| 129 | hsa-miR-718 | 1.55E-20 | Increase |
| 130 | hsa-miR-6780b-5p | 3.10E-20 | Increase |
| 131 | hsa-miR-6090 | 1.16E-19 | Increase |
| 132 | hsa-miR-6845-5p | 1.52E-19 | Increase |
| 133 | hsa-miR-4741 | 1.58E-19 | Increase |
| 134 | hsa-miR-4467 | 3.00E-19 | Increase |
| 135 | hsa-miR-4707-5p | 3.49E-19 | Increase |
| 136 | hsa-miR-4271 | 1.16E-18 | Decrease |
| 137 | hsa-miR-4673 | 1.20E-18 | Increase |
| 138 | hsa-miR-3184-5p | 1.69E-18 | Increase |
| 139 | hsa-miR-1469 | 1.98E-18 | Increase |
| 140 | hsa-miR-4640-5p | 2.13E-18 | Increase |
| 141 | hsa-miR-663a | 3.70E-18 | Increase |
| 142 | hsa-miR-6791-5p | 9.89E-18 | Increase |
| 143 | hsa-miR-6826-5p | 5.46E-17 | Decrease |
| 144 | hsa-miR-4433b-3p | 1.49E-16 | Increase |
| 145 | hsa-miR-1915-3p | 1.61E-16 | Increase |
| 146 | hsa-miR-4417 | 3.24E-16 | Increase |
| 147 | hsa-miR-4449 | 6.68E-16 | Increase |
| 148 | hsa-miR-4707-3p | 9.83E-16 | Increase |
| 149 | hsa-miR-3180-3p | 1.06E-15 | Increase |
| 150 | hsa-miR-5585-3p | 3.68E-15 | Increase |
| 151 | hsa-miR-1268a | 4.67E-15 | Increase |
| 152 | hsa-miR-8072 | 8.29E-15 | Increase |
| 153 | hsa-miR-296-5p | 1.70E-14 | Decrease |
| 154 | hsa-miR-204-3p | 2.59E-14 | Decrease |
| 155 | hsa-miR-4454 | 2.97E-14 | Decrease |
| 156 | hsa-miR-6722-3p | 3.47E-14 | Increase |
| 157 | hsa-miR-1290 | 5.12E-14 | Increase |
| 158 | hsa-miR-3622a-5p | 5.86E-14 | Decrease |
| 159 | hsa-miR-939-5p | 9.18E-14 | Increase |
| 160 | hsa-miR-675-5p | 1.25E-13 | Decrease |
| 161 | hsa-miR-3131 | 1.26E-13 | Decrease |
| 162 | hsa-miR-4648 | 1.93E-13 | Increase |
| 163 | hsa-miR-1268b | 2.06E-13 | Increase |
| 164 | hsa-miR-6741-5p | 2.25E-13 | Decrease |
| 165 | hsa-miR-6893-5p | 5.59E-13 | Decrease |
| 166 | hsa-miR-3162-5p | 1.01E-12 | Decrease |
| 167 | hsa-miR-642b-3p | 1.51E-12 | Decrease |
| 168 | hsa-miR-4734 | 7.47E-12 | Increase |
| 169 | hsa-miR-150-3p | 3.02E-11 | Decrease |
| 170 | hsa-miR-8089 | 3.88E-11 | Decrease |
| 171 | hsa-miR-6805-3p | 4.65E-11 | Increase |
| 172 | hsa-miR-7113-3p | 6.07E-11 | Increase |
| 173 | hsa-miR-6850-5p | 1.45E-10 | Increase |
| 174 | hsa-miR-6799-5p | 2.02E-10 | Decrease |
| 175 | hsa-miR-6768-5p | 2.10E-10 | Increase |
| 176 | hsa-miR-92b-5p | 2.27E-10 | Increase |
| 177 | hsa-miR-3679-5p | 3.05E-10 | Increase |
| 178 | hsa-miR-4792 | 4.13E-10 | Increase |
| 179 | hsa-miR-3656 | 8.02E-10 | Increase |
| 180 | hsa-miR-92a-2-5p | 9.84E-10 | Increase |
| 181 | hsa-miR-4466 | 1.70E-09 | Increase |
| 182 | hsa-miR-4513 | 1.79E-09 | Decrease |
| 183 | hsa-miR-6781-5p | 2.02E-09 | Increase |
| 184 | hsa-miR-4649-5p | 3.34E-09 | Decrease |
| 185 | hsa-miR-6775-5p | 7.94E-09 | Decrease |
| 186 | hsa-miR-4651 | 2.08E-08 | Decrease |
| 187 | hsa-miR-3195 | 4.19E-08 | Increase |
| 188 | hsa-miR-6726-5p | 4.45E-08 | Decrease |
| 189 | hsa-miR-6872-3p | 7.91E-08 | Decrease |
| 190 | hsa-miR-371a-5p | 1.32E-07 | Decrease |
| 191 | hsa-miR-6777-5p | 2.82E-07 | Decrease |
| 192 | hsa-miR-6789-5p | 5.95E-07 | Increase |
| 193 | hsa-miR-7975 | 1.00E-06 | Decrease |
| 194 | hsa-miR-6821-5p | 1.36E-06 | Decrease |
| 195 | hsa-miR-4534 | 5.94E-06 | Decrease |
| 196 | hsa-miR-619-5p | 6.51E-06 | Increase |
| 197 | hsa-miR-7107-5p | 1.06E-05 | Decrease |
| 198 | hsa-miR-1228-3p | 1.07E-05 | Increase |
| 199 | hsa-miR-6774-5p | 1.08E-05 | Decrease |
| 200 | hsa-miR-6805-5p | 1.10E-05 | Increase |
| 201 | hsa-miR-23a-3p | 2.26E-05 | Decrease |
| 202 | hsa-miR-4665-5p | 3.25E-05 | Decrease |
| 203 | hsa-miR-4505 | 7.17E-05 | Increase |
| 204 | hsa-miR-4638-5p | 7.59E-05 | Decrease |
| 205 | hsa-miR-24-3p | 8.75E-05 | Decrease |
| 206 | hsa-miR-3135b | 1.20E-04 | Decrease |
| 207 | hsa-miR-4745-5p | 1.24E-04 | Increase |
| 208 | hsa-miR-128-1-5p | 1.45E-04 | Increase |
| 209 | hsa-miR-4476 | 1.81E-04 | Decrease |
| 210 | hsa-miR-4687-3p | 1.94E-04 | Decrease |
| 211 | hsa-miR-3665 | 2.61E-04 | Increase |
| 212 | hsa-miR-6806-5p | 3.17E-04 | Decrease |
| 213 | hsa-miR-3937 | 3.45E-04 | Increase |
| 214 | hsa-miR-711 | 6.28E-04 | Increase |
| 215 | hsa-miR-3141 | 6.80E-04 | Increase |
| 216 | hsa-miR-3188 | 6.88E-04 | Increase |
| 217 | hsa-miR-4281 | 9.34E-04 | Decrease |
| 218 | hsa-miR-5196-5p | 1.33E-03 | Decrease |
| 219 | hsa-miR-6880-5p | 1.48E-03 | Increase |
| 220 | hsa-miR-3960 | 2.60E-03 | Increase |
| 221 | hsa-miR-3648 | 3.23E-03 | Increase |
| 222 | hsa-miR-6721-5p | 3.69E-03 | Increase |
| 223 | hsa-miR-4492 | 4.89E-03 | Increase |
| 224 | hsa-miR-744-5p | 8.59E-03 | Increase |
| 225 | hsa-miR-7704 | 8.95E-03 | Increase |
| 226 | hsa-miR-4749-5p | 8.96E-03 | Increase |

**[Table 3]**

| | Training cohort | | | Validation cohort | | |
|---|---|---|---|---|---|---|
| SEQ ID NO: | Accuracy (%) | Sensitivity (%) | Specificity (%) | Accuracy (%) | Sensitivity (%) | Specificity (%) |
| 1 | 89.8 | 96.2 | 87 | 85.1 | 84.6 | 85.2 |
| 2 | 89.8 | 96.2 | 87 | 89.7 | 100 | 85.2 |
| 3 | 87.5 | 86.8 | 87.8 | 94.3 | 92.3 | 95.1 |
| 4 | 88.1 | 96.2 | 84.6 | 90.8 | 92.3 | 90.2 |
| 5 | 88.6 | 94.3 | 86.2 | 85.1 | 88.5 | 83.6 |
| 6 | 88.6 | 86.8 | 89.4 | 89.7 | 84.6 | 91.8 |
| 7 | 84.1 | 86.8 | 82.9 | 92 | 92.3 | 91.8 |
| 8 | 85.8 | 79.2 | 88.6 | 89.7 | 88.5 | 90.2 |
| 9 | 84.1 | 84.9 | 83.7 | 79.3 | 73.1 | 82 |
| 10 | 85.8 | 84.9 | 86.2 | 86.2 | 88.5 | 85.2 |
| 11 | 84.7 | 88.7 | 82.9 | 81.6 | 76.9 | 83.6 |
| 12 | 86.9 | 92.5 | 84.6 | 83.9 | 76.9 | 86.9 |
| 13 | 85.8 | 94.3 | 82.1 | 83.9 | 88.5 | 82 |
| 14 | 84.7 | 79.2 | 87 | 74.7 | 61.5 | 80.3 |
| 15 | 84.1 | 84.9 | 83.7 | 80.5 | 76.9 | 82 |
| 16 | 83 | 83 | 82.9 | 78.2 | 73.1 | 80.3 |
| 17 | 83 | 77.4 | 85.4 | 80.5 | 73.1 | 83.6 |
| 18 | 85.2 | 90.6 | 82.9 | 85.1 | 92.3 | 82 |
| 19 | 81.2 | 83 | 80.5 | 79.3 | 61.5 | 86.9 |
| 20 | 84.7 | 84.9 | 84.6 | 82.8 | 80.8 | 83.6 |
| 21 | 86.9 | 80.8 | 89.4 | 85.1 | 80.8 | 86.9 |
| 22 | 81.8 | 73.6 | 85.4 | 74.7 | 61.5 | 80.3 |
| 23 | 79.5 | 83 | 78 | 74.7 | 76.9 | 73.8 |
| 24 | 86.9 | 81.1 | 89.4 | 80.5 | 88.5 | 77 |
| 25 | 84.1 | 94.3 | 79.7 | 81.6 | 88.5 | 78.7 |
| 26 | 79.5 | 81.1 | 78.9 | 78.2 | 76.9 | 78.7 |
| 27 | 80.1 | 86.8 | 77.2 | 74.7 | 73.1 | 75.4 |
| 28 | 83 | 81.1 | 83.7 | 89.7 | 92.3 | 88.5 |
| 29 | 78.4 | 75.5 | 79.7 | 79.1 | 61.5 | 86.7 |
| 30 | 78.4 | 73.6 | 80.5 | 83.9 | 80.8 | 85.2 |
| 31 | 79.5 | 83 | 78 | 86.2 | 96.2 | 82 |
| 32 | 79 | 94.3 | 72.4 | 83.9 | 100 | 77 |
| 33 | 80.1 | 81.1 | 79.7 | 77 | 80.8 | 75.4 |
| 34 | 77.8 | 86.8 | 74 | 81.6 | 80.8 | 82 |
| 35 | 79.5 | 84.9 | 77.2 | 82.8 | 88.5 | 80.3 |
| 36 | 81.2 | 84.9 | 79.7 | 79.3 | 76.9 | 80.3 |
| 37 | 77.3 | 71.7 | 79.7 | 75.9 | 61.5 | 82 |
| 38 | 75.6 | 79.2 | 74 | 73.6 | 73.1 | 73.8 |
| 39 | 76.7 | 75.5 | 77.2 | 70.1 | 65.4 | 72.1 |
| 40 | 75 | 75.5 | 74.8 | 74.7 | 80.8 | 72.1 |
| 41 | 73.9 | 71.7 | 74.8 | 83.9 | 92.3 | 80.3 |
| 42 | 79.5 | 69.8 | 83.7 | 75.9 | 73.1 | 77 |
| 43 | 80.7 | 81.1 | 80.5 | 83.9 | 69.2 | 90.2 |
| 44 | 75.6 | 79.2 | 74 | 74.7 | 80.8 | 72.1 |
| 45 | 77.8 | 75.5 | 78.9 | 78.2 | 69.2 | 82 |
| 46 | 75.6 | 77.4 | 74.8 | 67.8 | 61.5 | 70.5 |
| 47 | 76.1 | 86.8 | 71.5 | 72.4 | 65.4 | 75.4 |
| 48 | 75 | 77.4 | 74 | 69 | 69.2 | 68.9 |
| 49 | 74.4 | 71.7 | 75.6 | 69 | 57.7 | 73.8 |
| 50 | 79 | 83 | 77.2 | 79.3 | 84.6 | 77 |
| 51 | 77.8 | 77.4 | 78 | 77 | 76.9 | 77 |
| 52 | 73.9 | 81.1 | 70.7 | 74.7 | 73.1 | 75.4 |
| 53 | 71 | 81.1 | 66.7 | 71.3 | 69.2 | 72.1 |
| 54 | 73.9 | 67.9 | 76.4 | 79.3 | 73.1 | 82 |
| 55 | 78.4 | 62.3 | 85.4 | 74.7 | 61.5 | 80.3 |
| 56 | 73.9 | 67.9 | 76.4 | 72.4 | 73.1 | 72.1 |
| 57 | 77.3 | 86.8 | 73.2 | 74.7 | 80.8 | 72.1 |
| 58 | 73.9 | 73.6 | 74 | 69 | 57.7 | 73.8 |
| 59 | 71.6 | 81.1 | 67.5 | 78.2 | 73.1 | 80.3 |
| 60 | 76.1 | 71.7 | 78 | 77 | 69.2 | 80.3 |
| 61 | 72.7 | 75.5 | 71.5 | 78.2 | 84.6 | 75.4 |
| 62 | 69.9 | 71.7 | 69.1 | 73.6 | 73.1 | 73.8 |
| 63 | 74.4 | 86.8 | 69.1 | 72.4 | 73.1 | 72.1 |
| 64 | 71.6 | 75.5 | 69.9 | 66.7 | 80.8 | 60.7 |
| 65 | 69.9 | 67.9 | 70.7 | 67.8 | 73.1 | 65.6 |
| 66 | 67.6 | 62.3 | 69.9 | 73.6 | 69.2 | 75.4 |
| 67 | 72.2 | 73.6 | 71.5 | 66.7 | 69.2 | 65.6 |
| 68 | 72.2 | 71.7 | 72.4 | 63.2 | 57.7 | 65.6 |
| 69 | 68.2 | 79.2 | 63.4 | 72.4 | 80.8 | 68.9 |
| 70 | 68.8 | 75.5 | 65.9 | 62.1 | 65.4 | 60.7 |
| 71 | 71 | 62.3 | 74.8 | 73.3 | 68 | 75.4 |
| 72 | 67 | 75.5 | 63.4 | 70.1 | 80.8 | 65.6 |
| 73 | 68.8 | 62.3 | 71.5 | 70.1 | 69.2 | 70.5 |
| 74 | 68.8 | 69.8 | 68.3 | 66.7 | 73.1 | 63.9 |
| 75 | 69.3 | 66 | 70.7 | 74.7 | 80.8 | 72.1 |
| 76 | 72.7 | 69.8 | 74 | 73.6 | 57.7 | 80.3 |
| 77 | 64.8 | 73.6 | 61 | 58.6 | 73.1 | 52.5 |
| 78 | 65.9 | 66 | 65.9 | 73.6 | 73.1 | 73.8 |
| 79 | 72.7 | 83 | 68.3 | 60.9 | 46.2 | 67.2 |
| 80 | 64.8 | 69.8 | 62.6 | 64.4 | 53.8 | 68.9 |
| 81 | 64.2 | 69.8 | 61.8 | 66.7 | 76.9 | 62.3 |
| 82 | 66.5 | 69.8 | 65 | 69 | 76.9 | 65.6 |
| 83 | 60.8 | 52.8 | 64.2 | 70.1 | 65.4 | 72.1 |

**[Table 4]**

| SEQ ID NO: | Discriminant coefficient | Constant term |
|---|---|---|
| 1 | 3.10 | 39.85 |
| 2 | 2.84 | 16.65 |
| 3 | 2.22 | 13.44 |
| 4 | 1.76 | 10.14 |
| 5 | 2.75 | 16.32 |
| 6 | 3.19 | 22.93 |
| 7 | 6.93 | 64.26 |
| 8 | 3.85 | 31.55 |
| 9 | 2.35 | 17.01 |
| 10 | 2.72 | 16.12 |
| 11 | 3.83 | 24.06 |
| 12 | 3.64 | 27.80 |
| 13 | 3.40 | 27.68 |
| 14 | 3.50 | 36.83 |
| 15 | 1.68 | 9.99 |
| 16 | 2.03 | 13.73 |
| 17 | 2.79 | 19.34 |
| 18 | 2.79 | 17.67 |
| 19 | 3.46 | 23.85 |
| 20 | 2.16 | 12.27 |
| 21 | 1.69 | 11.03 |
| 22 | 2.22 | 19.50 |
| 23 | 1.82 | 14.51 |
| 24 | 2.08 | 13.00 |
| 25 | 2.36 | 17.76 |
| 26 | 4.24 | 50.31 |
| 27 | 2.19 | 15.19 |
| 28 | 2.97 | 17.23 |
| 29 | 2.73 | 15.72 |
| 30 | 4.77 | 60.37 |
| 31 | 2.88 | 16.50 |
| 32 | 3.36 | 40.40 |
| 33 | 4.45 | 47.32 |
| 34 | 3.10 | 26.84 |
| 35 | 2.73 | 22.91 |
| 36 | 3.64 | 26.76 |
| 37 | 2.04 | 21.42 |
| 38 | 3.84 | 30.11 |
| 39 | 2.80 | 20.07 |
| 40 | 3.78 | 31.18 |
| 41 | 3.93 | 28.97 |
| 42 | 3.87 | 27.95 |
| 43 | 3.61 | 27.20 |
| 44 | 4.63 | 58.85 |
| 45 | 1.91 | 10.91 |
| 46 | 2.27 | 24.74 |
| 47 | 2.57 | 15.29 |
| 48 | 5.47 | 46.73 |
| 49 | 1.56 | 10.17 |
| 50 | 1.61 | 10.45 |
| 51 | 1.31 | 7.87 |
| 52 | 3.03 | 21.43 |
| 53 | 1.93 | 11.42 |
| 54 | 3.60 | 47.33 |
| 55 | 2.26 | 18.49 |
| 56 | 2.73 | 29.67 |
| 57 | 1.87 | 14.47 |
| 58 | 2.21 | 18.13 |
| 59 | 2.26 | 13.31 |
| 60 | 5.71 | 76.75 |
| 61 | 2.63 | 15.46 |
| 62 | 1.12 | 6.78 |
| 63 | 1.37 | 8.50 |
| 64 | 3.59 | 30.25 |
| 65 | 2.51 | 15.04 |
| 66 | 4.85 | 54.20 |
| 67 | 2.42 | 15.94 |
| 68 | 2.46 | 21.44 |
| 69 | 3.86 | 43.50 |
| 70 | 2.76 | 28.72 |
| 71 | 1.34 | 13.78 |
| 72 | 2.57 | 15.69 |
| 73 | 2.33 | 14.12 |
| 74 | 2.22 | 13.04 |
| 75 | 1.22 | 6.90 |
| 76 | 1.12 | 6.52 |
| 77 | 1.35 | 7.66 |
| 78 | 2.49 | 14.91 |
| 79 | 2.22 | 17.09 |
| 80 | 2.59 | 17.43 |
| 81 | 2.64 | 17.94 |
| 82 | 1.95 | 11.84 |
| 83 | 3.21 | 44.93 |

**[Table 5-1]**

| Training cohort | | | | |
|---|---|---|---|---|
| Sample name | Disease | Stage of progression | CEA(ng/mL) | CA19-9(U/mL) |
| IP013 | IPMC | | 4.6 | 66.6 |
| IP023 | IPMC | | 4.7 | 8 |
| IP063 | IPMC | | 3 | 193.4 |
| IP147 | IPMC | | 3.2 | 183.3 |
| IP148 | IPMC | | 11.9 | 1158 |
| IP149 | IPMA | high grade | 3.6 | 35.6 |
| IP151 | IPMA | high grade | 2.3 | 0.1 |
| IP153 | IPMA | low grade | 3.5 | 4.4 |
| IP154 | IPMA | low grade | 1.8 | 7.2 |
| IP157 | IPMA | high grade | 4 | 0.1 |
| IP159 | IPMC | | 6.1 | 22.8 |
| IP160 | IPMC | | 2.8 | 26.1 |
| IP161 | IPMC | | 2.7 | 16.8 |
| IP166 | IPMA | low grade | 1.1 | 2.9 |
| IP167 | IPMA | high grade | 4.5 | 0.6 |
| IP168 | IPMA | low grade | 2.4 | 17 |
| IP172 | IPMC | | 6.2 | 60.2 |
| IP175 | IPMC | | 75 | 2246 |
| IP178 | IPMC | | 1.9 | 30 |
| IP182 | IPMA | high grade | | |
| IP205 | | | | |
| P_06 | | 2b | 1.7 | 435.1 |
| P_07 | | 2b | 1 | 149.8 |
| P_09 | | 2b | 3.5 | 22100 |
| P_10 | | 2b | 0.8 | 62 |
| P_11 | | 2a | 2.7 | 0.4 |
| P_13 | | 2a | 4.6 | 66.6 |
| P_15 | | 2a | 2.4 | 735 |
| P_18 | | 2a | 1.9 | 33.6 |
| P_19 | | 2b | 8.9 | 47.1 |
| P_20 | | 2b | 1.7 | 30.3 |
| P_21 | | 2b | 14.8 | 22.3 |
| P_25 | IPMC Early pancreatic cancer | 2b | 12.1 | 3274 |
| P_27 | | 2b | 4.6 | 282.8 |
| P_29 | | 2a | 1.5 | 37.8 |
| P_30 | | 2a | 1.7 | 20.9 |
| P_31 | | 2a | 2.1 | 387.9 |
| P_32 | | 2a | 1.1 | 15.4 |
| P_33 | | 2b | 3.1 | 160.6 |
| P_35 | | 2b | 0.3 | 9.9 |
| P_40 | | 2b | 1.4 | 221.5 |
| P_42 | | 2b | 34.6 | 168.8 |
| P_45 | | 2b | 9.1 | 569 |
| P_46 | | 2b | 3.3 | 80.5 |
| P_47 | | 2b | 1.8 | 97.5 |
| P_48 | | 2b | 3.1 | 405.7 |
| P_50 | | 2b | 0.9 | 8.1 |
| P_51 | | 2a | 2.3 | 2118 |
| P_54 | | 2b | 1.9 | 250.4 |
| P_56 | | 2b | 3.3 | 1870 |
| P_59 | | 2b | 2.1 | 95.1 |
| P_60 | | 2b | 3.4 | 79.2 |
| Sensitivity (%) | | | 18 | 58 |

**[Table 5-2]**

| Validation cohort | | | | |
|---|---|---|---|---|
| Sample name | Disease | Stage of progression | CEA(ng/mL) | CA19-9(U/mL) |
| IP008 | IPMC | | 1.8 | 19.5 |
| IP155 | IPMA | low grade | 1.9 | 18.6 |
| IP156 | IPMA | high grade | 0.7 | 8.8 |
| IP162 | IPMA | high grade | 1.5 | 10.9 |
| IP163 | IPMC | | 3 | 103.4 |
| IP164 | IPMA | high grade | 3.5 | 122.9 |
| IP165 | IPMC | | 5.6 | 401.7 |
| IP169 | IPMC | | 3.1 | 1143 |
| IP170 | IPMA | low grade | 1 | 7.1 |
| IP173 | IPMC | | 2.7 | 51.3 |
| IP174 | IPMC | | 3.9 | 23.2 |
| IP176 | IPMA | low grade | 2.2 | 28.3 |
| P_01 | | 2b | 4.5 | 55.9 |
| P_02 | | 2b | 2.6 | 327.6 |
| P_04 | | 2b | 4.1 | 950 |
| P_14 | | 2b | 3.2 | 137.8 |
| P_28 | | 2a | 3.8 | 348.8 |
| P_36 | Early pancreatic cancer | 2b | 5.2 | 1251 |
| P_52 | | 2b | 2.8 | 41.6 |
| P_53 | | 2b | 4 | 279.9 |
| P_55 | | 2a | 6.4 | 2848 |
| P_57 | | 2b | 5.9 | 454.3 |
| P_58 | | 2a | 2.1 | 0.1 |
| P_61 | | 2b | 10.3 | 283.5 |
| P_62 | | 2b | 4.2 | 268 |
| Sensitivity (%) | | | 20 | 68 |

**[Table 6]**

| | Training cohort | | | Validation cohort | | |
|---|---|---|---|---|---|---|
| SEQ ID NO: | Accuracy (%) | Sensitivity (%) | Specificity (%) | Accuracy (%) | Sensitivity (%) | Specificity (%) |
| 1_2 | 90.3 | 98.1 | 87 | 87.4 | 92.3 | 85.2 |
| 1_3 | 96.6 | 96.2 | 96.7 | 94.3 | 88.5 | 96.7 |
| 1_4 | 90.3 | 96.2 | 87.8 | 85.1 | 84.6 | 85.2 |
| 1_5 | 92.6 | 96.2 | 91.1 | 87.4 | 88.5 | 86.9 |
| 1_6 | 89.8 | 96.2 | 87 | 83.9 | 84.6 | 83.6 |
| 1_7 | 90.9 | 94.3 | 89.4 | 86.2 | 88.5 | 85.2 |
| 1_8 | 93.2 | 96.2 | 91.9 | 89.7 | 84.6 | 91.8 |
| 1_9 | 89.8 | 96.2 | 87 | 85.1 | 80.8 | 86.9 |
| 1_10 | 93.2 | 94.3 | 92.7 | 88.5 | 88.5 | 88.5 |
| 1_11 | 93.8 | 96.2 | 92.7 | 87.4 | 84.6 | 88.5 |
| 1_12 | 89.8 | 96.2 | 87 | 86.2 | 84.6 | 86.9 |
| 1_13 | 90.3 | 96.2 | 87.8 | 85.1 | 84.6 | 85.2 |
| 1_14 | 89.8 | 96.2 | 87 | 83.9 | 80.8 | 85.2 |
| 1_15 | 94.3 | 92.5 | 95.1 | 90.8 | 80.8 | 95.1 |
| 1_16 | 89.8 | 96.2 | 87 | 85.1 | 84.6 | 85.2 |
| 1_17 | 92 | 96.2 | 90.2 | 83.9 | 84.6 | 83.6 |
| 1_18 | 95.5 | 94.3 | 95.9 | 89.7 | 84.6 | 91.8 |
| 1_19 | 92.6 | 94.3 | 91.9 | 88.5 | 88.5 | 88.5 |
| 1_20 | 95.5 | 94.3 | 95.9 | 93.1 | 88.5 | 95.1 |
| 1_21 | 93.7 | 90.4 | 95.1 | 94.3 | 88.5 | 96.7 |
| 1_22 | 89.2 | 94.3 | 87 | 81.6 | 76.9 | 83.6 |
| 1_23 | 90.3 | 96.2 | 87.8 | 85.1 | 84.6 | 85.2 |
| 1_24 | 95.5 | 94.3 | 95.9 | 90.8 | 88.5 | 91.8 |
| 1_25 | 90.9 | 96.2 | 88.6 | 83.9 | 84.6 | 83.6 |
| 1_26 | 90.9 | 94.3 | 89.4 | 83.9 | 80.8 | 85.2 |
| 1_27 | 90.3 | 96.2 | 87.8 | 85.1 | 84.6 | 85.2 |
| 1_28 | 94.3 | 94.3 | 94.3 | 95.4 | 96.2 | 95.1 |
| 1_29 | 89.8 | 96.2 | 87 | 86 | 80.8 | 88.3 |
| 1_30 | 90.3 | 96.2 | 87.8 | 85.1 | 84.6 | 85.2 |
| 1_31 | 90.9 | 96.2 | 88.6 | 87.4 | 84.6 | 88.5 |
| 1_32 | 89.2 | 96.2 | 86.2 | 85.1 | 84.6 | 85.2 |
| 1_33 | 90.3 | 96.2 | 87.8 | 85.1 | 84.6 | 85.2 |
| 1_34 | 89.8 | 96.2 | 87 | 85.1 | 84.6 | 85.2 |
| 1_35 | 92 | 94.3 | 91.1 | 87.4 | 88.5 | 86.9 |
| 1_36 | 89.8 | 96.2 | 87 | 85.1 | 84.6 | 85.2 |
| 1_37 | 88.6 | 96.2 | 85.4 | 85.1 | 84.6 | 85.2 |
| 1_38 | 89.8 | 96.2 | 87 | 85.1 | 84.6 | 85.2 |
| 1_39 | 93.2 | 94.3 | 92.7 | 85.1 | 80.8 | 86.9 |
| 1_40 | 92.6 | 94.3 | 91.9 | 83.9 | 80.8 | 85.2 |
| 1_41 | 89.8 | 96.2 | 87 | 83.9 | 88.5 | 82 |
| 1_42 | 90.3 | 96.2 | 87.8 | 88.5 | 88.5 | 88.5 |
| 1_43 | 89.8 | 96.2 | 87 | 85.1 | 84.6 | 85.2 |
| 1_44 | 90.3 | 96.2 | 87.8 | 85.1 | 84.6 | 85.2 |
| 1_45 | 89.2 | 96.2 | 86.2 | 83.9 | 84.6 | 83.6 |
| 1_46 | 91.5 | 96.2 | 89.4 | 85.1 | 84.6 | 85.2 |
| 1_47 | 90.9 | 94.3 | 89.4 | 82.8 | 80.8 | 83.6 |
| 1_48 | 92 | 94.3 | 91.1 | 82.8 | 80.8 | 83.6 |
| 1_49 | 90.3 | 96.2 | 87.8 | 85.1 | 84.6 | 85.2 |
| 1_50 | 94.3 | 92.5 | 95.1 | 90.8 | 84.6 | 93.4 |
| 1_51 | 89.2 | 96.2 | 86.2 | 85.1 | 84.6 | 85.2 |
| 1_52 | 92 | 94.3 | 91.1 | 85.1 | 84.6 | 85.2 |
| 1_53 | 93.2 | 94.3 | 92.7 | 88.5 | 84.6 | 90.2 |
| 1_54 | 91.5 | 96.2 | 89.4 | 85.1 | 84.6 | 85.2 |
| 1_55 | 89.2 | 96.2 | 86.2 | 85.1 | 84.6 | 85.2 |
| 1_56 | 90.3 | 96.2 | 87.8 | 85.1 | 84.6 | 85.2 |
| 1_57 | 89.8 | 96.2 | 87 | 82.8 | 84.6 | 82 |
| 1_58 | 90.3 | 96.2 | 87.8 | 85.1 | 84.6 | 85.2 |
| 1_59 | 90.9 | 94.3 | 89.4 | 85.1 | 84.6 | 85.2 |
| 1_60 | 90.3 | 96.2 | 87.8 | 85.1 | 84.6 | 85.2 |
| 1_61 | 93.2 | 94.3 | 92.7 | 89.7 | 84.6 | 91.8 |
| 1_62 | 93.8 | 92.5 | 94.3 | 93.1 | 88.5 | 95.1 |
| 1_63 | 94.9 | 94.3 | 95.1 | 89.7 | 80.8 | 93.4 |
| 1_64 | 91.5 | 94.3 | 90.2 | 82.8 | 80.8 | 83.6 |
| 1_65 | 92.6 | 94.3 | 91.9 | 89.7 | 84.6 | 91.8 |
| 1_66 | 89.2 | 96.2 | 86.2 | 85.1 | 84.6 | 85.2 |
| 1_67 | 89.2 | 96.2 | 86.2 | 85.1 | 84.6 | 85.2 |
| 1_68 | 89.8 | 96.2 | 87 | 85.1 | 84.6 | 85.2 |
| 1_69 | 93.2 | 94.3 | 92.7 | 87.4 | 84.6 | 88.5 |
| 1_70 | 89.8 | 94.3 | 87.8 | 85.1 | 84.6 | 85.2 |
| 1_71 | 89.8 | 96.2 | 87 | 84.9 | 84 | 85.2 |
| 1_72 | 93.2 | 94.3 | 92.7 | 90.8 | 84.6 | 93.4 |
| 1_73 | 89.8 | 96.2 | 87 | 83.9 | 84.6 | 83.6 |
| 1_74 | 92 | 94.3 | 91.1 | 87.4 | 88.5 | 86.9 |
| 1_75 | 92 | 96.2 | 90.2 | 83.9 | 76.9 | 86.9 |
| 1_76 | 90.3 | 96.2 | 87.8 | 85.1 | 84.6 | 85.2 |
| 1_77 | 91.5 | 96.2 | 89.4 | 83.9 | 84.6 | 83.6 |
| 1_78 | 90.9 | 96.2 | 88.6 | 83.9 | 84.6 | 83.6 |
| 1_79 | 89.8 | 96.2 | 87 | 83.9 | 84.6 | 83.6 |
| 1_80 | 91.5 | 96.2 | 89.4 | 85.1 | 84.6 | 85.2 |
| 1_81 | 93.2 | 94.3 | 92.7 | 87.4 | 84.6 | 88.5 |
| 1_82 | 94.3 | 94.3 | 94.3 | 86.2 | 80.8 | 88.5 |
| 1_83 | 91.5 | 96.2 | 89.4 | 85.1 | 84.6 | 85.2 |
| 1_84 | 95.5 | 100 | 93.5 | 90.8 | 96.2 | 88.5 |
| 1_85 | 97.2 | 100 | 95.9 | 97.7 | 100 | 96.7 |
| 1_86 | 96.6 | 96.2 | 96.7 | 93.1 | 84.6 | 96.7 |
| 1_87 | 96.6 | 100 | 95.1 | 95.4 | 100 | 93.4 |
| 1_88 | 96.6 | 98.1 | 95.9 | 93.1 | 92.3 | 93.4 |
| 1_89 | 96 | 98.1 | 95.1 | 89.7 | 88.5 | 90.2 |
| 1_90 | 92.6 | 100 | 89.4 | 92 | 100 | 88.5 |
| 1_91 | 94.9 | 96.2 | 94.3 | 90.8 | 88.5 | 91.8 |
| 1_92 | 94.9 | 92.5 | 95.9 | 95.4 | 96.2 | 95.1 |
| 1_93 | 93.2 | 96.2 | 91.9 | 88.5 | 84.6 | 90.2 |
| 1_94 | 94.3 | 96.2 | 93.5 | 90.8 | 84.6 | 93.4 |
| 1_95 | 89.2 | 96.2 | 86.2 | 85.1 | 84.6 | 85.2 |
| 1_96 | 94.3 | 92.5 | 95.1 | 90.8 | 84.6 | 93.4 |
| 1_97 | 94.3 | 96.2 | 93.5 | 90.8 | 96.2 | 88.5 |
| 1_98 | 95.5 | 96.2 | 95.1 | 93.1 | 88.5 | 95.1 |
| 1_99 | 92 | 96.2 | 90.2 | 85.1 | 88.5 | 83.6 |
| 1_100 | 89.2 | 96.2 | 86.2 | 85.1 | 84.6 | 85.2 |
| 1_101 | 90.3 | 96.2 | 87.8 | 88.5 | 88.5 | 88.5 |
| 1_102 | 96 | 98.1 | 95.1 | 90.8 | 92.3 | 90.2 |
| 1_103 | 92 | 96.2 | 90.2 | 87.4 | 84.6 | 88.5 |
| 1_104 | 94.3 | 94.3 | 94.3 | 87.4 | 80.8 | 90.2 |
| 1_105 | 94.9 | 94.3 | 95.1 | 94.3 | 88.5 | 96.7 |
| 1_106 | 88.6 | 96.2 | 85.4 | 87.2 | 88 | 86.9 |
| 1_107 | 94.3 | 94.3 | 94.3 | 85.1 | 76.9 | 88.5 |
| 1_108 | 89.2 | 96.2 | 86.2 | 83.9 | 80.8 | 85.2 |
| 1_109 | 96 | 94.3 | 96.7 | 94.3 | 88.5 | 96.7 |
| 1_110 | 88.6 | 96.2 | 85.4 | 85.1 | 84.6 | 85.2 |
| 1_111 | 93.8 | 96.2 | 92.7 | 89.7 | 80.8 | 93.4 |
| 1_112 | 90.3 | 96.2 | 87.8 | 86.2 | 80.8 | 88.5 |
| 1_113 | 92 | 96.2 | 90.2 | 87.4 | 88.5 | 86.9 |
| 1_114 | 90.3 | 96.2 | 87.8 | 85.1 | 84.6 | 85.2 |
| 1_115 | 93.8 | 96.2 | 92.7 | 92 | 96.2 | 90.2 |
| 1_116 | 92 | 96.2 | 90.2 | 88.5 | 80.8 | 91.8 |
| 1_117 | 96.6 | 96.2 | 96.7 | 88.5 | 88.5 | 88.5 |
| 1_118 | 93.2 | 94.3 | 92.7 | 87.4 | 88.5 | 86.9 |
| 1_119 | 89.8 | 96.2 | 87 | 87.4 | 84.6 | 88.5 |
| 1_120 | 89.8 | 96.2 | 87 | 85.1 | 84.6 | 85.2 |
| 1_121 | 93.8 | 94.3 | 93.5 | 92 | 96.2 | 90.2 |
| 1_122 | 92 | 96.2 | 90.2 | 85.1 | 88.5 | 83.6 |
| 1_123 | 90.3 | 94.3 | 88.6 | 89.7 | 84.6 | 91.8 |
| 1_124 | 88.6 | 96.2 | 85.4 | 83.9 | 84.6 | 83.6 |
| 1_125 | 96 | 92.5 | 97.6 | 92 | 92.3 | 91.8 |
| 1_126 | 89.2 | 96.2 | 86.2 | 86.2 | 88.5 | 85.2 |
| 1_127 | 92 | 94.3 | 91.1 | 83.9 | 80.8 | 85.2 |
| 1_128 | 88.6 | 96.2 | 85.4 | 85.1 | 84.6 | 85.2 |
| 1_129 | 94.9 | 100 | 92.7 | 94.3 | 96.2 | 93.4 |
| 1_130 | 89.2 | 96.2 | 86.2 | 86.2 | 88.5 | 85.2 |
| 1_131 | 90.9 | 96.2 | 88.6 | 87.4 | 88.5 | 86.9 |
| 1_132 | 91.5 | 94.3 | 90.2 | 87.4 | 88.5 | 86.9 |
| 1_133 | 92 | 96.2 | 90.2 | 87.4 | 88.5 | 86.9 |
| 1_134 | 91.5 | 96.2 | 89.4 | 85.1 | 84.6 | 85.2 |
| 1_135 | 90.9 | 96.2 | 88.6 | 88.5 | 88.5 | 88.5 |
| 1_136 | 88.1 | 96.2 | 84.6 | 83.9 | 84.6 | 83.6 |
| 1_137 | 90.9 | 96.2 | 88.6 | 88.5 | 84.6 | 90.2 |
| 1_138 | 89.2 | 96.2 | 86.2 | 85.1 | 84.6 | 85.2 |
| 1_139 | 90.3 | 96.2 | 87.8 | 86.2 | 88.5 | 85.2 |
| 1_140 | 92.6 | 96.2 | 91.1 | 87.4 | 88.5 | 86.9 |
| 1_141 | 96 | 100 | 94.3 | 92 | 96.2 | 90.2 |
| 1_142 | 92 | 96.2 | 90.2 | 85.1 | 84.6 | 85.2 |
| 1_143 | 93.8 | 94.3 | 93.5 | 92 | 84.6 | 95.1 |
| 1_144 | 89.8 | 94.3 | 87.8 | 85.1 | 84.6 | 85.2 |
| 1_145 | 92.6 | 94.3 | 91.9 | 86.2 | 84.6 | 86.9 |
| 1_146 | 90.3 | 96.2 | 87.8 | 85.1 | 84.6 | 85.2 |
| 1_147 | 92.6 | 96.2 | 91.1 | 89.7 | 96.2 | 86.9 |
| 1_148 | 89.8 | 96.2 | 87 | 85.1 | 84.6 | 85.2 |
| 1_149 | 89.2 | 96.2 | 86.2 | 85.1 | 84.6 | 85.2 |
| 1_150 | 92.6 | 94.3 | 91.9 | 86.2 | 88.5 | 85.2 |
| 1_151 | 90.3 | 96.2 | 87.8 | 85.1 | 84.6 | 85.2 |
| 1_152 | 93.2 | 96.2 | 91.9 | 89.7 | 88.5 | 90.2 |
| 1_153 | 89.8 | 96.2 | 87 | 87.4 | 84.6 | 88.5 |
| 1_154 | 89.2 | 96.2 | 86.2 | 85.1 | 88.5 | 83.6 |
| 1_155 | 91.4 | 96.2 | 89.3 | 85.1 | 84.6 | 85.2 |
| 1_156 | 89.2 | 96.2 | 86.2 | 85.1 | 84.6 | 85.2 |
| 1_157 | 91.5 | 96.2 | 89.4 | 83.9 | 84.6 | 83.6 |
| 1_158 | 90.3 | 94.3 | 88.6 | 85.1 | 88.5 | 83.6 |
| 1_159 | 89.8 | 96.2 | 87 | 85.1 | 84.6 | 85.2 |
| 1_160 | 92 | 94.3 | 91.1 | 85.1 | 80.8 | 86.9 |
| 1_161 | 96 | 94.3 | 96.7 | 92 | 88.5 | 93.4 |
| 1_162 | 89.8 | 96.2 | 87 | 85.1 | 84.6 | 85.2 |
| 1_163 | 90.3 | 96.2 | 87.8 | 85.1 | 84.6 | 85.2 |
| 1_164 | 93.8 | 94.3 | 93.5 | 90.8 | 84.6 | 93.4 |
| 1_165 | 90.3 | 96.2 | 87.8 | 85.1 | 84.6 | 85.2 |
| 1_166 | 94.3 | 92.5 | 95.1 | 90.8 | 84.6 | 93.4 |
| 1_167 | 89.8 | 96.2 | 87 | 85.1 | 84.6 | 85.2 |
| 1_168 | 93.2 | 96.2 | 91.9 | 88.5 | 88.5 | 88.5 |
| 1_169 | 89.8 | 96.2 | 87 | 85.1 | 88.5 | 83.6 |
| 1_170 | 89.8 | 96.2 | 87 | 82.8 | 84.6 | 82 |
| 1_171 | 89.8 | 96.2 | 87 | 85.1 | 84.6 | 85.2 |
| 1_172 | 90.3 | 96.2 | 87.8 | 85.1 | 84.6 | 85.2 |
| 1_173 | 92 | 96.2 | 90.2 | 86.2 | 88.5 | 85.2 |
| 1_174 | 91.4 | 96.2 | 89.4 | 86.2 | 88.5 | 85.2 |
| 1_175 | 89.8 | 96.2 | 87 | 85.1 | 84.6 | 85.2 |
| 1_176 | 89.2 | 96.2 | 86.2 | 85.1 | 84.6 | 85.2 |
| 1_177 | 90.3 | 96.2 | 87.8 | 86.2 | 88.5 | 85.2 |
| 1_178 | 92.6 | 94.3 | 91.9 | 85.1 | 84.6 | 85.2 |
| 1_179 | 91.5 | 96.2 | 89.4 | 87.4 | 88.5 | 86.9 |
| 1_180 | 90.3 | 94.3 | 88.6 | 83.9 | 84.6 | 83.6 |
| 1_181 | 92 | 96.2 | 90.2 | 86.2 | 88.5 | 85.2 |
| 1_182 | 91.5 | 94.3 | 90.2 | 87.4 | 88.5 | 86.9 |
| 1_183 | 89.8 | 96.2 | 87 | 85.1 | 84.6 | 85.2 |
| 1_184 | 89.2 | 96.2 | 86.2 | 85.1 | 84.6 | 85.2 |
| 1_185 | 89.8 | 96.2 | 87 | 85.1 | 84.6 | 85.2 |
| 1_186 | 88.6 | 96.2 | 85.4 | 85.1 | 84.6 | 85.2 |
| 1_187 | 90.3 | 94.3 | 88.6 | 85.1 | 84.6 | 85.2 |
| 1_188 | 88.6 | 96.2 | 85.4 | 85.1 | 84.6 | 85.2 |
| 1_189 | 89.2 | 94.3 | 87 | 82.8 | 80.8 | 83.6 |
| 1_190 | 93.2 | 94.3 | 92.7 | 88.5 | 84.6 | 90.2 |
| 1_191 | 89.8 | 92.5 | 88.6 | 86.2 | 88.5 | 85.2 |
| 1_192 | 943 | 96.2 | 93.5 | 89.7 | 88.5 | 90.2 |
| 1_193 | 88.6 | 94.3 | 86.2 | 83.9 | 84.6 | 83.6 |
| 1_194 | 92.6 | 92.5 | 92.7 | 90.8 | 84.6 | 93.4 |
| 1_195 | 94.3 | 96.2 | 93.5 | 86.2 | 76.9 | 90.2 |
| 1_196 | 92 | 94.3 | 91.1 | 86.2 | 88.5 | 85.2 |
| 1_197 | 89.8 | 96.2 | 87 | 82.8 | 84.6 | 82 |
| 1_198 | 89.8 | 96.2 | 87 | 85.1 | 84.6 | 85.2 |
| 1_199 | 92.6 | 96.2 | 91.1 | 90.8 | 80.8 | 95.1 |
| 1_200 | 90.3 | 96.2 | 87.8 | 85.1 | 84.6 | 85.2 |
| 1_201 | 90.9 | 96.2 | 88.6 | 85.1 | 84.6 | 85.2 |
| 1_202 | 89.8 | 96.2 | 87 | 85.1 | 84.6 | 85.2 |
| 1_203 | 89.8 | 96.2 | 87 | 85.1 | 80.8 | 86.9 |
| 1_204 | 91.5 | 96.2 | 89.4 | 85.1 | 84.6 | 85.2 |
| 1_205 | 93.2 | 92.5 | 93.5 | 89.7 | 88.5 | 90.2 |
| 1_206 | 89.2 | 96.2 | 86.2 | 85.1 | 84.6 | 85.2 |
| 1_207 | 92 | 96.2 | 90.2 | 89.7 | 84.6 | 91.8 |
| 1_208 | 89.8 | 94.3 | 87.8 | 85.1 | 84.6 | 85.2 |
| 1_209 | 89.2 | 96.2 | 86.2 | 82.8 | 84.6 | 82 |
| 1_210 | 93.2 | 96.2 | 91.9 | 88.5 | 84.6 | 90.2 |
| 1_211 | 90.9 | 96.2 | 88.6 | 85.1 | 84.6 | 85.2 |
| 1_212 | 90.9 | 96.2 | 88.6 | 85.1 | 84.6 | 85.2 |
| 1_213 | 89.8 | 96.2 | 87 | 85.1 | 84.6 | 85.2 |
| 1_214 | 89.2 | 94.3 | 87 | 85.1 | 84.6 | 85.2 |
| 1_215 | 89.8 | 96.2 | 87 | 85.1 | 84.6 | 85.2 |
| 1_216 | 89.1 | 96.2 | 86.2 | 85.1 | 84.6 | 85.2 |
| 1_217 | 88.6 | 96.2 | 85.4 | 83.9 | 84.6 | 83.6 |
| 1_218 | 89.8 | 96.2 | 87 | 85.1 | 84.6 | 85.2 |
| 1_219 | 90.3 | 96.2 | 87.8 | 85.1 | 84.6 | 85.2 |
| 1_220 | 90.3 | 96.2 | 87.8 | 85.1 | 84.6 | 85.2 |
| 1_221 | 93.8 | 94.3 | 93.5 | 86.2 | 84.6 | 86.9 |
| 1_222 | 89.8 | 94.3 | 87.8 | 85.1 | 84.6 | 85.2 |
| 1_223 | 91.4 | 96.2 | 89.3 | 85.1 | 84.6 | 85.2 |
| 1_224 | 88.6 | 96.2 | 85.4 | 85.1 | 84.6 | 85.2 |
| 1_225 | 90.9 | 96.2 | 88.6 | 85.1 | 84.6 | 85.2 |
| 1_226 | 91.5 | 96.2 | 89.4 | 85.1 | 84.6 | 85.2 |

**[Table 7]**

| | Training cohort | | | Validation cohort | | |
|---|---|---|---|---|---|---|
| SEQ ID NO: | Accuracy (%) | Sensitivity (%) | Specificity (%) | Accuracy (%) | Sensitivity (%) | Specificity (%) |
| 2_18 | 98.9 | 100 | 98.4 | 97.7 | 100 | 96.7 |
| 2_53 | 98.9 | 98.1 | 99.2 | 97.7 | 100 | 96.7 |
| 2_20 | 98.9 | 96.2 | 100 | 98.9 | 100 | 98.4 |
| 2_3 | 98.3 | 98.1 | 98.4 | 98.9 | 100 | 98.4 |
| 2_50 | 98.3 | 98.1 | 98.4 | 98.9 | 100 | 98.4 |
| 85_3 | 98.9 | 100 | 98.4 | 100 | 100 | 100 |
| 84_3 | 98.9 | 98.1 | 99.2 | 97.7 | 100 | 96.7 |
| 90_3 | 98.3 | 100 | 97.6 | 98.9 | 100 | 98.4 |
| 87_3 | 97.7 | 100 | 96.7 | 98.9 | 100 | 98.4 |
| 3_137 | 97.7 | 94.3 | 99.2 | 100 | 100 | 100 |
| 90_18 | 99.4 | 100 | 99.2 | 97.7 | 100 | 96.7 |
| 87_18 | 98.9 | 98.1 | 99.2 | 98.9 | 100 | 98.4 |
| 89_18 | 98.3 | 96.2 | 99.2 | 98.9 | 100 | 98.4 |
| 18_137 | 98.3 | 94.3 | 100 | 98.9 | 96.2 | 100 |
| 84_18 | 97.7 | 98.1 | 97.6 | 96.6 | 100 | 95.1 |
| 86_12 | 97.2 | 96.2 | 97.6 | 97.7 | 92.3 | 100 |
| 109 12 | 97.2 | 96.2 | 97.6 | 96.6 | 92.3 | 98.4 |
| 85_12 | 96.6 | 100 | 95.1 | 97.7 | 100 | 96.7 |
| 88_12 | 96.6 | 100 | 95.1 | 94.3 | 92.3 | 95.1 |
| 105_12 | 96.6 | 98.1 | 95.9 | 97.7 | 92.3 | 100 |
| 85_20 | 98.3 | 100 | 97.6 | 100 | 100 | 100 |
| 84_20 | 98.3 | 98.1 | 98.4 | 97.7 | 100 | 96.7 |
| 106_20 | 98.3 | 98.1 | 98.4 | 97.7 | 92 | 100 |
| 90_20 | 97.7 | 100 | 96.7 | 98.9 | 100 | 98.4 |
| 87_20 | 97.7 | 98.1 | 97.6 | 96.6 | 100 | 95.1 |
| 87_15 | 98.9 | 100 | 98.4 | 98.9 | 100 | 98.4 |
| 85_15 | 98.3 | 96.2 | 99.2 | 100 | 100 | 100 |
| 2_15 | 97.7 | 98.1 | 97.6 | 97.7 | 100 | 96.7 |
| 88_15 | 97.2 | 94.3 | 98.4 | 100 | 100 | 100 |
| 15_137 | 97.2 | 94.3 | 98.4 | 97.7 | 96.2 | 98.4 |
| 85_50 | 98.3 | 98.1 | 98.4 | 100 | 100 | 100 |
| 87_50 | 97.7 | 100 | 96.7 | 97.7 | 100 | 96.7 |
| 84_50 | 97.7 | 98.1 | 97.6 | 97.7 | 100 | 96.7 |
| 106_50 | 97.7 | 96.2 | 98.4 | 98.8 | 96 | 100 |
| 90_50 | 97.2 | 98.1 | 96.7 | 97.7 | 100 | 96.7 |
| 2_63 | 98.3 | 98.1 | 98.4 | 100 | 100 | 100 |
| 85_63 | 97.7 | 100 | 96.7 | 100 | 100 | 100 |
| 90_63 | 97.7 | 100 | 96.7 | 97.7 | 100 | 96.7 |
| 84_63 | 97.7 | 98.1 | 97.6 | 94.3 | 92.3 | 95.1 |
| 87_63 | 97.2 | 100 | 95.9 | 97.7 | 100 | 96.7 |
| 84_72 | 98.3 | 100 | 97.6 | 97.7 | 100 | 96.7 |
| 85_72 | 97.7 | 100 | 96.7 | 100 | 100 | 100 |
| 88_72 | 97.7 | 100 | 96.7 | 97.7 | 96.2 | 98.4 |
| 87_72 | 97.7 | 98.1 | 97.6 | 98.9 | 100 | 98.4 |
| 93_72 | 97.7 | 98.1 | 97.6 | 94.3 | 92.3 | 95.1 |
| 94_5 | 98.3 | 96.2 | 99.2 | 94.3 | 92.3 | 95.1 |
| 85_5 | 97.7 | 98.1 | 97.6 | 97.7 | 100 | 96.7 |
| 87 5 | 97.2 | 100 | 95.9 | 94.3 | 100 | 91.8 |
| 5_10/ | 97.2 | 94.3 | 98.4 | 93.1 | 84.6 | 96.7 |
| 84_5 | 96.6 | 100 | 95.1 | 93.1 | 96.2 | 91.8 |
| 85_24 | 97.2 | 94.3 | 98.4 | 100 | 100 | 100 |
| 87_24 | 96.6 | 94.3 | 97.6 | 97.7 | 100 | 96.7 |
| 89_24 | 96.6 | 94.3 | 97.6 | 96.6 | 92.3 | 98.4 |
| 90_24 | 96.6 | 92.5 | 98.4 | 98.9 | 100 | 98.4 |
| 102_24 | 96.6 | 92.5 | 98.4 | 97.7 | 100 | 96.7 |
| 85_10 | 97.7 | 96.2 | 98.4 | 100 | 100 | 100 |
| 87_10 | 97.7 | 94.3 | 99.2 | 98.9 | 100 | 98.4 |
| 90_10 | 97.2 | 96.2 | 97.6 | 96.6 | 96.2 | 96.7 |
| 88_10 | 97.2 | 92.5 | 99.2 | 100 | 100 | 100 |
| 2_10 | 96.6 | 94.3 | 97.6 | 96.6 | 96.2 | 96.7 |
| 85_52 | 98.3 | 100 | 97.6 | 97.7 | 100 | 96.7 |
| 88_52 | 98.3 | 100 | 97.6 | 100 | 100 | 100 |
| 87_52 | 97.7 | 100 | 96.7 | 94.3 | 100 | 91.8 |
| 98_52 | 97.7 | 98.1 | 97.6 | 96.6 | 96.2 | 96.7 |
| 84_52 | 97.2 | 98.1 | 96.7 | 94.3 | 96.2 | 93.4 |
| 87_9 | 98.3 | 100 | 97.6 | 95.4 | 100 | 93.4 |
| 89_9 | 97.7 | 100 | 96.7 | 93.1 | 92.3 | 93.4 |
| 85_9 | 97.2 | 100 | 95.9 | 96.6 | 96.2 | 96.7 |
| 117_9 | 97.2 | 98.1 | 96.7 | 97.7 | 100 | 96.7 |
| 88_9 | 96.6 | 98.1 | 95.9 | 94.3 | 96.2 | 93.4 |
| 87_11 | 98.3 | 100 | 97.6 | 95.4 | 100 | 93.4 |
| 85_11 | 98.3 | 98.1 | 98.4 | 98.9 | 100 | 98.4 |
| 89_11 | 983 | 98.1 | 98.4 | 931 | 92.3 | 93.4 |
| 102_11 | 98.3 | 98.1 | 98.4 | 98.9 | 100 | 98.4 |
| 84_11 | 97.7 | 98.1 | 97.6 | 94.3 | 96.2 | 93.4 |
| 85_19 | 98.9 | 100 | 98.4 | 98.9 | 96.2 | 100 |
| 87_19 | 98.3 | 100 | 97.6 | 97.7 | 100 | 96.7 |
| 88_19 | 98.3 | 100 | 97.6 | 98.9 | 96.2 | 100 |
| 89_19 | 97.7 | 100 | 96.7 | 97.7 | 96.2 | 98.4 |
| 106_19 | 97.2 | 98.1 | 96.7 | 95.3 | 92 | 96.7 |
| 87_39 | 98.9 | 100 | 98.4 | 98.9 | 100 | 98.4 |
| 85_39 | 97.7 | 98.1 | 97.6 | 98.9 | 96.2 | 100 |
| 88_39 | 97.2 | 96.2 | 97.6 | 96.6 | 92.3 | 98.4 |
| 84_39 | 96.6 | 98.1 | 95.9 | 94.3 | 92.3 | 95.1 |
| 2_39 | 96.6 | 98.1 | 95.9 | 97.7 | 100 | 96.7 |
| 87_61 | 97.2 | 100 | 95.9 | 98.9 | 100 | 98.4 |
| 85_61 | 96.6 | 98.1 | 95.9 | 100 | 100 | 100 |
| 88_61 | 96.6 | 98.1 | 95.9 | 100 | 100 | 100 |
| 108_61 | 96 | 94.3 | 96.7 | 86.2 | 73.1 | 91.8 |
| 93_61 | 96 | 92.5 | 97.6 | 94.3 | 84.6 | 98.4 |
| 88_7 | 97.7 | 100 | 96.7 | 100 | 100 | 100 |
| 85_7 | 97.2 | 100 | 95.9 | 100 | 100 | 100 |
| 87_7 | 97.2 | 100 | 95.9 | 97.7 | 100 | 96.7 |
| 86_7 | 97.2 | 96.2 | 97.6 | 97.7 | 92.3 | 100 |
| 91_7 | 96.6 | 96.2 | 96.7 | 98.9 | 100 | 98.4 |
| 85_17 | 98.3 | 100 | 97.6 | 97.7 | 100 | 96.7 |
| 87_17 | 97.7 | 100 | 96.7 | 95.4 | 100 | 93.4 |
| 89_17 | 96.6 | 94.3 | 97.6 | 94.3 | 88.5 | 96.7 |
| 102_17 | 96.6 | 94.3 | 97.6 | 97.7 | 96.2 | 98.4 |
| 84_17 | 96 | 100 | 94.3 | 93.1 | 92.3 | 93.4 |
| 85_22 | 98.9 | 100 | 98.4 | 97.7 | 96.2 | 98.4 |
| 87_22 | 98.9 | 100 | 98.4 | 98.9 | 100 | 98.4 |
| 102_22 | 97.7 | 96.2 | 98.4 | 94.3 | 84.6 | 98.4 |
| 89_22 | 97.2 | 98.1 | 96.7 | 93.1 | 88.5 | 95.1 |
| 117_22 | 97.2 | 96.2 | 97.6 | 95.4 | 96.2 | 95.1 |
| 85_26 | 98.9 | 100 | 98.4 | 96.6 | 96.2 | 96.7 |
| 87_26 | 98.3 | 100 | 97.6 | 96.6 | 100 | 95.1 |
| 88_26 | 97.2 | 100 | 95.9 | 95.4 | 92.3 | 96.7 |
| 84_26 | 96 | 100 | 94.3 | 95.4 | 100 | 93.4 |
| 94 26 | 96 | 98.1 | 95.1 | 90.8 | 88.5 | 91.8 |
| 85_74 | 98.9 | 100 | 98.4 | 100 | 100 | 100 |
| 2_74 | 97.7 | 100 | 96.7 | 97.7 | 100 | 96.7 |
| 87_74 | 97.2 | 100 | 95.9 | 97.7 | 100 | 96.7 |
| 84 74 | 96.6 | 96.2 | 96.7 | 95.4 | 100 | 93.4 |
| 88_74 | 96.6 | 96.2 | 96.7 | 97.7 | 100 | 96.7 |
| 90_21 | 97.1 | 94.2 | 98.4 | 98.9 | 100 | 98.4 |
| 2_21 | 97.1 | 94.2 | 98.4 | 97.7 | 96.2 | 98.4 |
| 106_21 | 97.1 | 92.3 | 99.2 | 97.7 | 92 | 100 |
| 84_21 | 96.6 | 94.2 | 97.6 | 96.6 | 100 | 95.1 |
| 85_21 | 96.6 | 92.3 | 98.4 | 100 | 100 | 100 |
| 85_28 | 97.7 | 98.1 | 97.6 | 100 | 100 | 100 |
| 84_28 | 97.2 | 96.2 | 97.6 | 96.6 | 100 | 95.1 |
| 86_28 | 96.6 | 94.3 | 97.6 | 96.6 | 92.3 | 98.4 |
| 87_28 | 96.6 | 94.3 | 97.6 | 98.9 | 100 | 98.4 |
| 93_28 | 96.6 | 92.5 | 98.4 | 100 | 100 | 100 |

### [Example 3]

### <Selection of gene markers using all samples and method for evaluating early pancreatic cancer or pancreatic cancer precursor lesion discriminant performance of acquired gene markers>

In this Example, the samples of the training cohort and the validation cohort used in Examples 1 and 2 were integrated, and selection of a gene marker and evaluation of its early pancreatic cancer or pancreatic cancer precursor lesion discriminant performance were conducted using all of the samples.

Specifically, the miRNA expression levels in the sera of the pancreatic cancer precursor lesion patients (33 persons), early pancreatic cancer patients (44 persons) and the healthy subjects (184 persons) obtained in the preceding Reference Examples were normalized by global normalization. In order to acquire diagnosis markers with higher reliability, only genes having a gene expression level of 2⁶ or higher in 50% or more of the samples in either of the early pancreatic cancer or pancreatic cancer precursor lesion patient group or the healthy subject group were selected in the gene marker selection. In order to further acquire statistical significance for discriminating an early pancreatic cancer or pancreatic cancer precursor lesion patient group from a healthy subject group, the P value obtained by two-tailed t-test assuming equal variance as to each gene expression level was corrected by the Bonferroni method, and genes that satisfied p < 0.01 were selected as gene markers for use in explanatory variables of a discriminant and described in Table 8. In this way, hsa-miR-6794-5p, hsa-miR-6511a-5p, hsa-miR-6824-5p, hsa-miR-762, hsa-miR-6836-3p, hsa-miR-6727-5p, hsa-miR-4739, hsa-miR-7977, hsa-miR-4484, hsa-miR-6515-3p, hsa-miR-373-5p, hsa-miR-4258, hsa-miR-4674, hsa-miR-3180, hsa-miR-6076, hsa-miR-1238-5p, hsa-miR-4463, hsa-miR-4486, and hsa-miR-4730 genes, and the nucleotide sequences of SEQ ID NOs: 227 to 245 related thereto were found in addition to the genes described in Table 2. As with the nucleotide sequences of SEQ ID NOs: 1 to 226, the results obtained about the polynucleotides shown in SEQ ID NOs: 227 to 245 also showed that the measurement values were significantly lower (decrease) or higher (increase) in the early pancreatic cancer or pancreatic cancer precursor lesion patient group than in the healthy subject group (Table 8). These results were able to be validated in the validation cohort. Thus, the presence or absence of early pancreatic cancer or pancreatic cancer precursor lesion in the newly obtained samples can be determined by the methods described in Examples 1 and 2 by using, alone or in combination, the gene expression level measurement values described in Table 8.

**[Table 8]**

| SEQ ID NO: | Gene name | P value after Bonferroni correction | Change in expression level in early pancreatic cancer and pancreatic cancer precursor lesion patient relative to healthy subject |
|---|---|---|---|
| 227 | hsa-miR-6794-5p | 1.19E-03 | Decrease |
| 228 | hsa-miR-6511a-5p | 1.44E-03 | Decrease |
| 229 | hsa-miR-6824-5p | 2.18E-03 | Decrease |
| 230 | hsa-miR-762 | 2.05E-07 | Increase |
| 231 | hsa-miR-6836-3p | 9.60E-07 | Increase |
| 232 | hsa-miR-6727-5p | 1.30E-06 | Increase |
| 233 | hsa-miR-4739 | 5.12E-06 | Increase |
| 234 | hsa-miR-7977 | 1.01E-05 | Decrease |
| 235 | hsa-miR-4484 | 1.38E-05 | Increase |
| 236 | hsa miR 6515 3p | 4.31E 04 | Decrease |
| 237 | hsa-miR-373-5p | 6.79E-04 | Increase |
| 238 | hsa-miR-4258 | 9.00E-04 | Increase |
| 239 | hsa-miR-4674 | 9.95E-04 | Increase |
| 240 | hsa-miR-3180 | 1.43E-03 | Increase |
| 241 | hsa-miR-6076 | 2.65E-03 | Decrease |
| 242 | hsa-miR-1238-5p | 2.77E-03 | Decrease |
| 243 | hsa-miR-4463 | 3.16E-03 | Increase |
| 244 | hsa-miR-4486 | 6.37E-03 | Increase |
| 245 | hsa-miR-4730 | 9.34E-03 | Increase |

### [Example 4]

### <Method for evaluating early pancreatic cancer or pancreatic cancer precursor lesion-specific discriminant performance by combination of plurality of gene markers using samples of validation cohort>

In this Example, gene expression levels of miRNAs in sera were compared between a pancreatic cancer precursor lesion patient group and an early pancreatic cancer patient group as positive control groups and a healthy subject group, an advanced pancreatic cancer patient group, a bile duct cancer patient group, a breast cancer patient group, a prostate cancer patient group, a colorectal cancer patient group, a stomach cancer patient group, an esophageal cancer patient group, a liver cancer patient group, a benign pancreatic disease patient group, and a benign prostatic disease patient group as negative control groups in the same way as the method described in Example 1 with respect to the training cohort as the sample group described in Reference Example 2 to select an additional gene marker for diagnosis. The additional gene marker for diagnosis (SEQ ID NOs: 246 to 247) thus selected was combined with the gene markers selected in Example 1 to study a method for evaluating early pancreatic cancer or pancreatic cancer precursor lesion-specific discriminant performance.

Specifically, first, the miRNA expression levels of the training cohort and the validation cohort obtained in Reference Example 2 mentioned above were combined and normalized by global normalization. Next, Fisher's discriminant analysis was conducted as to combinations of 1 to 5 expression level measurement values comprising at least one of the expression level measurement values of the newly found polynucleotides consisting of the nucleotide sequences represented by SEQ ID NOs: 1 to 83, 227 to 229, 246, 248, and 250 among the polynucleotides consisting of the nucleotide sequences represented by SEQ ID NOs: 1 to 250, to construct a discriminant for determining the presence or absence of early pancreatic cancer or a pancreatic cancer precursor lesion. Next, accuracy, sensitivity, and specificity in the validation cohort were calculated using the discriminant thus prepared, with the pancreatic cancer precursor lesion patient group and the early pancreatic cancer patient group as positive control groups and the healthy subject group, the advanced pancreatic cancer patient group, the bile duct cancer patient group, the breast cancer patient group, the prostate cancer patient group, the colorectal cancer patient group, the stomach cancer patient group, the esophageal cancer patient group, the liver cancer patient group, the benign pancreatic disease patient group, and the benign prostatic disease patient group as negative sample groups. The discriminant performance of the selected polynucleotides was validated using independent samples.

Most of polynucleotides consisting of the nucleotide sequences represented by these SEQ ID NOs (SEQ ID NOs: 1 to 250 corresponding to the miRNA markers of Table 1) or complementary sequences thereof mentioned above were able to provide relatively high accuracy, sensitivity, and specificity in the determination of the presence or absence of early pancreatic cancer or a pancreatic cancer precursor lesion, and furthermore, were able to specifically discriminate early pancreatic cancer or a pancreatic cancer precursor lesion from the other cancers and benign diseases. For example, among the combinations of multiple polynucleotides selected from the group consisting of polynucleotides consisting of the nucleotide sequences represented by SEQ ID NOs: 119, 12, 28, 105, 137, 121, 109, 87, 5, 140, 106, 2, 175, 90, 237, 247, 103, 97, 124, 92, 100, 32, 1, 246, 84, 13, 85, 153, 111, 86, 141, 54, and 24 or complementary sequences thereof (the cancer type-specific polynucleotide group 1) as polynucleotides capable of specifically binding to target markers, combinations comprising at least one polynucleotides preferably selected from the group consisting of polynucleotides consisting of the nucleotide sequences represented by SEQ ID NOs: 119, 12, 28, 105, 137, 121, 109, 87, 5, 140, 106, 2, 175, 90, 237, and 247 or complementary sequences thereof (the cancer type-specific polynucleotide group 2) included in the cancer type-specific polynucleotide group 1 were able to specifically discriminate early pancreatic cancer or a pancreatic cancer precursor lesion from the other cancers and benign diseases with high accuracy.

The number of the polynucleotides with cancer type specificity in the combination mentioned above can be 1, 2, 3, 4, 5, 6, 7, 8, 9, 10 or more for the combination. The combinations of 4 or more of these polynucleotides were able to exhibit discrimination accuracy of 92% or higher or 95% or higher.

The probes used in the measurement were the above-defined nucleic acids capable of specifically binding to each polynucleotide as a target marker.

Specifically, the discrimination results of the measurement using the polynucleotide consisting of the nucleotide sequence represented by SEQ ID NO: 12 or a complementary sequence thereof as a target marker are shown in Table 9-1. The measurement using one polynucleotide consisting of the nucleotide sequence represented by SEQ ID NO: 12 or a complementary sequence thereof exhibited accuracy of 82.6% in the training cohort and accuracy of 82% in the validation cohort. Also, for example, the measurement using the combination of two polynucleotides comprising one polynucleotide consisting of the nucleotide sequence represented by SEQ ID NO: 12 or a complementary sequence thereof exhibited accuracy of 87% in the training cohort and accuracy of 88% in the validation cohort. Furthermore, for example, the measurement using the combination of three polynucleotides comprising one polynucleotide consisting of the nucleotide sequence represented by SEQ ID NO: 12 or a complementary sequence thereof exhibited accuracy of 91.4% in the training cohort and accuracy of 86.6% in the validation cohort. Furthermore, for example, the measurement using the combination of four polynucleotides comprising one polynucleotide consisting of the nucleotide sequence represented by SEQ ID NO: 12 or a complementary sequence thereof exhibited accuracy of 95.6% in the training cohort and accuracy of 95.1% in the validation cohort. Furthermore, for example, the measurement using the combination of five polynucleotides comprising one polynucleotide consisting of the nucleotide sequence represented by SEQ ID NO: 12 or a complementary sequence thereof exhibited the highest accuracy of 98.8% in the training cohort and the highest accuracy of 98.9% in the validation cohort.

Specifically, the discrimination results of the measurement using the polynucleotide consisting of the nucleotide sequence represented by SEQ ID NO: 28 or a complementary sequence thereof as a target marker are shown in Table 9-2. The measurement using one polynucleotide consisting of the nucleotide sequence represented by SEQ ID NO: 28 or a complementary sequence thereof exhibited accuracy of 81.6% in the training cohort and accuracy of 81.7% in the validation cohort. Also, for example, the measurement using the combination of two polynucleotides comprising one polynucleotide consisting of the nucleotide sequence represented by SEQ ID NO: 28 or a complementary sequence thereof exhibited accuracy of 84.9% in the training cohort and accuracy of 85.6% in the validation cohort. Furthermore, for example, the measurement using the combination of three polynucleotides comprising one polynucleotide consisting of the nucleotide sequence represented by SEQ ID NO: 28 or a complementary sequence thereof exhibited accuracy of 88.8% in the training cohort and accuracy of 86.3% in the validation cohort. Furthermore, for example, the measurement using the combination of four polynucleotides comprising one polynucleotide consisting of the nucleotide sequence represented by SEQ ID NO: 28 or a complementary sequence thereof exhibited accuracy of 92.4% in the training cohort and accuracy of 93.6% in the validation cohort. Furthermore, for example, the measurement using the combination of five polynucleotides comprising one polynucleotide consisting of the nucleotide sequence represented by SEQ ID NO: 28 or a complementary sequence thereof exhibited the highest accuracy of 97.7% in the training cohort and the highest accuracy of 98.6% in the validation cohort.

Specifically, the discrimination results of the measurement using the polynucleotide consisting of the nucleotide sequence represented by SEQ ID NO: 5 or a complementary sequence thereof as a target marker are shown in Table 9-3. The measurement using one polynucleotide consisting of the nucleotide sequence represented by SEQ ID NO: 5 or a complementary sequence thereof exhibited accuracy of 84% in the training cohort and accuracy of 87% in the validation cohort. Also, for example, the measurement using the combination of two polynucleotides comprising one polynucleotide consisting of the nucleotide sequence represented by SEQ ID NO: 5 or a complementary sequence thereof exhibited accuracy of 87.9% in the training cohort and accuracy of 88.4% in the validation cohort. Furthermore, for example, the measurement using the combination of three polynucleotides comprising one polynucleotide consisting of the nucleotide sequence represented by SEQ ID NO: 5 or a complementary sequence thereof exhibited accuracy of 90.4% in the training cohort and accuracy of 90.5% in the validation cohort. Furthermore, for example, the measurement using the combination of four polynucleotides comprising one polynucleotide consisting of the nucleotide sequence represented by SEQ ID NO: 5 or a complementary sequence thereof exhibited accuracy of 93.2% in the training cohort and accuracy of 93.7% in the validation cohort. Furthermore, for example, the measurement using the combination of five polynucleotides comprising one polynucleotide consisting of the nucleotide sequence represented by SEQ ID NO: 5 or a complementary sequence thereof exhibited the highest accuracy of 97.7% in the training cohort and the highest accuracy of 98.2% in the validation cohort.

Specifically, the discrimination results of the measurement using the polynucleotide consisting of the nucleotide sequence represented by SEQ ID NO: 2 or a complementary sequence thereof as a target marker are shown in Table 9-4. The measurement using one polynucleotide consisting of the nucleotide sequence represented by SEQ ID NO: 2 or a complementary sequence thereof exhibited accuracy of 86.8% in the training cohort and accuracy of 90.5% in the validation cohort. Also, for example, the measurement using the combination of two polynucleotides comprising one polynucleotide consisting of the nucleotide sequence represented by SEQ ID NO: 2 or a complementary sequence thereof exhibited accuracy of 88.4% in the training cohort and accuracy of 90.1% in the validation cohort. Furthermore, for example, the measurement using the combination of three polynucleotides comprising one polynucleotide consisting of the nucleotide sequence represented by SEQ ID NO: 2 or a complementary sequence thereof exhibited accuracy of 90.9% in the training cohort and accuracy of 92.6% in the validation cohort. Furthermore, for example, the measurement using the combination of four polynucleotides comprising one polynucleotide consisting of the nucleotide sequence represented by SEQ ID NO: 2 or a complementary sequence thereof exhibited accuracy of 93% in the training cohort and accuracy of 92.6% in the validation cohort. Furthermore, for example, the measurement using the combination of five polynucleotides comprising one polynucleotide consisting of the nucleotide sequence represented by SEQ ID NO: 2 or a complementary sequence thereof exhibited the highest accuracy of 97.7% in the training cohort and the highest accuracy of 98.2% in the validation cohort.

Specifically, the discrimination results of the measurement using the polynucleotides consisting of the nucleotide sequences represented by SEQ ID NOs: 12 and 28 or complementary sequences thereof as target markers are shown in Table 9-5. The measurement using the combination of two polynucleotides comprising the polynucleotides consisting of the nucleotide sequences represented by SEQ ID NOs: 12 and 28 or complementary sequences thereof exhibited accuracy of 90% in the training cohort and accuracy of 92.6% in the validation cohort. Also, for example, the measurement using the combination of three polynucleotides comprising the polynucleotides consisting of the nucleotide sequences represented by SEQ ID NOs: 12 and 28 or complementary sequences thereof exhibited accuracy of 92.3% in the training cohort and accuracy of 93.3% in the validation cohort. Furthermore, for example, the measurement using the combination of four polynucleotides comprising the polynucleotides consisting of the nucleotide sequences represented by SEQ ID NOs: 12 and 28 or complementary sequences thereof exhibited accuracy of 93.9% in the training cohort and accuracy of 93.7% in the validation cohort. Furthermore, for example, the measurement using the combination of five polynucleotides comprising the polynucleotides consisting of the nucleotide sequences represented by SEQ ID NOs: 12 and 28 or complementary sequences thereof exhibited the highest accuracy of 97.9% in the training cohort and the highest accuracy of 97.9% in the validation cohort.

Specifically, the discrimination results of the measurement using the polynucleotides consisting of the nucleotide sequences represented by SEQ ID NOs: 12 and 5 or complementary sequences thereof as target markers are shown in Table 9-6. The measurement using the combination of two polynucleotides comprising the polynucleotides consisting of the nucleotide sequences represented by SEQ ID NOs: 12 and 5 or complementary sequences thereof exhibited accuracy of 91.2% in the training cohort and accuracy of 89.4% in the validation cohort. Also, for example, the measurement using the combination of three polynucleotides comprising the polynucleotides consisting of the nucleotide sequences represented by SEQ ID NOs: 12 and 5 or complementary sequences thereof exhibited accuracy of 93% in the training cohort and accuracy of 92.6% in the validation cohort. Furthermore, for example, the measurement using the combination of four polynucleotides comprising the polynucleotides consisting of the nucleotide sequences represented by SEQ ID NOs: 12 and 5 or complementary sequences thereof exhibited accuracy of 95.1% in the training cohort and accuracy of 93% in the validation cohort. Furthermore, for example, the measurement using the combination of five polynucleotides comprising the polynucleotides consisting of the nucleotide sequences represented by SEQ ID NOs: 12 and 5 or complementary sequences thereof exhibited the highest accuracy of 98.1% in the training cohort and the highest accuracy of 97.9% in the validation cohort.

Specifically, the discrimination results of the measurement using the polynucleotides consisting of the nucleotide sequences represented by SEQ ID NOs: 12 and 2 or complementary sequences thereof as target markers are shown in Table 9-7. The measurement using the combination of two polynucleotides comprising the polynucleotides consisting of the nucleotide sequences represented by SEQ ID NOs: 12 and 2 or complementary sequences thereof exhibited accuracy of 91.2% in the training cohort and accuracy of 90.1% in the validation cohort. Also, for example, the measurement using the combination of three polynucleotides comprising the polynucleotides consisting of the nucleotide sequences represented by SEQ ID NOs: 12 and 2 or complementary sequences thereof exhibited accuracy of 93.9% in the training cohort and accuracy of 92.6% in the validation cohort. Furthermore, for example, the measurement using the combination of four polynucleotides comprising the polynucleotides consisting of the nucleotide sequences represented by SEQ ID NOs: 12 and 2 or complementary sequences thereof exhibited accuracy of 94.6% in the training cohort and accuracy of 93.3% in the validation cohort. Furthermore, for example, the measurement using the combination of five polynucleotides comprising the polynucleotides consisting of the nucleotide sequences represented by SEQ ID NOs: 12 and 2 or complementary sequences thereof exhibited the highest accuracy of 98.1% in the training cohort and the highest accuracy of 97.9% in the validation cohort.

Specifically, the discrimination results of the measurement using the polynucleotides consisting of the nucleotide sequences represented by SEQ ID NOs: 5 and 2 or complementary sequences thereof as target markers are shown in Table 9-8. The measurement using the combination of two polynucleotides comprising the polynucleotides consisting of the nucleotide sequences represented by SEQ ID NOs: 5 and 2 or complementary sequences thereof exhibited accuracy of 89.8% in the training cohort and accuracy of 92.3% in the validation cohort. Also, for example, the measurement using the combination of three polynucleotides comprising the polynucleotides consisting of the nucleotide sequences represented by SEQ ID NOs: 5 and 2 or complementary sequences thereof exhibited accuracy of 92.1% in the training cohort and accuracy of 94% in the validation cohort. Furthermore, for example, the measurement using the combination of four polynucleotides comprising the polynucleotides consisting of the nucleotide sequences represented by SEQ ID NOs: 5 and 2 or complementary sequences thereof exhibited accuracy of 93.9% in the training cohort and accuracy of 95.1% in the validation cohort. Furthermore, for example, the measurement using the combination of five polynucleotides comprising the polynucleotides consisting of the nucleotide sequences represented by SEQ ID NOs: 5 and 2 or complementary sequences thereof exhibited the highest accuracy of 97.2% in the training cohort and the highest accuracy of 97.9% in the validation cohort.

Specifically, the discrimination results of the measurement using the polynucleotides consisting of the nucleotide sequences represented by SEQ ID NOs: 12, 28, and 5 or complementary sequences thereof as target markers are shown in Table 9-9. The measurement using the combination of three polynucleotides comprising the polynucleotides consisting of the nucleotide sequences represented by SEQ ID NOs: 12, 28, and 5 or complementary sequences thereof exhibited accuracy of 93.3% in the training cohort and accuracy of 94.4% in the validation cohort. Also, for example, the measurement using the combination of four polynucleotides comprising the polynucleotides consisting of the nucleotide sequences represented by SEQ ID NOs: 12, 28, and 5 or complementary sequences thereof exhibited accuracy of 94.6% in the training cohort and accuracy of 96.5% in the validation cohort. Furthermore, for example, the measurement using the combination of five polynucleotides comprising the polynucleotides consisting of the nucleotide sequences represented by SEQ ID NOs: 12, 28, and 5 or complementary sequences thereof exhibited accuracy of 97.5% in the training cohort and accuracy of 96.8% in the validation cohort.

The expression level measurement values of the nucleotide sequences represented by SEQ ID NOs: 106, 12, 137, 119, and 105 were compared among 21 pancreatic cancer precursor lesion patients, 31 early pancreatic cancer patients, 128 healthy subjects, 61 advanced pancreatic cancer patients, 66 bile duct cancer patients, 51 breast cancer patients, 35 prostate cancer patients, 31 colorectal cancer patients, 32 stomach cancer patients, 34 esophageal cancer patients, 38 liver cancer patients, 15 benign pancreatic disease patients, and 26 benign prostatic disease patients in the training cohort. As a result, a scatter diagram that significantly separated the discriminant score of the early pancreatic cancer or pancreatic cancer precursor lesion patient group from the discriminant scores of the other groups was obtained in the training cohort (see the upper diagram of Figure 4). These results were also reproducible in the validation cohort (see the lower diagram of Figure 4).

**[Table 9-1]**

| | Training cohort | | | Validation cohort | | |
|---|---|---|---|---|---|---|
| SEQ ID NO: | Accuracy (%) | Sensitivity (%) | Specificity (%) | Accuracy (%) | Sensitivity (%) | Specificity (%) |
| 12 | 82.6 | 90.6 | 81.8 | 82 | 76.9 | 82.6 |
| 12_237 | 87 | 96.2 | 86.1 | 88 | 92.3 | 87.6 |
| 12_85_13 | 91.4 | 100 | 90.5 | 86.6 | 84.6 | 86.8 |
| 90_12_97_140 | 95.6 | 100 | 95.2 | 95.1 | 96.2 | 95 |
| 12_137_119_105_237 | 98.4 | 100 | 98.3 | 97.9 | 100 | 97.7 |
| 87_12_137_119_105 | 98.2 | 100 | 98.1 | 97.5 | 100 | 97.3 |
| 12_103_137_105_247 | 98.2 | 100 | 98.1 | 98.6 | 100 | 98.4 |
| 12_137_119_92_105 | 98.2 | 100 | 98.1 | 97.9 | 100 | 97.7 |
| 12_137_119_105_121 | 98.2 | 100 | 98.1 | 98.2 | 100 | 98.1 |
| 12_137_1_119_105 | 98.1 | 100 | 97.9 | 97.9 | 100 | 97.7 |
| 12_137_119_124_105 | 98.1 | 100 | 97.9 | 97.9 | 100 | 97.7 |
| 12_137_119_105_32 | 98.1 | 100 | 97.9 | 98.2 | 100 | 98.1 |
| 12_137_119_105_100 | 98.1 | 100 | 97.9 | 97.9 | 100 | 97.7 |
| 12_137_119_105_86 | 98.1 | 100 | 97.9 | 97.9 | 100 | 97.7 |
| 12_137_119_105_153 | 98.1 | 100 | 97.9 | 98.6 | 100 | 98.4 |
| 12_137_119_105_141 | 98.1 | 100 | 97.9 | 97.5 | 100 | 97.3 |
| 12_137_105_246_153 | 98.1 | 100 | 97.9 | 97.2 | 100 | 96.9 |
| 12_97_137_105_153 | 97.9 | 100 | 97.7 | 96.8 | 100 | 96.5 |
| 12_103_137_119_105 | 97.9 | 100 | 97.7 | 97.9 | 100 | 97.7 |
| 12_137_119_105_246 | 97.9 | 100 | 97.7 | 98.2 | 100 | 98.1 |
| 12_137_92_105_247 | 97.9 | 100 | 97.7 | 98.2 | 100 | 98.1 |
| 12_137_124_105_153 | 97.9 | 100 | 97.7 | 97.5 | 100 | 97.3 |
| 12_137_105_32_153 | 97.9 | 100 | 97.7 | 97.5 | 100 | 97.3 |
| 12_137 105_13_121 | 97.9 | 100 | 97.7 | 97.5 | 100 | 97.3 |
| 106_12_137_119_105 | 98.8 | 100 | 98.6 | 97.5 | 100 | 97.3 |
| 12_137_119_105_13 | 98.6 | 100 | 98.4 | 97.9 | 100 | 97.7 |
| 12_137_119_105_140 | 98.6 | 100 | 98.4 | 97.9 | 100 | 97.7 |
| 12_137_119_105_247 | 98.6 | 100 | 98.4 | 98.9 | 100 | 98.8 |
| 12_137_119_105_109 | 98.4 | 100 | 98.3 | 97.5 | 100 | 97.3 |
| 12_137_105_109_121 | 98.1 | 100 | 97.9 | 97.5 | 100 | 97.3 |
| 12_103_137_105_121 | 97.9 | 100 | 97.7 | 97.9 | 100 | 97.7 |
| 12_137 105_32_121 | 97.9 | 100 | 97.7 | 97.9 | 100 | 97.7 |
| 12_137_124_105_247 | 98.1 | 100 | 97.9 | 97.5 | 100 | 97.3 |
| 12_137_105_246_247 | 98.1 | 100 | 97.9 | 97.2 | 100 | 96.9 |
| 12_137_105_153_247 | 98.1 | 100 | 97.9 | 97.5 | 100 | 97.3 |
| 12_137_105_247_141 | 98.1 | 100 | 97.9 | 97.2 | 100 | 96.9 |
| 12_137_105_247 | 97.9 | 100 | 97.7 | 97.2 | 100 | 96.9 |
| 12_137_105_140_247 | 98.1 | 100 | 97.9 | 97.9 | 100 | 97.7 |
| 12_119_124_105_140 | 98.1 | 100 | 97.9 | 97.5 | 100 | 97.3 |
| 12_119_105_100_140 | 98.1 | 100 | 97.9 | 96.8 | 96.2 | 96.9 |
| 90_12_119_105_140 | 97.7 | 100 | 97.5 | 97.9 | 100 | 97.7 |
| 90_12_137_119_105 | 97.9 | 100 | 97.7 | 98.2 | 100 | 98.1 |
| 90_12_137_105_32 | 97.9 | 100 | 97.7 | 97.2 | 100 | 96.9 |
| 90_12_137_105_153 | 97.9 | 100 | 97.7 | 96.8 | 100 | 96.5 |
| 90_12_119_105_100 | 97.7 | 100 | 97.5 | 97.2 | 96.2 | 97.3 |
| 90_12_119_109_140 | 97.7 | 100 | 97.5 | 97.2 | 100 | 96.9 |
| 87_12_137_105_247 | 97.7 | 100 | 97.5 | 97.2 | 100 | 96.9 |
| 90_12_109_140_237 | 97.9 | 100 | 97.7 | 96.1 | 96.2 | 96.1 |
| 12_137_105_109_153 | 97.9 | 100 | 97.7 | 97.2 | 100 | 96.9 |
| 12_137_105_109_247 | 97.9 | 100 | 97.7 | 97.9 | 100 | 97.7 |
| 12_137_109_140_247 | 97.9 | 98.1 | 97.9 | 96.1 | 96.2 | 96.1 |
| 12_137_109_121_237 | 97.9 | 98.1 | 97.9 | 96.8 | 100 | 96.5 |
| 12_137_119_105_175 | 98.1 | 100 | 97.9 | 97.9 | 100 | 97.7 |
| 12_137_109_175_121 | 97.5 | 100 | 97.3 | 97.5 | 100 | 97.3 |
| 87_12_119_105_175 | 97.4 | 100 | 97.1 | 97.5 | 100 | 97.3 |
| 12_137_119_105_111 | 98.2 | 100 | 98.1 | 97.9 | 100 | 97.7 |
| 12_137_119_105_24 | 98.2 | 100 | 98.1 | 97.9 | 100 | 97.7 |
| 12_137_105_32_247 | 98.2 | 100 | 98.1 | 98.2 | 100 | 98.1 |
| 90_12_137_105_237 | 98.1 | 100 | 97.9 | 96.5 | 100 | 96.1 |
| 12_84_137_119_105 | 98.1 | 100 | 97.9 | 97.9 | 100 | 97.7 |
| 12_97_137_105_247 | 98.1 | 100 | 97.9 | 97.9 | 100 | 97.7 |
| 87_12_137_105_237 | 97.2 | 100 | 96.9 | 96.5 | 100 | 96.1 |
| 87_12_100_109_237 | 97.2 | 100 | 96.9 | 95.8 | 100 | 95.3 |
| 87_12_100_109_237 | 97.2 | 100 | 96.9 | 95.8 | 100 | 95.3 |
| 106_12_137_105_86 | 97.9 | 100 | 97.7 | 97.2 | 100 | 96.9 |
| 106_12_137_105_247 | 97.9 | 100 | 97.7 | 97.5 | 100 | 97.3 |
| 106_12_119_105_100 | 97.9 | 100 | 97.7 | 95.7 | 96 | 95.7 |
| 106_12_137_105_121 | 97.7 | 100 | 97.5 | 97.5 | 100 | 97.3 |

**[Table 9-2]**

| | Training cohort | | | Validation cohort | | |
|---|---|---|---|---|---|---|
| SEQ ID NO: | Accuracy (%) | Sensitivity (%) | Specificity (%) | Accuracy (%) | Sensitivity (%) | Specificity (%) |
| 28 | 81.6 | 83 | 81.4 | 81.7 | 96.2 | 80.2 |
| 103_28 | 84.9 | 84.9 | 84.9 | 85.6 | 96.2 | 84.5 |
| 105_28_109 | 88.8 | 88.7 | 88.8 | 86.3 | 96.2 | 85.3 |
| 84_105_28_100 | 92.4 | 94.3 | 92.2 | 93.6 | 100 | 93 |
| 137_119_105_28_237 | 97.7 | 98.1 | 97.7 | 98.6 | 100 | 100 |
| 87_106_119_28_121 | 97.4 | 100 | 97.1 | 97.5 | 100 | 100 |
| 106_137_119_28_121 | 97.4 | 100 | 97.1 | 97.5 | 100 | 100 |
| 90_119_105_28_237 | 97.4 | 98.1 | 97.3 | 97.9 | 100 | 100 |

**[Table 9-3]**

| | Training cohort | | | Validation cohort | | |
|---|---|---|---|---|---|---|
| SEQ ID NO: | Accuracy (%) | Sensitivity (%) | Specificity (%) | Accuracy (%) | Sensitivity (%) | Specificity (%) |
| 5 | 84 | 88.7 | 83.6 | 87 | 88.5 | 86.8 |
| 5_84 | 87.9 | 92.5 | 87.4 | 88.4 | 92.3 | 88 |
| 5_137_85 | 90.4 | 98.1 | 89.6 | 90.5 | 96.2 | 89.9 |
| 5_97_237_247 | 93.2 | 94.3 | 93 | 93.7 | 100 | 93 |
| 90_5_137_119_105 | 97.7 | 100 | 97.5 | 98.2 | 100 | 100 |
| 5_137_119_105_237 | 97.7 | 98.1 | 97.7 | 98.2 | 100 | 100 |
| 5_137_119_105_32 | 97.5 | 98.1 | 97.5 | 97.9 | 100 | 100 |

**[Table 9-4]**

| | Training cohort | | | Validation cohort | | |
|---|---|---|---|---|---|---|
| SEQ ID NO: | Accuracy (%) | Sensitivity (%) | Specificity (%) | Accuracy (%) | Sensitivity (%) | Specificity (%) |
| 2 | 86.8 | 96.2 | 85.9 | 90.5 | 96.2 | 89.9 |
| 2_1 | 88.4 | 98.1 | 87.4 | 90.1 | 96.2 | 89.5 |
| 2_87_111 | 90.9 | 100 | 89.9 | 92.6 | 100 | 91.9 |
| 2_87_90_103 | 93 | 98.1 | 92.5 | 92.6 | 100 | 91.9 |
| 2_137_119_105_237 | 97.7 | 98.1 | 97.7 | 98.2 | 100 | 100 |
| 2_87_137_119_105 | 97.4 | 100 | 97.1 | 97.9 | 100 | 100 |
| 2_137_119_105_13 | 97.4 | 100 | 97.1 | 98.2 | 100 | 100 |
| 2_137_119_105_121 | 97.4 | 100 | 97.1 | 98.2 | 100 | 100 |
| 2_137_119_105_247 | 97.4 | 98.1 | 97.3 | 97.9 | 100 | 100 |
| 2_87_119_109_247 | 97.2 | 100 | 96.9 | 97.5 | 100 | 100 |
| 2_90_137_119_105 | 97.2 | 100 | 96.9 | 97.5 | 100 | 100 |
| 2_137_119_105_140 | 97.2 | 100 | 96.9 | 98.2 | 100 | 100 |
| 2_87_119_105_237 | 97.2 | 100 | 96.9 | 96.8 | 100 | 100 |

**[Table 9-5]**

| | Training cohort | | | Validation cohort | | |
|---|---|---|---|---|---|---|
| SEQ ID NO: | Accuracy (%) | Sensitivity (%) | Specificity (%) | Accuracy (%) | Sensitivity (%) | Specificity (%) |
| 12_28 | 90 | 92.5 | 89.7 | 92.6 | 88.5 | 93 |
| 12_28_32 | 92.3 | 96.2 | 91.9 | 93.3 | 92.3 | 93.4 |
| 12_137_28_32 | 93.9 | 100 | 93.2 | 93.7 | 100 | 93 |
| 12_137_105_28_247 | 97.9 | 98.1 | 97.9 | 97.5 | 100 | 100 |
| 12_137_119_105_28 | 97.7 | 100 | 97.5 | 97.9 | 100 | 100 |
| 12_103_137_105_28 | 97.5 | 100 | 97.3 | 96.8 | 100 | 100 |
| 12_84_28_140_121 | 97.5 | 98.1 | 97.5 | 96.5 | 92.3 | 92.3 |
| 12_137_28_109_121 | 97.5 | 98.1 | 97.5 | 97.5 | 100 | 100 |
| 12_1_28_121_247 | 97.5 | 98.1 | 97.5 | 96.1 | 96.2 | 96.2 |
| 12_119_28_100_121 | 97.5 | 98.1 | 97.5 | 97.2 | 100 | 100 |
| 12_92_28_100_247 | 97.5 | 98.1 | 97.5 | 97.2 | 100 | 100 |
| 12_28_100_140_247 | 97.5 | 98.1 | 97.5 | 97.5 | 96.2 | 96.2 |
| 12_137_28_109_247 | 97.5 | 94.3 | 97.9 | 95.8 | 92.3 | 92.3 |
| 12_84_28_109_121 | 97.4 | 98.1 | 97.3 | 95.8 | 96.2 | 96.2 |
| 12_103_1_28_121 | 97.4 | 98.1 | 97.3 | 95.4 | 100 | 100 |
| 12_1_28_32_121 | 97.4 | 98.1 | 97.3 | 95.8 | 100 | 100 |
| 12_1_28_100_121 | 97.4 | 98.1 | 97.3 | 95.1 | 96.2 | 96.2 |
| 12_1_28_175_121 | 97.4 | 98.1 | 97.3 | 95.8 | 100 | 100 |

**[Table 9-6]**

| | Training cohort | | | Validation cohort | | |
|---|---|---|---|---|---|---|
| SEQ ID NO: | Accuracy (%) | Sensitivity (%) | Specificity (%) | Accuracy (%) | Sensitivity (%) | Specificity (%) |
| 5_12 | 91.2 | 98.1 | 90.5 | 89.4 | 84.6 | 89.9 |
| 5_12_92 | 93 | 98.1 | 92.5 | 92.6 | 96.2 | 92.2 |
| 5_12_105_13 | 95.1 | 98.1 | 94.8 | 93 | 92.3 | 93 |
| 5_12_137_119_105 | 97.9 | 100 | 97.7 | 97.9 | 100 | 100 |
| 90_5_12_119_105 | 97.7 | 100 | 97.5 | 96.8 | 100 | 100 |
| 5_12_137_105_121 | 97.7 | 100 | 97.5 | 97.5 | 100 | 100 |
| 5_12_119_92_121 | 97.7 | 100 | 97.5 | 96.1 | 100 | 100 |
| 90_5_12_109_247 | 97.7 | 98.1 | 97.7 | 95.8 | 96.2 | 96.2 |
| 5_12_137_109_121 | 97.7 | 98.1 | 97.7 | 97.5 | 100 | 100 |
| 5_12_137_109_247 | 97.7 | 96.2 | 97.9 | 97.9 | 100 | 100 |
| 90_5_106_12_109 | 97.5 | 100 | 97.3 | 95.4 | 96 | 96 |
| 90_5_12_137_105 | 97.5 | 100 | 97.3 | 96.8 | 100 | 100 |
| 90_5_12_119_109 | 97.5 | 100 | 97.3 | 96.1 | 100 | 100 |
| 90_5_12_105_109 | 97.5 | 100 | 97.3 | 96.1 | 100 | 100 |
| 5_12_137_105_153 | 97.5 | 100 | 97.3 | 97.5 | 100 | 100 |
| 5_12_119_105_54 | 97.5 | 100 | 97.3 | 97.5 | 96.2 | 96.2 |
| 87_5_106_12_109 | 97.4 | 100 | 97.1 | 96.1 | 96 | 96 |
| 87_5_12_137_105 | 97.4 | 100 | 97.1 | 96.1 | 100 | 100 |
| 90_5_12_1_105 | 97.4 | 100 | 97.1 | 95.4 | 96.2 | 96.2 |
| 90_5_12_109_86 | 97.4 | 100 | 97.1 | 95.4 | 96.2 | 96.2 |
| 5_12_137_105_247 | 98.1 | 100 | 97.9 | 97.5 | 100 | 100 |
| 90_5_12_109_175 | 97.5 | 100 | 97.3 | 95.1 | 100 | 100 |
| 5_12_100_109_121 | 97.9 | 100 | 97.7 | 96.1 | 96.2 | 96.2 |

**[Table 9-7]**

| | Training cohort | | | Validation cohort | | |
|---|---|---|---|---|---|---|
| SEQ ID NO: | Accuracy (%) | Sensitivity (%) | Specificity (%) | Accuracy (%) | Sensitivity (%) | Specificity (%) |
| 2_12 | 91.2 | 100 | 90.3 | 90.1 | 92.3 | 89.9 |
| 2_12_121 | 93.9 | 100 | 93.2 | 92.6 | 92.3 | 92.6 |
| 2_12_1_121 | 94.6 | 100 | 94 | 93.3 | 96.2 | 93 |
| 2_12_137_119_105 | 97.9 | 100 | 97.7 | 97.2 | 100 | 100 |
| 2_12_137_105_153 | 97.9 | 100 | 97.7 | 96.5 | 100 | 100 |
| 2_12_137_105_121 | 97.5 | 100 | 97.3 | 96.5 | 100 | 100 |
| 2_12_109_121_247 | 97.4 | 100 | 97.1 | 95.4 | 96.2 | 96.2 |
| 2_90_12_119_105 | 97.2 | 100 | 96.9 | 96.8 | 100 | 100 |
| 2_90_12_109_140 | 97.2 | 100 | 96.9 | 96.1 | 96.2 | 96.2 |
| 2_12_100 109 121 | 97.2 | 100 | 96.9 | 95.8 | 96.2 | 96.2 |
| 2_12_109_175_121 | 97.2 | 100 | 96.9 | 95.8 | 96.2 | 96.2 |
| 2_12_97_105_247 | 97.2 | 98.1 | 97.1 | 95.1 | 96.2 | 96.2 |
| 2_12_137_105_247 | 98.1 | 100 | 97.9 | 96.8 | 100 | 100 |
| 2_12_137_109_121 | 97.9 | 100 | 97.7 | 97.9 | 100 | 100 |

**[Table 9-8]**

| | Training cohort | | | Validation cohort | | |
|---|---|---|---|---|---|---|
| SEQ ID NO: | Accuracy (%) | Sensitivity (%) | Specificity (%) | Accuracy (%) | Sensitivity (%) | Specificity (%) |
| 2_5 | 89.8 | 100 | 88.8 | 92.3 | 100 | 91.5 |
| 2_5_247 | 92.1 | 98.1 | 91.5 | 94 | 100 | 93.4 |
| 2_5_97_153 | 93.9 | 98.1 | 93.4 | 95.1 | 100 | 94.6 |
| 2_90_5_119_105 | 97.2 | 100 | 96.9 | 97.9 | 100 | 100 |
| 2_5_119_109_121 | 97.2 | 100 | 96.9 | 97.9 | 100 | 100 |
| 2_5_119_86_121 | 97.2 | 100 | 96.9 | 96.5 | 100 | 100 |

**[Table 9-9]**

| | Training cohort | | | Validation cohort | | |
|---|---|---|---|---|---|---|
| SEQ ID NO: | Accuracy (%) | Sensitivity (%) | Specificity (%) | Accuracy (%) | Sensitivity (%) | Specificity (%) |
| 5_12_28 | 93.3 | 96.2 | 93 | 94.4 | 96.2 | 94.2 |
| 5_12_119_28 | 94.6 | 98.1 | 94.2 | 96.5 | 100 | 96.1 |
| 5_12_1_28_121 | 97.5 | 98.1 | 97.5 | 96.8 | 100 | 100 |

### [Example 5]

### <Method for evaluating early pancreatic cancer or pancreatic cancer precursor lesion discriminant performance by combination of multiple gene markers using samples in the validation cohort>

Example 2 showed that discriminant performance was improved by using a combination of the multiple gene markers selected in Example 1, as compared with using one of the gene marker. Thus, in this Example, even the gene markers that were not selected in Example 1 were studied as to whether high early pancreatic cancer or pancreatic cancer precursor lesion discriminant performance is obtained by combinations with the gene markers selected in Example 1.

Specifically, among the genes having a gene expression level of 2⁶ or higher in 50% or more of the samples in either of the early pancreatic cancer or pancreatic cancer precursor lesion patient group in the training cohort or the healthy subject group in the training cohort, genes that showed statistical significance for discriminating an early pancreatic cancer or pancreatic cancer precursor lesion patient group from a healthy subject group with the P value smaller than 0.05 calculated by two-tailed t-test assuming equal variance as to each gene expression level and corrected by the Bonferroni method, were examined. As a result, 248 genes containing the 226 genes selected in Example 1 were found. Fisher's discriminant analysis was conducted as to 30,876 combinations using one or two of these 248 genes, to construct a discriminant for determining the presence or absence of early pancreatic cancer or a pancreatic cancer precursor lesion. The discriminant performance of the selected combinations of 1 or 2 of the genes was validated in the same way as the method of Example 2.

As a result, some combinations of these genes exhibited accuracy of 85% or higher in both of the training cohort and the validation cohort and are shown in Table 10. For example, the newly found polynucleotide consisting of the nucleotide sequence represented by SEQ ID NO: 248 to 250 discriminated the early pancreatic cancer or pancreatic cancer precursor lesion patients from the healthy subjects with high discriminant performance when used in combination of at least two polynucleotides comprising any of the polynucleotides consisting of the nucleotide sequences represented by SEQ ID NOs: 1 to 226. More specifically, the polynucleotides consisting of the nucleotide sequences represented by SEQ ID NO: 248 to 250 was able to exhibit discrimination accuracy of 85% or higher between the early pancreatic cancer or pancreatic cancer precursor lesion patients and the healthy subjects in both of the training cohort and the validation cohort when used in combination of at least two polynucleotides comprising any of the polynucleotides consisting of the nucleotide sequences represented by SEQ ID NOs: 2, 3, 18, 12, 20, 1, 15, 50, 63, 72, 5, 24, 10, 52, 9, 11, 19, 39, 61, 7, 17, 22, 26, 74, 21, and 28. Examples of such combinations of two genes include combinations of SEQ ID NOs: 2 and 248, SEQ ID NOs: 3 and 249, SEQ ID NOs: 2 and 250, SEQ ID NOs: 1 and 249, SEQ ID NOs: 5 and 250, SEQ ID NOs: 3 and 248, SEQ ID NOs: 3 and 250, SEQ ID NOs: 1 and 250, SEQ ID NOs: 2 and 249, SEQ ID NOs: 21 and 248, SEQ ID NOs: 10 and 248, SEQ ID NOs: 5 and 248, SEQ ID NOs: 11 and 249, SEQ ID NOs: 9 and 250, SEQ ID NOs: 17 and 250, SEQ ID NOs: 21 and 249, SEQ ID NOs: 7 and 250, SEQ ID NOs: 15 and 248, SEQ ID NOs: 5 and 249, SEQ ID NOs: 12 and 248, SEQ ID NOs: 10 and 249, SEQ ID NOs: 28 and 250, SEQ ID NOs: 7 and 249, SEQ ID NOs: 18 and 249, SEQ ID NOs: 15 and 249, SEQ ID NOs: 20 and 249, SEQ ID NOs: 24 and 249, SEQ ID NOs: 11 and 250, and SEQ ID NOs: 18 and 248.

As one example, an attempt was made to discriminate the early pancreatic cancer or pancreatic cancer precursor lesion patients from the healthy subjects using the expression level measurement values of the nucleotide sequences represented by SEQ ID NO: 2 and SEQ ID NO: 248. As a result, discriminant performance as high as 93.2% accuracy, 96.2% sensitivity, and 91.9% specificity in the training cohort and 95.4% accuracy, 100% sensitivity, and 93.4% specificity in the validation cohort was obtained.

From these results, it can be concluded that all of the polynucleotides consisting of the nucleotide sequences represented by SEQ ID NOs: 248 to 250 are also excellent diagnostic markers.

Table 10 mentioned above is as follows.

**[Table 10]**

| | Training cohort | | | Validation cohort | | |
|---|---|---|---|---|---|---|
| SEQ ID NO: | Accuracy (%) | Sensitivity (%) | Specificity (%) | Accuracy (%) | Sensitivity (%) | Specificity (%) |
| 2_248 | 93.2 | 96.2 | 91.9 | 95.4 | 100 | 93.4 |
| 3_249 | 91.5 | 86.8 | 93.5 | 96.6 | 100 | 95.1 |
| 2_250 | 90.9 | 98.1 | 87.8 | 88.5 | 100 | 83.6 |
| 1_249 | 90.3 | 94.3 | 88.6 | 86.2 | 88.5 | 85.2 |
| 5_250 | 90.3 | 92.5 | 89.4 | 87.4 | 84.6 | 88.5 |
| 3_248 | 90.3 | 90.6 | 90.2 | 93.1 | 92.3 | 93.4 |
| 3_250 | 90.3 | 88.7 | 91.1 | 94.3 | 92.3 | 95.1 |
| 1_250 | 89.8 | 96.2 | 87 | 85.1 | 84.6 | 85.2 |
| 2_249 | 89.8 | 96.2 | 87 | 89.7 | 100 | 85.2 |
| 21_248 | 89.7 | 78.8 | 94.3 | 89.7 | 76.9 | 95.1 |
| 10_248 | 88.6 | 88.7 | 88.6 | 86.2 | 88.5 | 85.2 |
| 5_248 | 87.5 | 90.6 | 86.2 | 89.7 | 92.3 | 88.5 |
| 11_249 | 87.5 | 90.6 | 86.2 | 89.7 | 88.5 | 90.2 |
| 9_250 | 87.5 | 88.7 | 87 | 89.7 | 84.6 | 91.8 |
| 17_250 | 87.5 | 84.9 | 88.6 | 85.1 | 84.6 | 85.2 |
| 21_249 | 87.4 | 80.8 | 90.2 | 86.2 | 80.8 | 88.5 |
| 7_250 | 86.9 | 90.6 | 85.4 | 90.8 | 96.2 | 88.5 |
| 15_248 | 86.9 | 81.1 | 89.4 | 90.8 | 88.5 | 91.8 |
| 5_249 | 86.4 | 90.6 | 84.6 | 85.1 | 92.3 | 82 |
| 12_248 | 86.4 | 90.6 | 84.6 | 88.5 | 84.6 | 90.2 |
| 10_249 | 86.4 | 84.9 | 87 | 92 | 92.3 | 91.8 |
| 28_250 | 86.4 | 84.9 | 87 | 87.4 | 84.6 | 88.5 |
| 7_249 | 85.8 | 88.7 | 84.6 | 92 | 92.3 | 91.8 |
| 18_249 | 85.8 | 88.7 | 84.6 | 87.4 | 92.3 | 85.2 |
| 15_249 | 85.8 | 83 | 87 | 85.1 | 80.8 | 86.9 |
| 20_249 | 85.8 | 81.1 | 87.8 | 88.5 | 88.5 | 88.5 |
| 24_249 | 85.8 | 81.1 | 87.8 | 88.5 | 96.2 | 85.2 |
| 11_250 | 85.2 | 90.6 | 82.9 | 86.2 | 92.3 | 83.6 |
| 18_248 | 85.2 | 86.8 | 84.6 | 86.2 | 88.5 | 85.2 |

### [Comparative Example 1]

### <Early pancreatic cancer or pancreatic cancer precursor lesion discriminant performance of existing tumor markers in blood>

The concentrations of the existing tumor markers CEA and CA19-9 in blood were measured in the training cohort and the validation cohort obtained in the preceding Reference Examples. In principle, when the concentrations of these tumor markers in blood are higher than the reference values described in Non-Patent Literature 3 above (CEA: 5 ng/mL, CA19-9: 37 U/mL), subjects are usually suspected of having cancer. Thus, whether or not the concentrations of CEA and CA19-9 in blood exceeded their reference values was examined for each sample to decide whether early pancreatic cancer or pancreatic cancer precursor lesion patients were determined as early pancreatic cancer or pancreatic cancer precursor lesion patients, and the sensitivity of each existing marker was thereby calculated for the training cohort and validation cohort. The results are shown in Tables 5-1 and 5-2. The sensitivity of CEA and CA19-9 was as low as 18% and 58%, respectively, in the training cohort, and was as low as 20% and 68%, respectively, in the validation cohort, demonstrating that neither of the markers are useful in the detection of early pancreatic cancer or a pancreatic cancer precursor lesion (Tables 5-1 and 5-2). Furthermore, CEA and CA19-9 were totally unable to detect IPMA low grade, one type of pancreatic cancer precursor lesion with a low malignancy, in the training cohort and the validation cohort (Tables 5-1 and 5-2).

On the other hand, as shown above in Tables 3 and 6 of Examples 1 and 2, it can be concluded that in all of the polynucleotides consisting of the nucleotide sequences represented by SEQ ID NOs: 1 to 226, combinations of 1, 2 or more polynucleotides exhibiting sensitivity beyond the existing early pancreatic cancer or pancreatic cancer precursor lesion markers are present, and thus such polynucleotides serve as excellent diagnosis markers.

### [Comparative Example 2]

### <Performance of existing pancreatic cancer miRNA markers in blood for early pancreatic cancer or pancreatic cancer precursor lesion>

Combinations of one or more miRNAs selected from hsa-miR-4294 (SEQ ID NO: 125), hsa-miR-6836-3p (SEQ ID NO: 231), and hsa-miR-6880-5p (SEQ ID NO: 219) included in the present invention among hsa-miR-6075, hsa-miR-4294, hsa-miR-6836-3p, hsa-miR-4530, and hsa-miR-6880-5p described in Patent Literature 2 as being capable of specifically discriminating a pancreatic cancer patient group from other cancer patient groups were evaluated for their early pancreatic cancer or pancreatic cancer precursor lesion discriminant performance in the training cohort and the validation cohort obtained in the preceding Reference Examples. For example, the combination of the polynucleotides consisting of the nucleotide sequences represented by SEQ ID NOs: 219, 125, and 231 or complementary sequences thereof showed excellent accuracy of 93% in both of the training cohort and the validation cohort, but had sensitivity of 50% and specificity of 94.7% in the training cohort and sensitivity of 72.7% and specificity of 93.8% in the validation cohort and tended to differ in sensitivity between the training cohort and the validation cohort (Table 11).

**[Table 11]**

| | Training cohort | | | Validation cohort | | |
|---|---|---|---|---|---|---|
| SEQ ID NO: | Accuracy (%) | Sensitivity (%) | Specificity (%) | Accuracy (%) | Sensitivity (%) | Specificity (%) |
| 219_125_231 | 93 | 50 | 94.7 | 93 | 72.7 | 93.8 |

On the other hand, as shown in Tables 9-1 to 9-9 in Example 4, for example, the measurement using the combination of three polynucleotides comprising at least one polynucleotide selected from the group consisting of the polynucleotides consisting of the nucleotide sequences represented by SEQ ID NOs: 119, 12, 28, 105, 137, 121, 109, 87, 5, 140, 106, 2, 175, 90, 237, and 247 or complementary sequences thereof had the highest sensitivity of 100%, the lowest sensitivity of 88.7%, the highest specificity of 93.2%, and the lowest specificity of 88.8% in the training cohort and the highest sensitivity of 100%, the lowest sensitivity of 84.6%, the highest specificity of 94.2%, and the lowest specificity of 85.3% in the validation cohort and thus showed equivalently high sensitivity and specificity between the training cohort and the validation cohort. From these results, it can be concluded that the combination of two or more, preferably three or more, more preferably four or more or five or more polynucleotides comprising at least one polynucleotide selected from the group consisting of the polynucleotides consisting of the nucleotide sequences represented by SEQ ID NOs: 119, 12, 28, 105, 137, 121, 109, 87, 5, 140, 106, 2, 175, 90, 237, and 247 or complementary sequences thereof exhibits higher discriminant performance than that of the combination of existing pancreatic cancer miRNA markers in blood and serves as excellent diagnostic markers.

### [Comparative Example 3]

### <<Performance of existing pancreatic cancer miRNA markers in blood for early pancreatic cancer or pancreatic cancer precursor lesion>

hsa-miR-145-5p (SEQ ID NO: 813), hsa-let-7f-5p (SEQ ID NO: 814), hsa-miR-146a-5p (SEQ ID NO: 815), hsa-let-7d-5p (SEQ ID NO: 816), and hsa-let-7a-5p (SEQ ID NO: 817) having 3.0 or more fold change in their gene expression levels in a pancreatic cancer precursor lesion patient group relative to a healthy subject group were selected from among top 30 miRNAs that had statistically significant difference in their expression levels between a healthy subject group and an IPMN patient group in Patent Literature 5 and evaluated for their early pancreatic cancer or pancreatic cancer precursor lesion discriminant performance in the training cohort and the validation cohort obtained in the preceding Reference Examples. The combination of the polynucleotides consisting of the nucleotide sequences represented by SEQ ID NOs: 813 to 817 or complementary sequences thereof had sensitivity of 63.6% and specificity of 74.5% in the training cohort and sensitivity of 54.5% and specificity of 74.7% in the validation cohort (Table 12).

**[Table 12]**

| | Training cohort | | | Validation cohort | | |
|---|---|---|---|---|---|---|
| SEQ ID NO: | Accuracy (%) | Sensitivity (%) | Specificity (%) | Accuracy (%) | Sensitivity (%) | Specificity (%) |
| 813_814_815_816_817 | 74 | 63.6 | 74.5 | 73.9 | 54.5 | 74.7 |

On the other hand, as shown in Tables 9-1 to 9-9 in Example 4 described above, for example, the measurement using the combination of five polynucleotides comprising at least one polynucleotide selected from the group consisting of the polynucleotides consisting of the nucleotide sequences represented by SEQ ID NOs: 119, 12, 28, 105, 137, 121, 109, 87, 5, 140, 106, 2, 175, 90, 237, and 247 or complementary sequences thereof showed the highest sensitivity of 100%, the lowest sensitivity of 94.3%, the highest specificity of 98.6%, and the lowest specificity of 96.9% in the training cohort and the highest sensitivity of 100%, the lowest sensitivity of 92.3%, the highest specificity of 100%, and the lowest specificity of 92.3% in the validation cohort. From these results, it can be concluded that the combination of two or more, preferably three or more, more preferably four or more or five or more polynucleotides comprising at least one polynucleotide selected from the group consisting of the polynucleotides consisting of the nucleotide sequences represented by SEQ ID NOs: 119, 12, 28, 105, 137, 121, 109, 87, 5, 140, 106, 2, 175, 90, 237, and 247 or complementary sequences thereof exhibits higher discriminant performance than that of the combination of existing pancreatic cancer and pancreatic cancer precursor lesion miRNA markers in blood and serves as excellent diagnostic markers.

As shown in these Examples and Comparative Examples, the kit and the method of the present invention can detect early pancreatic cancer or a pancreatic cancer precursor lesion with higher sensitivity and specificity than the existing tumor markers and therefore permit early treatment and early decision to carry out the surgical resection of a cancer site. As a result, improvement in 5-year survival rate and reduction in the rate of recurrence can be achieved.

### INDUSTRIAL APPLICABILITY

According to the present invention, early pancreatic cancer or a pancreatic cancer precursor lesion can be detected in a subject with much higher sensitivity, specificity, and accuracy than conventional methods. This enables early detection, diagnosis and treatment of early pancreatic cancer or a pancreatic cancer precursor lesion. According to the present invention, early pancreatic cancer or a pancreatic cancer precursor lesion can be detected with limited invasiveness using the blood of a subject. This allows early pancreatic cancer or a pancreatic cancer precursor lesion to be determined conveniently, rapidly, and inexpensively.

All publications, patents, and patent applications cited herein are incorporated herein by reference in their entirety.
The following labelled statement set out further aspects of the present invention:
A1. A kit for the detection of early pancreatic cancer or a pancreatic cancer precursor lesion, comprising a nucleic acid(s) capable of specifically binding to at least one polynucleotides selected from the group consisting of the following early pancreatic cancer or pancreatic cancer precursor lesion markers: miR-6784-5p, miR-1181, miR-671-5p, miR-6857-5p, miR-4276, miR-1914-3p, miR-149-3p, miR-937-5p, miR-4675, miR-6795-5p, miR-4731-5p, miR-5090, miR-3620-5p, miR-1343-5p, miR-6717-5p, miR-6825-5p, miR-6738-5p, miR-6769a-5p, miR-4728-5p, miR-652-5p, miR-4257, miR-6785-5p, miR-7110-5p, miR-6887-5p, miR-887-3p, miR-1228-5p, miR-5572, miR-6782-5p, miR-4298, miR-6786-5p, miR-5010-5p, miR-6087, miR-6765-5p, miR-6732-5p, miR-6787-5p, miR-6737-5p, miR-128-2-5p, miR-4270, miR-6861-5p, miR-6756-5p, miR-1229-5p, miR-6891-5p, miR-6848-5p, miR-1237-5p, miR-30c-1-3p, miR-1233-5p, miR-211-3p, miR-4758-5p, miR-614, miR-6746-5p, miR-1915-5p, miR-4688, miR-3917, miR-5787, miR-4632-5p, miR-6126, miR-135a-3p, miR-8063, miR-5698, miR-6089, miR-498, miR-296-3p, miR-4419b, miR-6802-5p, miR-6829-5p, miR-6803-5p, miR-1199-5p, miR-6840-3p, miR-6752-5p, miR-6798-5p, miR-6131, miR-4667-5p, miR-6510-5p, miR-4690-5p, miR-920, miR-23b-3p, miR-4448, miR-2110, miR-4706, miR-7845-5p, miR-6808-5p, miR-4447, miR-6869-5p, miR-6794-5p, miR-6511a-5p, miR-6824-5p, miR-6766-3p, miR-6511a-5p, and miR-6749-5p.
A2. The kit according to clause A1, wherein miR-6784-5p is hsa-miR-6784-5p, miR-1181 is hsa-miR-1181, miR-671-5p is hsa-miR-671-5p, miR-6857-5p is hsa-miR-6857-5p, miR-4276 is hsa-miR-4276, miR-1914-3p is hsa-miR-1914-3p, miR-149-3p is hsa-miR-149-3p, miR-937-5p is hsa-miR-937-5p, miR-4675 is hsa-miR-4675, miR-6795-5p is hsa-miR-6795-5p, miR-4731-5p is hsa-miR-4731-5p, miR-5090 is hsa-miR-5090, miR-3620-5p is hsa-miR-3620-5p, miR-1343-5p is hsa-miR-1343-5p, miR-6717-5p is hsa-miR-6717-5p, miR-6825-5p is hsa-miR-6825-5p, miR-6738-5p is hsa-miR-6738-5p, miR-6769a-5p is hsa-miR-6769a-5p, miR-4728-5p is hsa-miR-4728-5p, miR-652-5p is hsa-miR-652-5p, miR-4257 is hsa-miR-4257, miR-6785-5p is hsa-miR-6785-5p, miR-7110-5p is hsa-miR-7110-5p, miR-6887-5p is hsa-miR-6887-5p, miR-887-3p is hsa-miR-887-3p, miR-1228-5p is hsa-miR-1228-5p, miR-5572 is hsa-miR-5572, miR-6782-5p is hsa-miR-6782-5p, miR-4298 is hsa-miR-4298, miR-6786-5p is hsa-miR-6786-5p, miR-5010-5p is hsa-miR-5010-5p, miR-6087 is hsa-miR-6087, miR-6765-5p is hsa-miR-6765-5p, miR-6732-5p is hsa-miR-6732-5p, miR-6787-5p is hsa-miR-6787-5p, miR-6737-5p is hsa-miR-6737-5p, miR-128-2-5p is hsa-miR-128-2-5p, miR-4270 is hsa-miR-4270, miR-6861-5p is hsa-miR-6861-5p, miR-6756-5p is hsa-miR-6756-5p, miR-1229-5p is hsa-miR-1229-5p, miR-6891-5p is hsa-miR-6891-5p, miR-6848-5p is hsa-miR-6848-5p, miR-1237-5p is hsa-miR-1237-5p, miR-30c-1-3p is hsa-miR-30c-1-3p, miR-1233-5p is hsa-miR-1233-5p, miR-211-3p is hsa-miR-211-3p, miR-4758-5p is hsa-miR-4758-5p, miR-614 is hsa-miR-614, miR-6746-5p is hsa-miR-6746-5p, miR-1915-5p is hsa-miR-1915-5p, miR-4688 is hsa-miR-4688, miR-3917 is hsa-miR-3917, miR-5787 is hsa-miR-5787, miR-4632-5p is hsa-miR-4632-5p, miR-6126 is hsa-miR-6126, miR-135a-3p is hsa-miR-135a-3p, miR-8063 is hsa-miR-8063, miR-5698 is hsa-miR-5698, miR-6089 is hsa-miR-6089, miR-498 is hsa-miR-498, miR-296-3p is hsa-miR-296-3p, miR-4419b is hsa-miR-4419b, miR-6802-5p is hsa-miR-6802-5p, miR-6829-5p is hsa-miR-6829-5p, miR-6803-5p is hsa-miR-6803-5p, miR-1199-5p is hsa-miR-1199-5p, miR-6840-3p is hsa-miR-6840-3p, miR-6752-5p is hsa-miR-6752-5p, miR-6798-5p is hsa-miR-6798-5p, miR-6131 is hsa-miR-6131, miR-4667-5p is hsa-miR-4667-5p, miR-6510-5p is hsa-miR-6510-5p, miR-4690-5p is hsa-miR-4690-5p, miR-920 is hsa-miR-920, miR-23b-3p is hsa-miR-23b-3p, miR-4448 is hsa-miR-4448, miR-2110 is hsa-miR-2110, miR-4706 is hsa-miR-4706, miR-7845-5p is hsa-miR-7845-5p, miR-6808-5p is hsa-miR-6808-5p, miR-4447 is hsa-miR-4447, miR-6869-5p is hsa-miR-6869-5p, miR-6794-5p is hsa-miR-6794-5p, miR-6511a-5p is hsa-miR-6511a-5p, miR-6824-5p is hsa-miR-6824-5p, miR-6766-3p is hsa-miR-6766-3p, miR-6511a-5p is hsa-miR-6511a-5p, and miR-6749-5p is hsa-miR-6749-5p.
A3. The kit according to clause A1 or A2, wherein the nucleic acid(s) is a polynucleotide(s) selected from the group consisting of the following polynucleotides (a) to (e):
   (a) a polynucleotide consisting of a nucleotide sequence represented by any of SEQ ID NOs: 1 to 83, 227 to 229, 246, 248, and 250 or a nucleotide sequence derived from the nucleotide sequence by the replacement of u with t, a variant thereof, a derivative thereof, or a fragment thereof comprising 15 or more consecutive nucleotides;
   (b) a polynucleotide comprising a nucleotide sequence represented by any of SEQ ID NOs: 1 to 83, 227 to 229, 246, 248, and 250;
   (c) a polynucleotide consisting of a nucleotide sequence complementary to a nucleotide sequence represented by any of SEQ ID NOs: 1 to 83, 227 to 229, 246, 248, and 250 or a nucleotide sequence derived from the nucleotide sequence by the replacement of u with t, a variant thereof, a derivative thereof, or a fragment thereof comprising 15 or more consecutive nucleotides;
   (d) a polynucleotide comprising a nucleotide sequence complementary to a nucleotide sequence represented by any of SEQ ID NOs: 1 to 83, 227 to 229, 246, 248, and 250 or a nucleotide sequence derived from the nucleotide sequence by the replacement of u with t; and
   (e) a polynucleotide hybridizing under stringent conditions to any of the polynucleotides (a) to (d).
A4. The kit according to any one of clauses A1 to A3, wherein the kit further comprises a nucleic acid or nucleic acids capable of specifically binding to at least one polynucleotides selected from the group consisting of the following other early pancreatic cancer or pancreatic cancer precursor lesion markers: miR-1908-5p, miR-6729-5p, miR-5195-3p, miR-638, miR-6125, miR-3178, miR-3196, miR-8069, miR-4723-5p, miR-4746-3p, miR-4689, miR-6816-5p, miR-6757-5p, miR-7109-5p, miR-6724-5p, miR-1225-3p, miR-6875-5p, miR-7108-5p, miR-4508, miR-6085, miR-6779-5p, miR-642a-3p, miR-4695-5p, miR-7847-3p, miR-3197, miR-6769b-5p, miR-7641, miR-187-5p, miR-3185, miR-2861, miR-3940-5p, miR-1203, miR-615-5p, miR-4787-5p, miR-1343-3p, miR-6813-5p, miR-1225-5p, miR-602, miR-4488, miR-125a-3p, miR-5100, miR-4294, miR-1231, miR-6765-3p, miR-4442, miR-718, miR-6780b-5p, miR-6090, miR-6845-5p, miR-4741, miR-4467, miR-4707-5p, miR-4271, miR-4673, miR-3184-5p, miR-1469, miR-4640-5p, miR-663a, miR-6791-5p, miR-6826-5p, miR-4433b-3p, miR-1915-3p, miR-4417, miR-4449, miR-4707-3p, miR-3180-3p, miR-5585-3p, miR-1268a, miR-8072, miR-296-5p, miR-204-3p, miR-4454, miR-6722-3p, miR-1290, miR-3622a-5p, miR-939-5p, miR-675-5p, miR-3131, miR-4648, miR-1268b, miR-6741-5p, miR-6893-5p, miR-3162-5p, miR-642b-3p, miR-4734, miR-150-3p, miR-8089, miR-6805-3p, miR-7113-3p, miR-6850-5p, miR-6799-5p, miR-6768-5p, miR-92b-5p, miR-3679-5p, miR-4792, miR-3656, miR-92a-2-5p, miR-4466, miR-4513, miR-6781-5p, miR-4649-5p, miR-6775-5p, miR-4651, miR-3195, miR-6726-5p, miR-6872-3p, miR-371a-5p, miR-6777-5p, miR-6789-5p, miR-7975, miR-6821-5p, miR-4534, miR-619-5p, miR-7107-5p, miR-1228-3p, miR-6774-5p, miR-6805-5p, miR-23a-3p, miR-4665-5p, miR-4505, miR-4638-5p, miR-24-3p, miR-3135b, miR-4745-5p, miR-128-1-5p, miR-4476, miR-4687-3p, miR-3665, miR-6806-5p, miR-3937, miR-711, miR-3141, miR-3188, miR-4281, miR-5196-5p, miR-6880-5p, miR-3960, miR-3648, miR-6721-5p, miR-4492, miR-744-5p, miR-7704, miR-4749-5p, miR-762, miR-6836-3p, miR-6727-5p, miR-4739, miR-7977, miR-4484, miR-6515-3p, miR-373-5p, miR-4258, miR-4674, miR-3180, miR-6076, miR-1238-5p, miR-4463, miR-4486, miR-4730, miR-4286, and miR-4739.
A5. The kit according to clause A4, wherein miR-1908-5p is hsa-miR-1908-5p, miR-6729-5p is hsa-miR-6729-5p, miR-5195-3p is hsa-miR-5195-3p, miR-638 is hsa-miR-638, miR-6125 is hsa-miR-6125, miR-3178 is hsa-miR-3178, miR-3196 is hsa-miR-3196, miR-8069 is hsa-miR-8069, miR-4723-5p is hsa-miR-4723-5p, miR-4746-3p is hsa-miR-4746-3p, miR-4689 is hsa-miR-4689, miR-6816-5p is hsa-miR-6816-5p, miR-6757-5p is hsa-miR-6757-5p, miR-7109-5p is hsa-miR-7109-5p, miR-6724-5p is hsa-miR-6724-5p, miR-1225-3p is hsa-miR-1225-3p, miR-6875-5p is hsa-miR-6875-5p, miR-7108-5p is hsa-miR-7108-5p, miR-4508 is hsa-miR-4508, miR-6085 is hsa-miR-6085, miR-6779-5p is hsa-miR-6779-5p, miR-642a-3p is hsa-miR-642a-3p, miR-4695-5p is hsa-miR-4695-5p, miR-7847-3p is hsa-miR-7847-3p, miR-3197 is hsa-miR-3197, miR-6769b-5p is hsa-miR-6769b-5p, miR-7641 is hsa-miR-7641, miR-187-5p is hsa-miR-187-5p, miR-3185 is hsa-miR-3185, miR-2861 is hsa-miR-2861, miR-3940-5p is hsa-miR-3940-5p, miR-1203 is hsa-miR-1203, miR-615-5p is hsa-miR-615-5p, miR-4787-5p is hsa-miR-4787-5p, miR-1343-3p is hsa-miR-1343-3p, miR-6813-5p is hsa-miR-6813-5p, miR-1225-5p is hsa-miR-1225-5p, miR-602 is hsa-miR-602, miR-4488 is hsa-miR-4488, miR-125a-3p is hsa-miR-125a-3p, miR-5100 is hsa-miR-5100, miR-4294 is hsa-miR-4294, miR-1231 is hsa-miR-1231, miR-6765-3p is hsa-miR-6765-3p, miR-4442 is hsa-miR-4442, miR-718 is hsa-miR-718, miR-6780b-5p is hsa-miR-6780b-5p, miR-6090 is hsa-miR-6090, miR-6845-5p is hsa-miR-6845-5p, miR-4741 is hsa-miR-4741, miR-4467 is hsa-miR-4467, miR-4707-5p is hsa-miR-4707-5p, miR-4271 is hsa-miR-4271, miR-4673 is hsa-miR-4673, miR-3184-5p is hsa-miR-3184-5p, miR-1469 is hsa-miR-1469, miR-4640-5p is hsa-miR-4640-5p, miR-663a is hsa-miR-663a, miR-6791-5p is hsa-miR-6791-5p, miR-6826-5p is hsa-miR-6826-5p, miR-4433b-3p is hsa-miR-4433b-3p, miR-1915-3p is hsa-miR-1915-3p, miR-4417 is hsa-miR-4417, miR-4449 is hsa-miR-4449, miR-4707-3p is hsa-miR-4707-3p, miR-3180-3p is hsa-miR-3180-3p, miR-5585-3p is hsa-miR-5585-3p, miR-1268a is hsa-miR-1268a, miR-8072 is hsa-miR-8072, miR-296-5p is hsa-miR-296-5p, miR-204-3p is hsa-miR-204-3p, miR-4454 is hsa-miR-4454, miR-6722-3p is hsa-miR-6722-3p, miR-1290 is hsa-miR-1290, miR-3622a-5p is hsa-miR-3622a-5p, miR-939-5p is hsa-miR-939-5p, miR-675-5p is hsa-miR-675-5p, miR-3131 is hsa-miR-3131, miR-4648 is hsa-miR-4648, miR-1268b is hsa-miR-1268b, miR-6741-5p is hsa-miR-6741-5p, miR-6893-5p is hsa-miR-6893-5p, miR-3162-5p is hsa-miR-3162-5p, miR-642b-3p is hsa-miR-642b-3p, miR-4734 is hsa-miR-4734, miR-150-3p is hsa-miR-150-3p, miR-8089 is hsa-miR-8089, miR-6805-3p is hsa-miR-6805-3p, miR-7113-3p is hsa-miR-7113-3p, miR-6850-5p is hsa-miR-6850-5p, miR-6799-5p is hsa-miR-6799-5p, miR-6768-5p is hsa-miR-6768-5p, miR-92b-5p is hsa-miR-92b-5p, miR-3679-5p is hsa-miR-3679-5p, miR-4792 is hsa-miR-4792, miR-3656 is hsa-miR-3656, miR-92a-2-5p is hsa-miR-92a-2-5p, miR-4466 is hsa-miR-4466, miR-4513 is hsa-miR-4513, miR-6781-5p is hsa-miR-6781-5p, miR-4649-5p is hsa-miR-4649-5p, miR-6775-5p is hsa-miR-6775-5p, miR-4651 is hsa-miR-4651, miR-3195 is hsa-miR-3195, miR-6726-5p is hsa-miR-6726-5p, miR-6872-3p is hsa-miR-6872-3p, miR-371a-5p is hsa-miR-371a-5p, miR-6777-5p is hsa-miR-6777-5p, miR-6789-5p is hsa-miR-6789-5p, miR-7975 is hsa-miR-7975, miR-6821-5p is hsa-miR-6821-5p, miR-4534 is hsa-miR-4534, miR-619-5p is hsa-miR-619-5p, miR-7107-5p is hsa-miR-7107-5p, miR-1228-3p is hsa-miR-1228-3p, miR-6774-5p is hsa-miR-6774-5p, miR-6805-5p is hsa-miR-6805-5p, miR-23a-3p is hsa-miR-23a-3p, miR-4665-5p is hsa-miR-4665-5p, miR-4505 is hsa-miR-4505, miR-4638-5p is hsa-miR-4638-5p, miR-24-3p is hsa-miR-24-3p, miR-3135b is hsa-miR-3135b, miR-4745-5p is hsa-miR-4745-5p, miR-128-1-5p is hsa-miR-128-1-5p, miR-4476 is hsa-miR-4476, miR-4687-3p is hsa-miR-4687-3p, miR-3665 is hsa-miR-3665, miR-6806-5p is hsa-miR-6806-5p, miR-3937 is hsa-miR-3937, miR-711 is hsa-miR-711, miR-3141 is hsa-miR-3141, miR-3188 is hsa-miR-3188, miR-4281 is hsa-miR-4281, miR-5196-5p is hsa-miR-5196-5p, miR-6880-5p is hsa-miR-6880-5p, miR-3960 is hsa-miR-3960, miR-3648 is hsa-miR-3648, miR-6721-5p is hsa-miR-6721-5p, miR-4492 is hsa-miR-4492, miR-744-5p is hsa-miR-744-5p, miR-7704 is hsa-miR-7704, miR-4749-5p is hsa-miR-4749-5p, miR-762 is hsa-miR-762, miR-6836-3p is hsa-miR-6836-3p, miR-6727-5p is hsa-miR-6727-5p, miR-4739 is hsa-miR-4739, miR-7977 is hsa-miR-7977, miR-4484 is hsa-miR-4484, miR-6515-3p is hsa-miR-6515-3p, miR-373-5p is hsa-miR-373-5p, miR-4258 is hsa-miR-4258, miR-4674 is hsa-miR-4674, miR-3180 is hsa-miR-3180, miR-6076 is hsa-miR-6076, miR-1238-5p is hsa-miR-1238-5p, miR-4463 is hsa-miR-4463, miR-4486 is hsa-miR-4486, miR-4730 is hsa-miR-4730, miR-4286 is hsa-miR-4286, and miR-4739 is hsa-miR-4739.
A6. The kit according to clause A4 or A5, wherein the nucleic acid(s) is a polynucleotide(s) selected from the group consisting of the following polynucleotides (f) to (j):
   (f) a polynucleotide consisting of a nucleotide sequence represented by any of SEQ ID NOs: 84 to 226, 230 to 245, 247, and 249 or a nucleotide sequence derived from the nucleotide sequence by the replacement of u with t, a variant thereof, a derivative thereof, or a fragment thereof comprising 15 or more consecutive nucleotides;
   (g) a polynucleotide comprising a nucleotide sequence represented by any of SEQ ID NOs: 84 to 226, 230 to 245, 247, and 249;
   (h) a polynucleotide consisting of a nucleotide sequence complementary to a nucleotide sequence represented by any of SEQ ID NOs: 84 to 226, 230 to 245, 247, and 249 or a nucleotide sequence derived from the nucleotide sequence by the replacement of u with t, a variant thereof, a derivative thereof, or a fragment thereof comprising 15 or more consecutive nucleotides;
   (i) a polynucleotide comprising a nucleotide sequence complementary to a nucleotide sequence represented by any of SEQ ID NOs: 84 to 226, 230 to 245, 247, and 249 or a nucleotide sequence derived from the nucleotide sequence by the replacement of u with t; and
   (j) a polynucleotide hybridizing under stringent conditions to any of the polynucleotides (f) to (i).
A7. A device for the detection of early pancreatic cancer or a pancreatic cancer precursor lesion, comprising a nucleic acid(s) capable of specifically binding to at least one polynucleotide selected from the following early pancreatic cancer or pancreatic cancer precursor lesion markers: miR-6784-5p, miR-1181, miR-671-5p, miR-6857-5p, miR-4276, miR-1914-3p, miR-149-3p, miR-937-5p, miR-4675, miR-6795-5p, miR-4731-5p, miR-5090, miR-3620-5p, miR-1343-5p, miR-6717-5p, miR-6825-5p, miR-6738-5p, miR-6769a-5p, miR-4728-5p, miR-652-5p, miR-4257, miR-6785-5p, miR-7110-5p, miR-6887-5p, miR-887-3p, miR-1228-5p, miR-5572, miR-6782-5p, miR-4298, miR-6786-5p, miR-5010-5p, miR-6087, miR-6765-5p, miR-6732-5p, miR-6787-5p, miR-6737-5p, miR-128-2-5p, miR-4270, miR-6861-5p, miR-6756-5p, miR-1229-5p, miR-6891-5p, miR-6848-5p, miR-1237-5p, miR-30c-1-3p, miR-1233-5p, miR-211-3p, miR-4758-5p, miR-614, miR-6746-5p, miR-1915-5p, miR-4688, miR-3917, miR-5787, miR-4632-5p, miR-6126, miR-135a-3p, miR-8063, miR-5698, miR-6089, miR-498, miR-296-3p, miR-4419b, miR-6802-5p, miR-6829-5p, miR-6803-5p, miR-1199-5p, miR-6840-3p, miR-6752-5p, miR-6798-5p, miR-6131, miR-4667-5p, miR-6510-5p, miR-4690-5p, miR-920, miR-23b-3p, miR-4448, miR-2110, miR-4706, miR-7845-5p, miR-6808-5p, miR-4447, miR-6869-5p, miR-6794-5p, miR-6511a-5p, miR-6824-5p, miR-6766-3p, miR-6511a-5p, and miR-6749-5p.
A8. The device according to clause A7, wherein miR-6784-5p is hsa-miR-6784-5p, miR-1181 is hsa-miR-1181, miR-671-5p is hsa-miR-671-5p, miR-6857-5p is hsa-miR-6857-5p, miR-4276 is hsa-miR-4276, miR-1914-3p is hsa-miR-1914-3p, miR-149-3p is hsa-miR-149-3p, miR-937-5p is hsa-miR-937-5p, miR-4675 is hsa-miR-4675, miR-6795-5p is hsa-miR-6795-5p, miR-4731-5p is hsa-miR-4731-5p, miR-5090 is hsa-miR-5090, miR-3620-5p is hsa-miR-3620-5p, miR-1343-5p is hsa-miR-1343-5p, miR-6717-5p is hsa-miR-6717-5p, miR-6825-5p is hsa-miR-6825-5p, miR-6738-5p is hsa-miR-6738-5p, miR-6769a-5p is hsa-miR-6769a-5p, miR-4728-5p is hsa-miR-4728-5p, miR-652-5p is hsa-miR-652-5p, miR-4257 is hsa-miR-4257, miR-6785-5p is hsa-miR-6785-5p, miR-7110-5p is hsa-miR-7110-5p, miR-6887-5p is hsa-miR-6887-5p, miR-887-3p is hsa-miR-887-3p, miR-1228-5p is hsa-miR-1228-5p, miR-5572 is hsa-miR-5572, miR-6782-5p is hsa-miR-6782-5p, miR-4298 is hsa-miR-4298, miR-6786-5p is hsa-miR-6786-5p, miR-5010-5p is hsa-miR-5010-5p, miR-6087 is hsa-miR-6087, miR-6765-5p is hsa-miR-6765-5p, miR-6732-5p is hsa-miR-6732-5p, miR-6787-5p is hsa-miR-6787-5p, miR-6737-5p is hsa-miR-6737-5p, miR-128-2-5p is hsa-miR-128-2-5p, miR-4270 is hsa-miR-4270, miR-6861-5p is hsa-miR-6861-5p, miR-6756-5p is hsa-miR-6756-5p, miR-1229-5p is hsa-miR-1229-5p, miR-6891-5p is hsa-miR-6891-5p, miR-6848-5p is hsa-miR-6848-5p, miR-1237-5p is hsa-miR-1237-5p, miR-30c-1-3p is hsa-miR-30c-1-3p, miR-1233-5p is hsa-miR-1233-5p, miR-211-3p is hsa-miR-211-3p, miR-4758-5p is hsa-miR-4758-5p, miR-614 is hsa-miR-614, miR-6746-5p is hsa-miR-6746-5p, miR-1915-5p is hsa-miR-1915-5p, miR-4688 is hsa-miR-4688, miR-3917 is hsa-miR-3917, miR-5787 is hsa-miR-5787, miR-4632-5p is hsa-miR-4632-5p, miR-6126 is hsa-miR-6126, miR-135a-3p is hsa-miR-135a-3p, miR-8063 is hsa-miR-8063, miR-5698 is hsa-miR-5698, miR-6089 is hsa-miR-6089, miR-498 is hsa-miR-498, miR-296-3p is hsa-miR-296-3p, miR-4419b is hsa-miR-4419b, miR-6802-5p is hsa-miR-6802-5p, miR-6829-5p is hsa-miR-6829-5p, miR-6803-5p is hsa-miR-6803-5p, miR-1199-5p is hsa-miR-1199-5p, miR-6840-3p is hsa-miR-6840-3p, miR-6752-5p is hsa-miR-6752-5p, miR-6798-5p is hsa-miR-6798-5p, miR-6131 is hsa-miR-6131, miR-4667-5p is hsa-miR-4667-5p, miR-6510-5p is hsa-miR-6510-5p, miR-4690-5p is hsa-miR-4690-5p, miR-920 is hsa-miR-920, miR-23b-3p is hsa-miR-23b-3p, miR-4448 is hsa-miR-4448, miR-2110 is hsa-miR-2110, miR-4706 is hsa-miR-4706, miR-7845-5p is hsa-miR-7845-5p, miR-6808-5p is hsa-miR-6808-5p, miR-4447 is hsa-miR-4447, miR-6869-5p is hsa-miR-6869-5p, miR-6794-5p is hsa-miR-6794-5p, miR-6511a-5p is hsa-miR-6511a-5p, miR-6824-5p is hsa-miR-6824-5p, miR-6766-3p is hsa-miR-6766-3p, miR-6511a-5p is hsa-miR-6511a-5p, and miR-6749-5p is hsa-miR-6749-5p.
A9. The device according to clause A7 or A8, wherein the nucleic acid(s) is a polynucleotide(s) selected from the group consisting of the following polynucleotides (a) to (e):
   (a) a polynucleotide consisting of a nucleotide sequence represented by any of SEQ ID NOs: 1 to 83, 227 to 229, 246, 248, and 250 or a nucleotide sequence derived from the nucleotide sequence by the replacement of u with t, a variant thereof, a derivative thereof, or a fragment thereof comprising 15 or more consecutive nucleotides;
   (b) a polynucleotide comprising a nucleotide sequence represented by any of SEQ ID NOs: 1 to 83, 227 to 229, 246, 248, and 250;
   (c) a polynucleotide consisting of a nucleotide sequence complementary to a nucleotide sequence represented by any of SEQ ID NOs: 1 to 83, 227 to 229, 246, 248, and 250 or a nucleotide sequence derived from the nucleotide sequence by the replacement of u with t, a variant thereof, a derivative thereof, or a fragment thereof comprising 15 or more consecutive nucleotides;
   (d) a polynucleotide comprising a nucleotide sequence complementary to a nucleotide sequence represented by any of SEQ ID NOs: 1 to 83, 227 to 229, 246, 248, and 250 or a nucleotide sequence derived from the nucleotide sequence by the replacement of u with t; and
   (e) a polynucleotide hybridizing under stringent conditions to any of the polynucleotides (a) to (d).
A10. The device according to any one of clauses A7 to A9, wherein the device further comprises a nucleic acid(s) capable of specifically binding to at least one polynucleotides selected from the group consisting of the following other early pancreatic cancer or pancreatic cancer precursor lesion markers: miR-1908-5p, miR-6729-5p, miR-5195-3p, miR-638, miR-6125, miR-3178, miR-3196, miR-8069, miR-4723-5p, miR-4746-3p, miR-4689, miR-6816-5p, miR-6757-5p, miR-7109-5p, miR-6724-5p, miR-1225-3p, miR-6875-5p, miR-7108-5p, miR-4508, miR-6085, miR-6779-5p, miR-642a-3p, miR-4695-5p, miR-7847-3p, miR-3197, miR-6769b-5p, miR-7641, miR-187-5p, miR-3185, miR-2861, miR-3940-5p, miR-1203, miR-615-5p, miR-4787-5p, miR-1343-3p, miR-6813-5p, miR-1225-5p, miR-602, miR-4488, miR-125a-3p, miR-5100, miR-4294, miR-1231, miR-6765-3p, miR-4442, miR-718, miR-6780b-5p, miR-6090, miR-6845-5p, miR-4741, miR-4467, miR-4707-5p, miR-4271, miR-4673, miR-3184-5p, miR-1469, miR-4640-5p, miR-663a, miR-6791-5p, miR-6826-5p, miR-4433b-3p, miR-1915-3p, miR-4417, miR-4449, miR-4707-3p, miR-3180-3p, miR-5585-3p, miR-1268a, miR-8072, miR-296-5p, miR-204-3p, miR-4454, miR-6722-3p, miR-1290, miR-3622a-5p, miR-939-5p, miR-675-5p, miR-3131, miR-4648, miR-1268b, miR-6741-5p, miR-6893-5p, miR-3162-5p, miR-642b-3p, miR-4734, miR-150-3p, miR-8089, miR-6805-3p, miR-7113-3p, miR-6850-5p, miR-6799-5p, miR-6768-5p, miR-92b-5p, miR-3679-5p, miR-4792, miR-3656, miR-92a-2-5p, miR-4466, miR-4513, miR-6781-5p, miR-4649-5p, miR-6775-5p, miR-4651, miR-3195, miR-6726-5p, miR-6872-3p, miR-371a-5p, miR-6777-5p, miR-6789-5p, miR-7975, miR-6821-5p, miR-4534, miR-619-5p, miR-7107-5p, miR-1228-3p, miR-6774-5p, miR-6805-5p, miR-23a-3p, miR-4665-5p, miR-4505, miR-4638-5p, miR-24-3p, miR-3135b, miR-4745-5p, miR-128-1-5p, miR-4476, miR-4687-3p, miR-3665, miR-6806-5p, miR-3937, miR-711, miR-3141, miR-3188, miR-4281, miR-5196-5p, miR-6880-5p, miR-3960, miR-3648, miR-6721-5p, miR-4492, miR-744-5p, miR-7704, miR-4749-5p, miR-762, miR-6836-3p, miR-6727-5p, miR-4739, miR-7977, miR-4484, miR-6515-3p, miR-373-5p, miR-4258, miR-4674, miR-3180, miR-6076, miR-1238-5p, miR-4463, miR-4486, miR-4730, miR-4286, and miR-4739.
A11. The device according to clause A10, wherein miR-1908-5p is hsa-miR-1908-5p, miR-6729-5p is hsa-miR-6729-5p, miR-5195-3p is hsa-miR-5195-3p, miR-638 is hsa-miR-638, miR-6125 is hsa-miR-6125, miR-3178 is hsa-miR-3178, miR-3196 is hsa-miR-3196, miR-8069 is hsa-miR-8069, miR-4723-5p is hsa-miR-4723-5p, miR-4746-3p is hsa-miR-4746-3p, miR-4689 is hsa-miR-4689, miR-6816-5p is hsa-miR-6816-5p, miR-6757-5p is hsa-miR-6757-5p, miR-7109-5p is hsa-miR-7109-5p, miR-6724-5p is hsa-miR-6724-5p, miR-1225-3p is hsa-miR-1225-3p, miR-6875-5p is hsa-miR-6875-5p, miR-7108-5p is hsa-miR-7108-5p, miR-4508 is hsa-miR-4508, miR-6085 is hsa-miR-6085, miR-6779-5p is hsa-miR-6779-5p, miR-642a-3p is hsa-miR-642a-3p, miR-4695-5p is hsa-miR-4695-5p, miR-7847-3p is hsa-miR-7847-3p, miR-3197 is hsa-miR-3197, miR-6769b-5p is hsa-miR-6769b-5p, miR-7641 is hsa-miR-7641, miR-187-5p is hsa-miR-187-5p, miR-3185 is hsa-miR-3185, miR-2861 is hsa-miR-2861, miR-3940-5p is hsa-miR-3940-5p, miR-1203 is hsa-miR-1203, miR-615-5p is hsa-miR-615-5p, miR-4787-5p is hsa-miR-4787-5p, miR-1343-3p is hsa-miR-1343-3p, miR-6813-5p is hsa-miR-6813-5p, miR-1225-5p is hsa-miR-1225-5p, miR-602 is hsa-miR-602, miR-4488 is hsa-miR-4488, miR-125a-3p is hsa-miR-125a-3p, miR-5100 is hsa-miR-5100, miR-4294 is hsa-miR-4294, miR-1231 is hsa-miR-1231, miR-6765-3p is hsa-miR-6765-3p, miR-4442 is hsa-miR-4442, miR-718 is hsa-miR-718, miR-6780b-5p is hsa-miR-6780b-5p, miR-6090 is hsa-miR-6090, miR-6845-5p is hsa-miR-6845-5p, miR-4741 is hsa-miR-4741, miR-4467 is hsa-miR-4467, miR-4707-5p is hsa-miR-4707-5p, miR-4271 is hsa-miR-4271, miR-4673 is hsa-miR-4673, miR-3184-5p is hsa-miR-3184-5p, miR-1469 is hsa-miR-1469, miR-4640-5p is hsa-miR-4640-5p, miR-663a is hsa-miR-663a, miR-6791-5p is hsa-miR-6791-5p, miR-6826-5p is hsa-miR-6826-5p, miR-4433b-3p is hsa-miR-4433b-3p, miR-1915-3p is hsa-miR-1915-3p, miR-4417 is hsa-miR-4417, miR-4449 is hsa-miR-4449, miR-4707-3p is hsa-miR-4707-3p, miR-3180-3p is hsa-miR-3180-3p, miR-5585-3p is hsa-miR-5585-3p, miR-1268a is hsa-miR-1268a, miR-8072 is hsa-miR-8072, miR-296-5p is hsa-miR-296-5p, miR-204-3p is hsa-miR-204-3p, miR-4454 is hsa-miR-4454, miR-6722-3p is hsa-miR-6722-3p, miR-1290 is hsa-miR-1290, miR-3622a-5p is hsa-miR-3622a-5p, miR-939-5p is hsa-miR-939-5p, miR-675-5p is hsa-miR-675-5p, miR-3131 is hsa-miR-3131, miR-4648 is hsa-miR-4648, miR-1268b is hsa-miR-1268b, miR-6741-5p is hsa-miR-6741-5p, miR-6893-5p is hsa-miR-6893-5p, miR-3162-5p is hsa-miR-3162-5p, miR-642b-3p is hsa-miR-642b-3p, miR-4734 is hsa-miR-4734, miR-150-3p is hsa-miR-150-3p, miR-8089 is hsa-miR-8089, miR-6805-3p is hsa-miR-6805-3p, miR-7113-3p is hsa-miR-7113-3p, miR-6850-5p is hsa-miR-6850-5p, miR-6799-5p is hsa-miR-6799-5p, miR-6768-5p is hsa-miR-6768-5p, miR-92b-5p is hsa-miR-92b-5p, miR-3679-5p is hsa-miR-3679-5p, miR-4792 is hsa-miR-4792, miR-3656 is hsa-miR-3656, miR-92a-2-5p is hsa-miR-92a-2-5p, miR-4466 is hsa-miR-4466, miR-4513 is hsa-miR-4513, miR-6781-5p is hsa-miR-6781-5p, miR-4649-5p is hsa-miR-4649-5p, miR-6775-5p is hsa-miR-6775-5p, miR-4651 is hsa-miR-4651, miR-3195 is hsa-miR-3195, miR-6726-5p is hsa-miR-6726-5p, miR-6872-3p is hsa-miR-6872-3p, miR-371a-5p is hsa-miR-371a-5p, miR-6777-5p is hsa-miR-6777-5p, miR-6789-5p is hsa-miR-6789-5p, miR-7975 is hsa-miR-7975, miR-6821-5p is hsa-miR-6821-5p, miR-4534 is hsa-miR-4534, miR-619-5p is hsa-miR-619-5p, miR-7107-5p is hsa-miR-7107-5p, miR-1228-3p is hsa-miR-1228-3p, miR-6774-5p is hsa-miR-6774-5p, miR-6805-5p is hsa-miR-6805-5p, miR-23a-3p is hsa-miR-23a-3p, miR-4665-5p is hsa-miR-4665-5p, miR-4505 is hsa-miR-4505, miR-4638-5p is hsa-miR-4638-5p, miR-24-3p is hsa-miR-24-3p, miR-3135b is hsa-miR-3135b, miR-4745-5p is hsa-miR-4745-5p, miR-128-1-5p is hsa-miR-128-1-5p, miR-4476 is hsa-miR-4476, miR-4687-3p is hsa-miR-4687-3p, miR-3665 is hsa-miR-3665, miR-6806-5p is hsa-miR-6806-5p, miR-3937 is hsa-miR-3937, miR-711 is hsa-miR-711, miR-3141 is hsa-miR-3141, miR-3188 is hsa-miR-3188, miR-4281 is hsa-miR-4281, miR-5196-5p is hsa-miR-5196-5p, miR-6880-5p is hsa-miR-6880-5p, miR-3960 is hsa-miR-3960, miR-3648 is hsa-miR-3648, miR-6721-5p is hsa-miR-6721-5p, miR-4492 is hsa-miR-4492, miR-744-5p is hsa-miR-744-5p, miR-7704 is hsa-miR-7704, miR-4749-5p is hsa-miR-4749-5p, miR-762 is hsa-miR-762, miR-6836-3p is hsa-miR-6836-3p, miR-6727-5p is hsa-miR-6727-5p, miR-4739 is hsa-miR-4739, miR-7977 is hsa-miR-7977, miR-4484 is hsa-miR-4484, miR-6515-3p is hsa-miR-6515-3p, miR-373-5p is hsa-miR-373-5p, miR-4258 is hsa-miR-4258, miR-4674 is hsa-miR-4674, miR-3180 is hsa-miR-3180, miR-6076 is hsa-miR-6076, miR-1238-5p is hsa-miR-1238-5p, miR-4463 is hsa-miR-4463, miR-4486 is hsa-miR-4486, miR-4730 is hsa-miR-4730, miR-4286 is hsa-miR-4286, and miR-4739 is hsa-miR-4739.
A12. The device according to clause A10 or A11, wherein the nucleic acid(s) is/are a polynucleotide(s) selected from the group consisting of the following polynucleotides (f) to (j):
   (f) a polynucleotide consisting of a nucleotide sequence represented by any of SEQ ID NOs: 84 to 226, 230 to 245, 247, and 249 or a nucleotide sequence derived from the nucleotide sequence by the replacement of u with t, a variant thereof, a derivative thereof, or a fragment thereof comprising 15 or more consecutive nucleotides;
   (g) a polynucleotide comprising a nucleotide sequence represented by any of SEQ ID NOs: 84 to 226, 230 to 245, 247, and 249;
   (h) a polynucleotide consisting of a nucleotide sequence complementary to a nucleotide sequence represented by any of SEQ ID NOs: 84 to 226, 230 to 245, 247, and 249 or a nucleotide sequence derived from the nucleotide sequence by the replacement of u with t, a variant thereof, a derivative thereof, or a fragment thereof comprising 15 or more consecutive nucleotides;
   (i) a polynucleotide comprising a nucleotide sequence complementary to a nucleotide sequence represented by any of SEQ ID NOs: 84 to 226, 230 to 245, 247, and 249 or a nucleotide sequence derived from the nucleotide sequence by the replacement of u with t; and
   (j) a polynucleotide hybridizing under stringent conditions to any of the polynucleotides (f) to (i).
A13. The device according to any one of clauses A7 to A12, wherein the device is a device for measurement by a hybridization technique.
A14. The device according to clause A13, wherein the hybridization technique is a nucleic acid array technique.
A15. A method for detecting early pancreatic cancer or a pancreatic cancer precursor lesion in a subject, comprising: measuring an expression level(s) of a target nucleic acid(s) in a sample from the subject using a kit according to any one of clauses A1 to A6 or a device according to any one of clauses A7 to A14; and evaluating *in vitro* whether or not the subject has early pancreatic cancer or a pancreatic cancer precursor lesion using both of the measured expression level(s) and a control expression level(s) in a sample from a healthy subject measured in the same way, to detect the presence or absence of early pancreatic cancer or a pancreatic cancer precursor lesion in the subject.
A16. A method for detecting early pancreatic cancer or a pancreatic cancer precursor lesion in a subject, comprising: measuring an expression level(s) of a target gene(s) in a sample from the subject using a kit according to any one of clauses A1 to A6 or a device according to any one of clauses A7 to A14; and assigning the expression level(s) of the target gene(s) in the sample from the subject to a discriminant to evaluate the presence or absence of early pancreatic cancer or a pancreatic cancer precursor lesion, wherein the discriminant is prepared with the gene expression level(s) in a sample(s) from a subject(s) known to have early pancreatic cancer or a pancreatic cancer precursor lesion and the gene expression level(s) in a sample(s) from a healthy subject(s) as supervising samples and is capable of discriminating an early pancreatic cancer or pancreatic cancer precursor lesion patient from a healthy subject.
A17. The method according to clause A15 or A16, wherein the subject is a human.
A18. The method according to any one of clauses A15 to A17, wherein the sample is blood, serum, or plasma.

## Claims

1. *In vitro* use of a kit for the detection of early pancreatic cancer or a pancreatic cancer precursor lesion, comprising a nucleic acid(s) capable of specifically binding to the early pancreatic cancer or pancreatic cancer precursor lesion marker: miR-4632-5p.

2. Use of the kit according to claim 1, wherein the nucleic acid is a polynucleotide selected from the group consisting of the following polynucleotides (a) to (e):
(a) a polynucleotide consisting of a nucleotide sequence represented by SEQ ID NO: 55, or a nucleotide sequence derived from the nucleotide sequence by the replacement of u with t, a variant thereof, a derivative thereof, or a fragment thereof comprising 15 or more consecutive nucleotides;
(b) a polynucleotide comprising a nucleotide sequence represented by SEQ ID NO: 55;
(c) a polynucleotide consisting of a nucleotide sequence complementary to a nucleotide sequence represented by SEQ ID NO: 55 or a nucleotide sequence derived from the nucleotide sequence by the replacement of u with t, a variant thereof, a derivative thereof, or a fragment thereof comprising 15 or more consecutive nucleotides;
(d) a polynucleotide comprising a nucleotide sequence complementary to a nucleotide sequence represented by SEQ ID NO: 55 or a nucleotide sequence derived from the nucleotide sequence by the replacement of u with t; and
(e) a polynucleotide hybridizing under stringent conditions to any of the polynucleotides (a) to (d).

3. Use of the kit according to claim 1 or 2, wherein the kit further comprises a nucleic acid or nucleotide acids capable of specifically binding to at least one polynucleotides selected from the group consisting of the following other early pancreatic cancer or pancreatic cancer precursor lesion markers: miR-1181, miR-671-5p, miR-6857-5p, miR-4276, miR-1914-3p, miR-149-3p, miR-937-5p, miR-4675, miR-6795-5p, miR-4731-5p, miR-5090, miR-3620-5p, miR-6717-5p, miR-6825-5p, miR-6738-5p, miR-6769a-5p, miR-4728-5p, miR-652-5p, miR-4257, miR-6785-5p, miR-7110-5p, miR-6887-5p, miR-887-3p, miR-1228-5p, miR-5572, miR-6782-5p, miR-4298, miR-6786-5p, miR-5010-5p, miR-6087, miR-6765-5p, miR-6732-5p, miR-6787-5p, miR-6737-5p, miR-128-2-5p, miR-4270, miR-6861-5p, miR-6756-5p, miR-1229-5p, miR-6891-5p, miR-6848-5p, miR-1237-5p, miR-30c-1-3p, miR-1233-5p, miR-211-3p, miR-4758-5p, miR-614, miR-6746-5p, miR-1915-5p, miR-4688, miR-3917, miR-5787, miR-6126, miR-135a-3p, miR-8063, miR-5698, miR-6089, miR-498, miR-296-3p, miR-4419b, miR-6802-5p, miR-6829-5p, miR-6803-5p, miR-1199-5p, miR-6840-3p, miR-6752-5p, miR-6798-5p, miR-6131, miR-4667-5p, miR-6510-5p, miR-4690-5p, miR-920, miR-23b-3p, miR-4448, miR-2110, miR-4706, miR-7845-5p, miR-6808-5p, miR-4447, miR-6869-5p, miR-6794-5p, miR-6511a-5p, miR-6824-5p, miR-6766-3p, miR-6511a-5p, and miR-6749-5p, miR-1908-5p, miR-6729-5p, miR-5195-3p, miR-638, miR-6125, miR-3178, miR-3196, miR-8069, miR-4723-5p, miR-4746-3p, miR-4689, miR-6816-5p, miR-6757-5p, miR-7109-5p, miR-6724-5p, miR-1225-3p, miR-6875-5p, miR-7108-5p, miR-4508, miR-6085, miR-6779-5p, miR-642a-3p, miR-4695-5p, miR-7847-3p, miR-3197, miR-6769b-5p, miR-7641, miR-187-5p, miR-3185, miR-2861, miR-3940-5p, miR-1203, miR-615-5p, miR-4787-5p, miR-1343-3p, miR-6813-5p, miR-1225-5p, miR-602, miR-4488, miR-125a-3p, miR-5100, miR-4294, miR-1231, miR-6765-3p, miR-4442, miR-718, miR-6780b-5p, miR-6090, miR-6845-5p, miR-4741, miR-4467, miR-4707-5p, miR-4271, miR-4673, miR-3184-5p, miR-1469, miR-4640-5p, miR-663a, miR-6791-5p, miR-6826-5p, miR-4433b-3p, miR-1915-3p, miR-4417, miR-4449, miR-4707-3p, miR-3180-3p, miR-5585-3p, miR-1268a, miR-8072, miR-296-5p, miR-204-3p, miR-4454, miR-6722-3p, miR-1290, miR-3622a-5p, miR-939-5p, miR-675-5p, miR-3131, miR-4648, miR-1268b, miR-6741-5p, miR-6893-5p, miR-3162-5p, miR-642b-3p, miR-4734, miR-150-3p, miR-8089, miR-6805-3p, miR-7113-3p, miR-6850-5p, miR-6799-5p, miR-6768-5p, miR-92b-5p, miR-3679-5p, miR-4792, miR-3656, miR-92a-2-5p, miR-4466, miR-4513, miR-6781-5p, miR-4649-5p, miR-6775-5p, miR-4651, miR-3195, miR-6726-5p, miR-6872-3p, miR-371a-5p, miR-6777-5p, miR-6789-5p, miR-7975, miR-6821-5p, miR-4534, miR-619-5p, miR-7107-5p, miR-1228-3p, miR-6774-5p, miR-6805-5p, miR-23a-3p, miR-4665-5p, miR-4505, miR-4638-5p, miR-24-3p, miR-3135b, miR-4745-5p, miR-128-1-5p, miR-4476, miR-4687-3p, miR-3665, miR-6806-5p, miR-3937, miR-711, miR-3141, miR-3188, miR-4281, miR-5196-5p, miR-6880-5p, miR-3960, miR-3648, miR-6721-5p, miR-4492, miR-744-5p, miR-7704, miR-4749-5p, miR-762, miR-6836-3p, miR-6727-5p, miR-4739, miR-7977, miR-4484, miR-6515-3p, miR-373-5p, miR-4258, miR-4674, miR-3180, miR-6076, miR-1238-5p, miR-4463, miR-4486, miR-4730, miR-4286, and miR-4739.

4. Use of the kit according to claim 3, wherein the nucleic acid(s) is a polynucleotide(s) selected from the group consisting of the following polynucleotides (f) to (j):
(f) a polynucleotide consisting of a nucleotide sequence represented by any of SEQ ID NOs: 2 to 13, 15 to 54, 56 to 83, 227 to 229, 246, 248, and 250, 84 to 226, 230 to 245, 247, and 249 or a nucleotide sequence derived from the nucleotide sequence by the replacement of u with t, a variant thereof, a derivative thereof, or a fragment thereof comprising 15 or more consecutive nucleotides;
(g) a polynucleotide comprising a nucleotide sequence represented by any of SEQ ID NOs: 2 to 13, 15 to 54, 56 to 83, 227 to 229, 246, 248, and 250, 84 to 226, 230 to 245, 247, and 249;
(h) a polynucleotide consisting of a nucleotide sequence complementary to a nucleotide sequence represented by any of SEQ ID NOs: 2 to 13, 15 to 54, 56 to 83, 227 to 229, 246, 248, and 250, 84 to 226, 230 to 245, 247, and 249 or a nucleotide sequence derived from the nucleotide sequence by the replacement of u with t, a variant thereof, a derivative thereof, or a fragment thereof comprising 15 or more consecutive nucleotides;
(i) a polynucleotide comprising a nucleotide sequence complementary to a nucleotide sequence represented by any of SEQ ID NOs: 2 to 13, 15 to 54, 56 to 83, 227 to 229, 246, 248, and 250, 84 to 226, 230 to 245, 247, and 249 or a nucleotide sequence derived from the nucleotide sequence by the replacement of u with t; and
(j) a polynucleotide hybridizing under stringent conditions to any of the polynucleotides (f) to (i).

5. *In vitro* use of a device for the detection of early pancreatic cancer or a pancreatic cancer precursor lesion, comprising a nucleic acid(s) capable of specifically binding to the early pancreatic cancer or pancreatic cancer precursor lesion marker: miR-4632-5p.

6. Use of the device according to claim 5, wherein the nucleic acid(s) is a polynucleotide(s) selected from the group consisting of the following polynucleotides (a) to (e):
(a) a polynucleotide consisting of a nucleotide sequence represented by SEQ ID NO: 55, or a nucleotide sequence derived from the nucleotide sequence by the replacement of u with t, a variant thereof, a derivative thereof, or a fragment thereof comprising 15 or more consecutive nucleotides;
(b) a polynucleotide comprising a nucleotide sequence represented by SEQ ID NO: 55;
(c) a polynucleotide consisting of a nucleotide sequence complementary to a nucleotide sequence represented by SEQ ID NO: 55 or a nucleotide sequence derived from the nucleotide sequence by the replacement of u with t, a variant thereof, a derivative thereof, or a fragment thereof comprising 15 or more consecutive nucleotides;
(d) a polynucleotide comprising a nucleotide sequence complementary to a nucleotide sequence represented by SEQ ID NO: 55 or a nucleotide sequence derived from the nucleotide sequence by the replacement of u with t; and
(e) a polynucleotide hybridizing under stringent conditions to any of the polynucleotides (a) to (d).

7. Use of the device according to claim 5 or 6, wherein the device further comprises a nucleic acid(s) capable of specifically binding to at least one polynucleotides selected from the group consisting of the following other early pancreatic cancer or pancreatic cancer precursor lesion markers: miR-1181, miR-671-5p, miR-6857-5p, miR-4276, miR-1914-3p, miR-149-3p, miR-937-5p, miR-4675, miR-6795-5p, miR-4731-5p, miR-5090, miR-3620-5p, miR-6717-5p, miR-6825-5p, miR-6738-5p, miR-6769a-5p, miR-4728-5p, miR-652-5p, miR-4257, miR-6785-5p, miR-7110-5p, miR-6887-5p, miR-887-3p, miR-1228-5p, miR-5572, miR-6782-5p, miR-4298, miR-6786-5p, miR-5010-5p, miR-6087, miR-6765-5p, miR-6732-5p, miR-6787-5p, miR-6737-5p, miR-128-2-5p, miR-4270, miR-6861-5p, miR-6756-5p, miR-1229-5p, miR-6891-5p, miR-6848-5p, miR-1237-5p, miR-30c-1-3p, miR-1233-5p, miR-211-3p, miR-4758-5p, miR-614, miR-6746-5p, miR-1915-5p, miR-4688, miR-3917, miR-5787, miR-6126, miR-135a-3p, miR-8063, miR-5698, miR-6089, miR-498, miR-296-3p, miR-4419b, miR-6802-5p, miR-6829-5p, miR-6803-5p, miR-1199-5p, miR-6840-3p, miR-6752-5p, miR-6798-5p, miR-6131, miR-4667-5p, miR-6510-5p, miR-4690-5p, miR-920, miR-23b-3p, miR-4448, miR-2110, miR-4706, miR-7845-5p, miR-6808-5p, miR-4447, miR-6869-5p, miR-6794-5p, miR-6511a-5p, miR-6824-5p, miR-6766-3p, miR-6511a-5p, and miR-6749-5p, miR-1908-5p, miR-6729-5p, miR-5195-3p, miR-638, miR-6125, miR-3178, miR-3196, miR-8069, miR-4723-5p, miR-4746-3p, miR-4689, miR-6816-5p, miR-6757-5p, miR-7109-5p, miR-6724-5p, miR-1225-3p, miR-6875-5p, miR-7108-5p, miR-4508, miR-6085, miR-6779-5p, miR-642a-3p, miR-4695-5p, miR-7847-3p, miR-3197, miR-6769b-5p, miR-7641, miR-187-5p, miR-3185, miR-2861, miR-3940-5p, miR-1203, miR-615-5p, miR-4787-5p, miR-1343-3p, miR-6813-5p, miR-1225-5p, miR-602, miR-4488, miR-125a-3p, miR-5100, miR-4294, miR-1231, miR-6765-3p, miR-4442, miR-718, miR-6780b-5p, miR-6090, miR-6845-5p, miR-4741, miR-4467, miR-4707-5p, miR-4271, miR-4673, miR-3184-5p, miR-1469, miR-4640-5p, miR-663a, miR-6791-5p, miR-6826-5p, miR-4433b-3p, miR-1915-3p, miR-4417, miR-4449, miR-4707-3p, miR-3180-3p, miR-5585-3p, miR-1268a, miR-8072, miR-296-5p, miR-204-3p, miR-4454, miR-6722-3p, miR-1290, miR-3622a-5p, miR-939-5p, miR-675-5p, miR-3131, miR-4648, miR-1268b, miR-6741-5p, miR-6893-5p, miR-3162-5p, miR-642b-3p, miR-4734, miR-150-3p, miR-8089, miR-6805-3p, miR-7113-3p, miR-6850-5p, miR-6799-5p, miR-6768-5p, miR-92b-5p, miR-3679-5p, miR-4792, miR-3656, miR-92a-2-5p, miR-4466, miR-4513, miR-6781-5p, miR-4649-5p, miR-6775-5p, miR-4651, miR-3195, miR-6726-5p, miR-6872-3p, miR-371a-5p, miR-6777-5p, miR-6789-5p, miR-7975, miR-6821-5p, miR-4534, miR-619-5p, miR-7107-5p, miR-1228-3p, miR-6774-5p, miR-6805-5p, miR-23a-3p, miR-4665-5p, miR-4505, miR-4638-5p, miR-24-3p, miR-3135b, miR-4745-5p, miR-128-1-5p, miR-4476, miR-4687-3p, miR-3665, miR-6806-5p, miR-3937, miR-711, miR-3141, miR-3188, miR-4281, miR-5196-5p, miR-6880-5p, miR-3960, miR-3648, miR-6721-5p, miR-4492, miR-744-5p, miR-7704, miR-4749-5p, miR-762, miR-6836-3p, miR-6727-5p, miR-4739, miR-7977, miR-4484, miR-6515-3p, miR-373-5p, miR-4258, miR-4674, miR-3180, miR-6076, miR-1238-5p, miR-4463, miR-4486, miR-4730, miR-4286, and miR-4739.

8. Use of the device according to claim 7, wherein the nucleic acid(s) is/are a polynucleotide(s) selected from the group consisting of the following polynucleotides (f) to (j):
(f) a polynucleotide consisting of a nucleotide sequence represented by any of SEQ ID NOs: 2 to 13, 15 to 54, 56 to 83, 227 to 229, 246, 248, 84 to 226, 230 to 245, 247, and 249 or a nucleotide sequence derived from the nucleotide sequence by the replacement of u with t, a variant thereof, a derivative thereof, or a fragment thereof comprising 15 or more consecutive nucleotides;
(g) a polynucleotide comprising a nucleotide sequence represented by any of SEQ ID NOs: 2 to 13, 15 to 54, 56 to 83, 227 to 229, 246, 248, 84 to 226, 230 to 245, 247, and 249;
(h) a polynucleotide consisting of a nucleotide sequence complementary to a nucleotide sequence represented by any of SEQ ID NOs: 2 to 13, 15 to 54, 56 to 83, 227 to 229, 246, 248, 84 to 226, 230 to 245, 247, and 249 or a nucleotide sequence derived from the nucleotide sequence by the replacement of u with t, a variant thereof, a derivative thereof, or a fragment thereof comprising 15 or more consecutive nucleotides;
(i) a polynucleotide comprising a nucleotide sequence complementary to a nucleotide sequence represented by any of SEQ ID NOs: 2 to 13, 15 to 54, 56 to 83, 227 to 229, 246, 248, 84 to 226, 230 to 245, 247, and 249 or a nucleotide sequence derived from the nucleotide sequence by the replacement of u with t; and
(j) a polynucleotide hybridizing under stringent conditions to any of the polynucleotides (f) to (i).

9. Use of the device according to any one of claims 5 to 8, wherein the device is a device for measurement by a hybridization technique, optionally wherein the hybridization technique is a nucleic acid array technique.

10. A method for detecting early pancreatic cancer or a pancreatic cancer precursor lesion in a subject, comprising:
measuring an expression level(s) of a target nucleic acid(s) in a sample from the subject using a kit according to any one of claims 1 to 4 or a device according to any one of claims 5 to 9; and
evaluating *in vitro* whether or not the subject has early pancreatic cancer or a pancreatic cancer precursor lesion using both of the measured expression level(s) and a control expression level(s) in a sample from a healthy subject measured in the same way, to detect the presence or absence of early pancreatic cancer or a pancreatic cancer precursor lesion in the subject.

11. A method for detecting early pancreatic cancer or a pancreatic cancer precursor lesion in a subject, comprising:
measuring an expression level(s) of a target gene(s) in a sample from the subject using a kit according to any one of claims 1 to 4 or a device according to any one of claims 5 to 9; and
assigning the expression level(s) of the target gene(s) in the sample from the subject to a discriminant to evaluate the presence or absence of early pancreatic cancer or a pancreatic cancer precursor lesion,
wherein the discriminant is prepared with the gene expression level(s) in a sample(s) from a subject(s) known to have early pancreatic cancer or a pancreatic cancer precursor lesion and the gene expression level(s) in a sample(s) from a healthy subject(s) as supervising samples and is capable of discriminating an early pancreatic cancer or pancreatic cancer precursor lesion patient from a healthy subject.

12. The method according to claim 10 or 11, wherein the subject is a human.

13. The method according to any one of claims 10 to 12, wherein the sample is blood, serum, or plasma.

14. Use of early pancreatic cancer or a pancreatic cancer precursor lesion marker polynucleotide: miR-4632-5p in an *in vitro* method of detection or diagnosis of early pancreatic cancer or a pancreatic cancer precursor lesion.

15. Use according to claim 14, wherein early pancreatic cancer or a pancreatic cancer precursor lesion marker polynucleotide is used together with at least one or more polynucleotide(s) selected from the group consisting of early pancreatic cancer or a pancreatic cancer precursor lesion markers: miR-1181, miR-671-5p, miR-6857-5p, miR-4276, miR-1914-3p, miR-149-3p, miR-937-5p, miR-4675, miR-6795-5p, miR-4731-5p, miR-5090, miR-3620-5p, miR-6717-5p, miR-6825-5p, miR-6738-5p, miR-6769a-5p, miR-4728-5p, miR-652-5p, miR-4257, miR-6785-5p, miR-7110-5p, miR-6887-5p, miR-887-3p, miR-1228-5p, miR-5572, miR-6782-5p, miR-4298, miR-6786-5p, miR-5010-5p, miR-6087, miR-6765-5p, miR-6732-5p, miR-6787-5p, miR-6737-5p, miR-128-2-5p, miR-4270, miR-6861-5p, miR-6756-5p, miR-1229-5p, miR-6891-5p, miR-6848-5p, miR-1237-5p, miR-30c-1-3p, miR-1233-5p, miR-211-3p, miR-4758-5p, miR-614, miR-6746-5p, miR-1915-5p, miR-4688, miR-3917, miR-5787, miR-6126, miR-135a-3p, miR-8063, miR-5698, miR-6089, miR-498, miR-296-3p, miR-4419b, miR-6802-5p, miR-6829-5p, miR-6803-5p, miR-1199-5p, miR-6840-3p, miR-6752-5p, miR-6798-5p, miR-6131, miR-4667-5p, miR-6510-5p, miR-4690-5p, miR-920, miR-23b-3p, miR-4448, miR-2110, miR-4706, miR-7845-5p, miR-6808-5p, miR-4447, miR-6869-5p, miR-6794-5p, miR-6511a-5p, miR-6824-5p, miR-6766-3p, miR-6511a-5p, and miR-6749-5p, miR-1908-5p, miR-6729-5p, miR-5195-3p, miR-638, miR-6125, miR-3178, miR-3196, miR-8069, miR-4723-5p, miR-4746-3p, miR-4689, miR-6816-5p, miR-6757-5p, miR-7109-5p, miR-6724-5p, miR-1225-3p, miR-6875-5p, miR-7108-5p, miR-4508, miR-6085, miR-6779-5p, miR-642a-3p, miR-4695-5p, miR-7847-3p, miR-3197, miR-6769b-5p, miR-7641, miR-187-5p, miR-3185, miR-2861, miR-3940-5p, miR-1203, miR-615-5p, miR-4787-5p, miR-1343-3p, miR-6813-5p, miR-1225-5p, miR-602, miR-4488, miR-125a-3p, miR-5100, miR-4294, miR-1231, miR-6765-3p, miR-4442, miR-718, miR-6780b-5p, miR-6090, miR-6845-5p, miR-4741, miR-4467, miR-4707-5p, miR-4271, miR-4673, miR-3184-5p, miR-1469, miR-4640-5p, miR-663a, miR-6791-5p, miR-6826-5p, miR-4433b-3p, miR-1915-3p, miR-4417, miR-4449, miR-4707-3p, miR-3180-3p, miR-5585-3p, miR-1268a, miR-8072, miR-296-5p, miR-204-3p, miR-4454, miR-6722-3p, miR-1290, miR-3622a-5p, miR-939-5p, miR-675-5p, miR-3131, miR-4648, miR-1268b, miR-6741-5p, miR-6893-5p, miR-3162-5p, miR-642b-3p, miR-4734, miR-150-3p, miR-8089, miR-6805-3p, miR-7113-3p, miR-6850-5p, miR-6799-5p, miR-6768-5p, miR-92b-5p, miR-3679-5p, miR-4792, miR-3656, miR-92a-2-5p, miR-4466, miR-4513, miR-6781-5p, miR-4649-5p, miR-6775-5p, miR-4651, miR-3195, miR-6726-5p, miR-6872-3p, miR-371a-5p, miR-6777-5p, miR-6789-5p, miR-7975, miR-6821-5p, miR-4534, miR-619-5p, miR-7107-5p, miR-1228-3p, miR-6774-5p, miR-6805-5p, miR-23a-3p, miR-4665-5p, miR-4505, miR-4638-5p, miR-24-3p, miR-3135b, miR-4745-5p, miR-128-1-5p, miR-4476, miR-4687-3p, miR-3665, miR-6806-5p, miR-3937, miR-711, miR-3141, miR-3188, miR-4281, miR-5196-5p, miR-6880-5p, miR-3960, miR-3648, miR-6721-5p, miR-4492, miR-744-5p, miR-7704, miR-4749-5p, miR-762, miR-6836-3p, miR-6727-5p, miR-4739, miR-7977, miR-4484, miR-6515-3p, miR-373-5p, miR-4258, miR-4674, miR-3180, miR-6076, miR-1238-5p, miR-4463, miR-4486, miR-4730, miR-4286, and miR-4739.
